# EUROPÄISCHE PATENTSCHRIFT

(11) **EP 2 334 669 B1**
(45) Veröffentlichungstag und Bekanntmachung des Hinweises auf die Patenterteilung: **23.12.2015**
(21) Anmeldenummer: 09778607.3
(22) Anmeldetag: 18.09.2009
(51) Int. Cl.: A01N 43/80

(54) **HETEROCYCLYL SUBSTITUIERTE THIAZOLE ALS PFLANZENSCHUTZMITTEL**
HETEROCYCLYL-SUBSTITUTED THIAZOLES AS PLANT PROTECTION AGENTS
THIAZOLES SUBSTITUÉS PAR UN HÉTÉROCYCLYLE COMME AGENT PHYTOSANITAIRE

(30) Priorität: 01.10.2008 EP 08165625
(43) Veröffentlichungstag der Anmeldung: 22.06.2011
(73) Patentinhaber: Bayer Intellectual Property GmbH, 40789 Monheim am Rhein (DE)
(72) Erfinder: CRISTAU, Pierre, 69009 Lyon (FR); RAHN, Nicola, 40589 Düsseldorf (DE); HERRMANN, Stefan, 40764 Langenfeld (DE); TSUCHIYA, Tomoki, 40227 Düsseldorf (DE); WACHENDORF-NEUMANN, Ulrike, 56566 Neuwied (DE); VOERSTE, Arnd, 50674 Köln (DE); BENTING, Jürgen, 42799 Leichlingen (DE); WASNAIRE, Pierre, 40591 Düsseldorf (DE); HOFFMANN, Sebastian, 41470 Neuss (DE)
(74) Vertreter: BIP Patents
(86) Internationale Anmeldenummer: PCT/EP2009/006755
(87) Internationale Veröffentlichungsnummer: WO 2010/037479

(56) Entgegenhaltungen:
- WO-A-2008/013925
- WO-A-2008/091580

## Beschreibung

Die Erfindung betrifft heterocyclyl substituierte Thiazole oder deren agrochemisch wirksamen Salze, deren Verwendung sowie Verfahren und Mittel zur Bekämpfung von pflanzenpathogenen Schadpilzen in und/oder auf Pflanzen oder in und/oder auf Saatgut von Pflanzen, Verfahren zur Herstellung solcher Mittel und behandeltes Saatgut sowie deren Verwendung zur Bekämpfung von pflanzenpathogenen Schadpilzen in der Land-, Garten- und Forstwirtschaft, in der Tiergesundheit, im Materialschutz sowie im Bereich Haushalt und Hygiene. Die vorliegende Erfindung betrifft weiterhin ein Verfahren zur Herstellung von heterocyclyl substituierten Thiazolen.

Es ist bereits bekannt, dass bestimmte heterocyclyl substituierte Thiazole als fungizide Pflanzenschutzmittel benutzt werden können (siehe WO 07/014290, WO 08/013925, WO 08/013622, WO 08/091594, WO 08/091580, WO 09/055514). Die fungizide Wirksamkeit dieser Verbindungen ist jedoch gerade bei niedrigeren Aufwandmengen nicht immer ausreichend.

Da sich die ökologischen und ökonomischen Anforderungen an moderne Pflanzenschutzmittel laufend erhöhen, beispielsweise was Wirkspektrum, Toxizität, Selektivität, Aufwandmenge, Rückstandsbildung und günstige Herstellbarkeit angeht, und außerdem z.B. Probleme mit Resistenzen auftreten können, besteht die ständige Aufgabe, neue Pflanzenschutzmittel, insbesondere Fungizide zu entwickeln, die zumindest in Teilbereichen Vorteile gegenüber den bekannten aufweisen.

Überraschenderweise wurde nun gefunden, dass die vorliegenden heterocyclyl-substituierten Thiazole die genannten Aufgaben zumindest in Teilaspekten lösen und sich als Pflanzenschutzmittel, insbesondere als Fungizide eignen.

Einige heterocyclyl-substituierte Thiazole sind bereits als pharmazeutisch wirksame Verbindungen bekannt (siehe z.B. EP 1832586, WO 04/058750, WO 04/058751, WO 05/003128, WO 06/032322, WO 07/104558, WO 07/115805, WO 08/083238) jedoch nicht deren überraschende fungizide Wirksamkeit.

Gegenstand der Erfindung sind Verbindungen der Formel **(I)**, 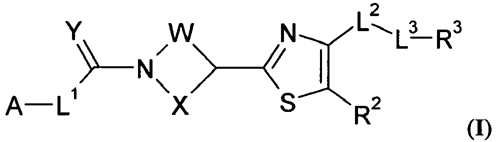 in welcher die Symbole folgende Bedeutungen haben:
- A: steht für unsubstituiertes oder substituiertes Phenyl,
wobei die Substituenten unabhängig voneinander ausgewählt sind aus folgender Liste
Cyano, Nitro, Halogen, C₁-C₆-Alkyl, C₁-C₃-Halogenalkyl, C₃-C₆-Cycloalkyl, C₃-C₆-Halogencycloalkyl, C₄-C₁₀-Cycloalkylalkyl, C₄-C₁₀-Alkylcycloalkyl, C₅-C₁₀-Alkylcycloalkylalkyl, C₂-C₆-Alkenyl, C₂-C₆-Halogenalkenyl, C₂-C₆-Alkinyl, C₂-C₆-Halogenalkinyl, Tri(C₁-C₂-alkyl)silyl, Benzyl, Phenyl, Hydroxy, C₁-C₄-Alkoxy, C₁-C₄-Halogenalkoxy, OCH₂OCH₃, SH, C₁-C₄-Alkylthio, C₁-C₆-Halogenalkylthio, CHO, COOH, (C₁-C₄-Alkoxy)carbonyl, CONR⁶R⁷, CR⁶=NOR⁷, (C₁-C₄-Alkyl)carbonyl, (C₁-C₄-Halogenalkyl)carbonyl, (C₁-C₄-Alkyl)carbonyloxy, (C₁-C₄-Alkyl)carbonylthio, C₁-C₄-Alkylsulfinyl, C₁-C₄-Halogenalkylsulfinyl, C₁-C₄-Alkylsulfonyl, C₁-C₄-Halogenalkylsulfonyl, NR⁶R⁷, NR⁶COR⁷, SF₅, SO₂NR⁶R⁷, C₂-C₄-Alkoxyalkyl, oder 1-Methoxycyclopropyl
oder
- A: ist ein gegebenenfalls benzokondensiertes, unsubstituiertes oder substituiertes 5-oder 6-gliedriges Heteroaryl, wobei die Substituenten unabhängig voneinander ausgewählt sind aus folgender Liste

### Substituenten am Kohlenstoff:

Cyano, Nitro, Halogen, C₁-C₆-Alkyl, C₁-C₃-Halogenalkyl, C₃-C₆-Cycloalkyl, C₃-C₆-Halogencycloalkyl C₄-C₁₀-Cycloalkylalkyl, C₄-C₁₀-Alkylcycloalkyl, C₅-C₁₀-Alkylcycloalkylalkyl, C₂-C₆-Alkenyl, C₂-C₆-Halogena!kenyl, C₂-C₆-Alkinyl, C₂-C₆-Halogenalkinyl, Tri(C₁-C₂-alkyl)silyl, Benzyl, Phenyl, Hydroxy, C₁-C₄-Alkoxy, C₁-C₄-Halogenalkoxy, OCH₂OCH₃, SH, C₁-C₄-Alkylthio, C₁-C₆-Halogenalkylthio, CHO, COOH, (C₁-C₄-Alkoxy)carbonyl, CONR⁶R⁷, CR⁶=NOR⁷, (C₁-C₄-Alkyl)carbonyl, (C₁-C₄-Halogenalkyl)carbonyl, (C₁-C₄-Alkyl)carbonyloxy, (C₁-C₄-Alkyl)carbonylthio, C₁-C₄-Alkylsulfinyl, C₁-C₄-Halogenalkylsulfinyl, C₁-C₄-Alkylsulfonyl, C₁-C₄-Halogenalkylsulfonyl, NR⁶R⁷, NR⁶COR⁷, SF₅, SO₂NR⁶R⁷, C₂-C₄-Alkoxyalkyl oder 1-Methoxycyclopropyl

### Substituenten am Stickstoff:

Hydroxy, NR⁶R⁷, C₁-C₆-Alkyl, C₁-C₃-Halogenalkyl, C₃-C₆-Cycloalkyl, C₃-C₆-Halogencycloalkyl, C₄-C₁₀-Alkylcycloalkyl, C₂-C₆-Alkenyl, C₂-C₆-Halogenalkenyl, C₂-C₆-Alkinyl, oder C₂-C₆-Halogenalkinyl
- L¹: ist (C(R¹)₂)ₙ
- mit n =: 0 bis 3
- R¹: ist gleich oder verschieden unabhängig voneinander Wasserstoff, Halogen, C₁-C₂-Alkyl, C₁-C₂-Halogenalkyl oder Cyano,
unter der Voraussetzung, dass L¹ maximal zwei R¹ enthalten kann, die von Wasserstoff verschieden sind
- Y: steht für Schwefel oder Sauerstoff,
- W: ist eine unsubstituierte oder einfach substituierte C₁- bis C₃-Kohlenstoffkette, wobei der Substituent ausgewählt ist aus Oxo, Hydroxy, Cyano oder C₁-C₄-Alkyl
- X: ist eine unsubstituierte oder einfach substituierte C₁- bis C₂-Kohlenstoffkette, wobei der Substituent ausgewählt ist aus Oxo, Hydroxy, Cyano oder C₁-C₄-Alkyl
- R²: steht für Wasserstoff, C₁-C₂-Alkyl, C₁-C₂-Halogenalkyl oder Halogen
- L²: steht für -C(R⁸)₂-C(R⁸)₂- oder -CR⁹=CR⁹- oder -C=C-
- L³: steht für eine direkte Bindung
oder
- L³: steht für eine C₁- bis C₄-Kohlenstoffkette, die bis zu zwei Substituenten enthalten kann, wobei die Substituenten unabhängig voneinander ausgewählt sind aus folgender Liste:
Halogen, C₁-C₂-Alkyl, C₁-C₂-Alkoxy, C₁-C₂-Halogenalkyl oder C₃-C₆-Cycloalkyl
- R³: steht für unsubstituiertes oder einfach substituiertes C₃-C₁₀-Cycloalkyl, wobei der Substituent ausgewählt ist aus folgender Liste:
Cyano, Halogen, C₁-C₆-Alkyl, C₁-C₃-Halogenalkyl, C₃-C₆-Cycloalkyl, C₂-C₆-Alkenyl, C₂-C₆-Halogenalkenyl, C₂-C₆-Alkinyl, C₂-C₆-Halogenalkinyl, Tri(C₁-C₂-alkyl)silyl, Phenyl, Hydroxy, Oxo, C₁-C₆-Alkoxy, C₁-C₆-Halogenalkoxy, C₂-C₆-Alkenyloxy, C₂-C₆-Alkinyloxy, C₁-C₆-Alkylthio, oder C₁-C₆-Halogenalkylthio,
oder
- R³: steht für unsubstituiertes oder substituiertes Phenyl,
wobei die Substituenten unabhängig voneinander ausgewählt sind aus folgender Liste:
Cyano, Nitro, Halogen, C₁-C₆-Alkyl, C₁-C₃-Halogenalkyl, C₃-C₆-Cycloalkyl, C₃-C₆-Halogencycloalkyl, C₄-C₁₀-Cycloalkylalkyl, C₄-C₁₀-Halogencycloalkylalkyl, C₄-C₁₀-Alkylcycloalkyl, C₅-C₁₀-Alkylcycloalkylalkyl, C₄-C₁₀-Cycloalkoxyalkyl, C₂-C₆-Alkenyl, C₂-C₆-Halogenalkenyl, C₃-C₈-Cycloalkenyl, C₃-C₈-Halocycloalkenyl, C₂-C₆-Alkinyl, C₂-C₆-Halogenalkinyl, C₂-C₆-Alkoxyalkyl, C₂-C₆-Halogenalkoxyalkyl, C₃-C₈-Alkoxyalkoxyalkyl, Tri(C₁-C₂-alkyl)silyl, Benzyl, Phenyl, Hydroxy, C₁-C₆-Alkoxy, C₂-C₆-Alkoxyalkoxy, C₁-C₆-Halogenalkoxy, C₂-C₆-Alkenyloxy, C₂-C₆-Halogenalkenyloxy, C₂-C₆-Alkinyloxy, C₂-C₆-Halogenalkinyloxy, C₃-C₆-Cycloalkoxy, C₃-C₆-Cycloalkyloxy, C₃-C₆-Halocycloalkoxy, C₄-C₁₀-Cycloalkylalkyloxy, NR⁶R⁷, SH, SF₅, C₁-C₆-Alkylthio, C₁-C₆-Halogenalkylthio, C₂-C₆-AlkylAlkylthio, C₃-C₆-Cycloalkylthio, CHO, COOH, (C₁-C₆-Alkoxy)carbonyl, CONR⁶R⁷, CR⁶=NOR⁷, (C₁-C₆-Alkyl)carbonyl, (C₁-C₆-Halogenalkyl)carbonyl, (C₁-C₆-Alkyl)carbonyloxy, (C₁-C₆-Halogenalkyl)carbonyloxy, (C₁-C₆-Alkyl)carbonylthio, C₁-C₆-Alkylsulfinyl, C₁-C₆-Halogenalkylsulfinyl, C₁-C₆-Alkylsulfonyl, C₁-C₆-Halogenalkylsulfonyl, NR⁶COR⁷ oder SO₂NR⁶R⁷
oder
- R³: steht für gesättigtes oder teilweise oder vollständig ungesättigtes Naphthyl oder Indenyl,
das bis zu zwei Substituenten enthalten kann, wobei die Substituenten unabhängig voneinander ausgewählt sind aus folgender Liste:
Cyano, Halogen, C₁-C₆-Alkyl, C₁-C₃-Halogenalkyl, C₃-C₆-Cycloalkyl, C₂-C₆-Alkenyl, C₂-C₆-Halogenalkenyl, C₂-C₆-Alkinyl, C₂-C₆-Halogenalkinyl, Tri(C₁-C₂-alkyl)silyl, Phenyl, Hydroxy, C₁-C₆-Alkoxy, C₁-C₆-Halogenalkoxy, C₂-C₆-Alkenyloxy, C₂-C₆-Alkinyloxy, C₁-C₆-Alkylthio oder C₁-C₆-Halogenalkylthio,
oder
- R³: steht für einen unsubstituierten oder substituierten 5-oder 6-gliedrigen Heteroarylrest,
wobei die Substituenten unabhängig voneinander ausgewählt sind aus folgender Liste:
Substituenten am Kohlenstoff: Cyano, Nitro, Halogen, C₁-C₆-Alkyl, C₁-C₃-Halogenalkyl, C₃-C₆-Cycloalkyl, C₃-C₆-Halogencycloalkyl C₄-C₁₀-Cycloalkylalkyl, C₄-C₁₀-Alkylcycloalkyl, C₅-C₁₀-Alkylcycloalkylalkyl, C₂-C₆-Alkenyl, C₂-C₆-Halogenalkenyl, C₂-C₆-Alkinyl, C₂-C₆-Halogenalkinyl, Tri(C₁-C₂-alkyl)silyl, Benzyl, Phenyl, Hydroxy, C₁-C₄-Alkoxy, C₁-C₄-Halogenalkoxy, OCH₂OCH₃, SH, C₁-C₄-Alkylthio, C₁-C₆-Halogenalkylthio, COOH, (C₁-C₄-Alkoxy)carbonyl, CONR⁶R⁷, (C₁-C₄-Alkyl)carbonyl, (C₁-C₄-Halogenalkyl)carbonyl, (C₁-C₄-Alkyl)carbonyloxy, (C₁-C₄-Alkyl)carbonylthio, C₁-C₄-Alkylsulfinyl, C₁-C₄-Halogenalkylsulfinyl, C₁-C₄-Alkylsulfonyl, C₁-C₄-Halogenalkylsulfonyl, NR⁶R⁷, NR⁶COR⁷, SF₅, SO₂NR⁶R⁷, C₂-C₄-Alkoxyalkyl oder 1-Methoxycyclopropyl
Substituenten am Stickstoff: Hydroxy, C₁-C₆-Alkyl, C₁-C₃-Halogenalkyl, C₃-C₆-Cycloalkyl, C₃-C₆-Halogencycloalkyl, C₄-C₁₀-Alkylcycloalkyl, C₂-C₆-Alkenyl, C₂-C₆-Halogenalkenyl, C₂-C₆-Alkinyl, C₂-C₆-Halogenalkinyl oder Phenyl
oder
- R³: steht für benzokondensiertes unsubstituiertes oder substituiertes 5- oder 6- gliedriges Heteroaryl, das bis zu zwei Substituenten enthalten kann, wobei die Substituenten unabhängig voneinander ausgewählt sind aus folgender Liste:

Substituenten am Kohlenstoff: Cyano, Nitro, Halogen, C₁-C₆-Alkyl, C₁-C₃-Halogenalkyl, C₃-C₆-Cycloalkyl, C₃-C₆-Halogencycloalkyl C₄-C₁₀-Cycloalkylalkyl, C₄-C₁₀-Alkylcycloalkyl, C₅-C₁₀-Alkylcycloalkylalkyl, C₂-C₆-Alkenyl, C₂-C₆-Halogenalkenyl, C₂-C₆-Alkinyl, C₂-C₆-Halogenalkinyl, Tri(C₁-C₂-alkyl)silyl, Benzyl, Phenyl, Hydroxy, C₁-C₄-Alkoxy, C₁-C₄-Halogenalkoxy, OCH₂OCH₃, SH, C₁-C₄-Alkylthio, C₁-C₆-Halogenalkylthio, COOH, (C₁-C₄-Alkoxy)carbonyl, CONR⁶R⁷, (C₁-C₄-Alkyl)carbonyl, (C₁-C₄-Halogenalkyl)carbonyl, (C₁-C₄-Alkyl)carbonyloxy, (C₁-C₄-Alkyl)carbonylthio, C₁-C₄-Alkylsulfinyl, C₁-C₄-Halogenalkylsulfinyl, C₁-C₄-Alkylsulfonyl, C₁-C₄-Halogenalkylsulfonyl, NR⁶R⁷, NR⁶COR⁷, SF₅, SO₂NR⁶R⁷, C₂-C₄-Alkoxyalkyl, oder 1-Methoxycyclopropyl
Substituenten am Stickstoff: C₁-C₆-Alkyl, C₁-C₃-Halogenalkyl, C₃-C₆-Cycloalkyl, C₃-C₆-Halogencycloalkyl, C₄-C₁₀-Alkylcycloalkyl, C₂-C₆-Alkenyl, C₂-C₆-Halogenalkenyl, C₂-C₆-Alkinyl, C₂-C₆-Halogenalkinyl oder Phenyl
oder
- R³: ist ein über ein Kohlenstoffatom gebundener, unsubstituierter oder einfach substituierter 5-bis 15-gliedriger Heterocyclylrest, der bis zu zwei weitere Heteroatome, ausgewählt aus Stickstoff, Sauerstoff und Schwefel enthalten kann, wobei der Substituent ausgewählt ist aus folgender Liste:

Substituenten am Kohlenstoff: Cyano, Halogen, C₁-C₆-Alkyl, C₁-C₃-Halogenalkyl, C₃-C₆-Cycloalkyl, C₂-C₆-Alkenyl, C₂-C₆-Halogenalkenyl, C₂-C₆-Alkinyl, C₂-C₆-Halogenalkinyl, Tri(C₁-C₂-alkyl)silyl, Phenyl, Hydroxy, C₁-C₆-Alkoxy, C₁-C₆-Halogenalkoxy, C₂-C₆-Alkenyloxy, C₂-C₆-Alkinyloxy, C₁-C₆-Alkylthio, oder C₁-C₆-Halogenalkylthio
Substituenten am Stickstoff: C₁-C₆-Alkyl, C₁-C₃-Halogenalkyl, C₃-C₆-Cycloalkyl, C₂-C₆-Alkenyl, C₂-C₆-Halogenalkenyl, C₂-C₆-Alkinyl, C₂-C₆-Halogenalkinyl, Tri(C₁-C₂-alkyl)silyl oder Phenyl
   - R⁶, R⁷: stehen unabhängig voneinander für Wasserstoff, C₁-C₄-Alkyl, C₁-C₃-Halogenalkyl, C₃-C₆-Cycloalkyl
   - R⁸: ist gleich oder verschieden unabhängig voneinander Wasserstoff, C₁-C₄-Alkyl,
   - R⁹: ist gleich oder verschieden unabhängig voneinander Wasserstoff, C₁-C₄-Alkyl,
   sowie agrochemisch wirksame Salze davon.

Ein weiterer Gegenstand ist die Verwendung der Verbindungen der Formel (I) als Fungizide.

Erfindungsgemäße heterocyclyl substituierte Thiazole Formel (I) sowie deren agrochemisch wirksame Salze eignen sich sehr gut als zur Bekämpfung pflanzenpathogener Schadpilze. Die vorgenannten erfindungsgemäßen Verbindungen zeigen vor allem eine starke fungizide Wirksamkeit und lassen sich sowohl im Pflanzenschutz, im Bereich Haushalt und Hygiene als auch im Materialschutz verwenden.

Die Verbindungen der Formel (I) können sowohl in reiner Form als auch als Mischungen verschiedener möglicher isomerer Formen, insbesondere von Stereoisomeren, wie E- und Z-, threo- und erythro-, sowie optischen Isomeren, wie R- und S-Isomeren oder Atropisomeren, gegebenenfalls aber auch von Tautomeren vorliegen. Es werden sowohl die E- als auch die Z-Isomeren, wie auch die threo- und erythro-, sowie die optischen Isomeren, beliebige Mischungen dieser Isomeren, sowie die möglichen tautomeren Formen beansprucht.

Bevorzugt werden Verbindungen der Formel (I), in denen ein oder mehrere der Symbole eine der folgenden Bedeutungen haben:
- A: steht für Phenyl, das bis zu zwei Substituenten enthalten kann, wobei die Substituenten unabhängig voneinander ausgewählt sind aus folgender Liste :
Cyano, Nitro, Halogen, C₁-C₄-Alkyl, C₁-C₃-Halogenalkyl, C₃-C₆-Cycloalkyl, C₂-C₄-Alkenyl, C₂-C₄-Alkinyl, Hydroxy, C₁-C₄-Alkoxy, C₁-C₄-Halogenalkoxy, C₁-C₄-Alkylthio, C₁-C₄-Halogenalkylthio, (C₁-C₄-Alkoxy)carbonyl, (C₁-C₄-Alkyl)carbonyl, (C₁-C₄-Halogenalkyl)carbonyl, C₁-C₄-Alkylsulfinyl, C₁-C₄-Halogenalkylsulfinyl, C₁-C₄-Alkylsulfonyl oder C₁-C₄-Halogenalkylsulfonyl,
oder
- A: steht für einen heteroaromatischen Rest ausgewählt aus folgender Gruppe: ein Furan-2-yl, Furan-3-yl, Thiophen-2-yl, Thiophen-3-yl, Isoxazol-3-yl, Isoxazol-4-yl, Isoxazol-5-yl, 1H-Pyrrol-1-yl, 1H-Pyrrol-2-yl, 1H-Pyrrol-3-yl, Oxazol-2-yl, Oxazol-4-yl, Oxazol-5-yl, Thiazol-2-yl, Thiazol-4-yl, Thiazol-5-yl, Isothiazol-3-yl, Isothiazol-4-yl, Isothiazol-5-yl, Pyrazol-1-yl, Pyrazol-3-yl, Pyrazol-4-yl, Imidazol-1-yl, Imidazol-2-yl, Imidazol-4-yl, 1,2,4-Oxadiazol-3-yl, 1,2,4-Oxadiazol-5-yl, 1,3,4-Oxadiazol-2-yl, 1,2,4-Thiadiazol-3-yl, 1,2,4-Thiadiazol-5-yl, 1,3,4-Thiadiazol-2-yl, 1,2,3-Triazol-1-yl, 1,2,3-Triazol-2-yl, 1,2,3-Triazol-4-yl, 1,2,4-Triazol-1-yl, 1,2,4-Triazol-3-yl, Pyridin-2-yl, Pyridin-3-yl, Pyridin-4-yl, Pyridazin-3-yl, Pyridazin-4-yl, Pyrimidin-2-yl, Pyrimidin-4-yl, Pyrimidin-5-yl, Pyrazin-2-yl, 1,3,5-Triazin-2-yl, 1,2,4-Triazin-3-yl, 1H-Indol-1-yl, 1H-Indol-2-yl, 1H-Indol-3-yl, 1H-Indol-4-yl, 1H-Indol-5-yl, 1H-Indol-6-yl, 1H-Indol-7-yl, 1H-Benzimidazol-1-yl, 1H-Benzimidazol-2-yl, 1H-Benzimidazol-4-yl, Benzimidazol-5-yl, 1H-Indazol-1-yl, 1H-Indazol-3-yl, 1H-Indazol-4-yl, 1H-Indazol-5-yl, 1H-Indazol-6-yl, 1H-Indazol-7-yl, 2H-Indazol-2-yl, 1-benzofuran-2-yl, 1-benzofuran-3-yl, 1-benzofuran-4-yl, 1-benzofuran-5-yl, 1-benzofuran-6-yl, 1-benzofuran-7-yl, 1-benzothiophen-2-yl, 1-benzothiophen-3-yl, 1-benzothiophen-4-yl, 1-benzothiophen-5-yl, 1-benzothiophen-6-yl, 1-benzothiophen-7-yl, 1,3-Benzothiazol-2-yl, 1,3-Benzoxazol-2-yl, Quinolin-2-yl, Quinolin-3-yl, Quinolin-4-yl, Quinolin-5-yl, Quinolin-6-yl, Quinolin-7-yl, Quinolin-8-yl, Isoquinolin-1-yl, Isoquinolin-3-yl, Isoquinolin-4-yl, Isoquinolin-5-yl, Isoquinolin-6-yl, Isoquinolin-7-yl, oder Isoquinolin-8-yl,
der bis zu zwei Substituenten enthalten kann, wobei die Substituenten unabhängig voneinander ausgewählt sind aus folgender Liste :
Substituenten am Kohlenstoff: Cyano, Halogen, C₁-C₄-Alkyl, C₁-C₃-Halogenalkyl, Hydroxy, C₁-C₄-Alkoxy, C₁-C₄-Halogenalkoxy, C₁-C₄-Alkylthio oder C₁-C₄-Halogenalkylthio,
Substituenten am Stickstoff: C₁-C₄-Alkyl, C₁-C₃-Halogenalkyl, Cyclopropyl, C₂-C₄-Alkenyl oder C₂-C₄-Alkinyl,
   - L¹: ist (C(R¹)₂)ₙ
mit n = 0 bis 3
- R¹: ist gleich oder verschieden unabhängig voneinander Wasserstoff, Chlor, Fluor, Methyl, CF₃ oder Cyano,
unter der Voraussetzung, dass L¹ maximal zwei R¹ enthalten kann, die von Wasserstoff verschieden sind,
- Y: steht für Schwefel oder Sauerstoff,
- W: ist eine unsubstituierte oder einfach substituierte C₁- bis C₂-Kohlenstoffkette, wobei der Substituent ausgewählt ist aus Cyano, oder C₁-C₂-Alkyl
- X: ist eine unsubstituierte oder einfach substituierte C₁- bis C₂-Kohlenstoffkette, wobei der Substituent ausgewählt ist aus Cyano, oder C₁-C₂-Alkyl
- R²: steht für Wasserstoff, C₁-C₂-Alkyl oder Halogen,
- L²: steht für -C(R⁸)₂-C(R⁸)₂- oder -CR⁹=CR⁹- oder -C=C-,
- L³: steht für eine direkte Bindung,
oder
- L³: steht für eine C₁- bis C₄-Kohlenstoffkette, die bis zu zwei Substituenten enthalten kann, wobei die Substituenten unabhängig voneinander ausgewählt sind aus folgender Liste:
C₁-C₂-Alkyl, C₁-C₂-Alkoxy, C₁-C₂-Halogenalkyl oder Cyclopropyl,

- R³: steht für unsubstituiertes oder einfach substituiertes C₃-C₁₀-Cycloalkyl, wobei der Substituent ausgewählt ist aus folgender Liste:
Halogen, C₁-C₆-Alkyl, C₃-C₆-Cycloalkyl, C₂-C₆-Alkenyl, C₂-C₆-Alkinyl, Tri(C₁-C₂-alkyl)silyl, Phenyl oder Oxo,
oder
- R³: steht für Phenyl, das bis zu drei Substituenten enthalten kann, wobei die Substituenten unabhängig voneinander ausgewählt sind aus folgender Liste:
Cyano, Nitro, Halogen, C₁-C₆-Alkyl, C₁-C₃-Halogenalkyl, C₃-C₆-Cycloalkyl, C₃-C₆-Halogencycloalkyl, C₂-C₆-Alkenyl, C₂-C₆-Halogenalkenyl, C₂-C₆-Alkinyl, C₂-C₆-Halogenalkinyl, Tri(C₁-C₂-alkyl)silyl, Phenyl, Hydroxy, C₁-C₆-Alkoxy, C₁-C₆-Halogenalkoxy, C₁-C₆-Alkylthio oder C₁-C₆-Halogenalkylthio,
oder
- R³: steht für Naphthalen-1-yl, Naphthalen-2-yl, 1,2,3,4-Tetrahydronaphthalen-1-yl, 1,2,3,4-Tetrahydronaphthalen-2-yl, 5,6,7,8-Tetrahydronaphthalen-1-yl, 5,6,7,8-Tetrahydronaphthalen-2-yl, Decalin-1-yl, Decalin-2-yl, 1H-Inden-1-yl, 1H-Inden-2-yl, 1H-Inden-3-yl, 1H-Inden-4-yl, 1H-Inden-5-yl, 1H-Inden-6-yl, 1H-Inden-7-yl, 2,3-Dihydro-1H-inden-1-yl, 2,3-Dihydro-1H-inden-2-yl, 2,3-Dihydro-1H-Inden-4-yl oder 2,3-Dihydro-1H-inden-5-yl,
das bis zu zwei Substituenten enthalten kann, wobei die Substituenten unabhängig voneinander ausgewählt sind aus folgender Liste:
Cyano, Halogen, C₁-C₆-Alkyl, C₁-C₃-Halogenalkyl, C₃-C₆-Cycloalkyl, Phenyl, Hydroxy, C₁-C₆-Alkoxy, C₁-C₃-Halogenalkoxy, C₁-C₆-Alkylthio oder C₁-C₃-Halogenalkylthio,
oder
- R³: steht für Furan-2-yl, Furan-3-yl, Thiophen-2-yl, Thiophen-3-yl, Isoxazol-3-yl, Isoxazol-4-yl, Isoxazol-5-yl, 1H-Pyrrol-1-yl, 1H-Pyrrol-2-yl, 1H-Pyrrol-3-yl, Oxazol-2-yl, Oxazol-4-yl, Oxazol-5-yl, Thiazol-2-yl, Thiazol-4-yl, Thiazol-5-yl, Isothiazol-3-yl, Isothiazol-4-yl, Isothiazol-5-yl, Pyrazol-1-yl, Pyrazol-3-yl, Pyrazol-4-yl, Imidazol-1-yl, Imidazol-2-yl, Imidazol-4-yl, 1,2,4-Oxadiazol-3-yl, 1,2,4-Oxadiazol-5-yl, 1,3,4-Oxadiazol-2-yl, 1,2,4-Thiadiazol-3-yl, 1,2,4-Thiadiazol-5-yl, 1,3,4-Thiadiazol-2-yl, 1,2,3-Triazol-1-yl, 1,2,3-Triazol-2-yl, 1,2,3-Triazol-4-yl, 1,2,4-Triazol-1-yl, 1,2,4-Triazol-3-yl, Pyridin-2-yl, Pyridin-3-yl, Pyridin-4-yl, Pyridazin-3-yl, Pyridazin-4-yl, Pyrimidin-2-yl, Pyrimidin-4-yl, Pyrimidin-5-yl, Pyrazin-2-yl, 1,3,5-Triazin-2-yl oder 1,2,4-Triazin-3-yl,
das bis zu zwei Substituenten enthalten kann, wobei die Substituenten unabhängig voneinander ausgewählt sind aus folgender Liste :
Substituenten am Kohlenstoff: Cyano, Halogen, C₁-C₆-Alkyl, C₁-C₃-Halogenalkyl, C₃-C₆-Cycloalkyl, Phenyl, Hydroxy, C₁-C₄-Alkoxy, C₁-C₄-Halogenalkoxy, C₁-C4-Alkylthio oder C₁-C₄-Halogenalkylthio,
Substituenten am Stickstoff: C₁-C₆-Alkyl, C₁-C₃-Halogenalkyl, C₃-C₆-Cycloalkyl, C₂-C₆-Alkenyl, C₂-C₆-Alkinyl oder Phenyl,
oder
- R³: steht für 1H-Indol-1-yl, 1H-Indol-2-yl, 1H-Indol-3-yl, 1H-Indol-4-yl, 1H-Indol-5-yl, 1H-Indol-6-yl, 1H-Indol-7-yl, 1H-Benzimidazol-1-yl, 1H-Benzimidazol-2-yl, 1H-Benzimidazol-4-yl, Benzimidazol-5-yl, 1H-Indazol-1-yl, 1H-Indazol-3-yl, 1H-Indazol-4-yl, 1H-Indazol-5-yl, 1H-Indazol-6-yl, 1H-Indazol-7-yl, 2H-Indazol-2-yl, 1-benzofuran-2-yl, 1-benzofuran-3-yl, 1-benzofuran-4-yl, 1-benzofuran-5-yl, 1-benzofuran-6-yl, 1-benzofuran-7-yl, 1-benzothiophen-2-yl, 1-benzothiophen-3-yl, 1-benzothiophen-4-yl, 1-benzothiophen-5-yl, 1-benzothiophen-6-yl, 1-benzothiophen-7-yl, Quinolin-2-yl, Quinolin-3-yl, Quinolin-4-yl, Quinolin-5-yl, Quinolin-6-yl, Quinolin-7-yl, Quinolin-8-yl, Isoquinolin-1-yl, Isoquinolin-3-yl, Isoquinolin-4-yl, Isoquinolin-5-yl, Isoquinolin-6-yl, Isoquinolin-7-yl, oder Isoquinolin-8-yl,
das bis zu zwei Substituenten enthalten kann, wobei die Substituenten unabhängig voneinander ausgewählt sind aus folgender Liste :
Substituenten am Kohlenstoff: Cyano, Halogen, C₁-C₆-Alkyl, C₁-C₃-Halogenalkyl, C₃-C₆-Cycloalkyl, Phenyl, Hydroxy, C₁-C₄-Alkoxy, C₁-C₄-Halogenalkoxy, C₁-C₄-Alkylthio oder C₁-C₄-Halogenalkylthio,
Substituenten am Stickstoff: C₁-C₆-Alkyl, C₁-C₃-Halogenalkyl, C₃-C₆-Cycloalkyl, C₂-C₆-Alkenyl, C₂-C₆-Alkinyl oder Phenyl,
oder
- R³: steht für einen über ein Kohlenstoffatom gebundenen, unsubstituierten oder einfach substituierten 5-bis 6-gliedrigen Heterocyclylrest, der bis zu zwei weitere Heteroatome, ausgewählt aus Stickstoff, Sauerstoff und Schwefel enthalten kann, wobei der Substituent ausgewählt ist aus folgender Liste:

Substituenten am Kohlenstoff: C₁-C₆-Alkyl, C₁-C₃-Halogenalkyl, C₃-C₆-Cycloalkyl, C₂-C₆-Alkenyl, C₂-C₆-Alkinyl, Tri(C₁-C₂-alkyl)silyl oder Phenyl,
Substituenten am Stickstoff: C₁-C₆-Alkyl, C₃-C₆-Cycloalkyl, C₂-C₆-Alkenyl, C₂-C₆-Alkinyl, Tri(C₁-C₂-alkyl)silyl oder Phenyl,
   - R⁸: ist gleich oder verschieden unabhängig voneinander Wasserstoff, C₁-C₂-Alkyl,
   - R⁹: ist gleich oder verschieden unabhängig voneinander Wasserstoff, C₁-C₂-Alkyl,
   sowie agrochemisch wirksame Salze davon.

Besonders bevorzugt werden Verbindungen der Formel (I), in denen ein oder mehrere der Symbole eine der folgenden Bedeutungen haben:
- A: steht für Phenyl, das bis zu zwei Substituenten enthalten kann, wobei die Substituenten unabhängig voneinander ausgewählt sind aus folgender Liste:
Cyano, Nitro, Halogen, C₁-C₂-Alkyl, C₁-C₂-Halogenalkyl, Hydroxy, C₁-C₂-Alkoxy oder C₁-C₂-Halogenalkoxy,
oder
- A: steht für einen heteroaromatischen Rest ausgewählt aus folgender Gruppe: Furan-2-yl, Furan-3-yl, Thiophen-2-yl, Thiophen-3-yl, Oxazol-2-yl, Oxazol-4-yl, Oxazol-5-yl, Thiazol-2-yl, Thiazol-4-yl, Thiazol-5-yl; Pyrazol-1-yl, Pyrazol-3-yl, Pyrazol-4-yl, Imidazol-1-yl, Imidazol-2-yl, Imidazol-4-yl, 1,2,4-Triazol-1-yl, Pyridin-2-yl, Pyridin-3-yl, Pyridin-4-yl, 1,2,4-Triazin-3-yl, 1H-Benzimidazol-2-yl oder 1,3-Benzothiazol-2-yl,
der bis zu zwei Substituenten enthalten kann, wobei die Substituenten unabhängig voneinander ausgewählt sind aus folgender Liste:
Substituenten am Kohlenstoff: Cyano, Halogen, C₁-C₂-Alkyl, C₁-C₂-Halogenalkyl, Hydroxy, C₁-C₂-Alkoxy oder C₁-C₂-Halogenalkoxy,
Substituenten am Stickstoff: C₁-C₂-Alkyl oder C₁-C₂-Halogenalkyl,
   - L¹: ist (C(R¹)₂)ₙ
mit n = 0 bis 3,
- R¹: ist gleich oder verschieden unabhängig voneinander Wasserstoff oder Methyl,
unter der Voraussetzung, dass L¹ maximal zwei Methylsubstituenten enthalten kann,
- Y: steht für Schwefel oder Sauerstoff,
- W: steht für -CH₂CH₂-,
- X: steht für -CH₂CH₂-,
- R²: steht für Wasserstoff, Methyl oder Halogen,
- L²: steht für -C(R⁸)₂-C(R⁸)₂- oder -CR⁹=CR⁹- oder -C=C-,
- L³: steht für eine direkte Bindung,
oder
- L³: steht für eine C₁- bis C₄-Kohlenstoffkette, die bis zu zwei Substituenten enthalten kann, wobei die Substituenten unabhängig voneinander ausgewählt sind aus folgender Liste:
Methyl, Methoxy oder CF₃,
- R³: steht für unsubstituiertes oder einfach substituiertes C₃-C₁₀-Cycloalkyl, wobei der Substituent ausgewählt ist aus folgender Liste:
Fluor, Chlor, Methyl, Ethyl, Cyclopropyl, Cyclopentyl oder Cyclohexyl,
oder
- R³: steht für Phenyl, das bis zu drei Substituenten enthalten kann, wobei die Substituenten unabhängig voneinander ausgewählt sind aus folgender Liste :
Cyano, Halogen, C₁-C₆-Alkyl, C₁-C₃-Halogenalkyl, Phenyl, Hydroxy, C₁-C₆-Alkoxy, C₁-C₆-Halogenalkoxy, C₁-C₆-Alkylthio oder C₁-C₆-Halogenalkylthio,
oder
- R³: steht für Naphthalen-1-yl, Naphthalen-2-yl, 1,2,3,4-Tetrahydronaphthalen-1-yl, 1,2,3,4-Tetrahydronaphthalen-2-yl, 5,6,7,8-Tetrahydronaphthalen-1-yl, 5,6,7,8-Tetrahydronaphthalen-2-yl, Decalin-1-yl, Decalin-2-yl, 1H-Inden-1-yl, 1H-Inden-2-yl, 1H-Inden-3-yl, 1H-Inden-4-yl, 1H-Inden-5-yl, 1H-Inden-6-yl, 1H-Inden-7-yl, 2,3-Dihydro-1H-inden-1-yl, 2,3-Dihydro-1H-inden-2-yl, 2,3-Dihydro-1H-Inden-4-yl oder 2,3-Dihydro-1H-inden-5-yl,
das bis zu zwei Substituenten enthalten kann, wobei die Substituenten unabhängig voneinander ausgewählt sind aus folgender Liste:
Fluor, Chlor, Brom, Jod, Methyl, Ethyl, CF₃, CHF₂, Cyclopropyl, Phenyl, Hydroxy, OMe, OEt, OCF₃, OCHF₂, OC₂F₅, SMe oder SCF₃,
oder
- R³: steht für Furan-2-yl, Furan-3-yl, Thiophen-2-yl, Thiophen-3-yl, Isoxazol-3-yl, Isoxazol-4-yl, Isoxazol-5-yl, 1H-Pyrrol-1-yl, 1H-Pyrrol-2-yl, 1H-Pyrrol-3-yl, Oxazol-2-yl, Oxazol-4-yl, Oxazol-5-yl, Thiazol-2-yl, Thiazol-4-yl, Thiazol-5-yl, Isothiazol-3-yl, Isothiazol-4-yl, Isothiazol-5-yl, Pyrazol-1-yl, Pyrazol-3-yl, Pyrazol-4-yl, Imidazol-1-yl, Imidazol-2-yl, Imidazol-4-yl, 1,2,4-Oxadiazol-3-yl, 1,2,4-Oxadiazol-5-yl, 1,3,4-Oxadiazol-2-yl, 1,2,4-Thiadiazol-3-yl, 1,2,4-Thiadiazol-5-yl, 1,3,4-Thiadiazol-2-yl, 1,2,3-Triazol-1-yl, 1,2,3-Triazol-2-yl, 1,2,3-Triazol-4-yl, 1,2,4-Triazol-1-yl, 1,2,4-Triazol-3-yl, Pyridin-2-yl, Pyridin-3-yl, Pyridin-4-yl, Pyridazin-3-yl, Pyridazin-4-yl, Pyrimidin-2-yl, Pyrimidin-4-yl, Pyrimidin-5-yl, Pyrazin-2-yl, 1,3,5-Triazin-2-yl oder 1,2,4-Triazin-3-yl,
das bis zu zwei Substituenten enthalten kann, wobei die Substituenten unabhängig voneinander ausgewählt sind aus folgender Liste :
Substituenten am Kohlenstoff: Cyano, Fluor, Chlor, Brom, Jod, Methyl, Ethyl, 1-Methylethyl, 1,1-Dimethylethyl, CF₃, CHF₂, C₂F₅, CCl₃, Cyclopropyl, Phenyl, Hydroxy, OMe, OEt, O*iso*Pr, OCF₃, OCHF₂, OC₂F₅, SMe oder SCF₃,
Substituenten am Stickstoff: Methyl, Ethyl, 1-Methylethyl, 1,1-Dimethylethyl, Cyclopropyl oder Phenyl,
oder
- R³: steht für 1H-Indol-1-yl, 1H-Indol-2-yl, 1H-Indol-3-yl, 1H-Indol-4-yl, 1H-Indol-5-yl, 1H-Indol-6-yl, 1H-Indol-7-yl, 1H-Benzimidazol-1-yl, 1H-Benzimidazol-2-yl, 1H-Benzimidazol-4-yl, Benzimidazol-5-yl, 1H-Indazol-1-yl, 1H-Indazol-3-yl, 1H-Indazol-4-yl, 1H-Indazol-5-yl, 1H-Indazol-6-yl, 1H-Indazol-7-yl, 2H-Indazol-2-yl, 1-benzofuran-2-yl, 1-benzofuran-3-yl, 1-benzofuran-4-yl, 1-benzofuran-5-yl, 1-benzofuran-6-yl, 1-benzofuran-7-yl, 1-benzothiophen-2-yl, 1-benzothiophen-3-yl, 1-benzothiophen-4-yl, 1-benzothiophen-5-yl, 1-benzothiophen-6-yl, 1-benzothiophen-7-yl, Quinolin-2-yl, Quinolin-3-yl, Quinolin-4-yl, Quinolin-5-yl, Quinolin-6-yl, Quinolin-7-yl, Quinolin-8-yl, Isoquinolin-1-yl, Isoquinolin-3-yl, Isoquinolin-4-yl, Isoquinolin-5-yl, Isoquinolin-6-yl, Isoquinolin-7-yl, oder Isoquinolin-8-yl,
das bis zu zwei Substituenten enthalten kann, wobei die Substituenten unabhängig voneinander ausgewählt sind aus folgender Liste :
Substituenten am Kohlenstoff: Cyano, Fluor, Chlor, Brom, Jod, Methyl, Ethyl, 1-Methylethyl, 1,1-Dimethylethyl, CF₃, CHF₂, C₂F₅, CCl₃, Cyclopropyl, Phenyl, Hydroxy, OMe, OEt, O*iso*Pr, OCF₃, OCHF₂, OC₂F₅, SMe oder SCF₃,
Substituenten am Stickstoff: Methyl, Ethyl, 1-Methylethyl, 1,1-Dimethylethyl, Cyclopropyl oder Phenyl,
oder
- R³: ist ein unsubstituiertes oder einfach substituiertes Pyrrolidin-2-yl, Pyrrolidin-3-yl, Morpholin-3-yl, Morpholin-2-yl, Piperidin-2-yl, Piperidin-3-yl, oder Piperazin-2-yl, wobei der Substituent ausgewählt ist aus folgender Liste:
Substituenten am Kohlenstoff: Methyl, Ethyl, CF₃, Cyclopropyl oder Phenyl,
Substituenten am Stickstoff: Methyl, Ethyl, Cyclopropyl oder Phenyl,
- R⁸: ist gleich oder verschieden unabhängig voneinander Wasserstoff, Methyl, Ethyl oder CF₃,
- R⁹: ist gleich oder verschieden unabhängig voneinander Wasserstoff, Methyl oder Ethyl,
sowie agrochemisch wirksame Salze davon.

Ganz besonders bevorzugt werden Verbindungen der Formel (I), in denen ein oder mehrere der Symbole eine der folgenden Bedeutungen haben:
steht für Phenyl, das bis zu zwei Substituenten enthalten kann, wobei die Substituenten unabhängig voneinander ausgewählt sind aus folgender Liste:
   Cyano, Fluor, Chlor, Brom, Jod, Methyl, Ethyl, CF₃, CHF₂, C₂F₅, CCl₃, Hydroxy, OMe, OCF₃, OCHF₂, OCH₂CF₃ oder OC₂F_{5.}
oder
- A: steht für einen heteroaromatischen Rest ausgewählt aus folgender Gruppe: Thiophen-3-yl, Pyrazol-1-yl, Pyrazol-4-yl, Pyridin-4-yl, 1H-Benzimidazol-2-yl oder 1,3-Benzothiazol-2-yl,
der bis zu zwei Substituenten enthalten kann, wobei die Substituenten unabhängig voneinander ausgewählt sind aus folgender Liste:
Substituenten am Kohlenstoff: Cyano, Fluor, Chlor, Brom, Jod, Methyl, Ethyl, CF₃, CHF₂, C₂F₅, CCl₃, Hydroxy, OMe, OCF₃, OCHF₂, OCH₂CF₃, oder OC₂F₅,
Substituenten am Stickstoff: Methyl, Ethyl oder CF₃,

- L¹: ist (C(R¹)₂)ₙ
mit n = 0 bis 3,
- R¹: ist gleich oder verschieden unabhängig voneinander Wasserstoff oder Methyl,
unter der Voraussetzung, dass L¹ maximal einen Methylsubstituenten enthalten kann,
- Y: steht für Schwefel oder Sauerstoff,
- W: steht für -CH₂CH₂-,
- X: steht für -CH₂CH₂-,
- R²: steht für Wasserstoff, Methyl, Chlor oder Brom,
- L²: steht für -C(R⁸)₂-C(R⁸)₂- oder -CR⁹=CR⁹- oder -C=C-,
- L³: steht für eine direkte Bindung,
oder
- L³: steht für eine C₁- bis C₄-Kohlenstoffkette,
- R³: steht für Cyclopropyl, Cyclobutyl, Cyclopentyl, Cyclohexyl, Cycloheptyl oder Cyclooctyl,
oder
- R³: steht für Phenyl, das bis zu zwei Substituenten enthalten kann, wobei die Substituenten unabhängig voneinander ausgewählt sind aus folgender Liste :
Cyano, Fluor, Chlor, Brom, Jod, Methyl, Ethyl, 1-Methylethyl, 1,1-Dimethylethyl, CF₃, CHF₂, C₂F₅, CCl₃, Phenyl, Hydroxy, OMe, OEt, O*iso*Pr, OCF₃, OCHF₂, OC₂F₅, SMe oder SCF₃,
oder
- R³: steht für Naphthalen-1-yl, Naphthalen-2-yl, 1,2,3,4-Tetrahydronaphthalen-1-yl, 1,2,3,4-Tetrahydronaphthalen-2-yl, 5,6,7,8-Tetrahydronaphthalen-1-yl, 5,6,7,8-Tetrahydronaphthalen-2-yl, Decalin-1-yl, Decalin-2-yl, 1H-Inden-1-yl, 1H-Inden-2-yl, 1H-Inden-3-yl, 1H-Inden-4-yl, 1H-Inden-5-yl, 1H-Inden-6-yl, 1H-Inden-7-yl, 2,3-Dihydro-1H-inden-1-yl, 2,3-Dihydro-1H-inden-2-yl, 2,3-Dihydro-1H-Inden-4-yl oder 2,3-Dihydro-1H-inden-5-yl,
oder
- R³: steht für Furan-2-yl, Furan-3-yl, Thiophen-2-yl, Thiophen-3-yl, Isoxazol-3-yl, Isoxazol-4-yl, Isoxazol-5-yl, 1H-Pyrrol-1-yl, 1H-Pyrrol-2-yl, 1H-Pyrrol-3-yl, Oxazol-2-yl, Oxazol-4-yl, Oxazol-5-yl, Thiazol-2-yl, Thiazol-4-yl, Thiazol-5-yl, Isothiazol-3-yl, Isothiazol-4-yl, Isothiazol-5-yl, Pyrazol-1-yl, Pyrazol-3-yl, Pyrazol-4-yl, Imidazol-1-yl, Imidazol-2-yl, Imidazol-4-yl, 1,2,4-Oxadiazol-3-yl, 1,2,4-Oxadiazol-5-yl, 1,3,4-Oxadiazol-2-yl, 1,2,4-Thiadiazol-3-yl, 1,2,4-Thiadiazol-5-yl, 1,3,4-Thiadiazol-2-yl, 1,2,3-Triazol-1-yl, 1,2,3-Triazol-2-yl, 1,2,3-Triazol-4-yl, 1,2,4-Triazol-1-yl, 1,2,4-Triazol-3-yl, Pyridin-2-yl, Pyridin-3-yl, Pyridin-4-yl, Pyridazin-3-yl, Pyridazin-4-yl, Pyrimidin-2-yl, Pyrimidin-4-yl, Pyrimidin-5-yl, Pyrazin-2-yl, 1,3,5-Triazin-2-yl oder 1,2,4-Triazin-3-yl,
oder
- R³: steht für 1H-Indol-1-yl, 1H-Indol-2-yl, 1H-Indol-3-yl, 1H-Indol-4-yl, 1H-Indol-5-yl, 1H-Indol-6-yl, 1H-Indol-7-yl, 1H-Benzimidazol-1-yl, 1H-Benzimidazol-2-yl, 1H-Benzimidazol-4-yl, Benzimidazol-5-yl, 1H-Indazol-1-yl, 1H-Indazol-3-yl, 1H-Indazol-4-yl, 1H-Indazol-5-yl, 1H-Indazol-6-yl, 1H-Indazol-7-yl, 2H-Indazol-2-yl, 1-benzofuran-2-yl, 1-benzofuran-3-yl, 1-benzofuran-4-yl, 1-benzofuran-5-yl, 1-benzofuran-6-yl, 1-benzofuran-7-yl, 1-benzothiophen-2-yl, 1-benzothiophen-3-yl, 1-benzothiophen-4-yl, 1-benzothiophen-5-yl, 1-benzothiophen-6-yl, 1-benzothiophen-7-yl, Quinolin-2-yl, Quinolin-3-yl, Quinolin-4-yl, Quinolin-5-yl, Quinolin-6-yl, Quinolin-7-yl, Quinolin-8-yl, Isoquinolin-1-yl, Isoquinolin-3-yl, Isoquinolin-4-yl, Isoquinolin-5-yl, Isoquinolin-6-yl, Isoquinolin-7-yl oder Isoquinolin-8-yl,
oder
- R³: steht für Pyrrolidin-2-yl, Pyrrolidin-3-yl, Morpholin-3-yl, Morpholin-2-yl, Piperidin-2-yl, Piperidin-3-yl, oder Piperazin-2-yl,
- R⁸: ist gleich oder verschieden unabhängig voneinander Wasserstoff, Methyl oder Ethyl,
- R⁹: ist gleich oder verschieden unabhängig voneinander Wasserstoff, Methyl oder Ethyl,
sowie agrochemisch wirksame Salze davon.

Insbesondere bevorzugt werden Verbindungen der Formel **(I)**, in denen ein oder mehrere der Symbole eine der folgenden Bedeutungen haben:
- A: steht für Methyl, 1,3-Dihydro-2H-indazol-2-yl, 3-Chlorphenyl, 2,5-Dichlorphenyl, 3-(Trifluormethyl)phenyl, 3,4-Dimethoxyphenyl, 2,5-Dibromphenyl, 2-Brom-5-(trifluormethyl)phenyl, 5-Brom-2-methylphenyl, 5-Jod-2-methylphenyl, 2,5-Bis(trifluormethyl)phenyl, 2-Chlor-5-(trifluormethyl)phenyl, 1H-Pyrazol-1-yl, 3-(Trifluormethyl)-1H-pyrazol-1-yl, 3,5-dimethyl-1H-pyrazol-1-yl, 5-Methyl-3-(trifluormethyl)-1H-pyrazol-1-yl, 4-Chlor-5-methyl-3-(trifluormethyl)-1H-pyrazol-1-yl, 3,5-Bis(trifluormethyl)-1H-pyrazol-1-yl, 5-(Difluormethyl)-3-methyl-1H-pyrazol-1-yl, 3,5-Dichlor-1H-pyrazol-1-yl, 3,5-Dibrom-1H-pyrazol-1-yl, 5-Chlor-1-methyl-1H-pyrazol-4-yl, Thiophen-3-yl, Pyridin-4-yl, 3-Chlor-5-(trifluormethyl)pyridin-2-yl , 1H-Benzimidazol-2-yl oder 1,3-Benzothiazol-2-yl,
- L¹: ist eine direkte Bindung,
oder
- L¹: steht für -CH₂-, -CH₂CH₂-, -CH₂CH₂CH₂- oder -CHCH₃-,
- Y: steht für Schwefel oder Sauerstoff,
- W: steht für -CH₂CH₂-,
- X: steht für -CH₂CH₂-,
- R²: steht für Wasserstoff, Methyl oder Brom,
- L²: steht für -CH₂CH₂-, -CH₂CHCH₃-, -CH=CH- (Z), -CH=CH- (E) oder -C=C-,
- L³: steht für eine direkte Bindung,
oder
- L³: steht für -CH₂-, -CH₂C(CH₃)₂-, -CH₂CHCH₃-,
- R³: steht für Methyl, -CH=CH₂, -C=CH, CF₃, Cyclohexyl, Phenyl, 4-Chlorphenyl, 4-Methoxyphenyl, 3-(Trifluormethyl)phenyl, Naphthalen-1-yl, Naphthalen-2-yl oder Pyridin-3-yl,
sowie agrochemisch wirksame Salze davon.

Weiterhin insbesondere bevorzugt werden Verbindungen der Formel **(I),** in denen
- A: für ein unsubstituiertes oder substituiertes Pyrazol-1-yl oder Pyrazol-4-yl steht,
der bis zu zwei Substituenten enthalten kann, wobei die Substituenten unabhängig voneinander ausgewählt sind aus folgender Liste:
Substituenten am Kohlenstoff: Cyano, Fluor, Chlor, Brom, Jod, Methyl, Ethyl, CF₃, CHF₂, C₂F₅, CCl₃, Hydroxy, OMe, OCF₃, OCHF₂, OCH₂CF₃, oder OC₂F₅,
Substituenten am Stickstoff: Methyl, Ethyl oder CF₃,
und L₁ für -CH₂- steht,
wobei die übrigen Substituenten eine oder mehrere der oben genannten Bedeutungen haben,
sowie die agrochemisch wirksamen Salzen davon.

Weiterhin insbesondere bevorzugt werden Verbindungen der Formel (I), in denen
- A: für ein unsubstituiertes oder substituiertes Phenyl steht,
wobei die Substituenten unabhängig voneinander ausgewählt sind aus folgender Liste:
Cyano, Fluor, Chlor, Brom, Jod, Methyl, Ethyl, CF₃, CHF₂, C₂F₅, CCl₃, Hydroxy, OMe, OCF₃, OCHF₂, OCH₂CF₃ oder OC₂F₅,
und L¹ steht für -CH₂-,
wobei die übrigen Substituenten eine oder mehrere der oben genannten Bedeutungen haben,
sowie die agrochemisch wirksamen Salzen davon.

Weiterhin insbesondere bevorzugt werden Verbindungen der Formel (I), in denen
- Y: für Sauerstoff steht,
wobei die übrigen Substituenten eine oder mehrere der oben genannten Bedeutungen haben,
sowie die agrochemisch wirksamen Salzen davon.

Weiterhin insbesondere bevorzugt werden Verbindungen der Formel (I), in denen ein oder mehrere der Symbole eine der folgenden Bedeutungen haben:
- W: steht für -CH₂CH₂-
- X: steht für -CH₂CH₂-
wobei die übrigen Substituenten eine oder mehrere der oben genannten Bedeutungen haben,
sowie die agrochemisch wirksamen Salzen davon.

Weiterhin insbesondere bevorzugt werden Verbindungen der Formel (I), in denen
- R²: für Wasserstoff, Halogen oder Methyl steht,
wobei die übrigen Substituenten eine oder mehrere der oben genannten Bedeutungen haben,
sowie die agrochemisch wirksamen Salzen davon.

Weiterhin insbesondere bevorzugt werden Verbindungen der Formel (I), in denen
- L²: für -CH₂CH₂- steht,
wobei die übrigen Substituenten eine oder mehrere der oben genannten Bedeutungen haben,
sowie die agrochemisch wirksamen Salzen davon.

Weiterhin insbesondere bevorzugt werden Verbindungen der Formel (I), in denen
- L²: für -CH=CH- (Z) steht,
wobei die übrigen Substituenten eine oder mehrere der oben genannten Bedeutungen haben,
sowie die agrochemisch wirksamen Salzen davon.

Weiterhin insbesondere bevorzugt werden Verbindungen der Formel (I), in denen
- L²: für -CH=CH- (E) steht,
wobei die übrigen Substituenten eine oder mehrere der oben genannten Bedeutungen haben,
sowie die agrochemisch wirksamen Salzen davon.

Weiterhin insbesondere bevorzugt werden Verbindungen der Formel (I), in denen
- L²: für -C=C- steht,
wobei die übrigen Substituenten eine oder mehrere der oben genannten Bedeutungen haben,
sowie die agrochemisch wirksamen Salzen davon.

Weiterhin insbesondere bevorzugt werden Verbindungen der Formel (I), in denen
- L³: für eine direkte Bindung steht,
wobei die übrigen Substituenten eine oder mehrere der oben genannten Bedeutungen haben,
sowie die agrochemisch wirksamen Salzen davon.

Weiterhin insbesondere bevorzugt werden Verbindungen der Formel (I), in denen
- L³: für -CH₂- steht,
wobei die übrigen Substituenten eine oder mehrere der oben genannten Bedeutungen haben,
sowie die agrochemisch wirksamen Salzen davon.

Weiterhin insbesondere bevorzugt werden Verbindungen der Formel (I), in denen
- R³: für ein unsubstituiertes Naphthalen-1-yl oder Naphthalen-2-yl steht,
wobei die übrigen Substituenten eine oder mehrere der oben genannten Bedeutungen haben,
sowie die agrochemisch wirksamen Salzen davon.

Weiterhin insbesondere bevorzugt werden Verbindungen der Formel (I), in denen
- R³: für ein unsubstituiertes Phenyl steht,
wobei die übrigen Substituenten eine oder mehrere der oben genannten Bedeutungen haben,
sowie die agrochemisch wirksamen Salzen davon.

Weiterhin insbesondere bevorzugt werden Verbindungen der Formel (I), in denen
- A: für 2,5-Dimethylphenyl, 2,3,6-Trifluorphenyl, 3,5-Bis(difluormethyl)-1H-pyrazol-1-yl, 3-(Propan-2-yl)-5-(trifluormethyl)-1H-pyrazol-1-yl, 3-tert-Butyl-5-(pentafluorethyl)-1H-pyrazol-1-yl, 3-tert-Butyl-5-(trifluormethyl)-1H-pyrazol-1-yl, 5-Ethyl-3-(trifluormethyl)-1H-pyrazol-1-yl, 5-tert-Butyl-3-(pentafluorethyl)-1H-pyrazol-1-yl, 5-tert-Butyl-3-(trifluormethyl)-1H-pyrazol-1-yl, 2H-Indazol-2-yl, 1,3-Benzodioxol-5-yl oder 1H-Benzimidazol-2-yl steht,
wobei die übrigen Substituenten eine oder mehrere der oben genannten Bedeutungen haben,
sowie die agrochemisch wirksamen Salzen davon.

Weiterhin insbesondere bevorzugt werden Verbindungen der Formel (I), in denen
- R³: für 2,4-Dichlorphenyl, 2,4-Dimethoxyphenyl, 2,5-Dimethoxyphenyl, 2-Methoxyphenyl, 2-Methylphenyl, 3,5-Dimethylisoxazol-4-yl, 3-Methylpyridin-2-yl, Chinolin-8-yl, Morpholin-4-yl, Naphthalen, tert-Butyl, Trimethylsilyl, 1,2,3,4-Tetrahydronaphthalen-1-yl, Cyclohexyl, 2-Bromphenyl, 2-Chlorphenyl, 2-Chlor-6-fluorphenyl, 2,6-Difluorphenyl steht,
wobei die übrigen Substituenten eine oder mehrere der oben genannten Bedeutungen haben,
sowie die agrochemisch wirksamen Salzen davon.

Die zuvor genannten Reste-Definitionen können untereinander in beliebiger Weise kombiniert werden. Außerdem können einzelne Definitionen entfallen.

Je nach Art der oben definierten Substituenten weisen die Verbindungen der Formel **(I)** saure oder basische Eigenschaften auf und können mit anorganischen oder organischen Säuren oder mit Basen oder mit Metallionen Salze, gegebenenfalls auch innere Salze oder Addukte bilden. Tragen die Verbindungen der Formel **(I)** Amino, Alkylamino oder andere, basische Eigenschaften induzierende Gruppen, so können diese Verbindungen mit Säuren zu Salzen umgesetzt werden oder fallen durch die Synthese direkt als Salze an. Tragen die Verbindungen der Formel **(I)** Hydroxy, Carboxy oder andere, saure Eigenschaften induzierende Gruppen, so können diese Verbindungen mit Basen zu Salzen umgesetzt werden. Geeignete Basen sind beispielsweise Hydroxide, Carbonate, Hydrogencarbonate der Alkali- und Erdalkalimetalle, insbesondere die von Natrium, Kalium, Magnesium und Calcium, weiterhin Ammoniak, primäre, sekundäre und tertiäre Amine mit (C₁-C₄-)-Alkyl-Gruppen, Mono-, Di- und Trialkanolamine von (C₁-C₄)-Alkanolen, Cholin sowie Chlorcholin.

Die so erhältlichen Salze weisen ebenfalls fungizide, herbizide und insektizide Eigenschaften auf.

Beispiele für anorganische Säuren sind Halogenwasserstoffsäuren wie Fluorwasserstoff, Chlorwasserstoff, Bromwasserstoff und Iodwasserstoff, Schwefelsäure, Phosphorsäure und Salpetersäure und saure Salze wie NaHSO₄ und KHSO₄. Als organische Säuren kommen beispielsweise Ameisensäure, Kohlensäure und Alkansäuren wie Essigsäure, Trifluoressigsäure, Trichloressigsäure und Propionsäure sowie Glycolsäure, Thiocyansäure, Milchsäure, Bernsteinsäure, Zitronensäure, Benzoesäure, Zimtsäure, Oxal-säure, Alkylsulfonsäuren (Sulfonsäuren mit geradkettigen oder verzweigten Alkylresten mit 1 bis 20 Kohlenstoffatomen), Arylsulfonsäuren oder -disulfonsäuren (aromatische Reste wie Phenyl und Naphthyl welche ein oder zwei Sulfonsäuregruppen tragen), Alkylphosphon-säuren (Phosphonsäuren mit geradkettigen oder verzweigten Alkylresten mit 1 bis 20 Kohlenstoffatomen), Arylphosphonsäuren oder - diphosphonsäuren (aromatische Reste wie Phenyl und Naphthyl welche ein oder zwei Phosphonsäurereste tragen), wobei die Alkyl- bzw. Arylreste weitere Substituenten tragen können, z.B. p-Toluolsulfonsäure, Salicylsäure, p-Aminosalicylsäure, 2-Phenoxybenzoesäure, 2-Acetoxybenzoesäure etc..

Als Metallionen kommen insbesondere die Ionen der Elemente der zweiten Hauptgruppe, insbesondere Calzium und Magnesium, der dritten und vierten Hauptgruppe, insbesondere Aluminium, Zinn und Blei, sowie der ersten bis achten Nebengruppe, insbesondere Chrom, Mangan, Eisen, Kobalt, Nickel, Kupfer, Zink und andere in Betracht. Besonders bevorzugt sind die Metallionen der Elemente der vierten Periode. Die Metalle können dabei in den verschiedenen ihnen zukommenden Wertigkeiten vorliegen.

Gegebenenfalls substituierte Gruppen können einfach oder mehrfach substituiert sein, wobei bei Mehrfachsubstitutionen die Substituenten gleich oder verschieden sein können.

Bei den in den vorstehenden Formeln angegebenen Definitionen der Symbole wurden Sammelbegriffe verwendet, die allgemein repräsentativ für die folgenden Substituenten stehen:
**Halogen:** Fluor, Chlor, Brom und Jod;
**Alkyl:** gesättigte, geradkettige oder verzweigte Kohlenwasserstoffreste mit 1 bis 8 Kohlenstoffatomen, z.B. (aber nicht beschränkt auf) C₁-C₆-Alkyl wie Methyl, Ethyl, Propyl, 1-Methylethyl, Butyl, 1-Methyl-propyl, 2-Methylpropyl, 1,1-Dimethylethyl, Pentyl, 1-Methylbutyl, 2-Methylbutyl, 3-Methylbutyl, 2,2-Di-methylpropyl, 1-Ethylpropyl, Hexyl, 1,1-Dimethylpropyl, 1,2-Dimethylpropyl,1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl;
**Alkenyl:** ungesättigte, geradkettige oder verzweigte Kohlenwasserstoffreste mit 2 bis 8 Kohlenstoffatomen und einer Doppelbindung in einer beliebigen Position, z.B. (aber nicht beschränkt auf) C₂-C₆-Alkenyl wie Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1,-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl und 1-Ethyl-2-methyl-2-propenyl;
**Alkinyl:** geradkettige oder verzweigte Kohlenwasserstoffgruppen mit 2 bis 8 Kohlenstoffatomen und einer Dreifachbindung in einer beliebigen Position, z.B. (aber nicht beschränkt auf) C₂-C₆-Alkinyl wie Ethinyl, 1-Propinyl, 2-Propinyl, 1-Butinyl, 2-Butinyl, 3-Butinyl, 1-Methyl-2-propinyl, 1-Pentinyl, 2-Pentinyl, 3-Pentinyl, 4-Pentinyl, 1-Methyl-2-butinyl, 1-Methyl-3-butinyl, 2-Methyl-3-butinyl, 3-Methyl-1-butinyl, 1,1-Dimethyl-2-propinyl, 1-Ethyl-2-propinyl, 1-Hexinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-1-pentinyl, 3-Methyl-4-pentinyl, 4-Methyl-1-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 3,3-Dimethyl-1-butinyl, 1-Ethyl-2-butinyl, 1-Ethyl-3-butinyl, 2-Ethyl-3-butinyl und 1-Ethyl-1-methyl-2-propinyl;
**Alkoxy:** gesättigte, geradkettige oder verzweigte Alkoxyreste mit 1 bis 8 Kohlenstoffatomen, z.B. (aber nicht beschränkt auf) C₁-C₆-Alkoxy wie Methoxy, Ethoxy, Propoxy, 1-Methylethoxy, Butoxy, 1-Methyl-propoxy, 2-Methylpropoxy, 1,1-Dimethylethoxy, Pentoxy, 1-Methylbutoxy, 2-Methylbutoxy, 3-Methylbutoxy, 2,2-Di-methylpropoxy, 1-Ethylpropoxy, Hexoxy, 1,1-Dimethylpropoxy, 1,2-Dimethylpropoxy,1-Methylpentoxy, 2-Methylpentoxy, 3-Methylpentoxy, 4-Methylpentoxy, 1,1-Dimethylbutoxy, 1,2-Dimethylbutoxy, 1,3-Dimethylbutoxy, 2,2-Dimethylbutoxy, 2,3-Dimethylbutoxy, 3,3-Dimethylbutoxy, 1-Ethylbutoxy, 2-Ethylbutoxy, 1,1,2-Trimethylpropoxy, 1,2,2-Trimethylpropoxy, 1-Ethyl-1-methylpropoxy und 1-Ethyl-2-methylpropoxy;
**Alkylthio:** gesättigte, geradkettige oder verzweigte Alkylthioreste mit 1 bis 8 Kohlenstoffatomen, z.B. (aber nicht beschränkt auf) C₁-C₆-Alkylthio wie Methylthio, Ethylthio, Propylthio, 1-Methylethylthio, Butylthio, 1-Methyl-propylthio, 2-Methylpropylthio, 1,1-Dimethylethylthio, Pentylthio, 1-Methylbutylthio, 2-Methylbutylthio, 3-Methylbutylthio, 2,2-Di-methylpropylthio, 1-Ethylpropylthio, Hexylthio, 1,1-Dimethylpropylthio, 1,2-Dimethylpropylthio,1-Methylpentylthio, 2-Methylpentylthio, 3-Methyl-pentylthio, 4-Methylpentylthio, 1,1-Dimethylbutylthio, 1,2-Dimethylbutylthio, 1,3-Dimethyl-butylthio, 2,2-Dimethylbutylthio, 2,3-Dimethylbutylthio, 3,3-Dimethylbutylthio, 1-Ethylbutylthio, 2-Ethylbutylthio, 1,1,2-Trimethylpropylthio, 1,2,2-Trimethylpropylthio, 1-Ethyl-1-methylpropyl-thio und 1-Ethyl-2-methylpropylthio;
**Alkoxycarbonyl:** eine Alkoxygruppe mit 1 bis 6 Kohlenstoffatomen (wie vorstehend genannt), welche über eine Carbonylgruppe (-CO-) an das Gerüst gebunden ist;
**Alkylsulfinyl:** gesättigte, geradkettige oder verzweigte Alkylsulfinylreste mit 1 bis 8 Kohlenstoffatomen, z.B. (aber nicht beschränkt auf) C₁-C₆-Alkylsulfinyl wie Methylsulfinyl, Ethylsulfinyl, Propylsulfinyl, 1-Methylethylsulfinyl, Butylsulfinyl, 1-Methyl-propylsulfinyl, 2-Methylpropylsulfinyl, 1,1-Dimethylethylsulfinyl, Pentylsulfinyl, 1-Methylbutylsulfinyl, 2-Methylbutylsulfinyl, 3-Methylbutylsulfinyl, 2,2-Di-methylpropylsulfinyl, 1-Ethylpropylsulfinyl, Hexylsulfinyl, 1,1-Dimethylpropylsulfmyl, 1,2-Dimethylpropylsulfinyl,1-Methylpentylsulfinyl, 2-Methylpentyl-sulfinyl, 3-Methylpentylsulfinyl, 4-Methylpentylsulfinyl, 1,1-Dimethylbutylsulfinyl, 1,2-Dimethylbutylsulfinyl, 1,3-Dimethylbutylsulfinyl, 2,2-Dimethylbutylsulfinyl, 2,3-Dimethylbutylsulfinyl, 3,3-Dimethylbutylsulfinyl, 1-Ethylbutylsulfinyl, 2-Ethylbutylsulfinyl, 1,1,2-Trimethylpropylsulfinyl, 1,2,2-Trimethylpropylsulfinyl, 1-Ethyl-1-methylpropylsulfinyl und 1-Ethyl-2-methylpropylsulfinyl;
**Alkylsulfonyl:** gesättigte, geradkettige oder verzweigte Alkylsulfonylreste mit 1 bis 8 Kohlenstoffatomen, z.B. (aber nicht beschränkt auf) C₁-C₆-Alkylsulfonyl wie Methylsulfonyl, Ethylsulfonyl, Propylsulfonyl, 1-Methylethylsulfonyl, Butylsulfonyl, 1-Methyl-propylsulfonyl, 2-Methylpropylsulfonyl, 1,1-Dimethylethylsulfonyl, Pentylsulfonyl, 1-Methylbutylsulfonyl, 2-Methylbutylsulfonyl, 3-Methylbutylsulfonyl, 2,2-Di-methylpropylsulfonyl, 1-Ethylpropylsulfonyl, Hexylsulfonyl, 1,1-Dimethylpropylsulfonyl, 1,2-Dimethylpropylsulfonyl,1-Methylpentylsulfonyl, 2-Methylpentyl-sulfonyl, 3-Methylpentylsulfonyl, 4-Methylpentylsulfonyl, 1,1-Dimethyl-butylsulfonyl, 1,2-Dimethylbutylsulfonyl, 1,3-Dimethylbutylsulfonyl, 2,2-Dimethylbutylsulfonyl, 2,3-Dimethylbutylsulfonyl, 3,3-Dimethylbutylsulfonyl, 1-Ethylbutylsulfonyl, 2-Ethylbutylsulfonyl, 1,1,2-Trimethylpropylsulfonyl, 1,2,2-Trimethylpropylsulfonyl, 1-Ethyl-1-methylpropylsulfonyl und 1-Ethyl-2-methylpropylsulfonyl;
**Cycloalkyl:** monocyclische, gesättigte Kohlenwasserstoffgruppen mit 3 bis 10 Kohlenstoffringgliedern, z.B. (aber nicht beschränkt auf) Cyclopropyl, Cyclopentyl und Cyclohexyl;
**Halogenalkyl:** geradkettige oder verzweigte Alkylgruppen mit 1 bis 8 Kohlenstoffatomen (wie vorstehend genannt), wobei in diesen Gruppen teilweise oder vollständig die Wasserstoffatome durch Halogenatome wie vorstehend genannt ersetzt sein können, z.B. (aber nicht beschränkt auf) C₁-C₃-Halogenalkyl wie Chlormethyl, Brommethyl, Dichlormethyl, Trichlormethyl, Fluormethyl, Difluormethyl, Trifluormethyl, Chlorfluormethyl, Dichlorfluormethyl, Chlordifluormethyl, 1-Chlorethyl, 1-Bromethyl, 1-Fluorethyl, 2-Fluorethyl, 2,2-Difluorethyl, 2,2,2-Trifluorethyl, 2-Chlor-2-fluorethyl, 2-Chlor,2-difluorethyl, 2,2-Dichlor-2-fluorethyl, 2,2,2-Trichlorethyl, Pentafluorethyl und 1,1,1-Trifluorprop-2-yl;
**Halogenalkoxy:** geradkettige oder verzweigte Alkoxygruppen mit 1 bis 8 Kohlenstoffatomen (wie vorstehend genannt), wobei in diesen Gruppen teilweise oder vollständig die Wasserstoffatome durch Halogenatome wie vorstehend genannt ersetzt sein können, z.B. (aber nicht beschränkt auf) C₁-C₃-Halogenalkoxy wie Chlormethoxy, Brommethoxy, Dichlormethoxy, Trichlormethoxy, Fluormethoxy, Difluormethoxy, Trifluormethoxy, Chlorfluormethoxy, Dichlorfluormethoxy, Chlordifluormethoxy, 1-Chlorethoxy, 1-Bromethoxy, 1-Fluorethoxy, 2-Fluorethoxy, 2,2-Difluorethoxy, 2,2,2-Trifluorethoxy, 2-Chlor-2-fluorethoxy, 2-Chlor,2-difluorethoxy, 2,2-Dichlor-2-fluorethoxy, 2,2,2-Trichlorethoxy, Pentafluor-ethoxy und 1,1,1-Trifluorprop-2-oxy;
**Halogenalkylthio:** geradkettige oder verzweigte Alkylthiogruppen mit 1 bis 8 Kohlenstoffatomen (wie vorstehend genannt), wobei in diesen Gruppen teilweise oder vollständig die Wasserstoffatome durch Halogenatome wie vorstehend genannt ersetzt sein können, z.B. (aber nicht beschränkt auf) C₁-C₃-Halogenalkylthio wie Chlormethylthio, Brommethylthio, Dichlormethylthio, Trichlormethylthio, Fluormethylthio, Difluormethylthio, Trifluormethylthio, Chlorfluormethylthio, Dichlorfluor-methylthio, Chlordifluormethylthio, 1-Chlorethylthio, 1-Bromethylthio, 1-Fluorethylthio, 2-Fluorethylthio, 2,2-Difluorethylthio, 2,2,2-Trifluorethylthio, 2-Chlor-2-fluorethylthio, 2-Chlor,2-difluorethylthio, 2,2-Dichlor-2-fluorethylthio, 2,2,2-Trichlorethylthio, Pentafluorethylthio und 1,1,1-Trifluorprop-2-ylthio;
**Heteroaryl:** 5 oder 6-gliedriges, vollständig ungesättigtes monocyclisches Ringsystem, enthaltend ein bis vier Heteroatome aus der Gruppe Sauerstoff, Stickstoff oder Schwefel, enthält der Ring mehrere Sauerstoffatome, so stehen diese nicht direkt benachbart;
**5-gliedriges Heteroaryl: enthaltend ein bis vier Stickstoffatome oder ein bis drei Stickstoffatome und ein Schwefel- oder Sauerstoffatom:** 5-Ring Heteroarylgruppen, welche neben Kohlenstoffatomen ein bis vier Stickstoffatome oder ein bis drei Stickstoffatome und ein Schwefel- oder Sauerstoffatom als Ringglieder enthalten können, z.B. (aber nicht beschränkt auf) 2-Furyl, 3-Furyl, 2-Thienyl, 3-Thienyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 3-Pyrazolyl, 4-Pyrazolyl, 5-Pyrazolyl, 2-Oxazolyl, 4-Oxazolyl, 5- Oxazolyl, 2-Thiazolyl, 4-Thiazolyl, 5-Thiazolyl, 2-Imidazolyl, 4-Imidazolyl, 1,2,4-Oxadiazol-3-yl, 1,2,4-Oxadiazol-5-yl, 1,2,4-Thiadiazol-3-yl, 1,2,4-Thiadiazol-5-yl, 1,2,4-Triazol-3-yl, 1,3,4-Oxadiazol-2-yl, 1,3,4-Thiadiazol-2-yl und 1,3,4-Triazol-2-yl;
**über Stickstoff gebundenes 5-gliedriges Heteroaryl, enthaltend ein bis vier Stickstoffatome, oder über Stickstoff gebundenes benzokondensiertes 5-gliederiges Heteroaryl, enthaltend ein bis drei Stickstoffatome:** 5-Ring Heteroarylgruppen, welche neben Kohlenstoffatomen ein bis vier Stickstoffatome bzw. ein bis drei Stickstoffatome als Ringglieder enthalten können, und in welchen zwei benachbarte Kohlenstoffringglieder oder ein Stickstoff- und ein benachbartes Kohlenstoffringglied durch eine Buta-1,3-dien-1,4-diylgruppe verbrückt sein können, in der ein oder zwei C-Atome durch N-Atome ersetzt sein können, in der ein oder zwei C-Atome durch N-Atome ersetzt sein können, wobei diese Ringe über eines der Stickstoffringglieder an das Gerüst gebunden sind, z.B. (aber nicht beschränkt auf) 1-Pyrrolyl, 1-Pyrazolyl, 1,2,4-Triazol-1-yl, 1-Imidazolyl, 1,2,3-Triazol-1-yl, 1,3,4-Triazol-1-yl;
**6-gliedriges Heteroaryl, enthaltend ein bis vier Stickstoffatome:** 6-Ring Heteroarylgruppen, welche neben Kohlenstoffatomen ein bis drei bzw. ein bis vier Stickstoffatome als Ringglieder enthalten können, beispielsweise (aber nicht beschränkt auf) 2-Pyridinyl, 3-Pyridinyl, 4-Pyridinyl, 3-Pyridazinyl, 4-Pyridazinyl, 2-Pyrimidinyl, 4-Pyrimidinyl, 5-Pyrimidinyl, 2-Pyrazinyl, 1,3,5-Triazin-2-yl, 1,2,4-Triazin-3-yl und 1,2,4,5-Tetrazin-3-yl;
**Benzokondensiertes 5-gliedriges Heteroaryl, enthaltend ein bis drei Stickstoffatome oder ein Stickstoffatom und ein Sauerstoff- oder Schwefelatom:** z.B. (aber nicht beschränkt auf) 1H-Indol-1-yl, 1H-Indol-2-yl, 1H-Indol-3-yl, 1H-Indol-4-yl, 1H-Indol-5-yl, 1H-Indol-6-yl, 1H-Indol-7-yl, 1H-Benzimidazol-1-yl, 1H-Benzimidazol-2-yl, 1H-Benzimidazol-4-yl, Benzimidazol-5-yl, 1H-Indazol-l-yl, 1H-Indazol-3-yl, 1H-Indazol-4-yl, 1H-Indazol-5-yl, 1H-Indazol-6-yl, 1H-Indazol-7-yl, 2H-Indazol-2-yl, 1-benzofuran-2-yl, 1-benzofuran-3-yl, 1-benzofuran-4-yl, 1-benzofuran-5-yl, 1-benzofuran-6-yl, 1-benzofuran-7-yl, 1-benzothiophen-2-yl, 1-benzothiophen-3-yl, 1-benzothiophen-4-yl, 1-benzothiophen-5-yl, 1-benzothiophen-6-yl, 1-benzothiophen-7-yl, 1,3-Benzothiazol-2-yl und 1,3-Benzoxazol-2-yl,
**Benzokondensiertes 6-gliedriges Heteroaryl, enthaltend ein bis drei Stickstoffatome: :** z.B. (aber nicht beschränkt auf) Quinolin-2-yl, Quinolin-3-yl, Quinolin-4-yl, Quinolin-5-yl, Quinolin-6-yl, Quinolin-7-yl, Quinolin-8-yl, Isoquinolin-1-yl, Isoquinolin-3-yl, Isoquinolin-4-yl, Isoquinolin-5-yl, Isoquinolin-6-yl, Isoquinolin-7-yl, und Isoquinolin-8-yl;
**Heterocyclyl:** drei- bis fünfzehngliedriger gesättigter oder partiell ungesättigter Heterocyclus, enthaltend ein bis vier Heteroatome aus der Gruppe Sauerstoff, Stickstoff oder Schwefel: mono-, bi- oder tricyclische Heterocyclen enthaltend neben Kohlenstoffringgliedern ein bis drei Stickstoffatome und/oder ein Sauerstoff- oder Schwefelatom oder ein oder zwei Sauerstoff- und/oder Schwefelatome; enthält der Ring mehrere Sauerstoffatome, so stehen diese nicht direkt benachbart; wie z.B. (aber nicht beschränkt auf) Oxiranyl, Aziridinyl, 2-Tetrahydrofuranyl, 3-Tetrahydrofuranyl, 2-Tetrahydrothienyl, 3-Tetrahydrothienyl, 2-Pyrrolidinyl, 3-Pyrrolidinyl, 3-Isoxazolidinyl, 4-Isoxazolidinyl, 5-Isoxazolidinyl, 3-Isothiazolidinyl, 4-Isothiazolidinyl, 5-Isothiazolidinyl, 3-Pyrazolidinyl, 4-Pyrazolidinyl, 5-Pyrazolidinyl, 2-Oxazolidinyl, 4-Oxazolidinyl, 5-Oxazolidinyl, 2-Thiazolidinyl, 4-thiazolidinyl, 5-Thiazolidinyl, 2-Imidazolidinyl, 4-Imidazolidinyl, 1,2,4-Oxadiazolidin-3-yl, 1,2,4-Oxadiazolidin-5-yl, 1,2,4-Thiadiazolidin-3-yl, 1,2,4-Thiadiazolidin-5-yl, 1,2,4-Triazolidin-3-yl, 1,3,4-Oxadiazolidin-2-yl, 1,3,4-Thiadiazolidin-2-yl, 1,3,4-Triazolidin-2-yl, 2,3-Dihydrofur-2-yl, 2,3-Dihydrofur-3-yl, 2,4-Dihydrofur-2-yl, 2,4-Dihydrofur-3-yl, 2,3-Dihydrothien-2-yl, 2,3-Dihydrothien-3-yl, 2,4-Dihydrothien-2-yl, 2,4-Dihydrothien-3-yl, 2-Pyrrolin-2-yl, 2-Pyrrolin-3-yl, 3-Pyrrolin-2-yl, 3-Pyrrolin-3-yl, 2-Isoxazolin-3-yl, 3-Isoxazolin-3-yl, 4-Isoxazolin-3-yl, 2-Isoxazolin-4-yl, 3-Isoxazolin-4-yl, 4-Isoxazolin-4-yl, 2-Isoxazolin-5-yl, 3-Isoxazolin-5-yl, 4-Isoxazolin-5-yl, 2-Isothiazolin-3-yl, 3-Isothiazolin-3-yl, 4-Isothiazolin-3-yl, 2-Isothiazolin-4-yl, 3-Isothiazolin-4-yl, 4-Isothiazolin-4-yl, 2-Isothiazolin-5-yl, 3-Isothiazolin-5-yl, 4-Isothiazolin-5-yl, 2,3-Dihydropyrazol-l-yl, 2,3-Dihydropyrazol-2-yl, 2,3-Dihydropyrazol-3-yl, 2,3-Dihydropyrazol-4-yl, 2,3-Dihydropyrazol-5-yl, 3,4-Dihydropyrazol-1-yl, 3,4-Dihydropyrazol-3-yl, 3,4-Dihydropyrazol-4-yl, 3,4-Dihydropyrazol-5-yl, 4,5-Dihydroopyrazol-1-yl, 4,5-Dihydropyrazol-3-yl, 4,5-Dihydropyrazol-4-yl, 4,5-Dihydropyrazol-5-yl, 2,3-Dihydrooxazol-2-yl, 2,3-Dihydrooxazol-3-yl, 2,3-Dihydrooxazol-4-yl, 2,3-Dihydrooxazol-5-yl, 3,4-Dihydrooxazol-2-yl, 3,4-Dihydrooxazol-3-yl, 3,4-Dihydrooxazol-4-yl, 3,4-Dihydrooxazol-5-yl, 3,4-Dihydrooxazol-2-yl, 3,4-Dihydrooxazol-3-yl, 3,4-Dihydrooxazol-4-yl, 2-Piperidinyl, 3-Piperidinyl, 4-Piperidinyl, 1,3-Dioxan-5-yl, 2-Tetrahydropyranyl, 4-Tetrahydropyranyl, 2-Tetrahydrothienyl, 3-Hexahydropyridazinyl, 4-Hexahydropyridazinyl, 2-Hexahydropyrimidinyl, 4-Hexahydropyrimidinyl, 5-Hexahydropyrimidinyl, 2-Piperazinyl, 1,3,5-Hexahydro-triazin-2-yl und 1,2,4-Hexahydrotriazin-3-yl;
**Abgangsgruppe:** S_{N}1 oder S_{N}2-Abgangsgruppe, beispielsweise Halogen (Chlor, Brom, Jod), Alkylsulfonate (-OSO₂-Alkyl, z.B. -OSO₂CH₃, -OSO₂CF₃) oder Arylsulfonate (-OSO₂-Aryl, z.B. -OSO₂Ph, -OSO₂PhMe);

Nicht umfasst sind solche Kombinationen, die den Naturgesetzen widersprechen und die der Fachmann daher aufgrund seines Fachwissens ausgeschlossen hätte. Beispielsweise sind Ringstrukturen mit drei oder mehreren benachbarten O-Atomen ausgeschlossen.

Ein weiterer Gegenstand der vorliegenden Erfindung betrifft ein Verfahren zum Herstellen der erfindungsgemäßen heterocyclyl-substituierten Thiazole der Formeln (**I**), umfassend wenigstens einen der folgenden Schritte (a) bis (u):
(a) Umsetzung von Verbindungen der Formel (**VII**) zu Verbindungen der Formel (**VIII**) in Gegenwart einer metallorganischen Verbindung R⁹-M und gegebenenfalls in Gegenwart eines Lösungsmittels gemäß dem nachfolgenden Reaktionsschema (Schema 1): 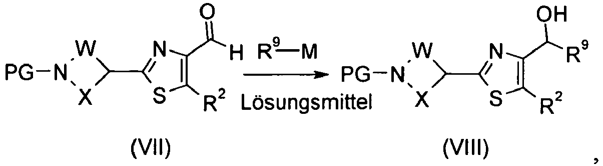 wobei
   PG = unsubstituiertes oder substituiertes Phenylmethyl (wobei die Substituenten unabhängig voneinenader ausgewählt sind aus Methyl, Methoxy, Nitro, Dioxolano), Acetyl, C₁-C₄-Alkoxycarbonyl, oder Benzyloxycarbonyl,
   M = z.B. MgCl, MgBr, MgI, Li,
   R⁹ = C₁-C₄-Alkyl oder C₃-C₆-Cycloalkyl,
   W, X, und R² wie für Formel (**I**) oben definiert.
(b) Umsetzung von Alkoholen der Formel (**VIII**) mit einem Oxidationsmittel in Gegenwart eines Lösungsmittels zu Ketonen der Formel (**IX**) gemäß dem nachfolgenden Reaktionsschema (Schema 2): 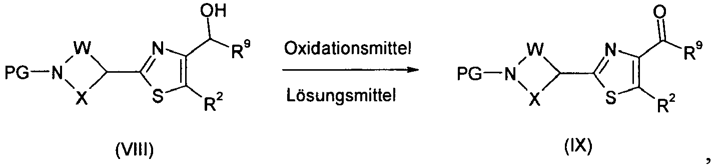 wobei
   PG = unsubstituiertes oder substituiertes Phenylmethyl (wobei die Substituenten unabhängig voneinenader ausgewählt sind aus Methyl, Methoxy, Nitro, Dioxolano), Acetyl, oder C₁-C₄-Alkoxycarbonyl,
   R⁹ = C₁-C₄-Alkyl oder C₃-C₆-Cycloalkyl,
   W, X, und R² wie für Formel (**I**) oben definiert.
(c) Umsetzung von Ketonen der Formel (**IX**) zu Verbindungen der Formel (**IVb**) in Gegenwart einer Base oder einer Säure und gegebenenfalls eines Lösungsmittels gemäß dem nachfolgenden Reaktionsschema (Schema 3): 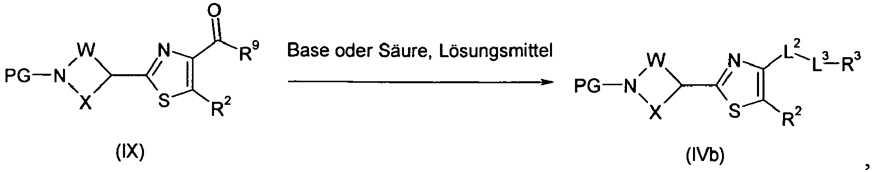 wobei
   PG = unsubstituiertes oder substituiertes Phenylmethyl (wobei die Substituenten unabhängig voneinenader ausgewählt sind aus Methyl, Methoxy, Nitro, Dioxolano), Acetyl, oder C₁-C₄-Alkoxycarbonyl,
   L² = -CR⁹-CR⁹-,
   W, X, L³, R², R³ und R⁹ wie für Formel (**I**) oben definiert.
(d) Umsetzung von Aldehyden der Formel (**VII**) zu Verbindungen der Formel (**IVb**) in Gegenwart einer Base oder einer Säure und gegebenenfalls eines Lösungsmittels gemäß dem nachfolgenden Reaktionsschema (Schema 4): 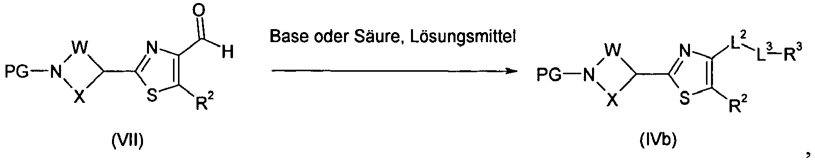 wobei
   PG = unsubstituiertes oder substituiertes Phenylmethyl (wobei die Substituenten unabhängig voneinenader ausgewählt sind aus Methyl, Methoxy, Nitro, Dioxolano), Acetyl, oder C₁-C₄-Alkoxycarbonyl,
   L² = -CR⁹=CR⁹-,
   W, X, L³, R², R³ und R⁹ wie für Formel (**I**) oben definiert.
(e) Umsetzung von Aldehyden der Formel (**VII**) zu Alkinen der Formel (**X**) in Gegenwart einer Base und gegebenenfalls eines Lösungsmittels gemäß dem nachfolgenden Reaktionsschema (Schema 5): 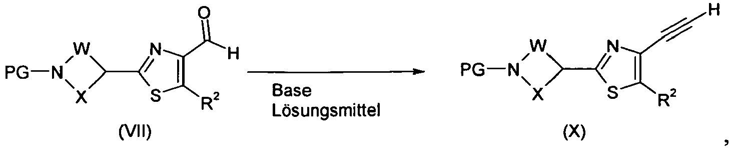 wobei
   PG = unsubstituiertes oder substituiertes Phenylmethyl (wobei die Substituenten unabhängig voneinander ausgewählt sind aus Methyl, Methoxy, Nitro, Dioxolano), Acetyl, oder C₁-C₄-Alkoxycarbonyl,
   W, X and R² wie für Formel (**I**) oben definiert.
(f) Umsetzung von Alkinen der Formel (**X**) mit einem R³-L³-Halogenid in einer ggf. katalysierten C-C-Kupplungsreaktion in Gegenwart einer Base und gegebenenfalls eines Lösungsmittels zu Verbindungen der Formel (**IVc**) gemäß dem nachfolgenden Reaktionsschema (Schema 6): 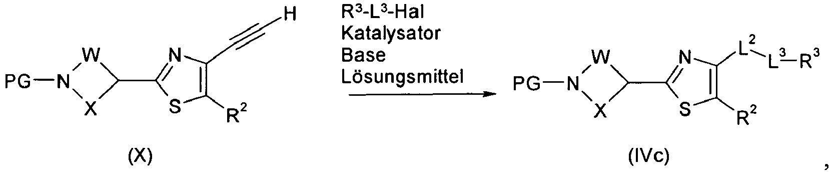 wobei
   PG = unsubstituiertes oder substituiertes Phenylmethyl (wobei die Substituenten unabhängig voneinenader ausgewählt sind aus Methyl, Methoxy, Nitro, Dioxolano), Acetyl, oder C₁-C₄-Alkoxycarbonyl,
   Hal = Cl, Br oder I,
   L² = -C≡C-,
   W, X, L³, R² und R³ wie für Formel (**I**) oben definiert.
(g) Umsetzung von Alkinen der Formel (**X**) mit ein Trialkylsilylchlorid (R¹⁰R¹¹R¹²)SiCl oder - triflat (R¹⁰R¹¹R¹²)SiOSO₂CF₃, oder anderen bekannten Silylierungsreagenzien in Gegenwart einer Base und gegebenenfalls eines Lösungsmittels zu Verbindungen der Formel (**XI**) gemäß dem nachfolgenden Reaktionsschema (Schema 7): 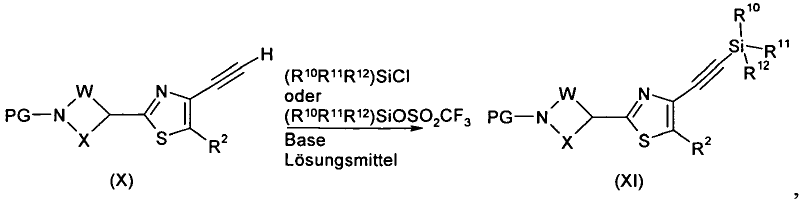 wobei
   PG = unsubstituiertes oder substituiertes Phenylmethyl (wobei die Substituenten unabhängig voneinenader ausgewählt sind aus Methyl, Methoxy, Nitro, Dioxolano), Acetyl, oder C₁-C₄-Alkoxycarbonyl,
   R¹⁰, R¹¹ und R¹² = C₁-C₄ alkyl oder Phenyl,
   W, X und R² wie für Formel (**I**) oben definiert.
(h) Umsetzung von Alkinen der Formel (**XI**) mit einer Verbindung R³-L³-Hal ggf. in Gegenwart eines Katalysators in Gegenwart einer Base und gegebenenfalls eines Lösungsmittels zu Verbindungen der Formel (**IVc**) gemäß dem nachfolgenden Reaktionsschema (Schema 8): 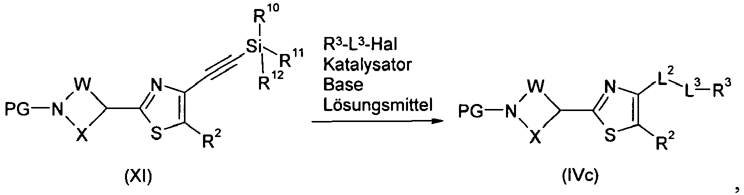 wobei
   PG = unsubstituiertes oder substituiertes Phenylmethyl (wobei die Substituenten unabhängig voneinenader ausgewählt sind aus Methyl, Methoxy, Nitro, Dioxolano), Acetyl, oder C₁-C₄-Alkoxycarbonyl,
   Hal = Cl, Br oder I,
   R² = Wasserstoff, C₁-C₂-Alkyl oder C₁-C₂-Halogenalkyl,
   L² = -C≡C-,
   R¹⁰, R¹¹ und R¹² = C₁-C₄-Alkyl oder Phenyl,
   W, X, L³ und R³ wie für Formel (**I**) oben definiert.
(i) Umsetzung von Thioamiden der Formel (**V**) mit Verbindungen der Formel (**VIa**) oder (**VIb**) zu Verbindungen der Formeln (**IVa**) oder (**IVb**), in Gegenwart einer Base und gegebenenfalls in Gegenwart eines Lösungsmittels gemäß dem nachfolgenden Reaktionsschema (Schema 9): 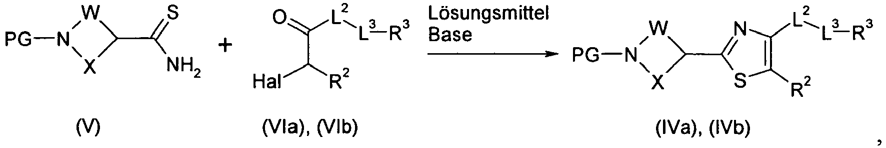 wobei
   PG = unsubstituiertes oder substituiertes Phenylmethyl (wobei die Substituenten unabhängig voneinenader ausgewählt sind aus Methyl, Methoxy, Nitro, Dioxolano), Acetyl, C₁-C₄-Alkoxycarbonyl oder Benzyloxycarbonyl,
   Hal = Cl, Br oder I,
   L² = -C(R⁸)₂-C(R⁸)₂- für Verbindungen mit der Formel (**IVa**) und (**VIa**),
   L² =-CR⁹=CR⁹- für Verbindungen mit der Formel (**IVb**) und (**VIb**),
   W, X, L³, R², R³, R⁸ und R⁹ wie für Formel (**I**) oben definiert.
(j) Umsetzung von Verbindungen der Formeln (**IVb**) oder (**IVc**) zu Verbindungen der Formel (**IVa**) in Gegenwart von Wasserstoff, eines Katalysators und gegebenenfalls eines Lösungsmittels gemäß dem nachfolgenden Reaktionsschema, (Schema 10): 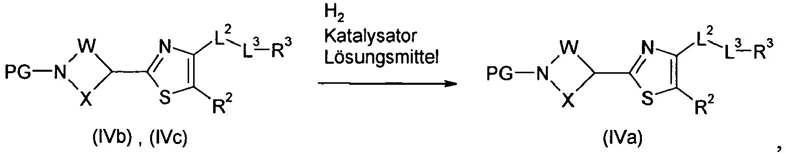 wobei
   PG = Acetyl oder C₁-C₄-Alkoxycarbonyl,
   L² = -C(R⁸)₂-C(R⁸)₂- für Verbindungen mit der Formel (**IVa**),
   L² = -CR⁹=CR⁹- für Verbindungen mit der Formel (**IVb**),
   L² = -C=C- für Verbindungen mit der Formel (**IVc**),
   W, X, L³, R², R³, R⁸ und R⁹ wie für Formel (**I**) oben definiert.
(k) Umsetzung von Verbindungen der Formel (**IV**) zu Verbindungen der Formel (**II**) gegebenenfalls in Gegenwart eines Lösungsmittels sowie ggf. in Gegenwart einer Säure oder ggf. in Gegenwart einer Base oder ggf. in Gegenwart einer Wasserstoffquelle gemäß dem nachfolgenden Reaktionsschema (Schema 11): 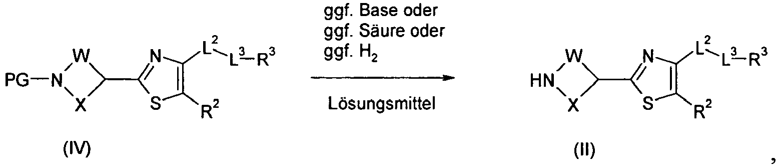 wobei
   PG = Acetyl, C₁-C₄-Alkoxycarbonyl oder Benzyloxycarbonyl,
   W, X, L², L³, R², und R³ wie für Formel (I) oben definiert.
(l) Umsetzung von Verbindungen der Formel (**IIa'**) zu Verbindungen der Formel (**IIa**) in Gegenwart eines Halogenierungsmittels, gegebenenfalls in Gegenwart einer Säure und eines Lösungsmittels gemäß dem nachfolgenden Reaktionsschema (Schema 12): 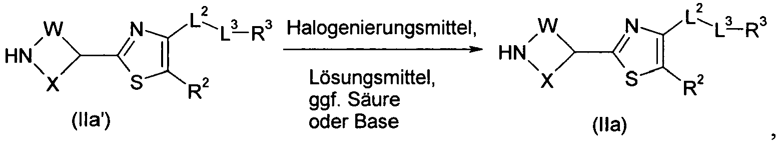 wobei
   L² = -C(R⁸)₂-C(R⁸)₂-,
   R² = I, Br oder Cl für Verbindungen mit der Formel (**IIa**),
   R² = Wasserstoff für Verbindungen mit der Formel (**IIa'**),
   W, X, L³, R³ und R⁸ wie für Formel (**I**) oben definiert.
(m) Umsetzung von Verbindungen der Formel (**III**) mit Verbindungen der Formel (**II**) zu Verbindungen der Formel (**I**) gegebenenfalls in Gegenwart eines Kupplungsreagenzes, einer Base und eines Lösungsmittels gemäß dem nachfolgenden Reaktionsschema (Schema 13): 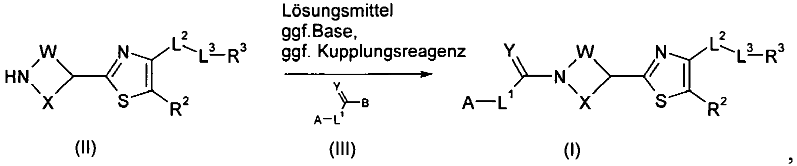 wobei
   B = OH, Chlor, Brom oder Jod,
   Y = Sauerstoff,
   A, W, X, L¹, L², L³, R², und R³ wie für Formel (**I**) oben definiert.
(n) Umsetzung von Verbindungen der Formel (**I**) zu Verbindungen der Formel (**I**) in Gegenwart eines Schwefelungsreagenzes und gegebenenfalls in Gegenwart eines Lösungsmittels gemäß dem nachfolgenden Reaktionsschema (Schema 14): 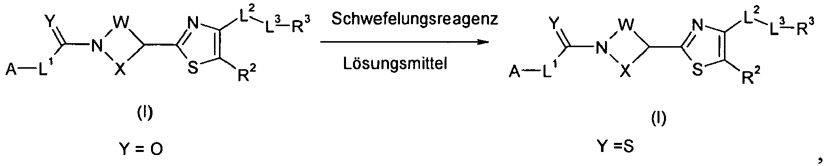 wobei
   A, W, X, L¹, L², L³, R², und R³ wie für Formel (**I**) oben definiert.
(o) Umsetzung von Aldehyden der Formel (**XII**) zu Alkinen der Formel (**XIII**) in Gegenwart einer Base und gegebenenfalls eines Lösungsmittels gemäß dem nachfolgenden Reaktionsschema (Schema 17): 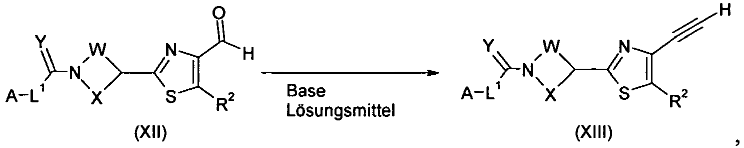 wobei
   A, W, X, Y, L¹ und R² wie für Formel (**I**) oben definiert.
(p) Umsetzung von Alkinen der Formel (**XIII**) mit ein Trialkylsilylchlorid (R¹⁰R¹¹R¹²)SiCl oder -triflat (R¹⁰R¹¹R¹²)SiOSO₂CF₃, oder anderen bekannten Silylierungsreagenzien in Gegenwart einer Base und gegebenenfalls eines Lösungsmittels zu Verbindungen der Formel (**XIV**) gemäß dem nachfolgenden Reaktionsschema (Schema 18): 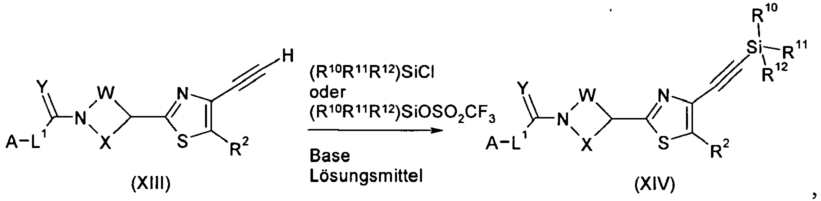 wobei
   R¹⁰, R¹¹ und R¹² = C₁-C₄ alkyl oder Phenyl,
   A, W, X, Y, L¹ und R² wie für Formel (**I**) oben definiert.
(q) Umsetzung von Alkinen der Formel (**XIII**) mit einem R³-L³-Halogenid in einer ggf. katalysierten C-C-Kupplungsreaktion in Gegenwart einer Base und gegebenenfalls eines Lösungsmittels zu Verbindungen der Formel (**Ic**) gemäß dem nachfolgenden Reaktionsschema (Schema 19): 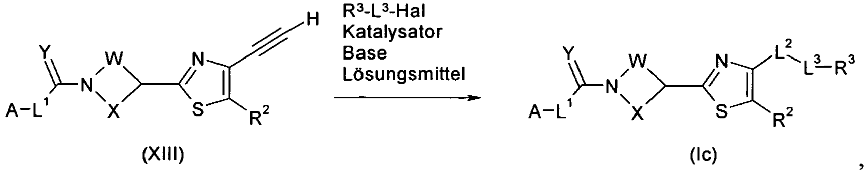 wobei
   Hal = Cl, Br oder I,
   L²= -C≡C-,
   A, W, X, Y, L¹ L³, R² und R³ wie für Formel (**I**) oben definiert.
(r) Umsetzung von Alkinen der Formel (**XIV**) mit einer Verbindung R³-L³-Hal ggf. in Gegenwart eines Katalysators in Gegenwart einer Base und gegebenenfalls eines Lösungsmittels zu Verbindungen der Formel (**Ic**) gemäß dem nachfolgenden Reaktionsschema (Schema 20): 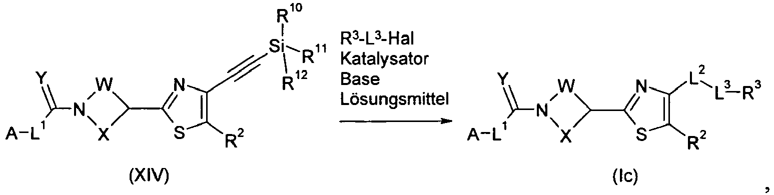 wobei
   Hal = Cl, Br oder I,
   L²=-C≡C-,
   R¹⁰, R¹¹ und R¹² = C₁-C₄-Alkyl oder Phenyl,
   A, W, X, Y, L¹ L³, R² und R³ wie für Formel (**I**) oben definiert.
(s) Umsetzung von Aldehyden der Formel (**XII**) zu Verbindungen der Formel (**Ib**) in Gegenwart einer Base oder einer Säure und gegebenenfalls eines Lösungsmittels gemäß dem nachfolgenden Reaktionsschema (Schema 21): 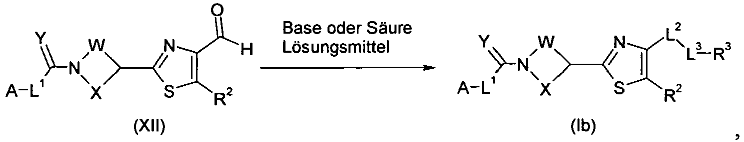 wobei
   L² = -CR⁹=CR⁹-,
   A, W, X, Y, L¹ L³, R², R³ und R⁹ wie für Formel (**I**) oben definiert.
(t) Umsetzung von Verbindungen der Formeln (**Ic**) zu Verbindungen der Formel (**Ib**) in Gegenwart von Wasserstoff, eines Katalysators und gegebenenfalls eines Lösungsmittels gemäß dem nachfolgenden Reaktionsschema, (Schema 22): 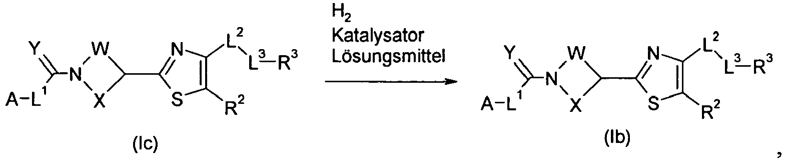 wobei
   L² = -CR⁹=CR⁹- für Verbindungen mit der Formel (**Ib**),
   L² = -C≡C- für Verbindungen mit der Formel (**Ic**),
   A, W, X, Y, L¹ L³, R², R³ und R⁹ wie für Formel (**I**) oben definiert.
(u) Umsetzung von Verbindungen der Formeln (**Ib**) oder (**Ic**) zu Verbindungen der Formel (**Ia**) in Gegenwart von Wasserstoff, eines Katalysators und gegebenenfalls eines Lösungsmittels gemäß dem nachfolgenden Reaktionsschema, (Schema 23): 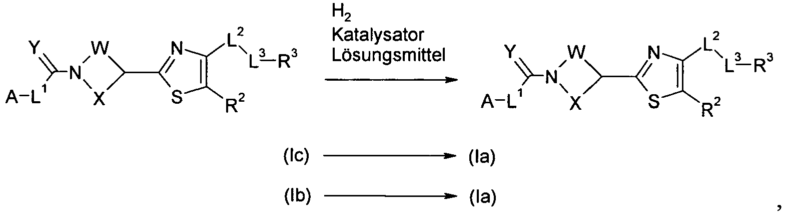 wobei
   L² = -C(R⁸)₂-C(R⁸)₂- für Verbindungen mit der Formel (**Ia**),
   L² = -CR⁹=CR⁹- für Verbindungen mit der Formel (**Ib**),
   L² = -C=C- für Verbindungen mit der Formel (**Ic**),
   A, W, X, Y, L¹ L³, R², R³, R⁸ und R⁹ wie für Formel (**I**) oben definiert.

Eine generelle Synthesewegeübersicht ist in Schema 15 und 24 dargestellt.

Eine Verbindung mit der allgemeinen Formel (**V**) wird mit einer Verbindung der Formel (**VIa**) oder (**VIb**) umgesetzt, um eine cyklische Verbindung mit der Formel (**IVa**) oder (**IVb**) zu erhalten (Schema 9). Eine andere Möglichkeit Verbindungen der Formel (**IV**) herzustellen, ist mit Verbindungen der Formel (**VII**) zu beginnen. Diese können einer Reaktion mit einem Substrat der allgemeinen Formel R⁹-M unterzogen werden (Schema 1). Es entsteht ein sekundärer Alkohol der Formel (**VIII**), der anschließend oxidiert wird, wobei eine Verbindung der Formel (**IX**) entsteht (Schema 2). Eine Verbindung der Formel (**VII**) oder (**IX**) wird einer Olefinierung unterzogen, um eine Verbindung der Formel (**IVb**) darzustellen (Schema 3 und Schema 4). Eine andere mögliche Route, Verbindungen der Formel (**IV**) herzustellen, beginnt ebenfalls mit einer Verbindung der Formel (**VII**). Eine Verbindung der Formel (**VII**) wird alkinyliert, um ein terminales Alkin der allgemeinen Formel (**X**) darzustellen (Schema 5). Eine Übergangsmetall-katalysierte C-C-Kupplungsreaktion einer Verbindung mit der Formel (**X**) mit einem Halogenid der Formel R³-L³-Hal ergibt eine Verbindung der Formel (**IVc**) (Schema 6). Eine Verbindung mit der Formel (**X**) kann auch mit einer Silylgruppe versehen werden, was zu einer Verbindung mit der Formel (**XI**) führt (Schema 7). Diese wird dann einer Übergangsmetall-katalysierten Kupplungsreaktion unterzogen, und zwar mit einem Substrat der allgemeinen Formel R³-L³-Hal, um eine Verbindung der Formel (**IVc**) (Schema 8) darzustellen. Die Doppel- und Dreifachbindungen einer Verbindung mit der Formel (**IVb**) und (**IVc**) können mit Wasserstoff zu einer Verbindung mit der Formel (**IVa**) hydriert werden (Schema 10). Die Schutzgruppe einer Verbindung mit der Formel (**IV**), die mit PG gekennzeichnet ist, wird dann entfernt, so dass eine Verbindung mit der Formel (**II**) oder das entsprechende Salz entsteht (Schema 11). Eine Verbindung mit der Formel (**IIa'**) kann halogeniert werden, so dass eine Verbindung mit der Formel (**IIa**) entsteht (Schema 12). Eine Verbindung mit der Formel (**II**) oder ein entsprechendes Salz wird mit einem Substrat der Formel (**III**) gekuppelt, wodurch eine Verbindung mit der Formel (**I**) dargestellt werden kann (Schema 13). Eine Verbindung mit der Formel (**I**) wird mit einem Schwefelungsreagenz versetzt, um eine Verbindung mit der Formel (**I**) zu generieren (Schema 14).

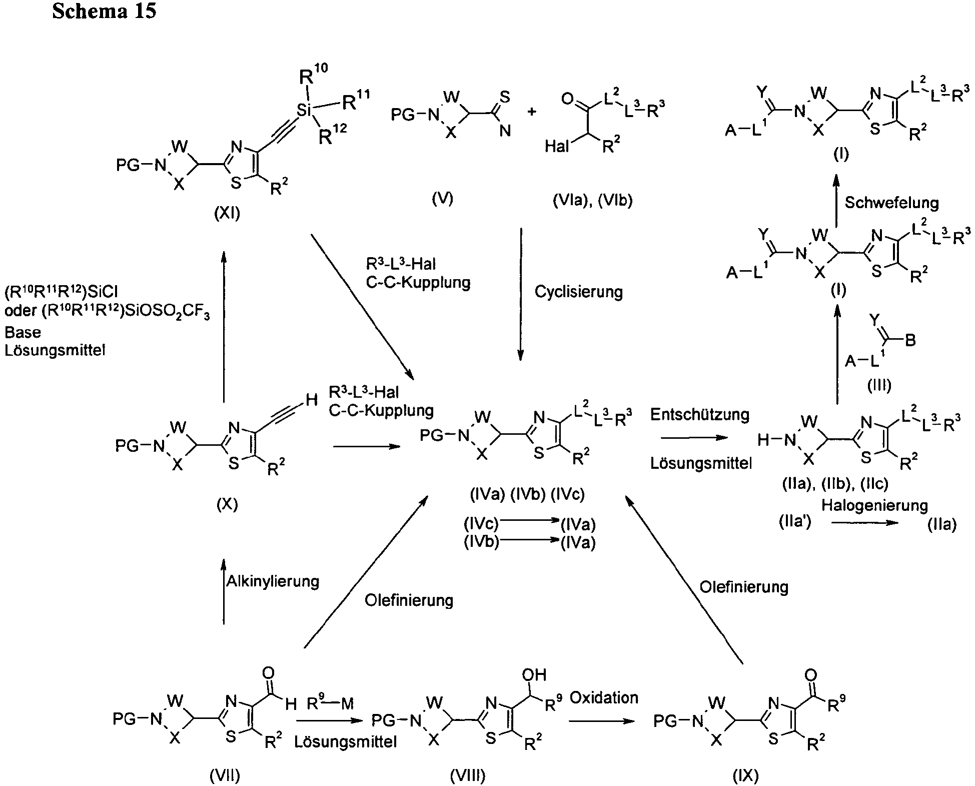

Eine andere mögliche Route, Verbindungen der Formel (**I**) herzustellen, beginnt mit einer Verbindung der Formel (**XII**) (Schema 24). Eine Verbindung der Formel (**XII**) wird alkinyliert, um ein terminales Alkin der allgemeinen Formel (**XIII**) darzustellen (Schema 17). Eine Übergangsmetall-katalysierte C-C-Kupplungsreaktion einer Verbindung mit der Formel (**XIII**) mit einem Halogenid der Formel R³-L³-Hal ergibt eine Verbindung der Formel (**Ic**) (Schema 19). Eine Verbindung mit der Formel (**XIII**) kann auch mit einer Silylgruppe versehen werden, was zu einer Verbindung mit der Formel (**XIV**) führt (Schema 18). Diese wird dann einer Übergangsmetall-katalysierten Kupplungsreaktion unterzogen, und zwar mit einem Substrat der allgemeinen Formel R³-L³-Hal, um eine Verbindung der Formel (**Ic**) (Schema 20) darzustellen. Eine Verbindung der Formel (**XII**) wird einer Olefinierung unterzogen, um eine Verbindung der Formel (**Ib**) darzustellen (Schema 21). Die Dreifachbindung einer Verbindung mit der Formel (**Ic**) kann mit Wasserstoff zu einer Verbindung mit der Formel (**Ib**) hydriert werden (Schema 22). Die Doppel- und Dreifachbindungen einer Verbindung mit der Formel (**Ib**) und (**Ic**) können mit Wasserstoff zu einer Verbindung mit der Formel (**Ia**) hydriert werden (Schema 23).

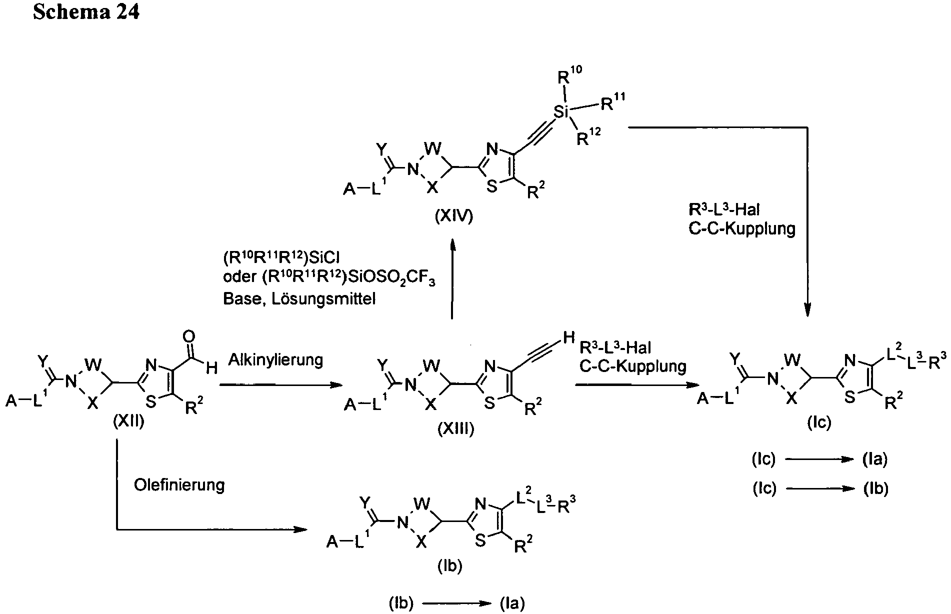

Eine Möglichkeit das Zwischenprodukt (**VIII**) aus Verbindung (**VII**) darzustellen ist in Schema 1 gezeigt.

Edukte der Formel (**VIII**) werden aus dem Aldehyd (**VII**) durch Addition eines metallorganischen Reagenzes R⁹-M (beispielsweise M = Mg, Li) hergestellt. Bevorzugt werden die Alkohole (**VIII**) hergestellt durch Addition eines Grignard Reagenzes R⁹-MgX (X = Cl, Br, oder I) zu Verbindungen (**VII**) in Tetrahydrofuran bei -78°C, und zwar unter Inertatmosphäre (siehe beispielsweise WO 07/039177).

Der Aldehyd (**VII**) ist kommerziell verfügbar (z.B. Maybridge) oder kann aus kommerziell erhältlichen Vorstufen nach literaturbeschriebenen Vorschriften hergestellt werden. Der Aldehyd (**VII**) wird beispielsweise aus dem entsprechenden Methyl- oder Ethylester (**XV**) durch Reduktion mit Lithiumaluminiumhydrid in Tetrahydrofuran bei 0°C und dann durch eine Oxidation des entsprechenden Alkohols mit Dess Martin Reagenz bei Raumtemperatur in Dichloromethan dargestellt (siehe z.B. WO 07/147336 und WO 07/039177 für die Reduktion mit Lithiumaluminiumhydrid und J. Am. Chem. Soc. 1978, 100, 300-301; 1979, 101, 5294-5299; 1991, 113, 7277-7287 für die Oxidation mit Dess Martin Reagenz).

### Abbildung 1

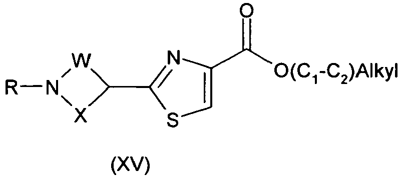

mit
R = H oder säurelabilen Aminschutzgruppen, wie z.B t-Butoxycarbonyl (tBoc) oder Benzyloxycarbonyl (Cbz) oder einer Benzylschutzgruppe, wie z.B Benzyl (Bn).
W und X wie wie für Formel (I) oben definiert.

Methyl-oder Ethylester (**XV**) sind bekannt und können aus kommerziell erhältlichen Vorstufen nach literaturbeschriebenen Vorschriften hergestellt werden, zum Beispiel aus Nitrilen der Formel (**XVI**), Carbonsäuren der Formel (**XVII**), Carbonsäurechloriden der Formel (**XVIII**), Amiden der Formel (**XIX**) oder Thioamiden der Formel (**V**) (Abbildung 2). Eine bevorzugte Methode ist die Hantzsche Thiazolsynthese. Ausgehend von (**V**) und kommerziell erhältlichem Ethyl- oder Methylhalpyruvate in Ethanol oder in *N,N*-Dimethylformamid in der Gegenwart von z.B. Triethylamin bei Raumtemperatur (Beispiele s. WO 07/014290 und darin zitierte Referenzen).

### Abbildung 2

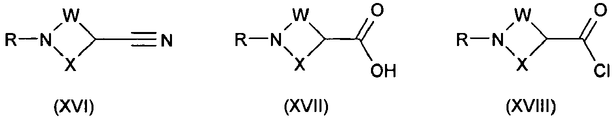

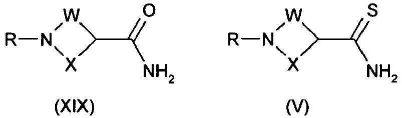

mit
R = H oder säurelabilen Aminschutzgruppen, wie z.B t-Butoxycarbonyl (tBoc) oder Benzyloxycarbonyl (Cbz) oder einer Benzylschutzgruppe, wie z.B Benzyl (Bn).
W und X wie für Formel (I) oben definiert.

Eine Möglichkeit Intermediat (**IX**) aus Verbindung (**VIII**) herzustellen wird in Schema 2 gezeigt.

Verbindungen der Formel (**IX**) werden durch Oxidation des Alkohols (**VIII**) hergestellt. In der Literatur finden sich zahlreiche Methoden für die Darstellung von Ketonen aus sekundären Alkoholen (siehe "Oxidation of Alcohols to Aldehydes and Ketones", Gabriel Tojo, Marcos Fernández, Springer Berlin, 2006, 1-97, und darin zitierte Literatur). Bevorzugt wird die Oxidation unter Swern Bedingungen oder mit Dess-Martin Periodinan durchgeführt (siehe z.B. J. Am. Chem. Soc. 1978, 100, 300-301). Es kommen alle Lösungsmittel infrage, die selbst nicht vom Oxidationsmittel oxidiert werden, wie beispielsweise Dichlormethan, Chloroform oder Acetonitril, gegebenenfalls in Gegenwart eines Reaktionshilfsmittels, beispielsweise einer Säure (z.B. Schwefelsaüre oder Salzsäure) oder auch Base (z.B Triethylamin oder Pyridin).

Eine Möglichkeit Intermediat (**IX**) aus Verbindung (**VIII**) zu erhalten ist z.B. mit Dess-Martin Periodinan (15% in Dichlormethan, kommerziell verfügbar beispielsweise von der Firma Acros).

Als Lösungsmittel können alle üblichen, unter den Reaktionsbedingungen inerten Lösungsmittel, wie z.B. zyklische und azyklische Ether (z.B. Diethylether, Tetrahydrofuran, Dioxane), aromatische Kohlenwasserstoffe (z.B. Benzol, Toluol, Xylol), halogenierte Kohlenwasserstoffe (z.B. Dichlormethan, Chloroform, Tetrachlorkohlenstoff), halogenierte aromatische Kohlenwasserstoffe (z.B. Chlorbenzol, Dichlorbenzol), Nitrile (z.B. Acetonitril), Carbonsäureester (z.B. Essigsäureethylester), Amide (z.B. N,N-Dimethylformamid, N,N-Dimethylacetamid), Dimethylsulfoxid, 1,3-Dimethyl-2-imidazolinon und Wasser verwendet werden und die Reaktion kann in Mischungen von zwei oder mehreren dieser Lösungsmittel ausgeführt werden.

Die Startmaterialien werden in equimolaren Mengen eingesetzt, aber das Oxidationsmittel kann, falls notwendig, auch im Überschuss verwendet werden. Die Reaktion wird normalerweise bei Temperaturen von 0 °C - 100 °C durchgeführt und vorzugsweise bei Raumtemperatur, aber sie kann auch bei Rückflusstemperatur der Reaktionsmischung durchgeführt werden. Die Reaktionszeit variiert in Abhängigkeit vom Maßstab der Reaktion und der Reaktionstemperatur, aber liegt im Allgemeinen zwischen einigen Minuten und 48 Stunden.

Nach Beendigung der Reaktion, wird Verbindung (**IX**) von der Reaktionsmischung durch eine der üblichen Trenntechniken getrennt. Falls notwendig werden die Verbindungen durch Umkristallisation, Destillation oder Chromatographie gereinigt oder können gegebenenfalls auch im nächsten Schritt ohne vorhergehende Reinigung eingesetzt werden.

Eine Möglichkeit das Zwischenprodukt (**IVb**) aus Verbindung (**IX**) oder (**VII**) darzustellen ist in den Schemata 3 und 4 gezeigt.

Ketone der Formel (**IX**) oder Aldehyde der Formel (**VII**) werden in *(Z)*- oder *(E)*-(**IVb**), z.B. durch eine Wittig oder Horner-Wadsworth-Emmons Olefinierung überführt (siehe z.B. Chem. Rev. 1989, 89, 863-927 und die dort zitierten Referenzen) oder z.B. Julia Olefinierung (siehe z.B. Tetrahedron Lett., 1973, 14, 4833-4836) oder z.B. durch eine Peterson-Olefinierung (siehe z.B. J. Org. Chem. 1968, 33, 780). Bevorzugt wird die Olefinierung unter Wittig oder Horner-Wadsworth-Emmons Bedingungen durchgeführt.

Die Reagenzien wie Phosphoniumsalze und Phosphonat-Reagenzien sind entweder kommerziell erhältlich oder nach in der Literatur beschriebener Weise zugänglich (siehe z.B. *"*The Chemistry of Organophosphorus Compounds"; John Wiley & Sons, 1994, Vol 3, 45-184, und darin zitierte Literatur).

Als Lösungsmittel können alle üblichen, unter den Reaktionsbedingungen inerten Lösungsmittel, wie z.B zyklisch und azyklische Ether (z.B. Diethylether, Tetrahydrofuran, Dioxane), aromatische Kohlenwasserstoffe (z.B. Benzol, Toluol, Xylol), halogenierte Kohlenwasserstoffe (z.B. Dichlormethan, Chloroform, Tetrachlorkohlenstoff), halogenierte aromatische Kohlenwasserstoffe (z.B. Chlorbenzol, Dichlorbenzol) verwendet werden und die Reaktion kann in Mischungen von zwei oder mehreren dieser Lösungsmittel ausgeführt werden. Die bevorzugten Lösungsmittel sind Tetrahydrofuran und Toluol.

Als Basen können solche eingesetzt werden, die üblicherweise in den zuvor genannten Olefinierungsreaktionen verwendet werden, z.B. NaH, tBuOK, nBuLi, (siehe z.B. Rev. 1989, 89, 863-927 und die dort zitierten Referenzen). Als Säuren kommen solche in Betracht, die üblicherweise in Olefinierungsreaktionen eingesetzt werden können, wie z.B. Sulfonsäuren oder Schwefelsäure.

Die Startmaterialien werden in equimolaren Mengen eingesetzt, aber die Phosphoniumsalze oder Phosphonate können falls notwendig im Überschuss verwendet werden. Die Reaktion wird normalerweise bei Temperaturen von 0 °C - 100 °C durchgeführt und vorzugsweise bei 0 °C - 25 °C, aber sie kann auch bei Rückflusstemperatur der Reaktionsmischung durchgeführt werden. Die Reaktionszeit variiert in Abhängigkeit vom Maßstab der Reaktion und der Reaktionstemperatur, aber liegt im Allgemeinen zwischen einigen Minuten und 48 Stunden.

Nach Beendigung der Reaktion, werden die Verbindungen (**IVb**) von der Reaktionsmischung durch eine der üblichen Trenntechniken getrennt. Falls notwendig werden die Verbindungen durch Umkristallisation, Destillation oder Chromatographie gereinigt oder können gegebenenfalls auch im nächsten Schritt ohne vorhergehende Reinigung eingesetzt werden.

Eine Möglichkeit, das Zwischenprodukt (**X**) aus Verbindung (**VII**) herzustellen, ist in Schema 5 gezeigt.

In der Literatur ist bekannt, dass Alkinylierungen von Aldehyden durch eine Corey-Fuchs-Reaktion (Tetrahedron Lett. 1972, 36, 3769-3772) oder eine Seyferth-Gilbert-Homologisierung (siehe z.B. J. Org. Chem., 1996, 61, 2540-2541) erzielt werden kann. Alternativ kann das Alkin (**X**) auch aus dem Aldehyd (**VII**) mit Bestmann-Ohira's Reagenz analog der Literaturvorschriften hergestellt werden (siehe z.B. Synthesis 2004, 1, 59-62).

Als Lösungsmittel können alle üblichen, unter den Reaktionsbedingungen inerten Lösungsmittel, wie z.B. zyklisch und azyklische Ether (z.B. Diethylether, Tetrahydrofuran, Dioxane), aromatische Kohlenwasserstoffe (z.B. Benzol, Toluol, Xylol), halogenierte Kohlenwasserstoffe (z.B. Dichlormethan, Chloroform, Tetrachlorkohlenstoff) und halogenierte aromatische Kohlenwasserstoffe (z.B. Chlorbenzol, Dichlorbenzol verwendet werden und die Reaktion kann in Mischungen von zwei oder mehreren dieser Lösungsmittel ausgeführt werden. Die bevorzugten Lösungsmittel sind Tetrahydrofuran und Toluol.

In der Reaktion können alle bekannten Basen eingesetzt werden, aber bevorzugt werden Alklimetall- und Erdalkalimetalloxide, Hydroxide and Carbonate verwendet. Wenigstens ein Äquivalent der Base muss zu dem Bestmann-Ohira's Reagenz zugegeben werden und falls nötig kann sie im Überschuss verwendet werden.

Die Startmaterialien werden in equimolaren Mengen eingesetzt, aber das Bestmann-Ohira's Reagenz kann falls notwendig im Überschuss verwendet werden. Die Reaktion wird normalerweise bei Temperaturen von 0 °C - 60 °C durchgeführt und vorzugsweise bei 0 °C - 40 °C, aber sie kann auch bei Rückflusstemperatur der Reaktionsmischung durchgeführt werden. Die Reaktionszeit variiert in Abhängigkeit vom Maßstab der Reaktion und der Reaktionstemperatur, aber liegt im Allgemeinen zwischen einigen Minuten und 48 Stunden.

Nach Beendigung der Reaktion, werden die Verbindungen **(VIc)** von der Reaktionsmischung durch eine der üblichen Trenntechniken getrennt. Falls notwendig werden die Verbindungen durch Umkristallisation, Destillation oder Chromatographie gereinigt oder können gegebenenfalls auch im nächsten Schritt ohne vorhergehende Reinigung eingesetzt werden.

Analog zu der zuvor beschriebenen Methode können die Verbindungen (**XIII**) aus entsprechenden Verbindungen (**XII**) synthetisiert werden wie in Schema 17 gezeigt. Der Aldehyd (**XII**) kann nach literaturbeschriebenen Vorschriften hergestellt werden (Beispiele s. WO 08/013925, WO 08/013622 und darin zitierte Referenzen).

Eine Möglichkeit das Zwischenprodukt (**IVc**) aus Verbindung (**X**) darzustellen ist in Schema 6 gezeigt.

Eine Verbindung der allgemeinen Formel (**IVc**) kann aus einem Alkin mit der allgemeinen Formel (**X**) durch eine Palladium-katalysierte Kreuzkupplung analog zu in der Literatur beschriebenen Vorschriften durchgeführt werden (siehe z.B. J. Am. Chem. Soc. 2003, 125, 13642-13643, Synlett. 2006, 18, 2941-2946), und zwar unter Verwendung eines Halogenids der Formel R³-L³-Hal, wobei das primäre Halogenid in der Gegenwart eines Katalysators wie z.B. [(π-allyl)PdCl]₂, Pd(OAc)₂, PdCl₂(CH₃CN)₂, Pd(PPh₃)₄ oder PdCl₂(PPh₃)₂ sowie gegebenenfalls in Gegenwart weiterer Cokatalysatoren wie etwa CuI, Cs₂CO₃ und 1,3-bis(1-adamantyl)imidazoliumchlorid z.B. in einem Lösungsmittelgemisch von DMF und Diethylether bei 0-80 °C umgesetzt wird.

Halogenide der Formel R³-L³-Hal sind entweder kommerziell erhältlich oder durch literaturbekannte Methoden erhältlich.

Alternativ kann das terminale Alkin auch mit einer Schutzgruppe versehen werden, wie z.B. durch Umsetzung mit Benzylchlorid in der Gegenwart eines Palladium(II)-Katalysators, wie z.B. Pd(OAc)₂ oder PdCl₂(CH₃CN)₂ und Caesiumcarbonat sowie 2-Dicyclohexylphosphino-2',4',6'-triisopropylbiphenyl, in Tetrahydrofuran oder Acetonitril bei 0-80 °C.

Nach Beendigung der Reaktion, werden die Verbindungen (**IVc**) von der Reaktionsmischung durch eine der üblichen Trenntechniken getrennt. Falls notwendig werden die Verbindungen durch Umkristallisation, Destillation oder Chromatographie gereinigt oder können gegebenenfalls auch im nächsten Schritt ohne vorhergehende Reinigung eingesetzt werden.

Analog zu der zuvor beschriebenen Methode können die Verbindungen (**Ic**) aus entsprechenden Verbindungen (**XIII**) synthetisiert werden wie in Schema 19 gezeigt.

Eine Möglichkeit das Zwischenprodukt (**XI**) aus Verbindung (**X**) darzustellen ist in Schema 7 gezeigt

Um eine direkte Bindung zwischen der Dreifachbindung (L² is -C≡C-) und R³ zu bilden, kann es notwendig sein, das terminale Alkin vor der Palladium-katalysierten Kupplungsreaktion zu silylieren. Silylierungen terminaler Alkine werden mit einer der bekannten Silylierungsmethoden für terminale Alkine durchgeführt, wie beispielsweise durch Versetzen des terminalen Alkins mit einer Base (wie z.B. Lithiumhexamethyldisilazid) bei -78 °C in einem polaren aprotischen Lösungsmittel und dem Abfangen des resultierenden Zwischenprodukts mit z.B. einem Silylhalogenid (CHEMBIOCHEM, 2001, 1, 69-75) oder -triflat.

Als Lösungsmittel kommen dabei alle üblichen, unter den Reaktionsbedingungen inerten Lösungsmittel, wie z.B zyklische und azyklische Ether (z.B. Diethylether, Tetrahydrofuran, Dioxane), aromatische Kohlenwasserstoffe (z.B. Benzol, Toluol, Xylol), halogenierte Kohlenwasserstoffe (z.B. Dichlormethan, Chloroform, Tetrachlorkohlenstoff) und halogenierte aromatische Kohlenwasserstoffe (z.B. Chlorbenzol, Dichlorbenzol) in Frage. Die Reaktion kann auch in Mischungen von zwei oder mehreren dieser Lösungsmittel ausgeführt werden. Die bevorzugten Lösungsmittel sind Tetrahydrofuran und Toluol.

Es können alle bekannten Basen eingesetzt werden, aber bevorzugt werden für die Reaktion Lithium-, Natrium-, Kaliumhexamethyldisilazid und Lithiumdiisopropylamid verwendet. Wenigstens ein Äquivalent der Base muss zu dem Alkin der allgemeinen Formel (**X**) zugegeben werden und falls nötig kann sie im Überschuss verwendet werden.

Mindestens ein Äquivalent Silylchlorid oder Silyltriflat kann als Quelle der Silylgruppe in der Reaktion verwendet werden.

Die Reaktion wird normalerweise bei Temperaturen von -78 °C - 100 °C durchgeführt und vorzugsweise bei -20 °C - 40 °C, aber sie kann auch bei Rückflusstemperatur der Reaktionsmischung durchgeführt werden. Die Reaktionszeit variiert in Abhängigkeit vom Maßstab der Reaktion und der Reaktionstemperatur, aber liegt im Allgemeinen zwischen einigen Minuten und 48 Stunden.

Nach Beendigung der Reaktion, werden die Verbindungen (**XI**) von der Reaktionsmischung durch eine der üblichen Trenntechniken getrennt. Falls notwendig werden die Verbindungen durch Umkristallisation, Destillation oder Chromatographie gereinigt oder können gegebenenfalls auch im nächsten Schritt ohne vorhergehende Reinigung eingesetzt werden.

Analog zu der zuvor beschriebenen Methode können die Verbindungen (**XIV**) aus entsprechenden Verbindungen (**XIII**) synthetisiert werden wie in Schema 18 gezeigt.

Eine Möglichkeit das Zwischenprodukt (**IVc**) aus Verbindung (**XI**) darzustellen ist in Schema 8 gezeigt.

Eine Verbindung der allgemeinen Formel (**IVc**) kann aus einem Alkin mit der allgemeinen Formel (**XI**) durch Palladium-katalysierte Kupplungsreaktionen, wie zum Beispiel der Sonogashira Reaktion (J. Med. Chem. 2006, 49, 1080-1110) hergestellt werden, wobei das Halogenid die allgemeine Formel R³-L³-Halogenid aufweist, in der L³ eine direkte Bindung ist.

Als Lösungsmittel können alle üblichen, unter den Reaktionsbedingungen inerten Lösungsmittel, wie beispielsweise zyklische und azyklische Ether (z.B. Diethylether, Tetrahydrofuran, Dioxane), aromatische Kohlenwasserstoffe (z.B. Benzol, Toluol, Xylol), halogenierte Kohlenwasserstoffe (z.B. Dichlormethan, Chloroform, Tetrachlorkohlenstoff), Carbonsäureester (z.B. Essigsäureethylester), Amide (z.B. N,N-Dimethylformamid, N,N-Dimethylacetamid), Dimethylsulfoxid und 1,3-Dimethyl-2-imidazolinon verwendet werden und die Reaktion kann in Mischungen von zwei oder mehreren dieser Lösungsmittel ausgeführt werden. Das bevorzugte Lösungsmittel ist *N,N*-Dimethylformamid.

Die Reaktion sollte in Gegenwart der folgenden Additive ausgeführt werden: Triethylamin (wenigstens ein Äquivalent bezogen auf Verbindung (**XI**)), Kupfer(I)iodid (wenigstens 0.1 Äquivalent zu der verbindung (**XI**)) und eine Fluoridquelle wie beispielsweise Tetra-*n-*butylammoniumfluorid (wenigstens 1 Äquivalent bezogen auf Verbindung (**XI**) oder ein leichter Überschuss).

Es können zahlreiche der kommerziell erhältlichen Palladium(0)katalysatoren in der Reaktion verwendet werden, aber bevorzugt wird in der Reaktion Tetrakistriphenylphosphinpalladium (Aldrich) verwendet. Die Aufwandmenge an Katalysator beträgt mindestens 1% bis zu einem Überschuss in Abhängigkeit von der Ausgangsverbindung (**XI**).

Die Reaktion wird normalerweise bei Temperaturen von 25 °C - 120 °C durchgeführt und vorzugsweise bei 70 °C - 90 °C, aber sie kann auch bei Rückflusstemperatur der Reaktionsmischung durchgeführt werden. Die Reaktionszeit variiert in Abhängigkeit vom Maßstab der Reaktion und der Reaktionstemperatur, aber liegt im Allgemeinen zwischen einigen Minuten und 48 Stunden.

Nach Beendigung der Reaktion, werden die Verbindungen (**IVc**) von der Reaktionsmischung durch eine der üblichen Trenntechniken getrennt. Falls notwendig werden die Verbindungen durch Umkristallisation, Destillation oder Chromatographie gereinigt oder können gegebenenfalls auch im nächsten Schritt ohne vorhergehende Reinigung eingesetzt werden.

Analog zu der zuvor beschriebenen Methode können die Verbindungen (**Ic**) aus entsprechenden Verbindungen (**XIV**) synthetisiert werden wie in Schema 20 gezeigt.

Eine Möglichkeit zur Synthese von Verbindungen der Formel (**IVa**) oder (**IVb**) ist in Schema 9 gezeigt.

Thiocarboxamide (**V**) sind entweder kommerziell verfügbar oder nach literaturbekannten Methoden erhältlich, beispielsweise durch Schwefelung des kommerziell erhältlichen entsprechenden Carboxamids durch Verwendung von z.B. Lawesson's Reagenz (Org. Synth. Vol. 7, 1990, 372) bei Raumtemperatur in einer Mischung von Chloroform/Dimethoxyethan (1/2.5). Dabei steht PG für eine Schutzgruppe wie in Schema 2 definiert.

α-Haloketone (**VIa**) und (**VIb**) sind entweder kommerziell verfügbar oder nach literaturbekannten Methoden erhältlich (Beispiele s. Molecules, 2003, 8, 793-865). Alternativ können Enolate oder Silylenolether, die aus (**XIIa**) oder (**XIIb**) hergestellt wurden, mit einer Halogenquelle zu □-Haloketonen **(VIa)** oder (**VIb**) umgesetzt werden, (J. Med. Chem. 2006, 49, 7877-7886; J. Med. Chem. 2006, 49, 1080-1110; Org. Synth. Vol. 6, 1988, 175; Org. Synth. Vol. 53, 1973, 111). (Schema 16)

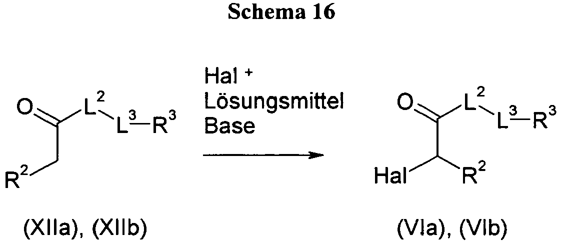

In Schema 16,
Hal = Cl, Br oder I,
L² = -C(R⁸)₂-C(R⁸)₂- für Verbindungen mit der Formel (**VIa**) und (**XIIa**),
L² = -CR⁹=CR⁹- für Verbindungen mit der Formel (VI**b**) und (**XIIb**),
L³, R², R³, R⁸ und R⁹ wie für Formel (**I**) oben definiert.

Die Thiazole (**IVa**) oder (**IVb**) werden durch eine Hantzsch-Thiazol-Synthese aus den Thiocarboxamiden (**V**) und α-Haloketonen (**VIa**) oder (**VIb**) erhalten (siehe z.B. "Comprehensive Heterocyclic Chemistry", Pergamon Press, 1984; Vol 6, Seite 235-363, "Comprehensive Heterocyclic Chemistry II", Pergamon Press, 1996; Vol 3, Seite 373-474 und darin zitierte Referenzen, und WO 07/014290).

Als Lösungsmittel können alle üblichen, unter den Reaktionsbedingungen inerten Lösungsmittel, wie z.B. Alkohole (z.B. Methanol, Ethanol, Propanol), zyklische und azyklische Ether (z.B. Diethylether, Tetrahydrofuran, Dioxane), aromatische Kohlenwasserstoffe (z.B. Benzol, Toluol, Xylol), halogenierte Kohlenwasserstoffe (z.B. Dichlormethan, Chloroform, Tetrachlorkohlenstoff), halogenierte aromatische Kohlenwasserstoffe (z.B. Chlorbenzol, Dichlorbenzol), Nitrile (z.B. Acetonitril), Carbonsäureester (z.B. Essigsäureethylester), Amide (z.B. N,N-Dimethylformamid, N,N-Dimethylacetamid), Dimethylsulfoxid, 1,3-Dimethyl-2-imidazolinon, Wasser und Essigsäure verwendet werden oder die Reaktion kann in Mischungen von zwei oder mehreren dieser Lösungsmittel ausgeführt werden. Die bevorzugten Lösungsmittel sind *N,N*-Dimethylformamid und Ethanol.

Gegebenenfalls wird eine Hilfsbase, wie beispielsweise Triethylamin, verwendet.

Die Ausgangsmaterialien werden in äquimolaren Mengen eingesetzt. (**V**) oder (**VI**) können aber auch jeweils im Überschuss verwendet werden. Die Reaktion wird normalerweise bei Temperaturen von 0 °C - 100 °C durchgeführt und vorzugsweise bei Raumtemperatur, aber sie kann auch bei Rückflusstemperatur der Reaktionsmischung durchgeführt werden. Die Reaktionszeit variiert in Abhängigkeit vom Maßstab der Reaktion und der Reaktionstemperatur, aber liegt im Allgemeinen zwischen einigen Minuten und 48 Stunden.

Falls notwendig werden die Verbindungen durch Umkristallisation oder Chromatographie gereinigt oder können gegebenenfalls auch im nächsten Schritt ohne vorhergehende Reinigung eingesetzt werden.

Eine Möglichkeit, das Zwischenprodukt (**IVa**) aus Verbindung (**IVb**) oder (**IVc**) darzustellen, ist in Schema 10 gezeigt.

Eine Dreifachbindung oder eine Doppelbindung wird durch eine Hydrierung unter Verwendung eines geeigneten Katalysators in eine Einfachbindung umgewandelt. Ein Katalysator für die Reaktion in Schema 10 wird aus den in der Literatur bekannten Katalysatoren zur Hydrierung *("*Reductions in Organic Chemistry", Miloš Hudlický, John Wiley & Sons, 1984) ausgewählt, wie z.B. Palladium auf Aktivkohle oder Pearlmans Katalysator (Pd(OH)₂ auf Aktivkohle). Durch die Verwendung eines solchen Katalysators kann eine Verbindung der Formel (**IVa**) aus einer Verbindung der Formel (**IVb**) oder (**IVc**) hergestellt werden.

Als Lösungsmittel können alle üblichen, unter den Reaktionsbedingungen inerten Lösungsmittel, wie z.B. Alkohole (z.B. Methanol, Ethanol, Propanol), zyklische und azyklische Ether (z.B. Diethylether, Tetrahydrofuran, Dioxan, Dimethoxyethan), aromatische Kohlenwasserstoffe (z.B. Benzol, Toluol, Xylol), halogenierte Kohlenwasserstoffe (z.B. Dichlormethan, Chloroform, Tetrachlorkohlenstoff), halogenierte aromatische Kohlenwasserstoffe (z.B. Chlorbenzol, Dichlorbenzol), Nitrile (z.B. Acetonitril), Carbonsäureester (z.B. Essigsäureethylester), Amide (z.B. N,N-Dimethylformamid, N,N-Dimethylacetamid), Dimethylsulfoxid, 1,3-Dimethyl-2-imidazolinon, Wasser und Essigsäure verwendet werden oder die Reaktion kann in Mischungen von zwei oder mehreren dieser Lösungsmittel ausgeführt werden. Die bevorzugten Lösungsmittel sind *N,N-*Dimethylformamid und Ethanol.

Die verwendete Katalysatormenge liegt bei 0,1-90mol% bezogen auf das Edukt, vorzugsweise werden 1-30mol% des Katalysators bezogen auf das Edukt verwendet. Die Reaktion kann bei Überdruck (1-1000 bar) oder bevorzugt bei atmosphärischem Druck durchgeführt werden. Die Reaktion wird normalerweise bei Temperaturen von 0°C - 150 °C durchgeführt und vorzugsweise bei Raumtemperatur, aber sie kann auch bei Rückflusstemperatur der Reaktionsmischung durchgeführt werden. Die Reaktionszeit variiert in Abhängigkeit vom Maßstab der Reaktion und der Reaktionstemperatur, aber liegt im Allgemeinen zwischen einer halben Stunde und 72 Stunden.

Nach Beendigung der Reaktion, werden die Verbindungen (**IVa**) von der Reaktionsmischung durch eine der üblichen Trenntechniken getrennt. Falls notwendig werden die Verbindungen durch Umkristallisation, Destillation oder Chromatographie gereinigt oder können gegebenenfalls auch im nächsten Schritt ohne vorhergehende Reinigung eingesetzt werden.

Eine Möglichkeit das Zwischenprodukt (**II**) aus entsprechenden Verbindungen (**IV**) darzustellen ist in Schema 11 gezeigt.

Eine Verbindung mit der allgemeinen Form (**IV**) wird in eine entsprechenden Verbindung mit der allgemeinen Formel (**II**) durch geeignete Methoden zur Entfernung von Schutzgruppen, die in der Literatur beschrieben sind ("Protective Groups in Organic Synthesis"; Third Edition; Theodora W. Greene, Peter G. M. Wuts; 494-653, und dort zitierte Literatur), überführt.

t-Butoxycarbonyl und Benzyloxycarbonyl Schutzgruppen können im sauren Medium entfernt werden (z.B mit Salzsäure oder der Trifluoressigsäure). Acetylschutzgruppen können unter basischen Bedingungen (z.B. mit Kaliumcarbonat oder Cäsiumcarbonat) entfernt werden. Benzylische Schutzgruppen können hydrogenolytisch mit Wasserstoff in Gegenwart eines Katalysators (z.B. Palladium auf Aktivkohle) entfernt werden.

Als Lösungsmittel können alle üblichen, unter den Reaktionsbedingungen inerten Lösungsmittel, wie z.B. Alkohole (z.B. Methanol, Ethanol, Propanol), zyklische und azyklische Ether (z.B. Diethylether, Tetrahydrofuran, Dioxane), aromatische Kohlenwasserstoffe (z.B. Benzol, Toluol, Xylol), halogenierte Kohlenwasserstoffe (z.B. Dichlormethan, Chloroform, Tetrachlorkohlenstoff), halogenierte aromatische Kohlenwasserstoffe (z.B. Chlorbenzol, Dichlorbenzol), Nitrile (z.B. Acetonitril), Carbonsäureester (z.B. Essigsäureethylester), Amide (z.B. N,N-Dimethylformamid, N,N-Dimethylacetamid), Dimethylsulfoxid, 1,3-Dimethyl-2-imidazolinon, Wasser und Essigsäure verwendet werden oder die Reaktion kann in Mischungen von zwei oder mehreren dieser Lösungsmittel ausgeführt werden.

Säuren, die für diese Reaktion, der Entschützung von t-Butoxycarbonyl und Benzyloxycarbonyl Gruppen verwendet werden können, sind z.B. Trifluoressigsäure, Salzsäure oder andere Säuren, wie sie in der Literatur beschrieben sind (z.B. "Protective Groups in Organic Synthesis"; Third Edition; Theodora W. Greene, Peter G. M. Wuts; S. 494-653).

Die Reaktion wird normalerweise bei Temperaturen von 0°C - 150 °C durchgeführt und vorzugsweise bei Raumtemperatur, aber sie kann auch bei Rückflusstemperatur der Reaktionsmischung durchgeführt werden. Die Reaktionszeit variiert in Abhängigkeit vom Maßstab der Reaktion und der Reaktionstemperatur, aber liegt im Allgemeinen zwischen einer halben Stunde und 72 Stunden.

Nach Beendigung der Reaktion, werden die Verbindungen (**II**) von der Reaktionsmischung durch eine der üblichen Trenntechniken getrennt. Falls notwendig werden die Verbindungen durch Umkristallisation, Destillation oder Chromatographie gereinigt oder wenn gewünscht auch im nächsten Schritt ohne vorhergehende Reinigung eingesetzt werden. Es ist außerdem möglich, die Verbindung der allgemeinen Formel (**II**) als Salz zu isolieren, z.B. als Salz der Salzsäure oder der Trifluoressigsäure.

Eine Möglichkeit das Zwischenprodukt (**IIa**) aus Verbindung (**IIa'**) darzustellen ist in Schema 12 gezeigt.

Eine Verbindung der allgemeinen Formel (**IIa**), bei der R² = Cl, Br und I ist, wird aus einer Verbindung der allgemeinen Formel (**IIa'**) mit R² = H durch Halogenierung erhalten, siehe beispielsweise Org. Lett., 2005, 7(2), 299-301; Angew. Chem., Int. Ed., 2004, 43(34), 4471-4475; Eur. J. Org. Chem., 2002, (13), 2126-2135.

Als Lösungsmittel können alle üblichen, unter den Reaktionsbedingungen inerten Lösungsmittel, wie z.B. Alkohole (z.B. Methanol, Ethanol, Propanol), zyklische und azyklische Ether (z.B. Diethylether, Tetrahydrofuran, Dioxane), aromatische Kohlenwasserstoffe (z.B. Benzol, Toluol, Xylol), halogenierte Kohlenwasserstoffe (z.B. Dichlormethan, Chloroform, Tetrachlorkohlenstoff), halogenierte aromatische Kohlenwasserstoffe (z.B. Chlorbenzol, Dichlorbenzol), Carbonsäureester (z.B. Essigsäureethylester), Amide (z.B. *N,N-*Dimethylformamid, *N,N*-Dimethylacetamid), Dimethylsulfoxid, 1,3-Dimethyl-2-imidazolinon, Wasser und Essigsäure verwendet werden oder die Reaktion kann in Mischungen von zwei oder mehreren dieser Lösungsmittel ausgeführt werden.

Als Halogenquelle kann z.B. *N-*Bromosuccinimid, *N-*Chlorosuccinimid, *N-*Iodosuccinimid, Pyridiniumtribromid, Brom, Iod, oder Chlor verwendet werden.

Gegebenenfalls kann eine Base, wie z.B. Lithiumdiisopropylamid oder Butyllithium verwendet werden, um die 5-Position des Thiazols zu lithiieren, bevor die elektrophile Halogenquelle zugegeben wird.

Die Reaktion kann gegebenenfalls mit einer Säure, wie z.B. Essigsäure, Schwefelsäure, Bromwasserstoff oder Salzsäure, durchgeführt werden.

Die Ausgangsmaterialien und das Halogenierungsmittel werden in equimolaren Mengen eingesetzt. Das Halogenierungsmittel kann auch im Überschuss verwendet werden. Die Reaktion wird normalerweise bei Temperaturen von 0 °C - 60 °C durchgeführt und vorzugsweise bei 0 °C - 30 °C, aber sie kann auch bei Rückflusstemperatur der Reaktionsmischung durchgeführt werden. Die Reaktionszeit variiert in Abhängigkeit vom Maßstab der Reaktion und der Reaktionstemperatur, aber liegt im Allgemeinen zwischen einigen Minuten und 48 Stunden.

Nach Beendigung der Reaktion, werden die Verbindungen (**II**) von der Reaktionsmischung durch eine der üblichen Trenntechniken getrennt. Falls notwendig werden die Verbindungen durch Umkristallisation, Destillation oder Chromatographie gereinigt oder können gegebenenfalls auch im nächsten Schritt ohne vorhergehende Reinigung eingesetzt werden.

Eine Möglichkeit Verbindungen der Formel (**I**), in denen Y = Sauerstoff ist, aus entsprechenden Verbindungen (**II**) darzustellen ist in Schema 13 gezeigt.

Säurehalogenide (**III**) (B = Halogen) oder die entsprechenden Carbonsäuren (**III**) (B = OH) sind kommerziell erhältlich oder durch in der Literatur beschriebene Prozesse darstellbar. Außerdem kann ein Substrat mit der allgemeinen Formel (**III**), mit B = Cl, aus der entsprechenden Säure (B = OH) durch Chlorierung unter Verwendung literaturbekannter Prozesse dargestellt werden. (z.B. Tetrahedron 2005, 61, 10827-10852, und darin zitierte Literatur).

Eine Verbindung mit der allgemeinen Formel (**I**), mit Y = O, kann synthetisiert werden durch eine Kupplungsreaktion einer Verbindung mit der entsprechenden allgemeinen Formel (**II**) mit einem Substrat der allgemeinen Formel (**III**) mit B = Cl, gegebenenfalls in der Gegenwart eines Säurefängers / Base.

Als Lösungsmittel können alle üblichen, unter den Reaktionsbedingungen inerten Lösungsmittel, wie z.B. Alkohole (z.B. Methanol, Ethanol, Propanol), zyklische und azyklische Ether (z.B. Diethylether, Tetrahydrofuran, Dioxane), aromatische Kohlenwasserstoffe (z.B. Benzol, Toluol, Xylol), halogenierte Kohlenwasserstoffe (z.B. Dichlormethan, Chloroform, Tetrachlorkohlenstoff), halogenierte aromatische Kohlenwasserstoffe (z.B. Chlorbenzol, Dichlorbenzol) und Nitrile (z.B. Acetonitril) verwendet werden oder die Reaktion kann in Mischungen von zwei oder mehreren dieser Lösungsmittel ausgeführt werden. Die bevorzugten Lösungsmittel sind Tetrahydrofuran und Dichlormethan.

Wenigstens ein Äquivalent eines Säurefängers / einer Base (z.B. Hünig Base, Triethylamin oder kommerziell erhältliche polymere Säurefänger) im Verhältnis zum Startmaterial der allgemeinen Formel (**II**) wird verwendet. Ist das Startmaterial ein Salz, werden wenigstens zwei Äquivalente des Säurefängers benötigt.

Die Reaktion wird normalerweise bei Temperaturen von 0 °C - 100 °C durchgeführt und vorzugsweise bei 20 °C - 30 °C, aber sie kann auch bei Rückflusstemperatur der Reaktionsmischung durchgeführt werden. Die Reaktionszeit variiert in Abhängigkeit vom Maßstab der Reaktion und der Reaktionstemperatur, aber liegt im Allgemeinen zwischen einigen Minuten und 48 Stunden.

Nach Beendigung der Reaktion, werden die Verbindungen (**I**), von der Reaktionsmischung durch eine der üblichen Trenntechniken getrennt. Falls notwendig werden die Verbindungen durch Umkristallisation, Destillation oder Chromatographie gereinigt oder können gegebenenfalls auch im nächsten Schritt ohne vorhergehende Reinigung eingesetzt werden.

Alternativ kann eine Verbindung mit der Formel (**I**), mit Y = O auch aus der entsprechenden Verbindung mit der allgemeinen Formel (**II**) mit einem Substrat der allgemeinen Formel (**III**) mit B = OH in der Gegenwart eines Kupplungsreagenzes synthetisiert werden analog zu in der Literatur beschriebenen Vorschriften (z.B. Tetrahedron 2005, 61, 10827-10852, und darin zitierte Referenzen).

Geeignete Kupplungsreagenzien sind beispielsweise Peptidkupplungsreagenzien (zum Beispiel, 1-Ethyl-3-(30-dimethylamino)carbodiimid gemischt mit 4-Dimethylaminopyridin, 1-Ethyl-3-(30-dimethylamino)carbodiimid gemischt mit Hydroxybenzotriazol, Bromotri(pyrrolidino)-phosphonium hexafluorophosphat, 2-(1H-7-Azabenzotriazol-1-yl)--1,1,3,3-tetramethyluroniumhexafluoro-phosphat methanaminium, etc.)

Gegebenenfalls kann eine Base, wie z.B. Triethylamin oder Hünig Base in der Reaktion verwendet werden.

Als Lösungsmittel können alle üblichen, unter den Reaktionsbedingungen inerten Lösungsmittel, wie z.B. Alkohole (z.B. Methanol, Ethanol, Propanol), zyklische und azyklische Ether (z.B. Diethylether, Tetrahydrofuran, Dioxane), aromatische Kohlenwasserstoffe (z.B. Benzol, Toluol, Xylol), halogenierte Kohlenwasserstoffe (z.B. Dichlormethan, Chloroform, Tetrachlorkohlenstoff), halogenierte aromatische Kohlenwasserstoffe (z.B. Chlorbenzol, Dichlorbenzol), Nitrile (z.B. Acetonitril) und Amide (z.B. N,N-Dimethylformamid, N,N-Dimethylacetamid) verwendet werden oder die Reaktion kann in Mischungen von zwei oder mehreren dieser Lösungsmittel ausgeführt werden. Die bevorzugten Lösungsmittel sind *N,N-*Dimethylformamid und Dichlormethan.

Die Reaktion wird normalerweise bei Temperaturen von 0 °C - 100 °C durchgeführt und vorzugsweise bei 0 °C - 30 °C, aber sie kann auch bei Rückflusstemperatur der Reaktionsmischung durchgeführt werden. Die Reaktionszeit variiert in Abhängigkeit vom Maßstab der Reaktion und der Reaktionstemperatur, aber liegt im Allgemeinen zwischen einigen Minuten und 48 Stunden.

Nach Beendigung der Reaktion, werden die Verbindungen (**I**) von der Reaktionsmischung durch eine der üblichen Trenntechniken getrennt. Falls notwendig werden die Verbindungen durch Umkristallisation, Destillation oder Chromatographie gereinigt oder können gegebenenfalls auch im nächsten Schritt ohne vorhergehende Reinigung eingesetzt werden.

Eine Möglichkeit Verbindungen der Formel (**I**), in denen Y = Schwefel ist aus entsprechenden Verbindungen (**I**), in denen Y gleich Sauerstoff ist, darzustellen ist in Schema 14 gezeigt.

Als Lösungsmittel können alle üblichen, unter den Reaktionsbedingungen inerten Lösungsmittel, wie z.B. Alkohole (z.B. Methanol, Ethanol, Propanol), zyklisch und azyklische Ether (z.B. Diethylether, Tetrahydrofuran, Dioxane), aromatische Kohlenwasserstoffe (z.B. Benzol, Toluol, Xylol), halogenierte Kohlenwasserstoffe (z.B. Dichlormethan, Chloroform, Tetrachlorkohlenstoff), halogenierte aromatische Kohlenwasserstoffe (z.B. Chlorbenzol, Dichlorbenzol), Nitrile (z.B. Acetonitril), Carbonsäureester (z.B. Essigsäureethylester) und Amide (z.B. N,N-Dimethylformamid, N,N-Dimethylacetamid) verwendet werden und die Reaktion kann in Mischungen von zwei oder mehreren dieser Lösungsmittel ausgeführt werden. Die bevorzugten Lösungsmittel sind Chloroform und 1,2-Dimethoxyethan

Geeignete Schwefelungsreagenzien sind beispielsweise Lawesson's Reagenz (s. Tetrahedron 1986, 42, 6555-6564, Tetrahedron Lett. 1993, 46, 7459-7462) und Phosphorpentasulfid. Das Startmaterial und das Schwefelungsreagenz werden in equimolaren Mengen verwendet, aber das Schwefelungsreagenz kann gegebenenfalls auch im Überschuss verwendet warden.

Die Reaktion wird normalerweise bei Temperaturen von 0 °C - 150 °C durchgeführt und vorzugsweise bei 0 °C - 100 °C, aber sie kann auch bei Rückflusstemperatur der Reaktionsmischung durchgeführt werden. Die Reaktionszeit variiert in Abhängigkeit vom Maßstab der Reaktion und der Reaktionstemperatur, aber liegt im Allgemeinen zwischen einigen Minuten und 48 Stunden.

Nach Beendigung der Reaktion, werden die Verbindungen (**I**), von der Reaktionsmischung durch eine der üblichen Trenntechniken getrennt. Falls notwendig werden die Verbindungen durch Umkristallisation, Destillation oder Chromatographie gereinigt.

Neu sind Verbindungen der Formel (**IX**), in denen 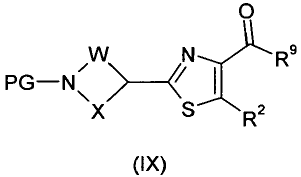 die Symbole die folgenden Bedeutungen haben
PG steht für unsubstituiertes oder substituiertes Phenylmethyl (wobei die Substituenten unabhängig voneinander ausgewählt sind aus Methyl, Methoxy, Nitro, Dioxolano), Acetyl, C₁-C₄-Alkoxycarbonyl oder Benzyloxycarbonyl,
R⁹ = C₁-C₄-Alkyl, C₃-C₆-Cycloalkyl,
W, X und R² haben die oben angegebenen allgemeinen, bevorzugten, besonders bevorzugten oder ganz besonders bevorzugten Bedeutungen.

Neu sind Verbindungen der Formel (**X**), in denen 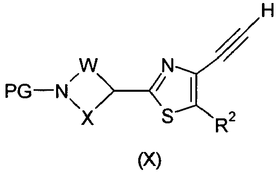 die Symbole die folgenden Bedeutungen haben:
PG steht für unsubstituiertes oder substituiertes Phenylmethyl (wobei die Substituenten unabhängig voneinander ausgewählt sind aus Methyl, Methoxy, Nitro, Dioxolano), Acetyl, C₁-C₄-Alkoxycarbonyl, oder Benzyloxycarbonyl,
W, X und R² haben die oben angegebenen allgemeinen, bevorzugten, besonders bevorzugten oder ganz besonders bevorzugten Bedeutungen.

Neu sind Verbindungen der Formel (**XI**), in denen 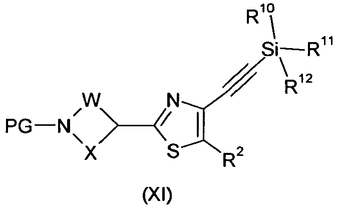 die Symbole die folgenden Bedeutungen haben
PG steht für unsubstituiertes oder substituiertes Phenylmethyl (wobei die Substituenten unabhängig voneinander ausgewählt sind aus Methyl, Methoxy, Nitro, Dioxolano), Acetyl, C₁-C₄-Alkoxycarbonyl, oder Benzyloxycarbonyl,
R¹⁰, R¹¹ und R¹² = C₁-C₄-Alkyl oder Phenyl,
W, X und R² haben die oben angegebenen allgemeinen, bevorzugten, besonders bevorzugten oder ganz besonders bevorzugten Bedeutungen.

Neu sind Verbindungen der Formel (**IV**) in denen 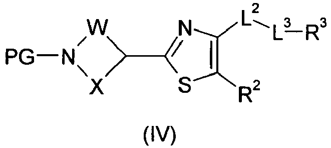 die Symbole die folgenden Bedeutungen haben
PG steht für unsubstituiertes oder substituiertes Phenylmethyl (wobei die Substituenten unabhängig voneinander ausgewählt sind aus Methyl, Methoxy, Nitro, Dioxolano), Acetyl, C₁-C₄-Alkoxycarbonyl oder Benzyloxycarbonyl,
W, X, L², L³, R², R³, R⁸ und R⁹ haben die oben angegebenen allgemeinen, bevorzugten, besonders bevorzugten oder ganz besonders bevorzugten Bedeutungen.

Neu sind Verbindungen der Formel (**IIb**) und (**IIc**), in denen 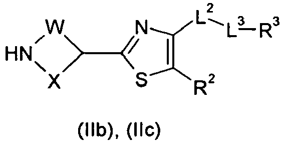 die Symbole die folgenden Bedeutungen haben
- L²: steht für -CR⁹=CR⁹- für Verbindungen mit der Formel (**IIb**)
- L²: steht für -C=C- für Verbindungen mit der Formel (**IIc**)
- W, X, L³, R² R³, und R⁹: haben die oben angegebenen allgemeinen, bevorzugten, besonders bevorzugten oder ganz besonders bevorzugten Bedeutungen.

Neu sind Verbindungen der Formel (**XIII**), in denen 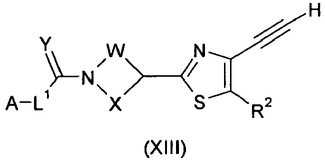 die Symbole die folgenden Bedeutungen haben
A, W, X, Y und R² haben die oben angegebenen allgemeinen, bevorzugten, besonders bevorzugten oder ganz besonders bevorzugten Bedeutungen.

Neu sind Verbindungen der Formel (**XIV**), in denen 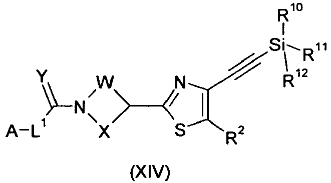 die Symbole die folgenden Bedeutungen haben
R¹⁰, R¹¹ und R¹² stehen für C₁-C₄-Alkyl oder Phenyl,
A, W, X, Y, L¹ und R² haben die oben angegebenen allgemeinen, bevorzugten, besonders bevorzugten oder ganz besonders bevorzugten Bedeutungen.

Die erfindungsgemäßen Verfahren zur Herstellung der Verbindungen der Formel **(I)** werden vorzugsweise unter Verwendung eines oder mehrerer Reaktionshilfsmittel durchgeführt.

Als Reaktionshilfsmittel kommen gegebenenfalls die üblichen anorganischen oder organischen Basen oder Säureakzeptoren in Betracht. Hierzu gehören vorzugsweise Alkalimetall- oder Erdalkalimetall- -acetate, -amide, -carbonate, -hydrogencarbonate, -hydride, -hydroxide oder -alkanolate, wie beispielsweise Natrium-, Kalium- oder Calcium-acetat, Lithium-, Natrium-, Kalium- oder Calcium-amid, Natrium-, Kalium- oder Calcium-carbonat, Natrium-, Kalium- oder Calcium-hydrogencarbonat, Lithium-, Natrium-, Kalium- oder Calcium-hydrid, Lithium-, Natrium- - Kalium- oder Calcium-hydroxid, Natrium- oder Kalium- -methanolat, -ethanolat, -n- oder -i-propanolat, -n-, -i-, -s- oder -tButanolat; weiterhin auch basische organische Stickstoffverbindungen, wie beispielsweise Trimethylamin, Triethylamin, Tripropylamin, Tributylamin, Ethyldiisopropylamin, N,N-Dimethyl-cyclohexylamin, Dicyclohexylamin, Ethyl-dicyclohexylamin, N,N-Dimethyl-anilin, N,N-Dimethyl-benzylamin, Pyridin, 2-Methyl-, 3-Methyl-, 4-Methyl-, 2,4-Dimethyl-, 2,6-Dimethyl-, 3,4-Dimethyl-und 3,5-Dimethyl-pyridin, 5-Ethyl-2-methyl-pyridin, 4-Dimethylamino-pyridin, N-Methyl-piperidin, 1,4-Diazabicyclo[2,2,2]-octan (DABCO), 1,5-Diazabicyclo[4,3,0]-non-5-en (DBN), oder 1,8 Diazabicyclo[5,4,0]-undec-7-en (DBU).

Die erfindungsgemäßen Verfahren werden vorzugsweise unter Verwendung eines oder mehrerer Verdünnungsmittel durchgeführt. Als Verdünnungsmittel kommen praktisch alle inerten organischen Lösungsmittel in Frage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl-und Dibutylether, Glykoldimethylether und Diglykoldimethylether, Tetrahydrofuran und Dioxan, Ketone wie Aceton, Methylethyl-, Methyl-isopropyl-oder Methyl-isobutyl-keton, Ester wie Essigsäuremethylester oder - ethylester, Nitrile wie z.B. Acetonitril oder Propionitril, Amide wie z.B. Dimethylformamid, Dimethylacetamid und N-Methyl-pyrrolidon, sowie Dimethylsulfoxid, Tetramethylensulfon und Hexamethylphosphorsäuretriamid und DMPU.

Die Reaktionstemperaturen können bei den erfindungsgemäßen Verfahren in einem größeren Bereich variiert werden. Im allgemeinen arbeitet man bei Temperaturen zwischen 0°C und 250°C, vorzugsweise bei Temperaturen zwischen 10°C und 185°C.

Die erfindungsgemäßen Verfahren werden im allgemeinen unter Normaldruck durchgeführt. Es ist jedoch auch möglich, unter erhöhtem oder vermindertem Druck zu arbeiten.

Zur Durchführung der erfindungsgemäßen Verfahren werden die jeweils benötigten Ausgangsstoffe im allgemeinen in angenähert äquimolaren Mengen eingesetzt. Es ist jedoch auch möglich, eine der jeweils eingesetzten Komponenten in einem größeren Überschuss zu verwenden. Die Aufarbeitung erfolgt bei den erfindungsgemäßen Verfahren jeweils nach üblichen Methoden (vgl. die Herstellungsbeispiele).

Ein weiterer Gegenstand der Erfindung betrifft die nichtmedizinische Verwendung der erfindungsgemäßen heterocyclyl substituierten Thiazole zum Bekämpfen unerwünschter Mikroorganismen.

Ein weiterer Gegenstand der Erfindung betrifft ein Mittel zum Bekämpfen unerwünschter Mikroorganismen, umfassend wenigstens ein heterocyclyl-substituiertes Thiazol gemäß der vorliegenden Erfindung.

Außerdem betrifft die Erfindung ein Verfahren zum Bekämpfen unerwünschter Mikroorganismen in und/oder auf Pflanzen oder in und/oder auf Saatgut von Pflanzen, dadurch gekennzeichnet, dass die erfindungsgemäßen heterocyclyl-substituierten Thiazole auf die Mikroorganismen und/oder in deren Lebensraum ausgebracht werden.

Weiterhin betrifft die Erfindung ein Saatgut, welches mit wenigstens einem erfindungsgemäßen heterocyclyl-substituierten Thiazol behandelt wurde.

Ein letzter Gegenstand der Erfindung betrifft ein Verfahren zum Schutz von Saatgut vor unerwünschten Mikroorganismen durch Verwendung eines mit wenigstens einem heterocyclyl-substituierten Thiazol gemäß der vorliegenden Erfindung behandelten Saatgutes.

Die erfindungsgemäßen Stoffe weisen eine starke mikrobizide Wirkung auf und können zur Bekämpfung von unerwünschten Mikroorganismen, wie Fungi und Bakterien, im Pflanzenschutz und im Materialschutz eingesetzt werden.

Die erfindungsgemäßen heterocyclyl-substituierten Thiazole der Formel **(I)** besitzen sehr gute fungizide Eigenschaften und lassen sich im Pflanzenschutz beispielsweise zur Bekämpfung von Plasmodiophoromyceten, Oomyceten, Chytridiomyceten, Zygomyceten, Ascomyceten, Basidiomyceten und Deuteromyceten einsetzen.

Bakterizide lassen sich im Pflanzenschutz beispielsweise zur Bekämpfung von Pseudomonadaceae, Rhizobiaceae, Enterobacteriaceae, Corynebacteriaceae und Streptomycetaceae einsetzen.Die erfindungsgemäßen fungiziden Mittel können zur Bekämpfung von phytopathogenen Pilzen kurativ oder protektiv eingesetzt werden. Die Erfindung betrifft daher auch kurative und protektive Verfahren zum Bekämpfen von phytopathogenen Pilzen durch die Verwendung der erfindungsgemäßen Wirkstoffe oder Mittel, welche auf das Saatgut, die Pflanze oder Pflanzenteile, die Früchten oder den Boden, in welcher die Pflanzen wachsen, ausgebracht wird.

Die erfindungsgemäßen Mittel zum Bekämpfen von phytopathogenen Pilzen im Pflanzenschutz umfassen eine wirksame, aber nicht-phytotoxische Menge der erfindungsgemäßen Wirkstoffe. "Wirksame, aber nicht-phytotoxische Menge" bedeutet eine Menge des erfindungsgemäßen Mittels, die ausreichend ist, um die Pilzerkrankung der Pflanze ausreichend zu kontrollieren oder ganz abzutöten und die gleichzeitig keine nennenswerten Symptome von Phytotoxizität mit sich bringt. Diese Aufwandmenge kann im Allgemeinen in einem größeren Bereich variieren. Sie hängt von mehreren Faktoren ab, z.B. vom zu bekämpfenden Pilz, der Pflanze, den klimatischen Verhältnissen und den Inhaltsstoffen der erfindungsgemäßen Mittel.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Spross, Blatt, Blüte und Wurzel verstanden werden, wobei beispielhaft Blätter, Nadeln, Stängel, Stämme, Blüten, Fruchtkörper, Früchte und Samen sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Samen.

Als Pflanzen, welche erfindungsgemäß behandelt werden können, seien folgende erwähnt: Baumwolle, Flachs, Weinrebe, Obst, Gemüse, wie *Rosaceae sp.* (beispielsweise Kernfrüchte wie Apfel und Birne, aber auch Steinfrüchte wie Aprikosen, Kirschen, Mandeln und Pfirsiche und Beerenfrüchte wie Erdbeeren), *Ribesioidae sp., Juglandaceae sp., Betulaceae sp., Anacardiaceae sp., Fagaceae sp., Moraceae sp., Oleaceae sp., Actinidaceae sp., Lauraceae sp., Muraceae sp.* (beispielsweise Bananenbäume und -plantagen), *Rubiaceae sp.* (beispielsweise Kaffee), *Theaceae sp., Sterculiceae sp., Rutaceae sp.* (beispielsweise Zitronen, Organen und Grapefruit); *Solanaceae sp.* (beispielsweise Tomaten), *Liliaceae sp., Asteraceae sp.* (beispielsweise Salat), *Umbelliferae sp., Cruciferae sp., Chenopodiaceae sp., Cucurbitaceae sp.* (beispielsweise Gurke), *Alliaceae sp.* (beispielsweise Lauch, Zwiebel), *Papilionaceae sp.* (beispielsweise Erbsen); Hauptnutzpflanzen, wie *Gramineae sp.* (beispielsweise Mais, Rasen, Getreide wie Weizen, Roggen, Reis, Gerste, Hafer, Hirse und Triticale), *Asteraceae sp.* (beispielsweise Sonnenblume), *Brassicaceae sp.* (beispielsweise Weißkohl, Rotkohl, Brokkoli, Blumenkohl, Rosenkohl, Pak Choi, Kohlrabi, Radieschen sowie Raps, Senf, Meerrettich und Kresse), *Fabacae sp.* (beispielsweise Bohne, Erdnüsse), *Papilionaceae sp.* (beispielsweise Sojabohne), *Solanaceae sp.* (beispielsweise Kartoffeln), *Chenopodiaceae sp.* (beispielsweise Zuckerrübe, Futterrübe, Mangold, Rote Rübe); Nutzpflanzen und Zierpflanzen in Garten und Wald; sowie jeweils genetisch modifizierte Arten dieser Pflanzen. Bevorzugt werden Getreidepflanzen erfindungsgemäß behandelt.

Beispielhaft, aber nicht begrenzend, seien einige Erreger von pilzlichen Erkrankungen, die erfindungsgemäß behandelt werden können, genannt:
Erkrankungen, hervorgerufen durch Erreger des Echten Mehltaus wie z.B. Blumeria-Arten, wie beispielsweise Blumeria graminis; Podosphaera-Arten, wie beispielsweise Podosphaera leucotricha; Sphaerotheca-Arten, wie beispielsweise Sphaerotheca fuliginea; Uncinula-Arten, wie beispielsweise Uncinula necator;
Erkrankungen, hervorgerufen durch Erreger von Rostkrankheiten wie z.B. Gymnosporangium-Arten, wie beispielsweise Gymnosporangium sabinae; Hemileia-Arten, wie beispielsweise Hemileia vastatrix; Phakopsora-Arten, wie beispielsweise Phakopsora pachyrhizi oder Phakopsora meibomiae; Puccinia-Arten, wie beispielsweise Puccinia recondita, Puccinia graminis oder Puccinia striiformis; Uromyces-Arten, wie beispielsweise Uromyces appendiculatus;
Erkrankungen, hervorgerufen durch Erreger der Gruppe der Oomyceten wie z.B. Albugo-Arten, wie beispielsweise Albugo candida; Bremia-Arten, wie beispielsweise Bremia lactucae; Peronospora-Arten, wie beispielsweise Peronospora pisi oder P. brassicae; Phytophthora-Arten, wie beispielsweise Phytophthora infestans; Plasmopara-Arten, wie beispielsweise Plasmopara viticola; Pseudoperonospora-Arten, wie beispielsweise Pseudoperonospora humuli oder Pseudoperonospora cubensis; Pythium-Arten, wie beispielsweise Pythium ultimum;
Blattfleckenkrankheiten und Blattwelken, hervorgerufen durch z.B. Alternaria-Arten, wie beispielsweise Alternaria solani; Cercospora-Arten, wie beispielsweise Cercospora beticola; Cladosporium-Arten, wie beispielsweise Cladosporium cucumerinum; Cochliobolus-Arten, wie beispielsweise Cochliobolus sativus (Konidienform: Drechslera, Syn: Helminthosporium) oder Cochliobolus miyabeanus; Colletotrichum-Arten, wie beispielsweise Colletotrichum lindemuthanium; Cycloconium-Arten, wie beispielsweise Cycloconium oleaginum; Diaporthe-Arten, wie beispielsweise Diaporthe citri; Elsinoe-Arten, wie beispielsweise Elsinoe fawcettii; Gloeosporium-Arten, wie beispielsweise Gloeosporium laeticolor; Glomerella-Arten, wie beispielsweise Glomerella cingulata; Guignardia-Arten, wie beispielsweise Guignardia bidwelli; Leptosphaeria-Arten, wie beispielsweise Leptosphaeria maculans; Magnaporthe-Arten, wie beispielsweise Magnaporthe grisea; Microdochium-Arten, wie beispielsweise Microdochium nivale; Mycosphaerella-Arten, wie beispielsweise Mycosphaerella graminicola, Mycosphaerella arachidicola oder Mycosphaerella fijiensis; Phaeosphaeria-Arten, wie beispielsweise Phaeosphaeria nodorum; Pyrenophora-Arten, wie beispielsweise Pyrenophora teres oder Pyrenophora tritici repentis; Ramularia-Arten, wie beispielsweise Ramularia collo-cygni oder Ramularia areola; Rhynchosporium-Arten, wie beispielsweise Rhynchosporium secalis; Septoria-Arten, wie beispielsweise Septoria apii oder Septoria lycopersici; Stagonospora-Arten, wie beispielsweise Stagonospora nodorum; Typhula-Arten, wie beispielsweise Typhula incarnata; Venturia-Arten, wie beispielsweise Venturia inaequalis;
Wurzel- und Stängelkrankheiten, hervorgerufen durch z.B. Corticium-Arten, wie beispielsweise Corticium graminearum; Fusarium-Arten, wie beispielsweise Fusarium oxysporum; Gaeumannomyces-Arten, wie beispielsweise Gaeumannomyces graminis; Plasmodiophora-Arten, wie beispielsweise Plasmodiophora brassicae; Rhizoctonia-Arten, wie beispielsweise Rhizoctonia solani; Sarocladium-Arten, wie beispielsweise Sarocladium oryzae; Sclerotium-Arten, wie beispielsweise Sclerotium oryzae; Tapesia-Arten, wie beispielsweise Tapesia acuformis; Thielaviopsis-Arten, wie beispielsweise Thielaviopsis basicola;
Ähren- und Rispenerkrankungen (inklusive Maiskolben), hervorgerufen durch z.B. Altemaria-Arten, wie beispielsweise Alternaria spp.; Aspergillus-Arten, wie beispielsweise Aspergillus flavus; Cladosporium-Arten, wie beispielsweise Cladosporium cladosporioides; Claviceps-Arten, wie beispielsweise Claviceps purpurea; Fusarium-Arten, wie beispielsweise Fusarium culmorum; Gibberella-Arten, wie beispielsweise Gibberella zeae; Monographella-Arten, wie beispielsweise Monographella nivalis; Stagonospora-Arten, wie beispielsweise Stagonospora nodorum;
Erkrankungen, hervorgerufen durch Brandpilze wie z.B. Sphacelotheca-Arten, wie beispielsweise Sphacelotheca reiliana; Tilletia-Arten, wie beispielsweise Tilletia caries oder Tilletia controversa; Urocystis-Arten, wie beispielsweise Urocystis occulta; Ustilago-Arten, wie beispielsweise Ustilago nuda;
Fruchtfäule hervorgerufen durch z.B. Aspergillus-Arten, wie beispielsweise Aspergillus flavus; Botrytis-Arten, wie beispielsweise Botrytis cinerea; Penicillium-Arten, wie beispielsweise Penicillium expansum oder Penicillium purpurogenum; Rhizopus -Arten, wie beispielsweise Rhizopus stolonifer; Sclerotinia-Arten, wie beispielsweise Sclerotinia sclerotiorum; Verticilium-Arten, wie beispielsweise Verticilium alboatrum;
Samen- und bodenbürtige Fäulen und Welken, sowie Sämlingserkrankungen, hervorgerufen durch z.B. Alternaria-Arten, wie beispielsweise Alternaria brassicicola; Aphanomyces-Arten, wie beispielsweise Aphanomyces euteiches; Ascochyta-Arten, wie beispielsweise Ascochyta lentis; Aspergillus-Arten, wie beispielsweise Aspergillus flavus; Cladosporium-Arten, wie beispielsweise Cladosporium herbarum; Cochliobolus-Arten, wie beispielsweise Cochliobolus sativus (Konidienform: Drechslera, Bipolaris Syn: Helminthosporium); Colletotrichum-Arten, wie beispielsweise Colletotrichum coccodes; Fusarium-Arten, wie beispielsweise Fusarium culmorum; Gibberella-Arten, wie beispielsweise Gibberella zeae; Macrophomina-Arten, wie beispielsweise Macrophomina phaseolina; Microdochium-Arten, wie beispielsweise Microdochium nivale; Monographella-Arten, wie beispielsweise Monographella nivalis; Penicillium-Arten, wie beispielsweise Penicillium expansum; Phoma-Arten, wie beispielsweise Phoma lingam; Phomopsis-Arten, wie beispielsweise Phomopsis sojae; Phytophthora Arten, wie beispielsweise Phytophthora cactorum; Pyrenophora-Arten, wie beispielsweise Pyrenophora graminea; Pyricularia-Arten, wie beispielsweise Pyricularia oryzae; Pythium-Arten, wie beispielsweise Pythium ultimum; Rhizoctonia-Arten, wie beispielsweise Rhizoctonia solani; Rhizopus-Arten, wie beispielsweise Rhizopus oryzae; Sclerotium-Arten, wie beispielsweise Sclerotium rolfsii; Septoria-Arten, wie beispielsweise Septoria nodorum; Typhula-Arten, wie beispielsweise Typhula incarnata; Verticillium-Arten, wie beispielsweise Verticillium dahliae;
Krebserkrankungen, Gallen und Hexenbesen, hervorgerufen durch z.B. Nectria-Arten, wie beispielsweise Nectria galligena;
Welkeerkrankungen hervorgerufen durch z.B. Monilinia-Arten, wie beispielsweise Monilinia laxa;
Deformationen von Blättern, Blüten und Früchten, hervorgerufen durch z.B. Exobasidium-Arten, wie beispielsweise Exobasidium vexans; Taphrina-Arten, wie beispielsweise Taphrina deformans;
Degenerationserkrankungen holziger pflanzen, hervorgerufen durch z.B. Esca-Arten, wie beispielsweise Phaeomoniella chlamydospora, Phaeoacremonium aleophilum oder Fomitiporia mediterranea; Ganoderma-Arten, wie beispielsweise Ganoderma boninense;
Blüten- und Samenerkrankungen, hervorgerufen durch z.B. Botrytis-Arten, wie beispielsweise Botrytis cinerea;
Erkrankungen von Pflanzenknollen, hervorgerufen durch z.B. Rhizoctonia-Arten, wie beispielsweise Rhizoctonia solani; Helminthosporium-Arten, wie beispielsweise Helminthosporium solani;
Erkrankungen, hervorgerufen durch bakterielle Erreger wie z.B. Xanthomonas-Arten, wie beispielsweise Xanthomonas campestris pv. oryzae; Pseudomonas-Arten, wie beispielsweise Pseudomonas syringae pv. lachrymans; Erwinia-Arten, wie beispielsweise Erwinia amylovora;
Bevorzugt können die folgenden Krankheiten von Soja-Bohnen bekämpft werden:
   Pilzkrankheiten an Blättern, Stängeln, Schoten und Samen verursacht durch z.B. Alternaria leaf spot (Altemaria spec. atrans tenuissima), Anthracnose (Colletotrichum gloeosporoides dematium var. truncatum), Brown spot (Septoria glycines), Cercospora leaf spot and blight (Cercospora kikuchii), Choanephora leaf blight (Choanephora infundibulifera trispora (Syn.)), Dactuliophora leaf spot (Dactuliophora glycines), Downy Mildew (Peronospora manshurica), Drechslera blight (Drechslera glycini), Frogeye Leaf spot (Cercospora sojina), Leptosphaerulina Leaf Spot (Leptosphaerulina trifolii), Phyllostica Leaf Spot (Phyllosticta sojaecola), Pod and Stem Blight (Phomopsis sojae), Powdery Mildew (Microsphaera diffusa), Pyrenochaeta Leaf Spot (Pyrenochaeta glycines), Rhizoctonia Aerial, Foliage, and Web Blight (Rhizoctonia solani), Rust (Phakopsora pachyrhizi, Phakopsora meibomiae), Scab (Sphaceloma glycines), Stemphylium Leaf Blight (Stemphylium botryosum), Target Spot (Corynespora cassiicola).

Pilzkrankheiten an Wurzeln und der Stängelbasis verursacht durch z.B. Black Root Rot (Calonectria crotalariae), Charcoal Rot (Macrophomina phaseolina), Fusarium Blight or Wilt, Root Rot, and Pod and Collar Rot (Fusarium oxysporum, Fusarium orthoceras, Fusarium semitectum, Fusarium equiseti), Mycoleptodiscus Root Rot (Mycoleptodiscus terrestris), Neocosmospora (Neocosmopspora vasinfecta), Pod and Stem Blight (Diaporthe phaseolorum), Stem Canker (Diaporthe phaseolorum var. caulivora), Phytophthora Rot (Phytophthora megasperma), Brown Stem Rot (Phialophora gregata), Pythium Rot (Pythium aphanidermatum, Pythium irregulare, Pythium debaryanum, Pythium myriotylum, Pythium ultimum), Rhizoctonia Root Rot, Stem Decay, and Damping-Off (Rhizoctonia solani), Sclerotinia Stem Decay (Sclerotinia sclerotiorum), Sclerotinia Southern Blight (Sclerotinia rolfsii), Thielaviopsis Root Rot (Thielaviopsis basicola).

Die erfindungsgemäßen Wirkstoffe weisen auch eine sehr gute stärkende Wirkung in Pflanzen auf. Sie eignen sich daher zur Mobilisierung pflanzeneigener Abwehrkräfte gegen Befall durch unerwünschte Mikroorganismen.

Unter pflanzenstärkenden (resistenzinduzierenden) Stoffen sind im vorliegenden Zusammenhang solche Substanzen zu verstehen, die in der Lage sind, das Abwehrsystem von Pflanzen so zu stimulieren, dass die behandelten Pflanzen bei nachfolgender Inokulation mit unerwünschten Mikroorganismen weitgehende Resistenz gegen diese Mikroorganismen entfalten.

Unter unerwünschten Mikroorganismen sind im vorliegenden Fall phytopathogene Pilze und Bakterien zu verstehen. Die erfindungsgemäßen Stoffe können also eingesetzt werden, um Pflanzen innerhalb eines gewissen Zeitraumes nach der Behandlung gegen den Befall durch die genannten Schaderreger zu schützen. Der Zeitraum, innerhalb dessen Schutz herbeigeführt wird, erstreckt sich im allgemeinen von 1 bis 10 Tage, vorzugsweise 1 bis 7 Tage nach der Behandlung der Pflanzen mit den Wirkstoffen.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz-und Saatgut, und des Bodens.

Dabei lassen sich die erfindungsgemäßen Wirkstoffe mit besonders gutem Erfolg zur Bekämpfung von Krankheiten im Wein-, Obst-, Kartoffel- und Gemüseanbau, wie beispielsweise insbesondere gegen falsche Mehltaupilze, Oomyceten, wie beispielsweise Phytophthora-, Plasmopara-, Pseudoperonospora- und Pythium-Arten, einsetzen.

Die erfindungsgemäßen Wirkstoffe eignen sich auch zur Steigerung des Ernteertrages. Sie sind außerdem mindertoxisch und weisen eine gute Pflanzenverträglichkeit auf.

Die erfindungsgemäßen Verbindungen können gegebenenfalls in bestimmten Konzentrationen bzw. Aufwandmengen auch als Herbizide, Safener, Wachstumsregulatoren oder Mittel zur Verbesserung der Pflanzeneigenschaften, oder als Mikrobizide, beispielsweise als Fungizide, Antimykotika, Bakterizide, Virizide (einschließlich Mittel gegen Viroide) oder als Mittel gegen MLO (Mycoplasma-like-organism) und RLO (Rickettsia-like-organism) verwendet werden. Sie lassen sich gegebenenfalls auch als Insektizide verwenden. Sie lassen sich gegebenenfalls auch als Zwischen- oder Vorprodukte für die Synthese weiterer Wirkstoffe einsetzen.

Die erfindungsgemäßen Wirkstoffe eignen sich bei guter Pflanzenverträglichkeit, günstiger Warmblütertoxizität und guter Umweltverträglichkeit zum Schutz von Pflanzen und Pflanzenorganen, zur Steigerung der Ernteerträge, Verbesserung der Qualität des Erntegutes in der Landwirtschaft, im Gartenbau, bei der Tierzucht, in Forsten, in Gärten und Freizeiteinrichtungen, im Vorrats- und Materialschutz sowie auf dem Hygienesektor. Sie können vorzugsweise als Pflanzenschutzmittel eingesetzt werden. Sie sind gegen normal sensible und resistente Arten sowie gegen alle oder einzelne Entwicklungsstadien wirksam.

Die erfindungsgemäße Behandlung der Pflanzen und Pflanzenteile mit den Wirkstoffen bzw. Mitteln erfolgt direkt oder durch Einwirkung auf deren Umgebung, Lebensraum oder Lagerraum nach den üblichen Behandlungsmethoden, z.B. durch Tauchen, (Ver-)Spritzen, (Ver-)Sprühen, Berieseln, Verdampfen, Zerstäuben, Vernebeln, (Ver-)Streuen, Verschäumen, Bestreichen, Verstreichen, Gießen (drenchen), Tröpfchenbewässerung und bei Vermehrungsmaterial, insbesondere bei Samen, weiterhin durch Trockenbeizen, Nassbeizen, Schlämmbeizen, Inkrustieren, ein- oder mehrschichtiges Umhüllen usw. Es ist ferner möglich, die Wirkstoffe nach dem Ultra-Low-Volume-Verfahren auszubringen oder die Wirkstoffzubereitung oder den Wirkstoff selbst in den Boden zu injizieren.

Die erfindungsgemäßen Wirkstoffe bzw. Mittel können außerdem im Materialschutz zum Schutz von technischen Materialien gegen Befall und Zerstörung durch unerwünschten Mikroorganismen, wie z.B. Pilzen, eingesetzt werden.

Unter technischen Materialien sind im vorliegenden Zusammenhang nichtlebende Materialien zu verstehen, die für die Verwendung in der Technik zubereitet worden sind. Beispielsweise können technische Materialien, die durch erfindungsgemäße Wirkstoffe vor mikrobieller Veränderung oder Zerstörung geschützt werden sollen, Klebstoffe, Leime, Papier und Karton, Textilien, Leder, Holz, Anstrichmittel und Kunststoffartikel, Kühlschmierstoffe und andere Materialien sein, die von Mikroorganismen befallen oder zersetzt werden können. Im Rahmen der zu schützenden Materialien seien auch Teile von Produktionsanlagen, beispielsweise Kühlwasserkreisläufe, genannt, die durch Vermehrung von Mikroorganismen beeinträchtigt werden können. Im Rahmen der vorliegenden Erfindung seien als technische Materialien vorzugsweise Klebstoffe, Leime, Papiere und Kartone, Leder, Holz, Anstrichmittel, Kühlschmiermittel und Wärmeübertragungsflüssigkeiten genannt, besonders bevorzugt Holz. Die erfindungsgemäßen Wirkstoffe bzw. Mittel können nachteilige Effekte wie Vermodern, Verfall, Ver-, Entfärbung oder Verschimmeln verhindern.

Das erfindungsgemäße Verfahren zum Bekämpfen von unerwünschten Pilzen kann auch zum Schutz von so genannte Storage Goods verwendet werden. Unter "Storage Goods" werden dabei natürliche Substanzen pflanzlichen oder tierischen Ursprungs oder deren Verarbeitungsprodukte, welche der Natur entnommen wurden und für die Langzeitschutz gewünscht ist, verstanden. Storage Goods pflanzlichen Ursprungs, wie z.B. Pflanzen oder Pflanzenteile, wie Stiele, Blätter, Knollen, Samen, Früchte, Körner, können in frisch geerntetem Zustand oder nach Verarbeitung durch (Vor-)Trocknen, Befeuchten, Zerkleinern, Mahlen, Pressen oder Rösten, geschützt werden. Storage Goods umfasst auch Nutzholz, sei es unverarbeitet, wie Bauholz, Stromleitungsmasten und Schranken, oder in Form fertiger Produkte, wie Möbel. Storage Goods tierischen Ursprungs sind beispielsweise Felle, Leder, Pelze und Haare. Die erfindungsgemäßen Wirkstoffe können nachteilige Effekte wie Vermodern, Verfall, Ver-, Entfärbung oder Verschimmeln verhindern.

Als Mikroorganismen, die einen Abbau oder eine Veränderung der technischen Materialien bewirken können, seien beispielsweise Bakterien, Pilze, Hefen, Algen und Schleimorganismen genannt. Vorzugsweise wirken die erfindungsgemäßen Wirkstoffe gegen Pilze, insbesondere Schimmelpilze, Holz verfärbende und Holz zerstörende Pilze (Basidiomyceten) sowie gegen Schleimorganismen und Algen. Es seien beispielsweise Mikroorganismen der folgenden Gattungen genannt: Alternaria, wie Alternaria tenuis; Aspergillus, wie Aspergillus niger; Chaetomium, wie Chaetomium globosum; Coniophora, wie Coniophora puetana; Lentinus, wie Lentinus tigrinus; Penicillium, wie Penicillium glaucum; Polyporus, wie Polyporus versicolor; Aureobasidium, wie Aureobasidium pullulans; Sclerophoma, wie Sclerophoma pityophila; Trichoderma, wie Trichoderma viride; Escherichia, wie Escherichia coli; Pseudomonas, wie Pseudomonas aeruginosa; Staphylococcus, wie Staphylococcus aureus.

Die vorliegende Erfindung betrifft weiterhin ein Mittel zum Bekämpfen unerwünschter Mikroorganismen in und/oder auf Pflanzen oder in und/oder auf Saatgut von Pflanzen, umfassend wenigstens eines der erfindungsgemäßen heterocyclyl substituierten Thiazole. Vorzugsweise handelt es sich um fungizide Mittel, welche landwirtschaftlich verwendbare Hilfsmittel, Solventien, Trägerstoffe, oberflächenaktive Stoffe oder Streckmittel enthalten.

Erfindungsgemäß bedeutet Trägerstoff eine natürliche oder synthetische, organische oder anorganische Substanz, mit welchen die Wirkstoffe zur besseren Anwendbarkeit, v.a. zum Aufbringen auf Pflanzen oder Pflanzenteile oder Saatgut, gemischt oder verbunden sind. Der Trägerstoff, welcher fest oder flüssig sein kann, ist im Allgemeinen inert und sollte in der Landwirtschaft verwendbar sein.

Als feste Trägerstoffe kommen infrage: z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Papier, Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-FettsäureEster, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage nicht-ionische und/oder ionische Stoffe, z.B. aus den Klassen der Alkohol-POE- und/oder POP-Ether, Säure- und/oder POP-POE-Ester, Alkyl-Aryl- und/oder POP- POE-Ether, Fett- und/oder POP- POE-Addukte, POE- und/oder POP-Polyol Derivate, POE- und/oder POP-Sorbitan- oder-Zucker-Addukte, Alky- oder Aryl-Sulfate, Sulfonate und Phosphate oder die entsprechenden PO-Ether-Addukte. Ferner geeignete Oligo- oder Polymere, z.B. ausgehend von vinylischen Monomeren, von Acrylsäure, aus EO und/oder PO allein oder in Verbindung mit z.B. (poly-) Alkoholen oder (poly-) Aminen. Ferner können Einsatz finden Lignin und seine Sulfonsäure-Derivate, einfache und modifizierte Cellulosen, aromatische und/oder aliphatische Sulfonsäuren sowie deren Addukte mit Formaldehyd.

Die Wirkstoffe können in die üblichen Formulierungen überführt werden, wie Lösungen, Emulsionen, Spritzpulver, wasser- und ölbasierte Suspensionen, Pulver, Stäubemittel, Pasten, lösliche Pulver, lösliche Granulate, Streugranulate, Suspensions-Emulsions-Konzentrate, Wirkstoffimprägnierte Naturstoffe, Wirkstoff-imprägnierte synthetische Stoffe, Düngemittel sowie Feinstverkapselungen in polymeren Stoffen.

Die Wirkstoffe können als solche, in Form ihrer Formulierungen oder den daraus bereiteten Anwendungsformen, wie gebrauchsfertige Lösungen, Emulsionen, wasser- oder ölbasierte Suspensionen, Pulver, Spritzpulver, Pasten, lösliche Pulver, Stäubemittel, lösliche Granulate, Streugranulate, Suspensions-Emulsions-Konzentrate, Wirkstoff-imprägnierte Naturstoffe, Wirkstoff-imprägnierte synthetische Stoffe, Düngemittel sowie Feinstverkapselungen in polymeren Stoffen angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Die genannten Formulierungen können in an sich bekannter Weise hergestellt werden, z.B. durch Vermischen der Wirkstoffe mit mindestens einem üblichen Streckmittel, Lösungs- bzw. Verdünnungsmittel, Emulgator, Dispergier- und/oder Binde- oder Fixiermittels, Netzmittel, Wasser-Repellent, gegebenenfalls Sikkative und UV-Stabilisatoren und gegebenenfalls Farbstoffen und Pigmenten, Entschäumer, Konservierungsmittel, sekundäre Verdickungsmittel, Kleber, Gibberelline sowie weiteren Verarbeitungshilfsmitteln.

Die erfindungsgemäßen Mittel umfassen nicht nur Formulierungen, welche bereits anwendungsfertig sind und mit einer geeigneten Apparatur auf die Pflanze oder das Saatgut ausgebracht werden können, sondern auch kommerzielle Konzentrate, welche vor Gebrauch mit Wasser verdünnt werden müssen.

Die erfindungsgemäßen Wirkstoffe können als solche oder in ihren (handelsüblichen) Formulierungen sowie in den aus diesen Formulierungen bereiteten Anwendungsformen in Mischung mit anderen (bekannten) Wirkstoffen, wie Insektiziden, Lockstoffen, Sterilantien, Bakteriziden, Akariziden, Nematiziden, Fungiziden, Wachstumsregulatoren, Herbiziden, Düngemitteln, Safener bzw. Semiochemicals vorliegen.

Als Hilfsstoffe können solche Stoffe Verwendung finden, die geeignet sind, dem Mittel selbst oder und/oder davon abgeleitete Zubereitungen (z.B. Spritzbrühen, Saatgutbeizen) besondere Eigenschaften zu verleihen, wie bestimmte technische Eigenschaften und/oder auch besondere biologische Eigenschaften. Als typische Hilfsmittel kommen in Frage: Streckmittel, Lösemittel und Trägerstoffe.

Als Streckmittel eignen sich z.B. Wasser, polare und unpolare organische chemische Flüssigkeiten z.B. aus den Klassen der aromatischen und nicht-aromatischen Kohlenwasserstoffe (wie Paraffine, Alkylbenzole, Alkylnaphthaline, Chlorbenzole), der Alkohole und Polyole (die ggf. auch substituiert, verethert und/oder verestert sein können), der Ketone (wie Aceton, Cyclohexanon), Ester (auch Fette und Öle) und (poly-)Ether, der einfachen und substituierten Amine, Amide, Lactame (wie N-Alkylpyrrolidone) und Lactone, der Sulfone und Sulfoxide (wie Dimethylsysulfoxid).

Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Im Falle der Benutzung von Wasser als Streckmittel können z.B. auch organische Lösungsmittel als Hilfslösungsmittel verwendet werden. Als flüssige Lösungsmittel kommen im Wesentlichen infrage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser.

Die erfindungsgemäßen Mittel können zusätzlich weitere Bestandteile enthalten, wie z.B. oberflächenaktive Stoffe. Als oberflächenaktive Stoffe kommen Emulgier- und/oder Schaum erzeugende Mittel, Dispergiermittel oder Benetzungsmittel mit ionischen oder nicht-ionischen Eigenschaften oder Mischungen dieser oberflächenaktiven Stoffe infrage. Beispiele hierfür sind Salze von Polyacrylsäure, Salze von Lignosulphonsäure, Salze von Phenolsulphonsäure oder Naphthalinsulphonsäure, Polykondensate von Ethylenoxid mit Fettalkoholen oder mit Fettsäuren oder mit Fettaminen, substituierten Phenolen (vorzugsweise Alkylphenole oder Arylphenole), Salze von Sulphobernsteinsäureestern, Taurinderivate (vorzugsweise Alkyltaurate), Phosphorsäureester von polyethoxylierten Alkoholen oder Phenole, Fettsäureester von Polyolen, und Derivate der Verbindungen enthaltend Sulphate, Sulphonate und Phosphate, z.B. Alkylarylpolyglycolether, Alkylsulfonate, Alkylsulfate, Arylsulfonate, Eiweißhydrolysate, Lignin-Sulfitablaugen und Methylcellulose. Die Anwesenheit einer oberflächenaktiven Substanz ist notwendig, wenn einer der Wirkstoff und/oder einer der inerten Trägerstoffe nicht in Wasser löslich ist und wenn die Anwendung in Wasser erfolgt. Der Anteil an oberflächenaktiven Stoffen liegt zwischen 5 und 40 Gewichtsprozent des erfindungsgemäßen Mittels.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Weitere Additive können Duftstoffe, mineralische oder vegetabile gegebenenfalls modifizierte Öle, Wachse und Nährstoffe (auch Spurennährstoffe), wie Salze von Eisen, Mangan, Bor, Kupfer, Kobalt, Molybdän und Zink sein.

Weiterhin enthalten sein können Stabilisatoren wie Kältestabilisatoren, Konservierungsmittel, Oxidationsschutzmittel, Lichtschutzmittel oder andere die chemische und / oder physikalische Stabilität verbessernde Mittel.

Gegebenenfalls können auch andere zusätzliche Komponenten enthalten sein, z.B. schützende Kolloide, Bindemittel, Klebstoffe, Verdicker, thixotrope Stoffe, Penetrationsförderer, Stabilisatoren, Sequestiermittel, Komplexbildner. Im Allgemeinen können die Wirkstoffe mit jedem festen oder flüssigen Additiv, welches für Formulierungszwecke gewöhnlich verwendet wird, kombiniert werden.

Die Formulierungen enthalten im Allgemeinen zwischen 0,05 und 99 Gew.-%, 0,01 und 98 Gew.-%, vorzugsweise zwischen 0,1 und 95 Gew.-%, besonders bevorzugt zwischen 0,5 und 90 % Wirkstoff, ganz besonders bevorzugt zwischen 10 und 70 Gewichtsprozent.

Die zuvor beschriebenen Formulierungen können in einem erfindungsgemäßen Verfahren zum Bekämpfen unerwünschter Mikroorganismen verwendet werden, bei dem die erfindungsgemäßen heterocyclyl substituierte Thiazole auf die Mikroorganismen und/oder in deren Lebensraum ausgebracht werden.

Die erfindungsgemäßen Wirkstoffe können als solche oder in ihren Formulierungen auch in Mischung mit bekannten Fungiziden, Bakteriziden, Akariziden, Nematiziden oder Insektiziden verwendet werden, um so z.B. das Wirkungsspektrum zu verbreitern oder Resistenzentwicklungen vorzubeugen.

Als Mischpartner kommen zum Beispiel bekannte Fungizide, Insektizide, Akarizide, Nematizide oder auch Bakterizide (siehe auch Pesticide Manual, 13th ed.) infrage.

Auch eine Mischung mit anderen bekannten Wirkstoffen, wie Herbiziden, oder mit Düngemitteln und Wachstumsregulatoren, Safenern bzw. Semiochemicals ist möglich.

Die Anwendung geschieht in einer den Anwendungsformen angepaßten üblichen Weise.

Die Erfindung umfasst weiterhin ein Verfahren zur Behandlung von Saatgut.

Ein weiterer Aspekt der vorliegenden Erfindung betrifft insbesondere ein Saatgut, welches mit wenigstens einem der erfindungsgemäßen heterocyclyl substituierte Thiazole behandelt ist. Die erfindungsgemäßen Saatgüter finden Anwendung in Verfahren zum Schutz von Saatgut vor pflanzenpathogenen Schadpilzen. Bei diesen wird ein mit wenigstens einem erfindungsgemäßen Wirkstoff behandeltes Saatgut verwendet.

Die erfindungsgemäßen Wirkstoffe bzw. Mittel sind auch geeignet für die Behandlung von Saatgut. Ein großer Teil des durch Schadorganismen hervorgerufenen Schadens an Kulturpflanzen wird durch den Befall des Saatguts während der Lagerung oder nach der Aussaat sowie während und nach der Keimung der Pflanze ausgelöst. Diese Phase ist besonders kritisch, weil die Wurzeln und Schösslinge der wachsenden Pflanze besonders empfindlich sind und auch nur eine kleine Schädigung zum Tod der Pflanze führen kann. Es besteht daher ein großes Interesse daran, das Saatgut und die keimende Pflanze durch Einsatz geeigneter Mittel zu schützen.

Die Bekämpfung von pflanzenpathogenen Schadpilzen durch die Behandlung des Saatguts von Pflanzen ist seit langem bekannt und ist Gegenstand ständiger Verbesserungen. Dennoch ergeben sich bei der Behandlung von Saatgut eine Reihe von Problemen, die nicht immer zufrieden stellend gelöst werden können. So ist es erstrebenswert, Verfahren zum Schutz des Saatguts und der keimenden Pflanze zu entwickeln, die das zusätzliche Ausbringen von Pflanzenschutzmitteln nach der Saat oder nach dem Auflaufen der Pflanzen überflüssig machen oder zumindest deutlich verringern. Es ist weiterhin erstrebenswert, die Menge des eingesetzten Wirkstoffs dahingehend zu optimieren, dass das Saatgut und die keimende Pflanze vor dem Befall durch phytopathogene Pilze bestmöglich geschützt wird, ohne jedoch die Pflanze selbst durch den eingesetzten Wirkstoff zu schädigen. Insbesondere sollten Verfahren zur Behandlung von Saatgut auch die intrinsischen fungiziden Eigenschaften transgener Pflanzen einbeziehen, um einen optimalen Schutz des Saatguts und der keimenden Pflanze bei einem minimalen Aufwand an Pflanzenschutzmitteln zu erreichen.

Die vorliegende Erfindung bezieht sich daher auch auf ein Verfahren zum Schutz von Saatgut und keimenden Pflanzen vor dem Befall von tierischen Schädlingen und/oder pflanzenpathogenen Schadpilzen, indem das Saatgut mit einem erfindungsgemäßen Mittel behandelt wird. Die Erfindung bezieht sich ebenfalls auf die Verwendung der erfindungsgemäßen Mittel zur Behandlung von Saatgut zum Schutz des Saatguts und der keimenden Pflanze vor phytopathogenen Pilzen in und/oder auf Pflanzen oder in und/oder auf Saatgut von Pflanzen. Weiterhin bezieht sich die Erfindung auf Saatgut, welches zum Schutz vor phytopathogenen Pilzen mit einem erfindungsgemäßen Mittel behandelt wurde.

Die Bekämpfung von tierischen Schädlingen und/oder pflanzenpathogenen Schadpilzen, die Pflanzen nach dem Auflaufen schädigen, erfolgt in erster Linie durch die Behandlung des Bodens und der oberirdischen Pflanzenteile mit Pflanzenschutzmitteln. Aufgrund der Bedenken hinsichtlich eines möglichen Einflusses der Pflanzenschutzmittel auf die Umwelt und die Gesundheit von Menschen und Tieren gibt es Anstrengungen, die Menge der ausgebrachten Wirkstoffe zu vermindern.

Einer der Vorteile der vorliegenden Erfindung ist es, dass aufgrund der besonderen systemischen Eigenschaften der erfindungsgemäßen Mittel die Behandlung des Saatguts mit diesen Mitteln nicht nur das Saatgut selbst, sondern auch die daraus hervorgehenden Pflanzen nach dem Auflaufen vor tierischen Schädlingen und/oder pflanzenpathogenen Schadpilzen schützt. Auf diese Weise kann die unmittelbare Behandlung der Kultur zum Zeitpunkt der Aussaat oder kurz danach entfallen.

Ebenso ist es als vorteilhaft anzusehen, dass die erfindungsgemäßen Wirkstoffe bzw. Mittel insbesondere auch bei transgenem Saatgut eingesetzt werden können, wobei die aus diesem Saatgut wachsende Pflanze in der Lage ist, ein Protein zu exprimieren, welches gegen Schädlinge wirkt. Durch die Behandlung solchen Saatguts mit den erfindungsgemäßen Wirkstoffen bzw. Mitteln können bereits durch die Expression des beispielsweise insektiziden Proteins bestimmte Schädlinge bekämpft werden. Überraschenderweise kann dabei ein weiterer synergistischer Effekt beobachtet werden, welcher zusätzlich die Effektivität zum Schutz gegen den Schädlingsbefall vergrößert.

Die erfindungsgemäßen Mittel eignen sich zum Schutz von Saatgut jeglicher Pflanzensorte, die in der Landwirtschaft, im Gewächshaus, in Forsten oder im Gartenbau eingesetzt wird. Insbesondere handelt es sich dabei um Saatgut von Getreide (wie Weizen, Gerste, Roggen, Hirse und Hafer), Mais, Baumwolle, Soja, Reis, Kartoffeln, Sonnenblume, Bohne, Kaffee, Rübe (z.B. Zuckerrübe und Futterrübe), Erdnuss, Gemüse (wie Tomate, Gurke, Zwiebeln und Salat), Rasen und Zierpflanzen. Besondere Bedeutung kommt der Behandlung des Saatguts von Getreide (wie Weizen, Gerste, Roggen und Hafer), Mais und Reis zu.

Wie auch weiter unten beschrieben, ist die Behandlung von transgenem Saatgut mit den erfindungsgemäßen Wirkstoffen bzw. Mitteln von besonderer Bedeutung. Dies betrifft das Saatgut von Pflanzen, die wenigstens ein heterologes Gen enthalten, das die Expression eines Polypeptids oder Proteins mit insektiziden Eigenschaften ermöglicht. Das heterologe Gen in transgenem Saatgut kann z.B. aus Mikroorganismen der Arten Bacillus, Rhizobium, Pseudomonas, Serratia, Trichoderma, Clavibacter, Glomus or Gliocladium stammen. Bevorzugt stammt dieses heterologe Gen aus Bacillus sp., wobei das Genprodukt eine Wirkung gegen den Maiszünsler (European corn borer) und/oder Western Corn Rootworm besitzt. Besonders bevorzugt stammt das heterologe Gen aus Bacillus thuringiensis.

Im Rahmen der vorliegenden Erfindung wird das erfindungsgemäßes Mittel alleine oder in einer geeigneten Formulierung auf das Saatgut aufgebracht. Vorzugsweise wird das Saatgut in einem Zustand behandelt, in dem so stabil ist, dass keine Schäden bei der Behandlung auftreten. Im Allgemeinen kann die Behandlung des Saatguts zu jedem Zeitpunkt zwischen der Ernte und der Aussaat erfolgen. Üblicherweise wird Saatgut verwendet, das von der Pflanze getrennt und von Kolben, Schalen, Stängeln, Hülle, Wolle oder Fruchtfleisch befreit wurde. So kann zum Beispiel Saatgut verwendet werden, das geerntet, gereinigt und bis zu einem Feuchtigkeitsgehalt von unter 15 Gew.-% getrocknet wurde. Alternativ kann auch Saatgut verwendet werden, das nach dem Trocknen z.B. mit Wasser behandelt und dann erneut getrocknet wurde.

Im Allgemeinen muss bei der Behandlung des Saatguts darauf geachtet werden, dass die Menge des auf das Saatgut aufgebrachten erfindungsgemäßen Mittels und/oder weiterer Zusatzstoffe so gewählt wird, dass die Keimung des Saatguts nicht beeinträchtigt bzw. die daraus hervorgehende Pflanze nicht geschädigt wird. Dies ist vor allem bei Wirkstoffen zu beachten, die in bestimmten Aufwandmengen phytotoxische Effekte zeigen können.

Die erfindungsgemäßen Mittel können unmittelbar aufgebracht werden, also ohne weitere Komponenten zu enthalten und ohne verdünnt worden zu sein. In der Regel ist es vorzuziehen, die Mittel in Form einer geeigneten Formulierung auf das Saatgut aufzubringen. Geeignete Formulierungen und Verfahren für die Saatgutbehandlung sind dem Fachmann bekannt und werden z.B. in den folgenden Dokumenten beschrieben: US 4,272,417 A, US 4,245,432 A, US 4,808,430 A, US 5,876,739 A, US 2003/0176428 A1, WO 2002/080675 A1, WO 2002/028186 A2.

Die erfindungsgemäß verwendbaren Wirkstoffe können in die üblichen Beizmittel-Formulierungen überführt werden, wie Lösungen, Emulsionen, Suspensionen, Pulver, Schäume, Slurries oder andere Hüllmassen für Saatgut, sowie ULV-Formulierungen.

Diese Formulierungen werden in bekannter Weise hergestellt, indem man die Wirkstoffe oder Wirkstoffkombinationen mit üblichen Zusatzstoffen vermischt, wie zum Beispiel übliche Streckmittel sowie Lösungs- oder Verdünnungsmittel, Farbstoffe, Netzmittel, Dispergiermittel, Emulgatoren, Entschäumer, Konservierungsmittel, sekundäre Verdickungsmittel, Kleber, Gibberelline und auch Wasser.

Als Farbstoffe, die in den erfindungsgemäß verwendbaren Beizmittel-Formulierungen enthalten sein können, kommen alle für derartige Zwecke üblichen Farbstoffe in Betracht. Dabei sind sowohl in Wasser wenig lösliche Pigmente als auch in Wasser lösliche Farbstoffe verwendbar. Als Beispiele genannt seien die unter den Bezeichnungen Rhodamin B, C.I. Pigment Red 112 und C.I. Solvent Red 1 bekannten Farbstoffe.

Als Netzmittel, die in den erfindungsgemäß verwendbaren Beizmittel-Formulierungen enthalten sein können, kommen alle zur Formulierung von agrochemischen Wirkstoffen üblichen, die Benetzung fördernden Stoffe in Frage. Vorzugsweise verwendbar sind Alkylnaphthalin-Sulfonate, wie Diisopropyl- oder Diisobutyl-naphthalin-Sulfonate.

Als Dispergiermittel und/oder Emulgatoren, die in den erfindungsgemäß verwendbaren Beizmittel-Formulierungen enthalten sein können, kommen alle zur Formulierung von agrochemischen Wirkstoffen üblichen nichtionischen, anionischen und kationischen Dispergiermittel in Betracht. Vorzugsweise verwendbar sind nichtionische oder anionische Dispergiermittel oder Gemische von nichtionischen oder anionischen Dispergiermitteln. Als geeignete nichtionische Dispergiermittel sind insbesondere Ethylenoxid-Propylenoxid Blockpolymere, Alkylphenolpolyglykolether sowie Tristryrylphenolpolyglykolether und deren phosphatierte oder sulfatierte Derivate zu nennen. Geeignete anionische Dispergiermittel sind insbesondere Ligninsulfonate, Polyacrylsäuresalze und Arylsulfonat-Formaldehydkondensate.

Als Entschäumer können in den erfindungsgemäß verwendbaren Beizmittel-Formulierungen alle zur Formulierung von agrochemischen Wirkstoffen üblichen schaumhemmenden Stoffe enthalten sein. Vorzugsweise verwendbar sind Silikonentschäumer und Magnesiumstearat.

Als Konservierungsmittel können in den erfindungsgemäß verwendbaren Beizmittel-Formulierungen alle für derartige Zwecke in agrochemischen Mitteln einsetzbaren Stoffe vorhanden sein. Beispielhaft genannt seien Dichlorophen und Benzylalkoholhemiformal.

Als sekundäre Verdickungsmittel, die in den erfindungsgemäß verwendbaren Beizmittel-Formulierungen enthalten sein können, kommen alle für derartige Zwecke in agrochemischen Mitteln einsetzbaren Stoffe in Frage. Vorzugsweise in Betracht kommen Cellulosederivate, Acrylsäurederivate, Xanthan, modifizierte Tone und hochdisperse Kieselsäure.

Als Kleber, die in den erfindungsgemäß verwendbaren Beizmittel-Formulierungen enthalten sein können, kommen alle üblichen in Beizmitteln einsetzbaren Bindemittel in Frage. Vorzugsweise genannt seien Polyvinylpyrrolidon, Polyvinylacetat, Polyvinylalkohol und Tylose.

Als Gibberelline, die in den erfindungsgemäß verwendbaren Beizmittel-Formulierungen enthalten sein können, kommen vorzugsweise die Gibberelline A1, A3 (= Gibberellinsäure), A4 und A7 infrage, besonders bevorzugt verwendet man die Gibberellinsäure. Die Gibberelline sind bekannt (vgl. R. Wegler "Chemie der Pflanzenschutz- und Schädlingsbekämpfungsmittel", Bd. 2, Springer Verlag, 1970, S. 401-412).

Die erfindungsgemäß verwendbaren Beizmittel-Formulierungen können entweder direkt oder nach vorherigem Verdünnen mit Wasser zur Behandlung von Saatgut der verschiedensten Art eingesetzt werden. So lassen sich die Konzentrate oder die daraus durch Verdünnen mit Wasser erhältlichen Zubereitungen einsetzen zur Beizung des Saatgutes von Getreide, wie Weizen, Gerste, Roggen, Hafer und Triticale, sowie des Saatgutes von Mais, Reis, Raps, Erbsen, Bohnen, Baumwolle, Sonnenblumen und Rüben oder auch von Gemüsesaatgut der verschiedensten Natur. Die erfindungsgemäß verwendbaren Beizmittel-Formulierungen oder deren verdünnte Zubereitungen können auch zum Beizen von Saatgut transgener Pflanzen eingesetzt werden. Dabei können im Zusammenwirken mit den durch Expression gebildeten Substanzen auch zusätzliche synergistische Effekte auftreten.

Zur Behandlung von Saatgut mit den erfindungsgemäß verwendbaren Beizmittel-Formulierungen oder den daraus durch Zugabe von Wasser hergestellten Zubereitungen kommen alle üblicherweise für die Beizung einsetzbaren Mischgeräte in Betracht. Im einzelnen geht man bei der Beizung so vor, dass man das Saatgut in einen Mischer gibt, die jeweils gewünschte Menge an Beizmittel-Formulierungen entweder als solche oder nach vorherigem Verdünnen mit Wasser hinzufügt und bis zur gleichmäßigen Verteilung der Formulierung auf dem Saatgut mischt. Gegebenenfalls schließt sich ein Trocknungsvorgang an.

Die Aufwandmenge an den erfindungsgemäß verwendbaren Beizmittel-Formulierungen kann innerhalb eines größeren Bereiches variiert werden. Sie richtet sich nach dem jeweiligen Gehalt der Wirkstoffe in den Formulierungen und nach dem Saatgut. Die Aufwandmengen an Wirkstoffkombination liegen im Allgemeinen zwischen 0,001 und 50 g pro Kilogramm Saatgut, vorzugsweise zwischen 0,01 und 15 g pro Kilogramm Saatgut.

Darüber hinaus weisen die erfindungsgemäßen Verbindungen der Formel **(I)** auch sehr gute antimykotische Wirkungen auf. Sie besitzen ein sehr breites antimykotisches Wirkungsspektrum, insbesondere gegen Dermatophyten und Sprosspilze, Schimmel und diphasische Pilze (z.B. gegen Candida-Spezies wie Candida albicans, Candida glabrata) sowie Epidermophyton floccosum, Aspergillus-Spezies wie Aspergillus niger und Aspergillus fumigatus, Trichophyton-Spezies wie Trichophyton mentagrophytes, Microsporon-Spezies wie Microsporon canis und audouinii. Die Aufzählung dieser Pilze stellt keinesfalls eine Beschränkung des erfassbaren mykotischen Spektrums dar, sondern hat nur erläuternden Charakter.

Die erfindungsgemäßen Wirkstoffe der Formel **(I)** können daher sowohl in medizinischen als auch in nicht-medizinischen Anwendungen eingesetzt werden.

Die Wirkstoffe können als solche, in Form ihrer Formulierungen oder den daraus bereiteten Anwendungsformen, wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Beim Einsatz der erfindungsgemäßen Wirkstoffe als Fungizide können die Aufwandmengen je nach Applikationsart innerhalb eines größeren Bereiches variiert werden. Die Aufwandmenge der erfindungsgemäßen Wirkstoffe beträgt
- bei der Behandlung von Pflanzenteilen, z.B. Blättern: von 0,1 bis 10 000 g/ha, bevorzugt von 10 bis 1 000 g/ha, besonders bevorzugt von 50 bis 300g/ha (bei Anwendung durch Gießen oder Tropfen kann die Aufwandmenge sogar verringert werden, vor allem wenn inerte Substrate wie Steinwolle oder Perlit verwendet werden);
- bei der Saatgutbehandlung: von 2 bis 200 g pro 100 kg Saatgut, bevorzugt von 3 bis 150 g pro 100 kg Saatgut, besonders bevorzugt von 2,5 bis 25 g pro 100 kg Saatgut, ganz besonders bevorzugt von 2,5 bis 12,5 g pro 100 kg Saatgut;
- bei der Bodenbehandlung: von 0,1 bis 10 000 g/ha, bevorzugt von 1 bis 5 000 g/ha.

Diese Aufwandmengen seien nur beispielhaft und nicht limitierend im Sinne der Erfindung genannt.

Die Anwendung der erfindungsgemäßen Wirkstoffe geschieht im Veterinärsektor und bei der Tierhaltung in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injektionen (intramuskulär, subcutan, intravenös, intraperitonal u.a.), Implantate, durch nasale Applikation, durch dermale Anwendung in Form beispielsweise des Tauchens oder Badens (Dippen), Sprühens (Spray), Aufgießens (Pour-on und Spot-on), des Waschens, des Einpuderns sowie mit Hilfe von wirkstoffhaltigen Formkörpern, wie Halsbändern, Ohrmarken, Schwanzmarken, Gliedmaßenbändern, Halftern, Markierungsvorrichtungen usw.

Bei der Anwendung für Vieh, Geflügel, Haustiere etc. kann man die Wirkstoffe der Formel (I) als Formulierungen (beispielsweise Pulver, Emulsionen, fließfähige Mittel), die die Wirkstoffe in einer Menge von 1 bis 80 Gew.-% enthalten, direkt oder nach 100 bis 10 000-facher Verdünnung anwenden oder sie als chemisches Bad verwenden.

Die anwendungsfertigen Mittel können gegebenenfalls noch weitere Insektizide und gegebenenfalls noch ein oder mehrere Fungizide enthalten.

Hinsichtlich möglicher zusätzlicher Zumischpartner sei auf die oben genannten Insektizide und Fungizide verwiesen.

Zugleich können die erfindungsgemäßen Verbindungen zum Schutz vor Bewuchs von Gegenständen, insbesondere von Schiffskörpern, Sieben, Netzen, Bauwerken, Kaianlagen und Signalanlagen, welche mit See- oder Brackwasser in Verbindung kommen, eingesetzt werden.

Weiter können die erfindungsgemäßen Verbindungen allein oder in Kombinationen mit anderen Wirkstoffen als Antifouling-Mittel eingesetzt werden.

Das erfindungsgemäße Behandlungsverfahren kann für die Behandlung von genetisch modifizierten Organismen (GMOs), z. B. Pflanzen oder Samen, verwendet werden. Genetisch modifizierte Pflanzen (oder transgene Pflanzen) sind Pflanzen, bei denen ein heterologes Gen stabil in das Genom integriert worden ist. Der Begriff "heterologes Gen" bedeutet im wesentlichen ein Gen, das außerhalb der Pflanze bereitgestellt oder assembliert wird und das bei Einführung in das Zellkerngenom, das Chloroplastengenom oder das Hypochondriengenom der transformierten Pflanze dadurch neue oder verbesserte agronomische oder sonstige Eigenschaften verleiht, daß es ein interessierendes Protein oder Polypeptid exprimiert oder daß es ein anderes Gen, das in der Pflanze vorliegt bzw. andere Gene, die in der Pflanze vorliegen, herunterreguliert oder abschaltet (zum Beispiel mittels Antisense-Technologie, Cosuppressionstechnologie oder RNAi-Technologie [RNA Interference]). Ein heterologes Gen, das im Genom vorliegt, wird ebenfalls als Transgen bezeichnet. Ein Transgen, das durch sein spezifisches Vorliegen im Pflanzengenom definiert ist, wird als Transformations- bzw. transgenes Event bezeichnet.

In Abhängigkeit von den Pflanzenarten oder Pflanzensorten, ihrem Standort und ihren Wachstumsbedingungen (Böden, Klima, Vegetationsperiode, Ernährung) kann die erfindungsgemäße Behandlung auch zu überadditiven ("synergistischen") Effekten führten. So sind zum Beispiel die folgenden Effekte möglich, die über die eigentlich zu erwartenden Effekte hinausgehen: verringerte Aufwandmengen und/oder erweitertes Wirkungsspektrum und/oder erhöhte Wirksamkeit der Wirkstoffe und Zusammensetzungen, die erfindungsgemäß eingesetzt werden können, besseres Pflanzenwachstum, erhöhte Toleranz gegenüber hohen oder niedrigen Temperaturen, erhöhte Toleranz gegenüber Trockenheit oder Wasser- oder Bodensalzgehalt, erhöhte Blühleistung, Ernteerleichterung, Reifebeschleunigung, höhere Erträge, größere Früchte, größere Pflanzenhöhe, intensiver grüne Farbe des Blatts, frühere Blüte, höhere Qualität und/oder höherer Nährwert der Ernteprodukte, höhere Zuckerkonzentration in den Früchten, bessere Lagerfähigkeit und/oder Verarbeitbarkeit der Ernteprodukte.

In gewissen Aufwandmengen können die erfindungsgemäßen Wirkstoffkombinationen auch eine stärkende Wirkung auf Pflanzen ausüben. Sie eignen sich daher für die Mobilisierung des pflanzlichen Abwehrsystems gegen Angriff durch unerwünschte phytopathogene Pilze und/oder Mikroorganismen und/oder Viren. Dies kann gegebenenfalls einer der Gründe für die erhöhte Wirksamkeit der erfindungsgemäßen Kombinationen sein, zum Beispiel gegen Pilze. Pflanzenstärkende (resistenzinduzierende) Substanzen sollen im vorliegenden Zusammenhang auch solche Substanzen oder Substanzkombinationen bedeuten, die fähig sind, das pflanzliche Abwehrsystem so zu stimulieren, daß die behandelten Pflanzen, wenn sie im Anschluß daran mit unerwünschten phytopathogenen Pilzen und/oder Mikroorganismen und/oder Viren inokkuliert werde, einen beträchtlichen Resistenzgrad gegen diese unerwünschten phytopathogenen Pilze und/oder Mikroorganismen und/oder Viren aufweisen. Im vorliegenden Fall versteht man unter unerwünschten phytopathogenen Pilzen und/oder Mikroorganismen und/oder Viren phytopathogene Pilze, Bakterien und Viren. Die erfindungsgemäßen Substanzen lassen sich daher zum Schutz von Pflanzen gegen Angriff durch die erwähnten Pathogene innerhalb eines gewissen Zeitraums nach der Behandlung einsetzen. Der Zeitraum, über den eine Schutzwirkung erzielt wird, erstreckt sich im allgemeinen von 1 bis 10 Tagen, vorzugsweise 1 bis 7 Tagen, nach der Behandlung der Pflanzen mit den Wirkstoffen.

Zu Pflanzen und Pflanzensorten, die vorzugsweise erfindungsgemäß behandelt werden, zählen alle Pflanzen, die über Erbgut verfügen, das diesen Pflanzen besonders vorteilhafte, nützliche Merkmale verleiht (egal, ob dies durch Züchtung und/oder Biotechnologie erzielt wurde).

Pflanzen und Pflanzensorten, die ebenfalls vorzugsweise erfindungsgemäß behandelt werden, sind gegen einen oder mehrere biotische Streßfaktoren resistent, d. h. diese Pflanzen weisen eine verbesserte Abwehr gegen tierische und mikrobielle Schädlinge wie Nematoden, Insekten, Milben, phytopathogene Pilze, Bakterien, Viren und/oder Viroide auf.

Pflanzen und Pflanzensorten, die ebenfalls erfindungsgemäß behandelt werden können, sind solche Pflanzen, die gegen einen oder mehrere abiotische Streßfaktoren resistent sind. Zu den abiotischen Streßbedingungen können zum Beispiel Dürre, Kälte- und Hitzebedingungen, osmotischer Streß, Staunässe, erhöhter Bodensalzgehalt, erhöhtes Ausgesetztsein an Mineralien, Ozonbedingungen, Starklichtbedingungen, beschränkte Verfügbarkeit von Stickstoffnährstoffen, beschränkte Verfügbarkeit von Phosphornährstoffen oder Vermeidung von Schatten zählen.

Pflanzen und Pflanzensorten, die ebenfalls erfindungsgemäß behandelt werden können, sind solche Pflanzen, die durch erhöhte Ertragseigenschaften gekennzeichnet sind. Ein erhöhter Ertrag kann bei diesen Pflanzen z. B. auf verbesserter Pflanzenphysiologie, verbessertem Pflanzenwuchs und verbesserter Pflanzenentwicklung, wie Wasserverwertungseffizienz, Wasserhalteeffizienz, verbesserter Stickstoffverwertung, erhöhter Kohlenstoffassimilation, verbesserter Photosynthese, verstärkter Keimkraft und beschleunigter Abreife beruhen. Der Ertrag kann weiterhin durch eine verbesserte Pflanzenarchitektur (unter Streß- und nicht-Streß-Bedingungen) beeinflußt werden, darunter frühe Blüte, Kontrolle der Blüte für die Produktion von Hybridsaatgut, Keimpflanzenwüchsigkeit, Pflanzengröße, Internodienzahl und -abstand, Wurzelwachstum, Samengröße, Fruchtgröße, Schotengröße, Schoten- oder Ährenzahl, Anzahl der Samen pro Schote oder Ähre, Samenmasse, verstärkte Samenfüllung, verringerter Samenausfall, verringertes Schotenplatzen sowie Standfestigkeit. Zu weiteren Ertragsmerkmalen zählen Samenzusammensetzung wie Kohlenhydratgehalt, Proteingehalt, Ölgehalt und Ölzusammensetzung, Nährwert, Verringerung der nährwidrigen Verbindungen, verbesserte Verarbeitbarkeit und verbesserte Lagerfähigkeit.

Pflanzen, die erfindungsgemäß behandelt werden können, sind Hybridpflanzen, die bereits die Eigenschaften der Heterosis bzw. des Hybrideffekts exprimieren, was im allgemeinen zu höherem Ertrag, höherer Wüchsigkeit, besserer Gesundheit und besserer Resistenz gegen biotische und abiotische Streßfaktoren führt. Solche Pflanzen werden typischerweise dadurch erzeugt, daß man eine ingezüchtete pollensterile Elternlinie (den weiblichen Kreuzungspartner) mit einer anderen ingezüchteten pollenfertilen Elternlinie (dem männlichen Kreuzungspartner) kreuzt. Das Hybridsaatgut wird typischerweise von den pollensterilen Pflanzen geerntet und an Vermehrer verkauft. Pollensterile Pflanzen können manchmal (z. B. beim Mais) durch Entfahnen (d. h. mechanischem Entfernen der männlichen Geschlechtsorgane bzw. der männlichen Blüten), produziert werden; es ist jedoch üblicher, daß die Pollensterilität auf genetischen Determinanten im Pflanzengenom beruht. In diesem Fall, insbesondere dann, wenn es sich bei dem gewünschten Produkt, da man von den Hybridpflanzen ernten will, um die Samen handelt, ist es üblicherweise günstig, sicherzustellen, daß die Pollenfertilität in Hybridpflanzen, die die für die Pollensterilität verantwortlichen genetischen Determinanten enthalten, völlig restoriert wird. Dies kann erreicht werden, indem sichergestellt wird, daß die männlichen Kreuzungspartner entsprechende Fertilitätsrestorergene besitzen, die in der Lage sind, die Pollenfertilität in Hybridpflanzen, die die genetischen Determinanten, die für die Pollensterilität verantwortlich sind, enthalten, zu restorieren. Genetische Determinanten für Pollensterilität können im Cytoplasma lokalisiert sein. Beispiele für cytoplasmatische Pollensterilität (CMS) wurden zum Beispiel für Brassica-Arten beschrieben. Genetische Determinanten für Pollensterilität können jedoch auch im Zellkerngenom lokalisiert sein. Pollensterile Pflanzen können auch mit Methoden der pflanzlichen Biotechnologie, wie Gentechnik, erhalten werden. Ein besonders günstiges Mittel zur Erzeugung von pollensterilen Pflanzen ist in WO 89/10396 beschrieben, wobei zum Beispiel eine Ribonuklease wie eine Barnase selektiv in den Tapetumzellen in den Staubblättern exprimiert wird. Die Fertilität kann dann durch Expression eines Ribonukleasehemmers wie Barstar in den Tapetumzellen restoriert werden.

Pflanzen oder Pflanzensorten (die mit Methoden der Pflanzenbiotechnologie, wie der Gentechnik, erhalten werden), die erfindungsgemäß behandelt werden können, sind herbizidtolerante Pflanzen, d. h. Pflanzen, die gegenüber einem oder mehreren vorgegebenen Herbiziden tolerant gemacht worden sind. Solche Pflanzen können entweder durch genetische Transformation oder durch Selektion von Pflanzen, die eine Mutation enthalten, die solch eine Herbizidtoleranz verleiht, erhalten werden.

Herbizidtolerante Pflanzen sind zum Beispiel glyphosatetolerante Pflanzen, d. h. Pflanzen, die gegenüber dem Herbizid Glyphosate oder dessen Salzen tolerant gemacht worden sind. So können zum Beispiel glyphosatetolerante Pflanzen durch Transformation der Pflanze mit einem Gen, das für das Enzym 5-Enolpyruvylshikimat-3-phosphatsynthase (EPSPS) kodiert, erhalten werden. Beispiele für solche EPSPS-Gene sind das AroA-Gen (Mutante CT7) des Bakterium *Salmonella typhimurium,* das CP4-Gen des Bakteriums *Agrobacterium sp*., die Gene, die für eine EPSPS aus der Petunie, für eine EPSPS aus der Tomate oder für eine EPSPS aus Eleusine kodieren. Es kann sich auch um eine mutierte EPSPS handeln. Glyphosatetolerante Pflanzen können auch dadurch erhalten werden, daß man ein Gen exprimiert, das für ein Glyphosate-Oxidoreduktase-Enzym kodiert. Glyphosatetolerante Pflanzen können auch dadurch erhalten werden, daß man ein Gen exprimiert, das für ein Glyphosate-acetyltransferase-Enzym kodiert. Glyphosatetolerante Pflanzen können auch dadurch erhalten werden, daß man Pflanzen, die natürlich vorkommende Mutationen der oben erwähnten Gene selektiert.

Sonstige herbizidresistente Pflanzen sind zum Beispiel Pflanzen, die gegenüber Herbiziden, die das Enzym Glutaminsynthase hemmen, wie Bialaphos, Phosphinotricin oder Glufosinate, tolerant gemacht worden sind. Solche Pflanzen können dadurch erhalten werden, daß man ein Enzym exprimiert, das das Herbizid oder eine Mutante des Enzyms Glutaminsynthase, das gegenüber Hemmung resistent ist, entgiftet. Solch ein wirksames entgiftendes Enzym ist zum Beispiel ein Enzym, das für ein Phosphinotricin-acetyltransferase kodiert (wie zum Beispiel das bar- oder pat-Protein aus Streptomyces-Arten). Pflanzen, die eine exogene Phosphinotricin-acetyltransferase exprimieren, sind beschrieben.

Weitere herbizidtolerante Pflanzen sind auch Pflanzen, die gegenüber den Herbiziden, die das Enzym Hydroxyphenylpyruvatdioxygenase (HPPD) hemmen, tolerant gemacht worden sind. Bei den Hydroxyphenylpyruvatdioxygenasen handelt es sich um Enzyme, die die Reaktion, in der para-Hydroxyphenylpyruvat (HPP) zu Homogentisat umgesetzt wird, katalysieren. Pflanzen, die gegenüber HPPD-Hemmern tolerant sind, können mit einem Gen, das für ein natürlich vorkommendes resistentes HPPD-Enzym kodiert, oder einem Gen, das für ein mutiertes HPPD-Enzym kodiert, transformiert werden. Eine Toleranz gegenüber HPPD-Hemmern kann auch dadurch erzielt werden, daß man Pflanzen mit Genen transformiert, die für gewisse Enzyme kodieren, die die Bildung von Homogentisat trotz Hemmung des nativen HPPD-Enzyms durch den HPPD-Hemmer ermöglichen. Die Toleranz von Pflanzen gegenüber HPPD-Hemmern kann auch dadurch verbessert werden, daß man Pflanzen zusätzlich zu einem Gen, das für ein HPPDtolerantes Enzym kodiert, mit einem Gen transformiert, das für ein Prephenatdehydrogenase-Enzym kodiert.

Weitere herbizidresistente Pflanzen sind Pflanzen, die gegenüber Acetolactatsynthase (ALS)-Hemmern tolerant gemacht worden sind. Zu bekannten ALS-Hemmern zählen zum Beispiel Sulfonylharnstoff, Imidazolinon, Triazolopyrimidine, Pyrimidinyloxy(thio)benzoate und/oder Sulfonylaminocarbonyltriazolinon-Herbizide. Es ist bekannt, daß verschiedene Mutationen im Enzym ALS (auch als Acetohydroxysäure-Synthase, AHAS, bekannt) eine Toleranz gegenüber unterschiedlichen Herbiziden bzw. Gruppen von Herbiziden verleihen. Die Herstellung von sulfonylharnstofftoleranten Pflanzen und imidazolinontoleranten Pflanzen ist in der internationalen Veröffentlichung WO 1996/033270 beschrieben. Weitere sulfonylharnstoff und imidazolinontolerante Pflanzen sind auch in z.B. WO 2007/024782 beschrieben.

Weitere Pflanzen, die gegenüber Imidazolinon und/oder Sulfonylharnstoff tolerant sind, können durch induzierte Mutagenese, Selektion in Zellkulturen in Gegenwart des Herbizids oder durch Mutationszüchtung erhalten werden.

Pflanzen oder Pflanzensorten (die nach Methoden der pflanzlichen Biotechnologie, wie der Gentechnik, erhalten wurden), die ebenfalls erfindungsgemäß behandelt werden können, sind insektenresistente transgene Pflanzen, d.h. Pflanzen, die gegen Befall mit gewissen Zielinsekten resistent gemacht wurden. Solche Pflanzen können durch genetische Transformation oder durch Selektion von Pflanzen, die eine Mutation enthalten, die solch eine Insektenresistenz verleiht, erhalten werden.

Der Begriff "insektenresistente transgene Pflanze" umfaßt im vorliegenden Zusammenhang jegliche Pflanze, die mindestens ein Transgen enthält, das eine Kodiersequenz umfaßt, die für folgendes kodiert:
1) ein insektizides Kristallprotein aus *Bacillus thuringiensis* oder einen insektiziden Teil davon, wie die insektiziden Kristallproteine, die online bei: http://www.lifesci.sussex.ac.uk/Home/Neil Crickmore/Bt/ beschrieben sind, zusammengestellt wurden, oder insektizide Teile davon, z.B. Proteine der Cry-Proteinklassen Cry1Ab, Cry1Ac, Cry1F, Cry2Ab, Cry3Ae oder Cry3Bb oder insektizide Teile davon; oder
2) ein Kristallprotein aus *Bacillus thuringiensis* oder einen Teil davon, der in Gegenwart eines zweiten, anderen Kristallproteins als *Bacillus thuringiensis* oder eines Teils davon insektizid wirkt, wie das binäre Toxin, das aus den Kristallproteinen Cy34 und Cy35 besteht; oder
3) ein insektizides Hybridprotein, das Teile von zwei unterschiedlichen insektiziden Kristallproteinen aus *Bacillus thuringiensis* umfaßt, wie zum Beispiel ein Hybrid aus den Proteinen von 1) oben oder ein Hybrid aus den Proteinen von 2) oben, z. B. das Protein Cry1A.105, das von dem Mais-Event MON98034 produziert wird (WO 2007/027777); oder
4) ein Protein gemäß einem der Punkte 1) bis 3) oben, in dem einige, insbesondere 1 bis 10, Aminosäuren durch eine andere Aminosäure ersetzt wurden, um eine höhere insektizide Wirksamkeit gegenüber einer Zielinsektenart zu erzielen und/oder um das Spektrum der entsprechenden Zielinsektenarten zu erweitern und/oder wegen Veränderungen, die in die Kodier- DNA während der Klonierung oder Transformation induziert wurden, wie das Protein Cry3Bb1 in Mais-Events MON863 oder MON88017 oder das Protein Cry3A im Mais-Event MIR 604;
5) ein insektizides sezerniertes Protein aus *Bacillus thuringiensis* oder *Bacillus cereus* oder einen insektiziden Teil davon, wie die vegetativ wirkenden insektentoxischen Proteine (vegetative insekticidal proteins, VIP), die unter http://www.lifesci.sussex.ac.uk/Home/Neil_CrickmoreBbvip.html angeführt sind, z. B. Proteine der Proteinklasse VIP3Aa; oder
6) ein sezerniertes Protein aus *Bacillus thuringiensis* oder *Bacillus cereus*, das in Gegenwart eines zweiten sezernierten Proteins aus *Bacillus thuringiensis* oder *B. cereus* insektizid wirkt, wie das binäre Toxin, das aus den Proteinen VIP1A und VIP2A besteht.
7) ein insektizides Hybridprotein, das Teile von verschiedenen sezernierten Proteinen von *Bacillus thuringiensis* oder *Bacillus cereus* umfaßt, wie ein Hybrid der Proteine von 1) oder ein Hybrid der Proteine von 2) oben; oder
8) ein Protein gemäß einem der Punkte 1) bis 3) oben, in dem einige, insbesondere 1 bis 10, Aminosäuren durch eine andere Aminosäure ersetzt wurden, um eine höhere insektizide Wirksamkeit gegenüber einer Zielinsektenart zu erzielen und/oder um das Spektrum der entsprechenden Zielinsektenarten zu erweitern und/oder wegen Veränderungen, die in die Kodier- DNA während der Klonierung oder Transformation induziert wurden (wobei die Kodierung für ein insektizides Protein erhalten bleibt), wie das Protein VIP3Aa im Baumwoll-Event COT 102.

Natürlich zählt zu den insektenresistenten transgenen Pflanzen im vorliegenden Zusammenhang auch jegliche Pflanze, die eine Kombination von Genen umfaßt, die für die Proteine von einer der oben genannten Klassen 1 bis 8 kodieren. In einer Ausführungsform enthält eine insektenresistente Pflanze mehr als ein Transgen, das für ein Protein nach einer der oben genannten 1 bis 8 kodiert, um das Spektrum der entsprechenden Zielinsektenarten zu erweitern oder um die Entwicklung einer Resistenz der Insekten gegen die Pflanzen dadurch hinauszuzögern, daß man verschiedene Proteine einsetzt, die für dieselbe Zielinsektenart insektizid sind, jedoch eine unterschiedliche Wirkungsweise, wie Bindung an unterschiedliche Rezeptorbindungsstellen im Insekt, aufweisen.

Pflanzen oder Pflanzensorten (die nach Methoden der pflanzlichen Biotechnologie, wie der Gentechnik, erhalten wurden), die ebenfalls erfindungsgemäß behandelt werden können, sind gegenüber abiotischen Streßfaktoren tolerant. Solche Pflanzen können durch genetische Transformation oder durch Selektion von Pflanzen, die eine Mutation enthalten, die solch eine Streßresistenz verleiht, erhalten werden. Zu besonders nützlichen Pflanzen mit Streßtoleranz zählen folgende:
a. Pflanzen, die ein Transgen enthalten, das die Expression und/oder Aktivität des Gens für die Poly(ADP-ribose)polymerase (PARP) in den Pflanzenzellen oder Pflanzen zu reduzieren vermag.
b. Pflanzen, die ein streßtoleranzförderndes Transgen enthalten, das die Expression und/oder Aktivität der für PARG kodierenden Gene der Pflanzen oder Pflanzenzellen zu reduzieren vermag;
c. Pflanzen, die ein streßtoleranzförderndes Transgen enthalten, das für ein in Pflanzen funktionelles Enzym des Nicotinamidadenindinukleotid-Salvage-Biosynthesewegs kodiert, darunter Nicotinamidase, Nicotinatphosphoribosyltransferase, Nicotinsäuremononukleotidadenyltransferase, Nicotinamidadenindinukleotidsynthetase oder Nicotinamidphosphoribosyltransferase.

Pflanzen oder Pflanzensorten (die nach Methoden der pflanzlichen Biotechnologie, wie der Gentechnik, erhalten wurden), die ebenfalls erfindungsgemäß behandelt werden können, weisen eine veränderte Menge, Qualität und/oder Lagerfähigkeit des Ernteprodukts und/oder veränderte Eigenschaften von bestimmten Bestandteilen des Ernteprodukts auf, wie zum Beispiel:
1) Transgene Pflanzen, die eine modifizierte Stärke synthetisieren, die bezüglich ihrer chemisch-physikalischen Eigenschaften, insbesondere des Amylosegehalts oder des Amylose/Amylopektin-Verhältnisses, des Verzweigungsgrads, der durchschnittlichen Kettenlänge, der Verteilung der Seitenketten, des Viskositätsverhaltens, der Gelfestigkeit, der Stärkekorngröße und/oder Stärkekornmorphologie im Vergleich mit der synthetisierten Stärke in Wildtyppflanzenzellen oder -pflanzen verändert ist, so daß sich diese modifizierte Stärke besser für bestimmte Anwendungen eignet.
2) Transgene Pflanzen, die Nichtstärkekohlenhydratpolymere synthetisieren, oder Nichtstärkekohlenhydratpolymere, deren Eigenschaften im Vergleich zu Wildtyppflanzen ohne genetische Modifikation verändert sind. Beispiele sind Pflanzen, die Polyfructose, insbesondere des Inulin- und Levantyps, produzieren, Pflanzen, die alpha-1,4-Glucane produzieren, Pflanzen, die alpha-1,6-verzweigte alpha-1,4-Glucane produzieren und Pflanzen, die Alternan produzieren.
3) Transgene Pflanzen, die Hyaluronan produzieren.

Pflanzen oder Pflanzensorten (die nach Methoden der pflanzlichen Biotechnologie, wie der Gentechnik, erhalten wurden), die ebenfalls erfindungsgemäß behandelt werden können, sind Pflanzen wie Baumwollpflanzen mit veränderten Fasereigenschaften. Solche Pflanzen können durch genetische Transformation oder durch Selektion von Pflanzen, die eine Mutation enthalten, die solche veränderten Fasereigenschaften verleiht, erhalten werden; dazu zählen:
a) Pflanzen wie Baumwollpflanzen, die eine veränderte Form von Cellulosesynthasegenen enthalten,
b) Pflanzen wie Baumwollpflanzen, die eine veränderte Form von rsw2- oder rsw3-homologen Nukleinsäuren enthalten;
c) Pflanzen wie Baumwollpflanzen mit einer erhöhten Expression der Saccharosephosphatsynthase;
d) Pflanzen wie Baumwollpflanzen mit einer erhöhten Expression der Saccharosesynthase;
e) Pflanzen wie Baumwollpflanzen bei denen der Zeitpunkt der Durchlaßsteuerung der Plasmodesmen an der Basis der Faserzelle verändert ist, z. B. durch Herunterregulieren der faserselektiven β-1,3-Glucanase;
f) Pflanzen wie Baumwollpflanzen mit Fasern mit veränderter Reaktivität, z. B. durch Expression des N-Acetylglucosamintransferasegens, darunter auch nodC, und von Chitinsynthasegenen.

Pflanzen oder Pflanzensorten (die nach Methoden der pflanzlichen Biotechnologie, wie der Gentechnik, erhalten wurden), die ebenfalls erfindungsgemäß behandelt werden können, sind Pflanzen wie Raps oder verwandte Brassica-Pflanzen mit veränderten Eigenschaften der Ölzusammensetzung. Solche Pflanzen können durch genetische Transformation oder durch Selektion von Pflanzen, die eine Mutation enthalten, die solche veränderten Öleigenschaften verleiht, erhalten werden; dazu zählen:
a) Pflanzen wie Rapspflanzen, die Öl mit einem hohen Ölsäuregehalt produziere;
b) Pflanzen wie Rapspflanzen, die Öl mit einem niedrigen Linolensäuregehalt produzieren.
c) Pflanzen wie Rapspflanzen, die Öl mit einem niedrigen gesättigten Fettsäuregehalt produzieren.

Besonders nützliche transgene Pflanzen, die erfindungsgemäß behandelt werden können, sind Pflanzen mit einem oder mehreren Genen, die für ein oder mehrere Toxine kodieren, sind die transgenen Pflanzen, die unter den folgenden Handelsbezeichnungen angeboten werden: YIELD GARD® (zum Beispiel Mais, Baumwolle, Sojabohnen), KnockOut® (zum Beispiel Mais), BiteGard® (zum Beispiel Mais), BT-Xtra® (zum Beispiel Mais), StarLink® (zum Beispiel Mais), Bollgard® (Baumwolle), Nucotn® (Baumwolle), Nucotn 33B® (Baumwolle), NatureGard® (zum Beispiel Mais), Protecta® und NewLeaf® (Kartoffel). Herbizidtolerante Pflanzen, die zu erwähnen sind, sind zum Beispiel Maissorten, Baumwollsorten und Sojabohnensorten, die unter den folgenden Handelsbezeichnungen angeboten werden: Roundup Ready® (Glyphosatetoleranz, zum Beispiel Mais, Baumwolle, Sojabohne), Liberty Link® (Phosphinotricintoleranz, zum Beispiel Raps), IMI® (Imidazolinontoleranz) und SCS® (Sylfonylharnstofftoleranz), zum Beispiel Mais. Zu den herbizidresistenten Pflanzen (traditionell auf Herbizidtoleranz gezüchtete Pflanzen), die zu erwähnen sind, zählen die unter der Bezeichnung Clearfield® angebotenen Sorten (zum Beispiel Mais).

Besonders nützliche transgene Pflanzen, die erfindungsgemäß behandelt werden können, sind Pflanzen, die Transformations-Events, oder eine Kombination von Transformations-Events, enthalten und die zum Beispiel in den Dateien von verschiedenen nationalen oder regionalen Behörden angeführt sind (siehe zum Beispiel http://gmoinfo.jrc.it/gmp_browse.aspx und http://www.agbios.com/dbase.php).

Die aufgeführten Pflanzen können besonders vorteilhaft erfindungsgemäß mit den Verbindungen der allgemeinen Formel **(I)** bzw. den erfindungsgemäßen Wirkstoffmischungen behandelt werden. Die bei den Wirkstoffen bzw. Mischungen oben angegebenen Vorzugsbereiche gelten auch für die Behandlung dieser Pflanzen. Besonders hervorgehoben sei die Pflanzenbehandlung mit den im vorliegenden Text speziell aufgeführten Verbindungen bzw. Mischungen.

Die erfindungsgemäßen Wirkstoffe bzw. Mittel können also eingesetzt werden, um Pflanzen innerhalb eines gewissen Zeitraumes nach der Behandlung gegen den Befall durch die genannten Schaderreger zu schützen. Der Zeitraum, innerhalb dessen Schutz herbeigeführt wird, erstreckt sich im Allgemeinen auf 1 bis 28 Tage, bevorzugt auf 1 bis 14 Tage, besonders bevorzugt auf 1 bis 10 Tage, ganz besonders bevorzugt auf 1 bis 7 Tage nach der Behandlung der Pflanzen mit den Wirkstoffen bzw. auf bis zu 200 Tage nach einer Saatgutbehandlung.

Die Herstellung und die Verwendung der erfindungsgemäßen Wirkstoffe der Formeln **(I)** geht aus den folgenden Beispielen hervor. Die Erfindung ist jedoch nicht auf diese Beispiele beschränkt.

Allgemeines: Wenn nicht anders angegeben, wurden alle chromatografischen Reinigungs- bzw. Trennungsschritte an Kieselgel und mit einem Lösungsmittelgradienten von 0:100 Essigsäureethylester/Cyclohexan zu 100:0 Essigsäure-ethylester/Cyclohexan durchgeführt

### Herstellung von Ausgangsstoffen der Formel (IX):

### tert-Butyl-4-(4-acetyl-1,3-thiazol-2-yl)piperidin-1-carboxylat (IX-1)

Zu einer Lösung von *tert*-Butyl-4-[4-(1-hydroxyethyl)-1,3-thiazol-2-yl]piperidin-1-carboxylat **(VIII-1,** 2.06 g) in Dichloromethan (40 mL) wird bei Raumtemperatur 1,1,1-triacetoxy-1,1-dihydro-1,2-benziodoxol-3(1H)-on (15% Lösung in Dichloromethan, 28 g) getropft. Das Reaktionsgemisch wird über Nacht bei Raumtemperatur gerührt dann 5 g Silicagel zugegeben und das Lösungsmittel im Vakuum entfernt. Der Rückstand wird chromatografisch gereinigt. Man erhält *tert*-Butyl-4-(4-acetyl-1,3-thiazol-2-yl)piperidin-1-carboxylat (1.65 g).
¹H NMR (DMSO-d6): δ 8.34 (s, 1H), 4.00 (d, 2H), 3.26 (m, 1H), 2.93 (td, 2H), 2.54 (s, 3H), 2.09-2.03 (m, 2H), 1.60 (qd, 2H), 1.41 (s, 9H)
MS (ESI): 255 (M-C(CH3)3+H)

### Herstellung von Ausgangsstoffen der Formel (IVa), (IVb) und (IVc) :

### tert-Butyl-4-[4-(2-phenylethyl)-1,3-thiazol-2-yl]piperidin-1-carboxylat (IV-1)

Zu einer Lösung von 1-Brom-4-phenylbutan-2-on (2.00 g) in Ethanol (20 mL) wird bei 0 °C eine Lösung von *tert*-Butyl-4-carbamothioylpiperidin-1-carboxylat (1.95 g) getropft. Das Reaktionsgemisch wird unter Argon über Nacht bei Raumtemperatur gerührt. Es wird Triethylamin (1.7 mL) zugegeben, das Gemisch mit Essigsäure-ethylester verdünnt und mit konzentrierter Kochsalzlösung gewaschen. Die wässrige Phase wird abgetrennt und mit Essigsäure-ethylester extrahiert. Die vereinigten organischen Phasen werden über Natriumsulfat getrocknet und unter vermindertem Druck eingeengt, wobei man ein Gemisch von *tert*-Butyl-4-[4-(2-phenylethyl)-1,3-thiazol-2-yl]piperidin-1-carboxylat und *tert*-Butyl-4-[4-hydroxy-4-(2-phenylethyl)-4,5-dihydro-1,3-thiazol-2-yl]piperidine-1-carboxylat erhält. Beide Verbindungen werden chromatografisch getrennt. Man erhält *tert*-Butyl-4-[4-(2-phenylethyl)-1,3-thiazol-2-yl]piperidin-1-carboxylat (800 mg) und *tert-*Butyl-4-[4-hydroxy-4-(2-phenylethyl)-4,5-dihydro-1,3-thiazol-2-yl]piperidine-1-carboxylat (640 mg). Beide Verbindungen können in Verfahren 1.2 eingesetzt werden, da sich letztere unter den Reaktionsbedingungen in die erste umwandelt.
¹H NMR (DMSO-d₆): δ 7.28-7.25 (m, 2H), 7.22-7.22 (m, 2H), 7.20-7.18 (m, 1H), 7.13 (s, 1H), 4.00 (bs, 2H), 3.35 (s, 2H), 3.16 (m, 1H), 2.95 (s, 4H), 2.00 (d, 2H), 1.52 (qd, 2H), 1.41 (s, 9H) ppm
MS (ESI): 373 ([M+H]⁺)

### tert-Butyl-4-{4-[(E)2-(naphthalen-1-yl)ethenyl]-1,3-thiazol-2-yl}piperidin-1-carboxylat (IV-2)

Diethyl-1-naphthylmethylphosphonat (1.88 g) wird in Tetrahydrofuran (20 mL) unter Argon gelöst und auf 0 °C abgekühlt. 757 mg Kalium-*tert*-butanolat wird zugegeben, wobei die Lösung dunkelrot wird. Nach weiteren 10 min Rühren wird *tert*-Butyl-4-(4-formyl-1,3-thiazol-2-yl)piperidin-1-carboxylat (1.00 g) zugegeben. Das Gemisch wird 30 min bei 0 °C gerührt und dann auf Raumtemperatur erwärmt. Nach weiteren 20 min wird mit konz. Ammoniumchloridlösung versetzt. Die wässrige Phase wird abgetrennt und mit Essigsäure-ethylester extrahiert. Die vereinigten organischen Phasen werden über Natriumsulfat getrocknet, eingeengt und der Rückstand chromatografisch gereinigt. Man erhält *tert*-Butyl-4-{4-[(E)-2-(naphthalen-1-yl)ethenyl]-1,3-thiazol-2-yl}piperidin-1-carboxylat (1.16 g) als *E*-Isomer.
¹H NMR (DMSO-d₆): δ 8.23 (d, 1H), 8.14 (d, 1H), 7.94 (d, 1H), 7.87 (d, 1H), 7.83 (d, 1H), 7.62 (s, 1H), 7.62-7.51 (m, 3H), 7.26 (d, 1H), 4.04 (d, 2H), 3.27 (m, 1H), 2.94 (t, 2H), 2.15-2.05 (m, 2H), 1.66 (qd, 2H), 1.42 (s, 9H) ppm
MS (ESI): 421 ([M+H]⁺)

### tert-Butyl-4-{4-[(Z)-2-(naphthalen-1-yl)ethenyl]-1,3-thiazol-2-yl}piperidin-1-carboxylat (IV-3)

(1-Naphthylmethyl)triphenylphosphonium chloride (2.96 g) wird in 20 mL Tetrahydrofuran gelöst, unter Argon auf 0 °C abgekühlt und mit Kalium-*tert*.butanolat (757 mg) versetzt, wobei die Lösung dunkelrot wird. Nach 10 min Rühren wird *tert*-Butyl-4-(4-formyl-1,3-thiazol-2-yl)piperidin-1-carboxylat (1.00 g) zugegeben. Das Gemisch wird 30 min bei 0 °C weitergerührt und dann langsam auf Raumtemperatur erwärmt. Nach weiteren 20 min wird mit gesättigter Ammoniumchloridlösung versetzt und die wässrige Phase abgetrennt. Nach Extraktion der wässrigen Phase mit Essigsäureethylester werden die vereinigten organischen Phasen über Natriumsulfat getrocknet und unter vermindertem Druck eingeengt. Nach chromatografischer Reinigung erhält man *tert*-Butyl-4-{4-[(Z)-2-(naphthalen-1-yl)ethenyl]-1,3-thiazol-2-yl}piperidin-1-carboxylat (1.2 g, Z-Isomer).
¹H NMR (DMSO-d₆): δ 7.92 (t, 2H), 7.87-7.80 (m, 1H), 7.50-7.48 (m, 4H), 7.10 (d, 1H), 6.91 (d, 1H), 6.86 (s, 1H), 3.75 (d, 2H), 2.98 (m, 1H), 2.80 (t, 2H), 1.80-1.70 (m, 2H), 1.40 (s, 9H), 1.30 (qd, 2H) ppm
MS (ESI): 421 ([M+H]⁺)

### tert-Butyl-4-{4-[(1Z)-4-phenylpent-1-en-1-yl]-1,3-thiazol-2-yl}piperidin-1-carboxylat (IV-4)

*tert*-Butyl-4-(4-formyl-1,3-thiazol-2-yl)piperidin-1-carboxylat (1.00 g) wird mit Triphenyl(3-phenylbutyl)phosphoniumiodid (3.53 g) umgesetzt und wie in IV-3 beschrieben gereinigt. Man erhält *tert*-Butyl-4-{4-[(1Z)-4-phenylpent-1-en-1-yl]-1,3-thiazol-2-yl}piperidin-1-carboxylat (1.23 g) in Form des Z-Isomers.
¹H NMR (DMSO-d₆): δ 7.30 (s, 1H), 7.26-7.22 (m, 4H), 7.15 (m, 1H), 6.33 (s, 1H), 5.59 (m, 1H), 3.98 (d, 2H), 3.19 (m, 1H), 3.00-2.83 (m, 5H), 2.06-1.97 (m, 2H), 1.60 (qd, 2H), 1.41 (s, 9H), 1.24 (d, 3H) ppm
MS (ESI): 413 ([M+H]⁺)

### tert-Butyl-4-[4-(3-phenylprop-1-in-1-yl)-1,3-thiazol-2-yl]piperidin-1-carboxylat (IV-5)

Zu einer entgasten Lösung von *tert*-Butyl-4-(4-ethinyl-1,3-thiazol-2-yl)piperidin-1-carboxylat (**X-1**, 310 mg) in Tetrahydrofuran (4 mL) werden unter Argon Benzylchlorid (134 mg), Bis(acetonitril)dichlorpalladium(II) (5.5 mg), 2-Dicyclohexylphosphino-2',4',6'-triisopropylbiphenyl (30 mg) und Cäsiumcarbonat (363 mg) gegeben. Das Gemisch wird dann auf 65 °C erwärmt. Nach anschließendem Abkühlen wird der Katalysator durch Filtration über Celite entfernt und unter vermindertem Druck eingeengt. Nach chromatografischer Reinigung erhält man *tert*-Butyl-4-[4-(3-phenylprop-1-in-1-yl)-1,3-thiazol-2-yl]piperidin-1-carboxylat (329 mg).
¹H NMR (CD₃CN): δ 7.40-7.20 (m, 6H), 4.10-4.05 (m, 2H), 3.83 (s, 2H), 3.15 (m, 1H), 2.94-2.35 (m, 2H), 2.08-2.00 (m, 2H), 1.63 (qd, 2H), 1.43 (s, 9H) ppm
MS (ESI): 383 ([M+H]⁺)

### tert-Butyl-4-[4-(naphthalen-1-ylethinyl)-1,3-thiazol-2-yl]piperidin-1-carboxylat (IV-6)

Zu einer Lösung von *tert*-Butyl-4-{4-[(trimethylsilyl)ethinyl]-1,3-thiazol-2-yl}piperidin-1-carboxylat **(XI-1,** 600 mg) und 1-Bromnaphthalen (375 mg) in entgastem N,N-Dimethylformamid werden Tetrakis(triphenylphosphin)palladium(0) (95 mg), Kupfer(I)iodid (31 mg) und Triethylamin (200 mg) gegeben. Nach Erwärmen auf 85 °C wird Tetra-n-butylammoniumfluorid (1M in THF, 1.81 mL) zugetropft. Das Reaktionsgemisch wird anschließend mit Wasser verdünnt und mit Essigsäureethylester extrahiert. Die organischen Extrakte werden über Natriumsulfat getrocknet und unter vermindertem Druck eingeengt. Nach chromatografischer Reinigung erhält man *tert*-Butyl-4-[4-(naphthalen-1-ylethinyl)-1,3-thiazol-2-yl]piperidin-1-carboxylat (576 mg).
¹H NMR (CD₃CN): δ 8.38 (d, 1H), 7.95 (d, 2H), 7.79 (d, 1H), 7.70 (s, 1H), 7.67-7.50 (m, 3H), 4.12 (d, 2H), 3.24 (m, 1H), 2.94 (t, 2H), 2.15-2.08 (m, 2H), 1.72 (qd, 2H), 1.45 (s, 9H) ppm
MS (ESI): 419 ([M+H]⁺)

### tert-Butyl-4-[4-(pyridin-3-ylethinyl)-1,3-thiazol-2-yl]piperidin-1-carboxylat (IV-7)

*tert*-Butyl-4-{4-[(trimethylsilyl)ethinyl]-1,3-thiazol-2-yl}piperidin-1-carboxylat (**XI-1**, 610 mg) wird analog zu Beispiel IV-6 mit 3-Brompyridin (291 mg) umgesetzt. Man erhält *tert*-Butyl-4-[4-(pyridin-3-ylethinyl)-1,3-thiazol-2-yl]piperidin-1-carboxylat (469 mg).
¹H NMR (CD₃CN): δ 8.74 (d, 1H), 8.55 (dd, 1H), 7.87 (s, 1H), 7.63 (m, 1H), 7.35 (dd, 1H), 4.10 (d, 2H), 3.20 (m, 2H), 2.93 (t, 2H), 2.13-2.05 (m, 2H), 1.69 (qd, 2H), 1.44 (s, 9H) ppm
MS (ESI): 270 ([M+2H-C=OOC(CH₃)₃]⁺)

### tert-Butyl-4-{4-[2-(naphthalen-1-yl)ethyl]-1,3-thiazol-2-yl}piperidin-1-carboxylat (IV-8)

*tert*-Butyl-4-{4-[(Z)-2-(naphthalen-1-yl)ethenyl]-1,3-thiazol-2-yl}piperidin-1-carboxylat (**IV-3**, 1.5 g) wird in 90 mL Methanol gelöst und bei 30 °C mit 10% Pd/C als Katalysator unter 10 bar Wasserstoff hydrogeniert. Nach Abfiltrieren des Katalysators und Entfernen des Lösungsmittels unter vermindertem Druck wird *tert*-Butyl-4-{4-[2-(naphthalen-1-yl)ethyl]-1,3-thiazol-2-yl}piperidin-1-carboxylat (1.48 g) als farbloses Öl erhalten.
¹H NMR (DMSO-d₆): δ 8.08 (d, 1H), 7.88 (d, 1H), 7.74 (d, 1H), 7.55-7.45 (m, 2H), 7.42-7.32 (m, 2H), 7.10 (s, 1H), 4.00 (d, 2H), 3.43 (t, 2H), 3.32-3.06 (m, 3H), 2.92 (t, 2H), 2.04-1.98 (m, 2H), 1.58 (qd, 2H), 1.42 (s, 9H) ppm
MS (ESI): 423 ([M+H]⁺)

### Herstellung von Ausgangsstoffen der Formel (X):

### tert-Butyl-4-(4-ethinyl-1,3-thiazol-2-yl)piperidin-1-carboxylat (X-1)

*tert*-Butyl-4-(4-formyl-1,3-thiazol-2-yl)piperidin-1-carboxylat (600 mg) wird unter Argon in Methanol gelöst und mit Kaliumcarbonat (839 mg) und Dimethyl-1-diazo-2-oxopropylphosphonat (786 mg) versetzt. Das Gemisch wird bei Raumtemperatur für 3 Stunden gerührt. Nach wässriger Aufarbeitung wird mit Essigsäure-ethylester extrahiert, die Extrakte mit Natriumsulfat getrocknet und unter vermindertem Druck eingeengt. Der Rückstand wird chromatografisch gereinigt. Man erhält *tert*-Butyl-4-(4-ethinyl-1,3-thiazol-2-yl)piperidin-1-carboxylat (493 mg).
¹H NMR (CD₃CN): δ 7.51 (s, 1H), 4.07 (d, 2H), 3.36 (s, 1H), 3.16 (m, 1H), 2.90 (t, 2H), 2.10-2.00 (m, 2H), 1.65 (qd, 2H), 1.43 (s, 9H) ppm
MS (ESI): 237 ([M+2H-C(CH₃)₃]⁺)

### Herstellung von Ausgangsstoffen der Formel (XI):

### tert-Butyl-4-{4-[(trimethylsilyl)ethinyl]-1,3-thiazol-2-yl}piperidin-1-carboxylat (XI-1)

*tert*-Butyl-4-(4-ethinyl-1,3-thiazol-2-yl)piperidin-1-carboxylat (**X-1**, 940 mg) wird unter Argon bei -78 °C in Tetrahydrofuran gelöst und Lithiumhexamethyldisilazan (1M in Hexan, 4.82 mL) wird zugetropft. Nach 30 min wird Trimethylsilylchlorid (699 mg) zugetropft und die Kühlung entfernt. Nach Rühren über Nacht wird mit gesättigter Ammoniumchloridlösung versetzt und mit Essigsäure-ethylester extrahiert. Die organischen Extrakte werden über Natriumsulfat getrocknet und unter vermindertem Druck eingeengt. Der Rückstand wird chromatografisch gereinigt. Man erhält *tert*-Butyl-4-{4-[(trimethylsilyl)ethinyl]-1,3-thiazol-2-yl}piperidin-1-carboxylat (1.28 g).
¹H NMR (CD₃CN): δ 7.42 (s, 1H), 4.05-3.97 (m, 2H), 3.09 (m, 1H), 2.83 (t, 2H), 2.05-1.90 (m, 2H), 1.59 (qd, 2H), 1.37 (s, 9H), 0.17 (s, 9H) ppm
MS (ESI): 309 ([M+2H-C(CH₃)₃]⁺)

### Herstellung von Ausgangsstoffen der Formel (IIa), (IIb) und (IIc) :

### 4-[4-(2-Phenylethyl)-1,3-thiazol-2-yl]piperidinhydrochlorid (II-1)

Zu einer Lösung von *tert*-Butyl-4-[4-(2-phenylethyl)-1,3-thiazol-2-yl]piperidin-1-carboxylat (**IV-1**, 640 mg) wird bei 0 °C eine 2-molare Lösung von Chlorwasserstoff in Diethylether getropft. Das Reaktionsgemisch wird bei 0 °C gerührt und dann langsam auf Raumtemperatur erwärmt. Nach Rühren über Nacht wird das Lösungsmittel und überschüssiger Chlorwasserstoff entfernt. Man erhält 4-[4-(2-Phenylethyl)-1,3-thiazol-2-yl]piperidinhydrochlorid (590 mg).
¹H NMR (DMSO-d₆): δ 7.25-7.23 (m, 2H), 7.19-7.16 (m, 3H), 7.11 (s, 1H), 3.40-3.30 (m, 5H), 2.98 (s, 4H), 2.21-2.14 (m, 2H), 1.95-1.87 (m, 2H) ppm
MS (ESI): 273 ([M-Cl]⁺)

### 4-{4-[2-(Naphthalen-1-yl)ethyl]-1,3-thiazol-2-yl}piperidinhydrochlorid (II-2)

Zu *tert*-Butyl-4-{4-[2-(naphthalen-1-yl)ethyl]-1,3-thiazol-2-yl}piperidin-1-carboxylat (**IV-2**, 1.40 g) wird unter Argon und bei 0 °C eine Lösung von Chlorwasserstoff in Diethylether (2 M, 25 mL) gegeben. Das Gemisch wird bei 0 °C gerührt und danach langsam auf Raumtemperatur erwärmt. Nach Rühren über Nacht werden die überschüssige Säure und das Lösungsmittel unter vermindertem Druck entfernt. Man erhält 4-{4-[2-(Naphthalen-1-yl)ethyl]-1,3-thiazol-2-yl}piperidinhydrochlorid (1.28 g).
¹H NMR (DMSO-d₆): δ 9.18 (bs, 1H), 8.93 (bs, 1H), 8.08 (d, 1H), 7.89 (d, 1H), 7.75 (d, 1H), 7.56-7.45 (m, 2H), 7.73-7.32 (m, 2H), 7.16 (s, 1H), 3.45 (d, 1H), 3.43 (d, 1H), 3.38-3.28 (m, 2H), 3.15-2.95 (m, 4H), 2.25-2.17 (m, 2H), 2.00 (qd, 2H) ppm
MS (ESI): 323 ([M-Cl]⁺)

### 4-[5-Brom-4-(2-phenylethyl)-1,3-thiazol-2-yl]piperidin (II-3)

Zu einer Lösung von 4-[4-(2-Phenylethyl)-1,3-thiazol-2-yl]piperidinhydrochlorid (**II-1,** 200 mg) in Essigsäure (2 mL) wird bei 0 °C Brom (123 mg) getropft. Das Gemisch wird 30 min gerührt und dann mit gesättigter Natriumbicarbonatlösung versetzt. Die wässrige Phase wird abgetrennt und mit Essigsäure-ethylester extrahiert. Die vereinigten organischen Phasen werden über wasserfreiem Natriumcarbonat getrocknet, unter vermindertem Druck eingeengt und chromatografisch gereinigt. Man erhält 4-[5-Brom-4-(2-phenylethyl)-1,3-thiazol-2-yl]piperidin (127 mg).
logP (pH2.3): 1.72
MS (ESI): 351, 353 ([M+1]⁺)

### Herstellung von Verbindungen der Formel (Ia)

### Methode A:

### 2-[5-Methyl-3-(trifluormethyl)-1H-pyrazol-1-yl]-1-{4-[4-(2-phenylethyl)-1,3-thiazol-2-yl]piperidin-1-yl}ethanon (I-53)

Zu einer Lösung von [5-Methyl-3-(trifluormethyl)-1H-pyrazol-1-yl]essigsäure (185 mg) in Dichlormethan werden bei 0 °C Oxalylchlorid (134 mg) und ein Tropfen N,N-Dimethylformamid gegeben. Das Reaktionsgemisch wird bei Raumtemperatur für 3 h gerührt. Das Lösungsmittel und das überschüssige Reagenz werden unter vermindertem Druck entfernt. Der feste Rückstand wird dann erneut in Dichlormethan gelöst und tropfenweise bei 0 °C zu einer Lösung von 4-[4-(2-Phenylethyl)-1,3-thiazol-2-yl]piperidinhydrochlorid (**II-1,** 250 mg) und Triethylamine (410 mg) in Dichlormethan gegeben. Daraufhin wird die Reaktionslösung mit konzentrierter Ammoniumchloridlösung versetzt, die wässrige Phase abgetrennt und mit Essigsäure-ethylester extrahiert. Die vereinigten organischen Phasen werden über wasserfreiem Natriumsulfat getrocknet und eingeengt. Man erhält 2-[5-Methyl-3-(trifluormethyl)-1H-pyrazol-1-yl]-1-{4-[4-(2-phenylethyl)-1,3-thiazol-2-yl]piperidin-1-yl}ethanon (122 mg).
¹H NMR (DMSO-d₆): δ 7.28-7.26 (m, 2H), 7.22-7.21 (m, 2H), 7.19-7.15 (m, 2H), 6.51 (s, 1H), 5.33 (d, 1H), 5.25 (d, 1H), 4.36 (d, 1H), 3.95 (d, 1H), 3.35-3.20 (m, 2H), 2.96 (s, 4H), 2.82 (t, 1H), 2.21 (s, 3H), 2.09 (d, 1H), 2.07 (d, 1H), 1.78 (m, 1H), 1.53 (m, 1H) ppm
MS (ESI): 463 ([M+H]⁺)

### 1-{4-[4-(2-Phenylethyl)-1,3-thiazol-2-yl]piperidin-1-yl}pentan-1-on (I-58)

Zu einer Lösung von 4-[4-(2-Phenylethyl)-1,3-thiazol-2-yl]piperidinhydrochlorid (**II-1**,309 mg) und Triethylamine (0.7 mL) in Dichlormethan (3 mL) wird Pentanoylchlorid (133 mg) gegeben. Das Gemisch wird über Nacht gerührt und dann mit Wasser versetzt. Die wässrige Phase wird abgetrennt und mit Essigsäure-ethylester extrahiert und dann die vereinigten organischen Phasen mit Natriumsulfat getrocknet und eingeengt. Der Rückstand wird gereinigt. Man erhält 1-{4-[4-(2-Phenylethyl)-1,3-thiazol-2-yl]piperidin-1-yl}pentan-1-on (179 mg).
¹H NMR (DMSO-d6): δ 7.27-7.21 (m, 2H), 7.20-7.14 (m, 3H), 7.05 (s, 1H), 4.37 (bs, 1H), 3.95 (bs, 1H), 3.26-3.18 (m, 2H), 2.96 (s, 4H), 2.80 (bs, 1H), 2.33 (t, 2H), 2.04 (d, 2H), 1.55 (bs, 2H), 1.50 (tt, 2H), 1.33 (tq, 2H), 0.90 (t, 3H) ppm
MS (ESI): 357 ([M+H]⁺)

### 2-[5-Methyl-3-(trifluormethyl)-1H-pyrazol-1-yl]-1-(4-{4-[2-(naphthalen-1-yl)ethyl]-1,3-thiazol-2-yl}piperidin-1-yl)ethanon (I-81)

Zu einer Lösung von [5-Methyl-3-(trifluormethyl)-1H-pyrazol-1-yl]essigsäure (100 mg) in Dichlormethan (5 mL) werden Oxalylchlorid (183 mg) und ein Tropfen N,N-Dimethylformamid gegeben. Das Reaktionsgemisch wird über Nacht bei Raumtemperatur gerührt. Nach Entfernen der Lösungsmittel unter vermindertem Druck wird der Rückstand dann in Dichlormethan (5 mL) gelöst und bei 0 °C tropfenweise mit einer Lösung von 4-{4-[2-(Naphthalen-1-yl)ethyl]-1,3-thiazol-2-yl}piperidinhydrochlorid (**II-2**, 172 mg) und von Hünigbase (186 mg) in Dichlormethan (5 mL) versetzt. Nach Zugabe von konz. Ammoniumchloridlösung wird die wässrige Phase abgetrennt und mit Essigsäureethylester extrahiert. Die vereinigten organischen Phasen werden über Natriumsulfat getrocknet und unter vermindertem Druck eingeengt. Der Rückstand wird chromatografisch gereinigt. Man erhält 2-[5-Methyl-3-(trifluormethyl)-1H-pyrazol-1-yl]-1-(4-{4-[2-(naphthalen-1-yl)ethyl]-1,3-thiazol-2-yl}piperidin-1-yl)ethanon (150 mg).
¹H NMR (DMSO-d₆): δ 8.09 (d, 1H), 7.90 (d, 1H), 7.75 (d, 1H), 7.56-7.46 (m, 2H), 7.42-7.33 (m, 2H), 7.12 (s, 1H), 6.45 (s, 1H), 5.22 (bs, 2H), 4.31 (bs, 1H), 3.98 (bs, 1H), 3.44 (t, 2H), 3.35-3.26 (m, 2H), 3.15-3.05 (m, 2H), 2.90 (bs, 1H), 2.23 (s, 3H), 2.15-2.05 (m, 2H), 1.75 (bs, 1H), 1.62 (bs, 1H) ppm
MS (ESI): 513 ([M+H]⁺)

### 2-[5-Methyl-3-(trifluormethyl)-1H-pyrazol-1-yl]-1-{4-[4-(4-phenylpentyl)-1,3-thiazol-2-yl]piperidin-1-yl}ethanon (I-80)

*tert*-Butyl-4-{4-[(1Z)-4-phenylpent-1-en-1-yl]-1,3-thiazol-2-yl}piperidin-1-carboxylat (IV-4, 1.00 g) wird in Methanol gelöst und bei 40 °C unter 10 bar H₂ für 5 Stunden in Gegenwart von Pd/C (10%) hydrogeniert. Nach Filtration und Entfernung des Lösungsmittels unter vermindertem Druck erhält man *tert*-Butyl-4-[4-(4-phenylpentyl)-1,3-thiazol-2-yl]piperidin-1-carboxylat (1.00 g) als farbloses Öl. Dieses wird dann analog zu Beispiel II-2 entschützt. Man erhält 850 mg von 4-[4-(4-phenylpentyl)-1,3-thiazol-2-yl]piperidinhydrochlorid. 169 mg werden dann analog zu Beispiel I-81 mit [5-Methyl-3-(trifluormethyl)-1H-pyrazol-1-yl]essigsäure (100 mg) umgesetzt. Nach chromatografischer Reinigung erhält man 2-[5-Methyl-3-(trifluorinethyl)-1H-pyrazol-1-yl]-1-{4-[4-(4-phenylpentyl)-1,3-thiazol-2-yl]piperidin-1-yl}ethanon (180 mg) als farbloses Öl.
¹H NMR (DMSO-d₆): δ 7.28-7.23 (m, 2H), 7.19-7.14 (m, 3H), 7.02 (s, 1H), 6.44 (s, 1H), 5.20 (bs, 2H), 4.30 (bs, 1H), 3.95 (bs, 1H), 3.30-3.20 (m, 2H), 2.87 (bs, 1H), 2.72 (m, 1H), 2.67-2.62 (m, 2H), 2.22 (s, 3H), 2.07-2.02 (m, 2H), 1.72 (bs, 1H), 1.63-1.47 (m, 5H), 1.19 (d, 3H) ppm
MS (ESI): 505 ([M+H]⁺)

### 1-{4-[5-Brom-4-(2-phenylethyl)-1,3-thiazol-2-yl]piperidin-1-yl}-2-[5-methyl-3-(trifluormethyl)-1H-pyrazol-1-yl]ethanon (I-78)

4-[5-Brom-4-(2-phenylethyl)-1,3-thiazol-2-yl]piperidin (**II-3,** 127 mg) wird analog zu Beispiel I-81 mit [5-Methyl-3-(trifluormethyl)-1H-pyrazol-1-yl]essigsäure (118 mg) umgesetzt. Nach chromatografischer Reinigung erhält man 1-{4-[5-Brom-4-(2-phenylethyl)-1,3-thiazol-2-yl]piperidin-1-yl}-2-[5-methyl-3-(trifluormethyl)-1H-pyrazol-1-yl]ethanon (52 mg).
¹H NMR (DMSO-d₆): δ 7.27-7.22 (m, 2H), 7.18-7.13 (m, 3H), 6.45 (s, 1H), 5.22 (bs, 2H), 4.38 (bs, 1H), 3.95 (bs, 1H), 3.33-3.20 (m, 2H), 2.94 (s, 4H), 2.93 (bs, 1H), 2.22 (s, 3H), 2.09-2.02 (m, 2H), 1.80-1.50 (m, 2H) ppm
MS (ESI): 541 ([M+H]⁺)

### Method B:

### 1-{4-[4-(2-Phenylethyl)-1,3-thiazol-2-yl]piperidin-1-yl}-2-(thiophen-3-yl)ethanon (I-63)

Thiophen-3-ylessigsäure (156 mg) und Hünigbase (323 mg) werden in Dichlormethan (10 mL) gelöst und für 30 min gerührt. 4-[4-(2-Phenylethyl)-1,3-thiazol-2-yl]piperidinhydrochlorid **(II-1,** 309 mg), hergestellt nach Verfahren 1.2, wird zugegeben und das Gemisch weitere 5 min gerührt, bevor Brom-tris-pyrrolidino-phosphoniumhexafluorophosphat (599 mg) zugegeben wird. Die Reaktionsmischung wird über Nacht bei Raumtemperatur gerührt. Nach Entfernen des Lösungsmittels unter vermindertem Druck wird der Rückstand chromatografisch gereinigt. Man erhält 1-{4-[4-(2-Phenylethyl)-1,3-thiazol-2-yl]piperidin-1-yl}-2-(thiophen-3-yl)ethanon (99 mg).
¹H NMR (DMSO-d₆): δ 7.44-7.43 (m, 1H), 7.28-7.12 (m, 6H), 7.05 (s, 1H), 6.99 (d, 1H), 4.38 (bs, 1H), 3.99 (bs, 1H), 3.72 (s, 2H), 3.22-3.15 (m, 2H), 2.96 (s, 4H), 2.81 (bs, 1H), 2.00 (d, 2H), 1.52 (m, 2H) ppm
MS (ESI): 397 ([M+H]⁺)

### 2-[2-Chlor-5-(trifluormethyl)phenyl]-1-{4-[4-(2-phenylethyl)-1,3-thiazol-2-yl]piperidin-1-yl}ethanon (I-67)

4-[4-(2-Phenylethyl)-1,3-thiazol-2-yl]piperidinhydrochlorid (**II-1,** 309 mg) wird analog zu Beispiel I-63 mit [2-Chlor-5-(trifluormethyl)phenyl]essigsäure (262 mg) umgesetzt. Man erhält nach chromatografischer Reinigung 2-[2-Chlor-5-(trifluormethyl)phenyl]-1-{4-[4-(2-phenylethyl)-1,3-thiazol-2-yl]piperidin-1-yl}ethanon (210 mg).
¹H NMR (DMSO-d₆): δ 7.71-7.58 (m, 3H), 7.27-7.12 (m, 5H), 7.07 (s, 1H), 4.37 (bs, 1H), 4.05 (bs, 1H), 3.97 (s, 2H), 3.35-3.23 (m, 2H), 2.97 (s, 4H), 2.88 (bs, 1H), 2.07 (d, 2H), 1.65 (bs, 2H) ppm
MS (ESI): 439 ([M+H]⁺)

### 2-(5-Chlor-1-methyl-1H-pyrazol-4-yl)-1-{4-[4-(2-phenylethyl)-1,3-thiazol-2-yl]piperidin-1-yl}ethanon (I-65)

4-[4-(2-Phenylethyl)-1,3-thiazol-2-yl]piperidinhydrochlorid (**II-1,** 280 mg) wird analog zu Beispiel I-63 mit (5-Chlor-l-methyl-1H-pyrazol-4-yl)essigsäure (174 mg) umgesetzt. Man erhält nach chromatografischer Reinigung 2-(5-Chlor-1-methyl-1H-pyrazol-4-yl)-1-{4-[4-(2-phenylethyl)-1,3-thiazol-2-yl]piperidin-1-yl}ethanon (89 mg).
¹H NMR (DMSO-d₆): δ 7.40 (s, 1H), 7.26-7.15 (m, 5H), 7.06 (s, 1H), 4.35 (bs, 1H), 4.05 (bs, 1H), 3.76 (s, 3H), 3.49 (s, 2H), 3.30-3.19 (m, 2H), 2.99 (s, 4H), 2.82 (bs, 1H), 2.08-2.00 (m, 2H), 1.58 (bs, 2H) ppm
MS (ESI): 429 ([M+H]⁺)

### 2-[4-Chlor-5-methyl-3-(trifluormethyl)-1H-pyrazol-1-yl]-1-{4-[4-(2-phenylethyl)-1,3-thiazol-2-yl]piperidin-1-yl}propan-1-on (I-64)

4-[4-(2-Phenylethyl)-1,3-thiazol-2-yl]piperidinhydrochlorid (**II-1,** 309 mg) wird analog zu Beispiel I-63 mit 2-[4-Chlor-5-methyl-3-(trifluoromethyl)-1H-pyrazol-1-yl]propansäure (282 mg) umgesetzt. Man erhält nach chromatografischer Reinigung 2-[4-Chlor-5-methyl-3-(trifluormethyl)-1H-pyrazol-1-yl]-1-{4-[4-(2-phenylethyl)-1,3-thiazol-2-yl]piperidin-1-yl}propan-1-on (165 mg).
¹H NMR (DMSO₋d6): δ 7.26-7.21 (m, 2H), 7.20-7.13 (m, 3H), 7.06 (s, 1H), 5.70 (m, 1H), 4.30 (bs, 1H), 4.00 (bs, 1H), 3.30-3.19 (m, 2H), 2.96 (s, 5H), 2.26 (d, 3H), 2.08-1.96 (m, 2H), 1.64-1.54 (m, 5H) ppm
MS (ESI): 511 ([M+H]⁺)

### [2,5-Bis(trifluormethyl)phenyl]{4-[4-(2-phenylethyl)-1,3-thiazol-2-yl] piperidin-1-yl}methanon (I-61)

4-[4-(2-Phenylethyl)-1,3-thiazol-2-yl]piperidinhydrochlorid (**II-1,** 309 mg) wird analog zu Beispiel I-63 mit 2,5-Bis(trifluormethyl)benzolcarbonylchlorid (304 mg) umgesetzt. Man erhält nach chromatografischer Reinigung [2,5-Bis(trifluormethyl)phenyl]{4-[4-(2-phenylethyl)-1,3-thiazol-2-yl]piperidin-1-yl}methanon (282 mg).
¹H NMR (DMSO-d₆): δ 8.07-7.90 (m, 3H), 7.26-7.21 (m, 2H), 7.20-7.14 (m, 3H), 7.08 (s, 1H), 4.50 (d, 1H), 3.35-3.23 (m, 4H), 2.97 (s, 4H), 2.22-2.10 (m, 1H), 2.00-1.87 (m, 2H), 1.87-1.45 (m, 2H) ppm
MS (ESI): 513 ([M+H]⁺)

### 3-(3,4-Dimethoxyphenyl)-1-{4-[4-(2-phenylethyl)-1,3-thiazol-2-yl]piperidin-1-yl}propan-1-on (I-66)

4-[4-(2-Phenylethyl)-1,3-thiazol-2-yl]piperidinhydrochlorid (**II-1,** 309 mg) wird analog zu Beispiel I-63 mit 3-(3,4-Dimethoxyphenyl)propansäure (231 mg) umgesetzt. Man erhält nach chromatografischer Reinigung 3-(3,4-Dimethoxyphenyl)-1-{4-[4-(2-phenylethyl)-1,3-thiazol-2-yl]piperidin-1-yl}propan-1-on (139 mg).
¹H NMR (DMSO-d₆): δ 7.26-7.21 (m, 2H), 7.20-7.12 (m, 3H), 7.05 (s, 1H), 6.85-6.80 (m, 3H), 6.75-6.71 (dd, 1H), 3.93 (bs, 1H), 3.74 (s, 3H), 3.69 (s, 3H), 3.20 (m, 1H), 2.96 (s, 4H), 2.80-2.74 (m, 2H), 2.65-2.55 (bs, 2H), 2.04-1.97 (m, 2H), 1.58-1.42 (m, 2H), 1.28 (t, 2H) ppm
MS (ESI): 465 ([M+H]⁺)

### 2-(1,3-Benzothiazol-2-yl)-1-{4-[4-(2-phenylethyl)-1,3-thiazol-2-yl]piperidin-1-yl}ethanon (I-71)

4-[4-(2-Phenylethyl)-1,3-thiazol-2-yl]piperidinhydrochlorid (**II-1**, 308 mg) wird analog zu Beispiel I-63 mit 1,3-Benzothiazol-2-ylessigsäure (252 mg) umgesetzt. Man erhält nach chromatografischer Reinigung 2-(1,3-Benzothiazol-2-yl)-1-{4-[4-(2-phenylethyl)-1,3-thiazol-2-yl]piperidin-1-yl}ethanon (109 mg).
¹H NMR (DMSO-d₆): δ 8.03 (d, 1H), 7.93 (d, 1H), 7.47 (t, 1H), 7.40 (t, 1H), 7.25-7.16 (m, 5H), 7.06 (s, 1H), 4.40 (bs, 1H), 4.34 (s, 2H), 4.09 (bs, 1H), 3.40-3.15 (m, 3H), 2.96 (s, 4H), 2.08-2.02 (m, 2H), 1.65 (bs, 2H) ppm
MS (ESI): 448 ([M+H]⁺)

### 2-(2H-Indazol-2-yl)-1-{4-[4-(2-phenylethyl)-1,3-thiazol-2-yl]piperidin-1-yl}ethanon (I-70)

4-[4-(2-Phenylethyl)-1,3-thiazol-2-yl]piperidinhydrochlorid (**II**-1, 309 mg), hergestellt nach Verfahren 1.2, wird analog zu Beispiel I-63 mit 2H-Indazol-2-ylessigsäure (194 mg) umgesetzt. Man erhält nach chromatografischer Reinigung 2-(2H-Indazol-2-yl)-1-{4-[4-(2-phenylethyl)-1,3-thiazol-2-yl]piperidin-1-yl}ethanon (67 mg).
¹H NMR (CD₃CN): δ 8.10 (s, 1H), 7.71 (d, 1H), 7.60 (d, 1H), 7.29-7.15 (m, 6H), 7.07 (dd, 1H), 6.87 (s, 1H), 5.45 (d, 1H), 5.36 (d, 1H), 4.48 (d, 1H), 4.00 (d, 1H), 3.35-3.23 (m, 2H), 3.00 (s, 4H), 2.86 (t, 1H), 2.18-2.06 (m, 2H), 1.80 (qd, 1H), 1.66 (qd, 1H) ppm
MS (ESI): 431 ([M+H]⁺)

### Method C:

### 2-[5-Methyl-3-(trifluormethyl)-1H-pyrazol-1-yl]-1-(4-{4-[2-(naphthalen-1-yl)ethyl]-1,3-thiazol-2-yl}piperidin-1-yl)ethanon (I-81)

2-[5-Methyl-3-(trifluormethyl)-1H-pyrazol-1-yl]-1-{4-[4-(naphthalen-1-ylethinyl)-1,3-thiazol-2-yl]piperidin-1-yl}ethanon (**I-50,** 157 mg) wird in Methanol (1.5 mL) gelöst und bei 60 °C mit Ammonium formiat (195 mg) und 20% Pd(OH)₂/C (4 mg) als Katalysator. Nach Abfiltrieren des Katalysators und Entfernen des Lösungsmittels unter vermindertem Druck wird 2-[5-Methyl-3-(trifluormethyl)-1H-pyrazol-1-yl]-1-(4-{4-[2-(naphthalen-1-yl)ethyl]-1,3-thiazol-2-yl}piperidin-1-yl)ethanon (86 mg) als farbloses Öl erhalten.

Das Produkt wurde wie die vorher beschriebene Verbindung charakterisiert.

### Herstellung von Verbindungen der Formel (Ib)

### 2-[5-Methyl-3-(trifluormethyl)-1H-pyrazol-1-yl]-1-(4-{4-[(E)-2-(naphthalen-1-yl)ethenyl]-1,3-thiazol-2-yl}piperidin-1-yl)ethanon (I-27)

*tert*-Butyl-4-{4-[(E)-2-(naphthalen-1-yl)ethenyl]-1,3-thiazol-2-yl}piperidin-1-carboxylat (**IV-2**, 171 mg) wird analog zu Beispiel II-2 entschützt und dann analog zu Beispiel I-81 mit [5-Methyl-3-(trifluormethyl)-1H-pyrazol-1-yl]essigsäure (100 mg) umgesetzt. Nach chromatografischer Reinigung erhält man 2-[5-Methyl-3-(trifluormethyl)-1H-pyrazol-1-yl]-1-(4-{4-[(E)-2-(naphthalen-1-yl)ethenyl]-1,3-thiazol-2-yl}piperidin-1-yl)ethanon (180 mg).
¹H NMR (DMSO-d₆): δ 8.23 (d, 1H), 8.16 (d, 1H), 7.95 (d, 1H), 7.88 (d, 1H), 7.83 (d, 1H), 7.64 (s, 1H), 7.63-7.51 (m, 3H), 7.27 (d, 1H), 6.46 (s, 1H), 5.24 (bs, 2H), 4.38 (bs, 1H), 4.03 (bs, 1H), 3.40 (m, 1H), 3.32 (bs, 1H), 2.94 (bs, 1H), 2.24 (s, 3H), 2.23-2.12 (m, 2H), 1.85 (bs, 1H), 1.70 (bs, 1H) ppm
MS (ESI): 511 ([M+H]⁺)

### 2-[5-Methyl-3-(trifluormethyl)-1H-pyrazol-1-yl]-1-(4-{4-[(Z)-2-(naphthalen-1-yl)ethenyl]-1,3-thiazol-2-yl}piperidin-1-yl)ethanon (I-2)

*tert*-Butyl-4-{4-[(Z)-2-(naphthalen-1-yl)ethenyl]-1,3-thiazol-2-yl}piperidin-1-carboxylat (**IV-3,** 171 mg) wird analog zu Beispiel II-2 entschützt und dann analog zu Beispiel I-81 mit [5-Methyl-3-(trifluormethyl)-1H-pyrazol-1-yl]essigsäure (100 mg) umgesetzt. Man erhält nach chromatografischer Reinigung 2-[5-Methyl-3-(trifluormethyl)-1H-pyrazol-1-yl]-1-(4-{4-[(E)-2-(naphthalen-1-yl)ethenyl]-1,3-thiazol-2-yl}piperidin-1-yl)ethanon (200 mg).
¹H NMR (DMSO-d₆): δ 7.95-7.92 (m, 2H), 7.87-7.84 (m, 1H), 7.52-7.43 (m, 4H), 7.11 (d, 1H), 6.93 (d, 1H), 6.88 (s, 1H), 6.45 (s, 1H), 5.14 (s, 2H), 4.05 (bs, 1H), 3.74 (bs, 1H), 3.20-3.05 (m, 2H), 2.88 (bs, 1H), 2.24 (s, 3H), 1.90-1.78 (bs, 2H), 1.50 (bs, 1H), 1.35 (bs, 1H) ppm
MS (ESI): 511 ([M+H]⁺)

### 2-[5-Methyl-3-(trifluormethyl)-1H-pyrazol-1-yl]-1-(4-{4-[(1Z)-4-phenylpent-1-en-1-yl]-1,3-thiazol-2-yl}piperidin-1-yl)ethanon (I-3)

*tert*-Butyl-4-{4-[(1Z)-4-phenylpent-1-en-1-yl]-1,3-thiazol-2-yl}piperidin-1-carboxylat (**IV-4**, 168 mg), wird analog zu Beispiel II-2 entschützt und dann analog zu Beispiel I-81 mit [5-Methyl-3-(trifluormethyl)-1H-pyrazol-1-yl]essigsäure (100 mg) umgesetzt. Man erhält 2-[5-Methyl-3-(trifluormethyl)-1H-pyrazol-1-yl]-1-(4-{4-[(1Z)-4-phenylpent-1-en-1-yl]-1,3-thiazol-2-yl}piperidin-1-yl)ethanon (200 mg).
¹H NMR (DMSO-d₆): δ 7.32 (s, 1H), 7.29-7.22 (m, 4H), 7.15 (m, 1H), 6.44 (s, 1H), 6.35 (d, 1H), 5.60 (m, 1H), 5.20 (bs, 2H), 4.30 (bs, 1H), 3.98 (bs, 1H), 3.38-3.25 (m, 2H), 3.05-2.84 (m, 4H), 2.22 (s, 3H), 2.17-2.05 (m, 2H), 1.85-1.56 (m, 2H), 1.26 (d, 3H) ppm
MS (ESI): 503 ([M+H]⁺)

### 2-[5-Methyl-3-(trifluormethyl)-1H-pyrazol-1-yl]-1-(4-{4-[(Z)-2-(2-naphthyl)vinyl]-1,3-thiazol-2-yl}piperidin-1-yl)ethanon (I-20)

(2-Naphthylmethyl)(triphenyl)phosphoniumchlorid (454 mg) wird in 5 mL Tetrahydrofuran gelöst, unter Argon auf 0 °C abgekühlt und mit Kalium-*tert*.butanolat (125 mg) versetzt, wobei die Lösung dunkelrot wird. Nach 10 min Rühren wird 2-(1-{[5-Methyl-3-(trifluormethyl)-1H-pyrazol-1-yl]acetyl}piperidin-4-yl)-1,3-thiazol-4-carbaldehyd (200 mg) zugegeben. Das Gemisch wird 30 min bei 0 °C weitergerührt und dann langsam auf Raumtemperatur erwärmt. Nach weiteren 20 min wird mit gesättigter Ammoniumchloridlösung versetzt und die wässrige Phase abgetrennt. Nach Extraktion der wässrigen Phase mit Essigsäure-ethylester werden die vereinigten organischen Phasen über Natriumsulfat getrocknet und unter vermindertem Druck eingeengt. Nach chromatografischer Reinigung erhält man 2-[5-Methyl-3-(trifluormethyl)-1H-pyrazol-1-yl]-1-(4-{4-[(Z)-2-(2-naphthyl)vinyl]-1,3-thiazol-2-yl}piperidin-1-yl)ethanon (260 mg).
MS (ESI): 511 ([M+H]⁺)

### 2-[5-Methyl-3-(trifluormethyl)-1H-pyrazol-1-yl]-1-(4-{4-[(E)-2-phenylethenyl]-1,3-thiazol-2-yl}piperidin-1-yl)ethanthion (I-93)

Zu einer Lösung von 2-[5-Methyl-3-(trifluormethyl)-1H-pyrazol-1-yl]-1-(4-{4-[(E)-2-phenylethenyl]-1,3-thiazol-2-yl}piperidin-1-yl)ethanon (141 mg) in Chloroform (1 mL) und 1,2-dimethoxyethane (2 mL) wird Methoxyphenyldithiophosphonsaeureanhydrid (62 mg) zugegeben. Das Reaktionsgemisch wird 24 Stunden bei Raumtemperatur und 3 Stunden bei 40°C gerührt. Nach Entfernen des Lösungsmittels unter vermindertem Druck wird der Rückstand chromatografisch gereinigt. Man erhält 2-[5-Methyl-3-(trifluormethyl)-1H-pyrazol-1-yl]-1-(4-{4-[(E)-2-phenylethenyl]-1,3-thiazol-2-yl}piperidin-1-yl)ethanthion (45 mg) als farbloses Öl.
¹H NMR (CD₃CN): δ 7.55 (d, 2H), 7.43 (d, 1H), 7.37 (t, 2H), 7.30-7.28 (m, 2H), 7.18 (d, 1H), 6.39 (s, 1H), 5.41 (d, 1H), 5.26 (s, 2H), 4.42 (d, 1H), 3.54 (t, 1H), 3.45 (m, 1H), 3.38 (t, 1H), 2.31 (s, 3H), 2.30-2.23 (m, 2H), 1.95-1.80 (m, 2H) ppm
MS (ESI): 477 ([M+H]⁺)

### Herstellung von Verbindungen der Formel (Ic)

### 2-[5-Methyl-3-(trinuormethyl)-1H-pyrazol-1-yl]-1-{4-[4-(naphthalen-1-ylethinyl)-1,3-thiazol-2-yl]piperidin-1-yl}ethanon (I-50)

*tert*-Butyl-4-[4-(naphthalen-1-ylethinyl)-1,3-thiazol-2-yl]piperidin-1-carboxylat (**IV-6**, 200 mg) wird analog zu Beispiel II-2 entschützt und dann analog zu Beispiel I-81 mit [5-Methyl-3-(trifluormethyl)-1H-pyrazol-1-yl]essigsäure (117 mg) umgesetzt. Man erhält nach chromatografischer Reinigung 2-[5-Methyl-3-(trifluormethyl)-1H-pyrazol-1-yl]-1-{4-[4-(naphthalen-1-ylethinyl)-1,3-thiazol-2-yl]piperidin-1-yl}ethanon (112 mg).
¹H NMR (CD₃CN): δ 8.39 (d, 1H), 7.96 (d, 2H), 7.80 (d, 1H), 7.72 (s, 1H), 7.67-7.50 (m, 3H), 6.37 (s, 1H), 5.05 (s, 2H), 4.45 (bs, 1H), 3.98 (bs, 1H), 3.40-3.20 (m, 2H), 2.93 (bs, 1H), 2.25 (s, 3H), 2.25-2.15 (m, 2H), 1.90-1.75 (m, 2H) ppm
MS (ESI): 509 ([M+H]⁺)

### 2-[5-Methyl-3-(trifluormethyl)-1H-pyrazol-1-yl]-1-{4-[4-(pyridin-3-ylethinyl)-1,3-thiazol-2-yl]piperidin-1-yl}ethanon (I-51)

*tert*-Butyl-4-[4-(pyridin-3-ylethinyl)-1,3-thiazol-2-yl]piperidin-1-carboxylat (**IV-7**, 469 mg) wird analog zu Beispiel II-2 entschützt, so dass 3-{[2-(Piperidin-4-yl)-1,3-thiazol-4-yl]ethinyl}pyridinhydrochlorid (407 mg) erhalten wird. Dieses wird umgehend bei Raumtemperatur mit [5-Methyl-3-(trifluormethyl)-1H-pyrazol-1-yl]essigsäure (277 mg) in Gegenwart von 4-Dimethylaminopyridin (16 mg) und 1-Ethyl-3-(3'-dimethylaminopropyl)carbodiimide (268 mg) in Dichlormethan (5 mL) umgesetzt. Nach Rühren bei Raumtemperatur über Nacht, Einengen und chromatografischer Reinigung erhält man 2-[5-Methyl-3-(trifluormethyl)-1H-pyrazol-1-yl]-1-{4-[4-(pyridin-3-ylethinyl)-1,3-thiazol-2-yl]piperidin-1-yl}ethanon (65 mg).
¹H NMR (CD₃CN): δ 8.74 (dd, 1H), 8.59 (dd, 1H), 7.99 (s, 1H), 7.96 (m, 1H), 7.45 (dd, 1H), 6.45 (s, 1H), 5.22 (bs, 2H), 4.35 (bs, 1H), 4.00 (bs, 1H), 3.40-3.20 (m, 3H), 2.22 (s, 3H), 2.17-2.10 (m, 2H), 1.90-1.55 (m, 2H) ppm
MS (ESI): 460 ([M+H]⁺)

### 2-[5-Methyl-3-(trifluormethyl)-1H-pyrazol-1-yl]-1-{4-[4-(3-phenylprop-1-in-1-yl)-1,3-thiazol-2-yl]piperidin-1-yl}ethanon (I-52)

*tert*-Butyl-4-[4-(3-phenylprop-1-in-1-yl)-1,3-thiazol-2-yl]piperidin-1-carboxylat (**IV-5**, 320 mg) wird dann analog zu Beispiel II-2 entschützt und dann analog zu Beispiel I-81 mit [5-Methyl-3-(trifluormethyl)-1H-pyrazol-1-yl]essigsäure (174 mg) umgesetzt. Nach chromatografischer Reinigung erhält man 2-[5-Methyl-3-(trifluormethyl)-1H-pyrazol-1-yl]-1-{4-[4-(3-phenylprop-1-in-1-yl)-1,3-thiazol-2-yl]piperidin-1-yl}ethanon (33 mg).
¹H NMR (CD₃CN): δ 7.46 (s, 1H), 7.42-7.34 (m, 4H), 7.30-7.25 (m, 1H), 6.39 (s, 1H), 5.09 (d, 1H), 5.02 (d, 1H), 4.45 (d, 1H), 3.91 (d, 1H), 3.84 (s, 2H), 3.25 (m, 2H), 2.82 (t, 1H), 2.23 (s, 3H), 2.18-2.05 (m, 2H), 1.82 (qd, 1H), 1.66 (qd, 1H) ppm
MS (ESI): 473 ([M+H]⁺)

### 1-[4-(4-Ethinyl-1,3-thiazol-2-yl)piperidin-1-yl]-2-[5-methyl-3-(trifluormethyl)-1H-pyrazol-1-yl]ethanon (I-86)

2-(1-{[5-Methyl-3-(trifluormethyl)-1H-pyrazol-1-yl]acetyl}piperidin-4-yl)-1,3-thiazol-4-carbaldehyd (200 mg) wird unter Argon in Methanol 5 mL) gelöst und mit Kaliumcarbonat (215 mg) und Dimethyl-1-diazo-2-oxopropylphosphonat (199 mg) versetzt. Das Gemisch wird bei 40 °C gerührt. Nach wässriger Aufarbeitung wird mit Essigsäure-ethylester extrahiert, die Extrakte mit Natriumsulfat getrocknet und unter vermindertem Druck eingeengt. Der Rückstand wird chromatografisch gereinigt. Man erhält 1-[4-(4-ethinyl-1,3-thiazol-2-yl)piperidin-1-yl]-2-[5-methyl-3-(trifluormethyl)-1H-pyrazol-1-yl]ethanon (67 mg).
¹H NMR (DMSO-d6): δ 7.86 (s, 1H), 6.44 (s, 1H), 5.20 (bs, 2H), 4.31 (bs, 1H), 4.08 (s, 1H), 3.98 (bs, 1H), 3.31 (m, 1H), 3.28 (bs, 1H), 2.88 (bs, 1H), 2.22 (s, 3H), 2.13-2.05 (m, 2H), 1.78 (bs, 1H), 1.61 (bs, 1 H) ppm
MS (ESI): 383 ([M+H]⁺)

### 2-[5-Methyl-3-(trifluormethyl)-1H-pyrazol-1-yl]-1-(4-{4-[(trimethylsilyl)ethinyl]-1,3-thiazol-2-yl}piperidin-1-yl)ethanon (I-98)

1-[4-(4-Ethinyl-1,3-thiazol-2-yl)piperidin-1-yl]-2-[5-methyl-3-(trifluormethyl)-1H-pyrazol-1-yl]ethanon (**I-86,** 525 mg) wird unter Argon bei -78 °C in Tetrahydrofuran (10 mL) gelöst und Lithiumhexamethyldisilazan (1M in Hexan, 1.40 mL) wird zugetropft. Nach 10 min wird Trimethylsilylchlorid (179 mg) zugetropft. Nach Rühren für 30 Minuten wird mit gesättigter Ammoniumchloridlösung versetzt und mit Essigsäure-ethylester extrahiert. Die organischen Extrakte werden über Natriumsulfat getrocknet und unter vermindertem Druck eingeengt. Der Rückstand wird chromatografisch gereinigt. Man erhält 2-[5-Methyl-3-(trifluormethyl)-1H-pyrazol-1-yl]-1-(4-{4-[(trimethylsilyl)ethinyl]-1,3-thiazol-2-yl}piperidin-1-yl)ethanon (540 mg).
¹H NMR (DMSO-d6): δ 7.70 (s, 1H), 6.27 (s, 1H), 5.08 (d, 1H), 5.01 (d, 1H), 4.12 (bd, 1H), 3.76 (bd, 1H), 3.05 (t, 1H), 2.59 (t, 1H), 1.97 (s, 3H), 1.88-1.77 (m, 2H), 1.56 (tdd, 1H), 1.30 (tdd, 1H), 0.00 (s, 9H) ppm
MS (ESI): 455 ([M+1]⁺)

### 1-(4-{4-[(4-Methoxyphenyl)ethinyl]-1,3-thiazol-2-yl}piperidin-1-yl)-2-[5-methyl-3-(trifluormethyl)-1H-pyrazol-1-yl]ethanon (I-100)

Zu einer Lösung von 2-[5-Methyl-3-(trifluormethyl)-1H-pyrazol-1-yl]-1-(4-{4-[(trimethylsilyl)ethinyl]-1,3-thiazol-2-yl}piperidin-1-yl)ethanon (**I-98,** 200 mg) und 1-Brom-4-methoxybenzol (91 mg) in entgastem N,N-Dimethylformamid (2 mL) werden Tetrakis(triphenylphosphin)palladium(0) (25 mg), Kupfer(I)iodid (8 mg) und Triethylamin (53 mg) gegeben. Nach Erwärmen auf 85 °C wird Tetra-n-butylammoniumfluorid (1M in THF, 0.48 mL) zugetropft. Das Reaktionsgemisch wird anschließend mit Wasser verdünnt und mit Essigsäureethylester extrahiert. Die organischen Extrakte werden über Natriumsulfat getrocknet und unter vermindertem Druck eingeengt. Nach chromatografischer Reinigung erhält man 1-(4-{4-[(4-Methoxyphenyl)ethinyl]-1,3-thiazol-2-yl}piperidin-1-yl)-2-[5-methyl-3-(trifluormethyl)-1H-pyrazol-1-yl]ethanon (84 mg).
¹H NMR (DMSO-d6): δ 7.84 (s, 1H), 7.49 (d, 2H), 6.98 (d, 2H), 6.45 (s, 1H), 5.22 (bs, 2H), 4.33 (bs, 1H), 3.98 (bs, 1H), 3.80 (s, 3H), 3.34 (m, 1H), 3.28 (bs, 1H), 2.89 (bs, 1H), 2.22 (s, 3H), 2.15-2.09 (m, 2H), 1.80 (bs, 1H), 1.63 (bs, 1H) ppm
MS (ESI): 489 ([M+H]⁺)

### 1-(4-{4-[(2-Methoxyphenyl)ethinyl]-1,3-thiazol-2-yl}piperidin-1-yl)-2-[5-methyl-3-(trifluormethyl)-1H-pyrazol-1-yl]ethanon (I-103)

Zu einer Lösung von 1-[4-(4-ethinyl-1,3-thiazol-2-yl)piperidin-1-yl]-2-[5-methyl-3-(trifluormethyl)-1H-pyrazol-1-yl]ethanon (**I-86,** 150 mg) und 1-Iod-2-methoxybenzol (101 mg) in entgastem N,N-Dimethylformamid (1.5 mL) werden Tetrakis(triphenylphosphin)palladium(0) (25 mg), Kupfer(I)iodid (7 mg) und Triethylamin (48 mg) gegeben. Nach Rühren bei 85 °C für 2 Stunden wird das Reaktionsgemisch anschließend mit Wasser verdünnt und mit Essigsäureethylester extrahiert. Die organischen Extrakte werden über Natriumsulfat getrocknet und unter vermindertem Druck eingeengt. Nach chromatografischer Reinigung erhält man 1-(4-{4-[(2-Methoxyphenyl)ethinyl]-1,3-thiazol-2-yl}piperidin-1-yl)-2-[5-methyl-3-(trifluormethyl)-1H-pyrazol-1-yl]ethanon (100 mg).
1H NMR (DMSO-d6): δ 7.86 (s, 1H), 7.47 (dd, 1H), 7.40 (td, 1H), 7.09 (d, 1H), 6.98 (td, 1H), 6.45 (s, 1H), 5.23 (bs, 2H), 4.33 (bs, 1H), 3.98 (bs, 1H), 3.86 (s, 3H), 3.35 (m, 1H), 3.29 (bs, 1H), 2.89 (bs, 1H), 2.23 (s, 3H), 2.15-2.08 (m, 2H), 1.80 (bs, 1H), 1.63 (bs, 1H) ppm
MS (ESI): 489 ([M+H]⁺)

In den folgenden Beispielen bezeichnet "*" Vergleichsbeispiele.

### Beispiele

Analog zu den zuvor angegebenen Methoden können die in der nachstehenden Tabelle 1 aufgeführten Verbindungen der Formel I erhalten werden. 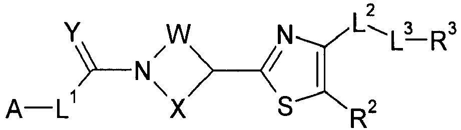 mit

**Tabelle I**

| **Bsp** | **A** | **L¹** | **Y** | **W** | **X** | **R²** | **L²** | **L³** | **R³** | **log p** |
|---|---|---|---|---|---|---|---|---|---|---|
| I-1 | 5-Methyl-3-(trifluormethyl)-1H-pyrazol-1-yl | CH₂ | O | CH₂CH₂ | CH₂CH₂ | H | CH=CH (Z) | Bindung | Phenyl | 3,91^{[b]} |
| I-2 | 5-Methyl-3-(trifluormethyl)-1H-pyrazol-1-yl | CH₂ | O | CH₂CH₂ | CH₂CH₂ | H | CH=CH (Z) | Bindung | Naphthalen-1-yl | 4,52^{[b]} |
| I-3 | 5-Methyl-3-(trifluormethyl)-1H-pyrazol-1-yl | CH₂ | O | CH₂CH₂ | CH₂CH₂ | H | CH=CH (Z) | CH₂CHCH₃ | Phenyl | 4,84^{[b]} |
| I-4 | 5-Methyl-3-(trifluormethyl)-1H-pyrazol-1-yl | CH₂ | O | CH₂CH₂ | CH₂CH₂ | H | CH=CH (Z) | Bindung | Cyclohexyl | |
| I-5 | 3-(Trifluormethyl)-1H-pyrazol-1-yl | CH₂ | O | CH₂CH₂ | CH₂CH₂ | H | CH=CH (Z) | Bindung | Cyclohexyl | |
| I-6 | 4-Chlor-5-methyl-3-(trifluormethyl)-1H-pyrazol-1-yl | CH₂ | O | CH₂CH₂ | CH₂CH₂ | H | CH=CH (Z) | Bindung | Cyclohexyl | |
| I-7 | 3-(Trifluormethyl)-1H-pyrazol-1-yl | CH₂ | O | CH₂CH₂ | CH₂CH₂ | H | CH=CH (Z) | Bindung | Phenyl | 3,72^{[b]} |
| I-8 | 4-Chlor-5-methyl-3-(trifluormethyl)-1H-pyrazol-1-yl | CH₂ | O | CH₂CH₂ | CH₂CH₂ | H | CH=CH (Z) | Bindung | Phenyl | 4,56^{[b]} |
| I-9 | 5-Methyl-3-(trifluormethyl)-1H-pyrazol-1-yl | CH₂ | O | CH₂CH₂ | CH₂CH₂ | H | CH=CH (Z) | Bindung | 4-Chlorphenyl | 4,41^{[b]} |
| I-10 | 3-(Trifluormethyl)-1H-pyrazol-1-yl | CH₂ | O | CH₂CH₂ | CH₂CH₂ | H | CH=CH (Z) | Bindung | 4-Chlorphenyl | 4,2^{[b]} |
| I-11 | 4-Chlor-5-methyl-3-(trifluormethyl)-1H-pyrazol-1-yl | CH₂ | O | CH₂CH₂ | CH₂CH₂ | H | CH=CH (Z) | Bindung | 4-Chlorphenyl | 4,92^{[b]} |
| I-12 | 5-Methyl-3-(trifluormethyl)-1H-pyrazol-1-yl | CH₂ | O | CH₂CH₂ | CH₂CH₂ | H | CH=CH (Z) | Bindung | 4-Methoxyphenyl | 3,9^{[b]} |
| I-13 | 3-(Trifluormethyl)-1H-pyrazol-1-yl | CH₂ | O | CH₂CH₂ | CH₂CH₂ | H | CH=CH (Z) | Bindung | 4-Methoxyphenyl | |
| I-14 | 4-Chlor-5-methyl-3-(trifluormethyl)-1H-pyrazol-1-yl | CH₂ | O | CH₂CH₂ | CH₂CH₂ | H | CH=CH (Z) | Bindung | 4-Methoxyphenyl | 4,46^{[b]} |
| I-15 | 5-Methyl-3-(trifluormethyl)-1H-pyrazol-1-yl | CH₂ | O | CH₂CH₂ | CH₂CH₂ | H | CH=CH (Z) | Bindung | 3-(Trifluormethyl)phenyl | 4,51^{[b]} |
| I-16 | 3-(Trifluormethyl)-1H-pyrazol-1-yl | CH₂ | O | CH₂CH₂ | CH₂CH₂ | H | CH=CH (Z) | Bindung | 3-(Trifluormethyl)phenyl | 4,3^{[b]} |
| I-17 | 4-Chlor-5-methyl-3-(trifluormethyl)-1H-pyrazol-1-yl | CH₂ | O | CH₂CH₂ | CH₂CH₂ | H | CH=CH (Z) | Bindung | 3-(Trifluormethyl)phenyl | 5,03^{[b]} |
| I-18 | 3-(Trifluormethyl)-1H-pyrazol-1-yl | CH₂ | O | CH₂CH₂ | CH₂CH₂ | H | CH=CH (Z) | Bindung | Naphthalen-1-yl | 4,35^{[b]} |
| I-19 | 4-Chlor-5-methyl-3-(trifluormethyl)-1H-pyrazol-1-yl | CH₂ | O | CH₂CH₂ | CH₂CH₂ | H | CH=CH (Z) | Bindung | Naphthalen-1-yl | 5,14^{[b]} |
| I-20 | 5-Methyl-3-(trifluormethyl)-1H-pyrazol-1-yl | CH₂ | O | CH₂CH₂ | CH₂CH₂ | H | CH=CH (Z) | Bindung | Naphthalen-2-yl | 4,61^{[b]} |
| I-21 | 3-(Trifluormethyl)-1H-pyrazol-1-yl | CH₂ | O | CH₂CH₂ | CH₂CH₂ | H | CH=CH (Z) | Bindung | Naphthalen-2-yl | 4,35^{[b]} |
| I-22 | 4-Chlor-5-methyl-3-(trifluormethyl)-1H-pyrazol-1-yl | CH₂ | O | CH₂CH₂ | CH₂CH₂ | H | CH=CH (Z) | Bindung | Naphthalen-2-yl | 5,08^{[b]} |
| I-23 | 5-Methyl-3-(trifluormethyl)-1H-pyrazol-1-yl | CH₂ | O | CH₂CH₂ | CH₂CH₂ | H | CH=CH (Z) | CH₂ | Phenyl | 4,41^{[b]} |
| I-24 | 3-(Trifluormethyl)-1H-pyrazol-1-yl | CH₂ | O | CH₂CH₂ | CH₂CH₂ | H | CH=CH (Z) | CH₂ | Phenyl | |
| I-25 | 4-Chlor-5-methyl-3-(trifluormethyl)-1 H-pyrazol-1-yl | CH₂ | O | CH₂CH₂ | CH₂CH₂ | H | CH=CH (Z) | CH₂ | Phenyl | |
| I-26 | 5-Methyl-3-(trifluormethyl)-1H-pyrazol-1-yl | CH₂ | O | CH₂CH₂ | CH₂CH₂ | H | CH=CH (E) | Bindung | Phenyl | 3,99^{[b]} |
| I-27 | 5-Methyl-3-(trifluormethyl)-1H-pyrazol-1-yl | CH₂ | O | CH₂CH₂ | CH₂CH₂ | H | CH=CH (E) | Bindung | Naphthalen-1-yl | 4,63^{[b]} |
| I-28* | 5-Methyl-3-(trifluormethyl)-1H-pyrazol-1-yl | CH₂ | O | CH₂CH₂ | CH₂CH₂ | H | CH=CH (E) | CH₂ | CF₃ | |
| I-29* | 5-Methyl-3-(trifluormethyl)-1H-pyrazol-1-yl | CH₂ | O | CH₂CH₂ | CH₂CH₂ | H | CH=CH (E) | CH₂ | tert-Butyl | |
| I-30* | 5-Methyl-3-(trifluormethyl)-1H-pyrazol-1-yl | CH₂ | O | CH₂CH₂ | CH₂CH₂ | H | CH=CH (E) | CH₂ | Ethenyl | |
| I-31* | 5-Methyl-3-(trifluormethyl)-1 H-pyrazol-1-yl | CH₂ | O | CH₂CH₂ | CH₂CH₂ | H | CH=CH (E) | CH₂ | Ethinyl | |
| I-32 | 5-Methyl-3-(trifluormethyl)-1H-pyrazol-1-yl | CH₂ | O | CH₂CH₂ | CH₂CH₂ | H | CH=CH (E) | CH₂ | Cyclohexyl | |
| I-33 | 5-Methyl-3-(trifluormethyl)-1H-pyrazol-1-yl | CH₂ | O | CH₂CH₂ | CH₂CH₂ | H | CH=CH (E) | CH₂ | Morpholin-4-yl | |
| I-34 | 5-Methyl-3-(trifluormethyl)-1H-pyrazol-1-yl | CH₂ | O | CH₂CH₂ | CH₂CH₂ | H | CH=CH (E) | Bindung | Pyridin-3-yl | |
| I-35 | 5-(Difluormethyl)-3-methyl-1H-pyrazol-1-yl | CH₂ | O | CH₂CH₂ | CH₂CH₂ | H | CH₂CH₂ | Bindung | Phenyl | 3,37^{[b]} |
| I-36 | 3,5-Dichlor-1H-pyrazol-1-yl | CH₂ | O | CH₂CH₂ | CH₂CH₂ | H | CH₂CH₂ | Bindung | Phenyl | |
| I-37 | 3,5-Dibrom-1H-pyrazol-1-yl | CH₂ | O | CH₂CH₂ | CH₂CH₂ | H | CH₂CH₂ | Bindung | Phenyl | |
| I-38 | 2-Brom-5-(trifluormethyl)phenyl | CH₂ | O | CH₂CH₂ | CH₂CH₂ | H | CH₂CH₂ | Bindung | Phenyl | |
| I-39 | 5-Brom-2-methylphenyl | CH₂ | O | CH₂CH₂ | CH₂CH₂ | H | CH₂CH₂ | Bindung | Phenyl | |
| I-40 | 2,5-Dichlorphenyl | CH₂ | O | CH₂CH₂ | CH₂CH₂ | H | CH₂CH₂ | Bindung | Phenyl | |
| I-41 | 2,5-Dibromphenyl | CH₂ | O | CH₂CH₂ | CH₂CH₂ | H | CH₂CH₂ | Bindung | Phenyl | |
| I-42 | 5-Iod-2-methylphenyl | CH₂ | O | CH₂CH₂ | CH₂CH₂ | H | CH₂CH₂ | Bindung | Phenyl | |
| I-43 | 3,5-Bis(trifluormethyl)-1H-pyrazol-1-yl | CH₂ | O | CH₂CH₂ | CH₂CH₂ | H | CH₂CH₂ | Bindung | Naphthalen-1-yl | 4,89^{[b]} |
| I-44 | 5-(Difluormethyl)-3-methyl-1H-pyrazol-1-yl | CH₂ | O | CH₂CH₂ | CH₂CH₂ | H | CH₂CH₂ | Bindung | Naphthalen-1-yl | |
| I-45 | 3,5-Dichlor-1H-pyrazol-1-yl | CH₂ | O | CH₂CH₂ | CH₂CH₂ | H | CH₂CH₂ | Bindung | Naphthalen-1-yl | |
| I-46 | 3,5-Dibrom-1H-pyrazol-1-yl | CH₂ | O | CH₂CH₂ | CH₂CH₂ | H | CH₂CH₂ | Bindung | Naphthalen-1-yl | |
| I-47 | 2-Chlor-5-(trifluormethyl)phenyl | CH₂ | O | CH₂CH₂ | CH₂CH₂ | H | CH₂CH₂ | Bindung | Naphthalen-1-yl | |
| I-48 | 2-Brom-5-(trifluormethyl)phenyl | CH₂ | O | CH₂CH₂ | CH₂CH₂ | H | CH₂CH₂ | Bindung | Naphthalen-1-yl | |
| I-49 | 5-Brom-2-methylphenyl | CH₂ | O | CH₂CH₂ | CH₂CH₂ | H | CH₂CH₂ | Bindung | Naphthalen-1-yl | |
| I-50 | 5-Methyl-3-(trifluormethyl)-1H-pyrazol-1-yl | CH₂ | O | CH₂CH₂ | CH₂CH₂ | H | C≡C | Bindung | Naphthalen-1-yl | 4,66^{[c]}; 4,5^{[b]} |
| I-51 | 5-Methyl-3-(trifluormethyl)-1H-pyrazol-1-yl | CH₂ | O | CH₂CH₂ | CH₂CH₂ | H | C≡C | Bindung | Pyridin-3-yl | 2,68^{[b]} |
| I-52 | 5-Methyl-3-(trifluormethyl)-1H-pyrazol-1-yl | CH₂ | O | CH₂CH₂ | CH₂CH₂ | H | C≡C | CH₂ | Phenyl | 3,88^{[b]} |
| I-53 | 5-Methyl-3-(trifluormethyl)-1H-pyrazol-1-yl | CH₂ | O | CH₂CH₂ | CH₂CH₂ | H | CH₂CH₂ | Bindung | Phenyl | 3,79^{[a]} |
| I-54 | 3,5-Dimethyl-1H-pyrazol-1-yl | CH₂ | O | CH₂CH₂ | CH₂CH₂ | H | CH₂CH₂ | Bindung | Phenyl | 2,86^{[a]} |
| I-55 | 3-(Trifluormethyl)-1H-pyrazol-1-yl | CH₂ | O | CH₂CH₂ | CH₂CH₂ | H | CH₂CH₂ | Bindung | Phenyl | 3,57^{[b]} |
| I-56 | 3-(Trifluormethyl)phenyl | CH₂ | O | CH₂CH₂ | CH₂CH₂ | H | CH₂CH₂ | Bindung | Phenyl | 4,26^{[b]} |
| I-57 | 3-Chlorphenyl | CH₂ | O | CH₂CH₂ | CH₂CH₂ | H | CH₂CH₂ | Bindung | Phenyl | 4,1^{[b]} |
| I-58* | CH₃ | CH₂CH₂C H₂ | O | CH₂CH₂ | CH₂CH₂ | H | CH₂CH₂ | Bindung | Phenyl | 3,73^{[b]} |
| I-59 | 3,5-Bis(trifluormethyl)-1H-pyrazol-1-yl | CH₂ | O | CH₂CH₂ | CH₂CH₂ | H | CH₂CH₂ | Bindung | Phenyl | 4,45^{[b]} |
| I-60 | 4-Chlor-5-methyl-3-(trifluormethyl)-1H-pyrazol-1-yl | CH₂ | O | CH₂CH₂ | CH₂CH₂ | H | CH₂CH₂ | Bindung | Phenyl | 4,34^{[b]} |
| I-61 | 2,5-Bis(trifluormethyl)phenyl | Bindung | O | CH₂CH₂ | CH₂CH₂ | H | CH₂CH₂ | Bindung | Phenyl | 4,67^{[b]} |
| I-62 | 3-(Trifluormethyl)phenyl | Bindung | O | CH₂CH₂ | CH₂CH₂ | H | CH₂CH₂ | Bindung | Phenyl | 4,23^{[b]} |
| I-63 | Thiophen-3-yl | CH₂ | O | CH₂CH₂ | CH₂CH₂ | H | CH₂CH₂ | Bindung | Phenyl | 3,47^{[b]} |
| I-64 | 4-Chlor-5-methyl-3-(trifluormethyl)-1 H-pyrazol-1-yl | CHCH₃ | O | CH₂CH₂ | CH₂CH₂ | H | CH₂CH₂ | Bindung | Phenyl | 4,62^{[b]} |
| I-65 | 5-Chlor-1-methyl-1H-pyrazol-4-yl | CH₂ | O | CH₂CH₂ | CH₂CH₂ | H | CH₂CH₂ | Bindung | Phenyl | 2,98^{[b]} |
| I-66 | 3,4-Dimethoxyphenyl | CH₂CH₂ | O | CH₂CH₂ | CH₂CH₂ | H | CH₂CH₂ | Bindung | Phenyl | 3,43^{[b]} |
| I-67 | 2-Chlor-5-(trifluormethyl)phenyl | CH₂ | O | CH₂CH₂ | CH₂CH₂ | H | CH₂CH₂ | Bindung | Phenyl | 4.73^{[b]} |
| I-68 | 3-(Trifluormethyl)-1H-pyrazol-1-yl | CHCH₃ | O | CH₂CH₂ | CH₂CH₂ | H | CH₂CH₂ | Bindung | Phenyl | 3,93^{[b]} |
| I-69 | 1H-Pyrazol-1-yl | CH₂ | O | CH₂CH₂ | CH₂CH₂ | H | CH₂CH₂ | Bindung | Phenyl | 2,56^{[b]} |
| I-70 | 2H-Indazol-2-yl | CH₂ | O | CH₂CH₂ | CH₂CH₂ | H | CH₂CH₂ | Bindung | Phenyl | 3,14^{[b]} |
| I-71 | 1,3-Benzothiazol-2-yl | CH₂ | O | CH₂CH₂ | CH₂CH₂ | H | CH₂CH₂ | Bindung | Phenyl | 3,68^{[b]} |
| I-72 | 1H-Benzimidazol-2-yl | CH₂ | O | CH₂CH₂ | CH₂CH₂ | H | CH₂CH₂ | Bindung | Phenyl | |
| I-73* | 5-Methyl-3-(trifluormethyl)-1H-pyrazol-1-yl | CH₂ | O | CH₂CH₂ | CH₂CH₂ | 2-Methylpro pyl | CH₂ | Bindung | H | 3,75^{[b]} |
| I-74 | 3,5-Dimethyl-1H-pyrazol-1-yl | CH₂ | O | CH₂CH₂ | CH₂CH₂ | H | C≡C | Bindung | Naphthalen-1-yl | 3,85^{[c]} |
| I-75 | Pyridin-4-yl | CH₂ | O | CH₂CH₂ | CH₂CH₂ | H | CH₂CH₂ | Bindung | Phenyl | |
| I-76 | 3,5-Dimethyl-1H-pyrazol-1-yl | CH₂ | O | CH₂CH₂ | CH₂CH₂ | H | CH=CH (Z) | Bindung | Naphthalen-1-yl | 3,73^{[b]} |
| I-77 | 3,5-Dimethyl-1H-pyrazol-1-yl | CH₂ | O | CH₂CH₂ | CH₂CH₂ | H | CH=CH (E) | Bindung | Naphthalen-1-yl | 3,58^{[b]} |
| I-78 | 5-Methyl-3-(trifluormethyl)-1H-pyrazol-1-yl | CH₂ | O | CH₂CH₂ | CH₂CH₂ | Br | CH₂CH₂ | Bindung | Phenyl | 4,71^{[b]} |
| I-79 | 3,5-Dimethyl-1H-pyrazol-1-yl | CH₂ | O | CH₂CH₂ | CH₂CH₂ | H | CH₂CH₂ | Bindung | Naphthalen-1-yl | 3,39^{[c]} |
| I-80 | 5-Methyl-3-(trifluormethyl)-1H-pyrazol-1-yl | CH₂ | O | CH₂CH₂ | CH₂CH₂ | H | CH₂CH₂ | CH₂CHCH₃ | Phenyl | 4,77^{[b]} |
| I-81 | 5-Methyl-3-(trifluormethyl)-1H-pyrazol-1-yl | CH₂ | O | CH₂CH₂ | CH₂CH₂ | H | CH₂CH₂ | Bindung | Naphthalen-1-yl | 4,37^{[b]} |
| I-82 | 5-Methyl-3-(trifluormethyl)-1H-pyrazol-1-yl | CH₂ | O | CH₂CH₂ | CH₂CH₂ | H | CH₂CH₂ | Bindung | Pyridin-3-yl | |
| I-83 | 3,5-Dimethyl-1H-pyrazol-1-yl | CH₂ | O | CH₂CH₂ | CH₂CH₂ | H | CH₂CH₂ | CH₂CHCH₃ | Phenyl | 3,83^{[b]} |
| I-84 | 3,5-Dimethyl-1H-pyrazol-1-yl | CH₂ | O | CH₂CH₂ | CH₂CH₂ | H | CH=CH (E) | Bindung | Phenyl | 3,04^{[b]} |
| I-85 | 3,5-Dimethyl-1H-pyrazol-1-yl | CH₂ | O | CH₂CH₂ | CH₂CH₂ | H | CH=CH (E) | CH₂CHCH₃ | Phenyl | 3,83^{[c]} |
| I-86* | 5-Methyl-3 -(trifluormethyl)-1H-pyrazol-1-yl | CH₂ | O | CH₂CH₂ | CH₂CH₂ | H | C≡C | Bindung | H | 2,57^{[b]} |
| I-87 | 2,5-Dichlorphenyl | CH₂ | O | CH₂CH₂ | CH₂CH₂ | H | CH₂CH₂ | Bindung | Naphthalen-1-yl | |
| I-88 | 2,5-Dibromphenyl | CH₂ | O | CH₂CH₂ | CH₂CH₂ | H | CH₂CH₂ | Bindung | Naphthalen-1-yl | |
| I-89 | 5-Iod-2-methylphenyl | CH₂ | O | CH₂CH₂ | CH₂CH₂ | H | CH₂CH₂ | Bindung | Naphthalen-1-yl | |
| I-90 | 3-Chlor-5-(trifluormethyl)pyridin-2-yl | CH₂ | O | CH₂CH₂ | CH₂CH₂ | H | CH₂CH₂ | Bindung | Phenyl | |
| I-91 | 3-Chlor-5-(trifluormethyl)pyridin-2-yl | CH₂ | O | CH₂CH₂ | CH₂CH₂ | H | CH₂CH₂ | Bindung | Naphthalen-1-yl | |
| I-92 | 5-Methyl-3-(trifluormethyl)-1H-pyrazol-1-yl | CH₂ | S | CH₂CH₂ | CH₂CH₂ | H | CH₂CH₂ | Bindung | Naphthalen-1-yl | |
| I-93 | 5-Methyl-3-(trifluormethyl)-1H-pyrazol-1-yl | CH₂ | S | CH₂CH₂ | CH₂CH₂ | H | CH=CH (E) | Bindung | Phenyl | 4,65^{[b]} |
| I-94 | 5-Methyl-3-(trifluormethyl)-1H-pyrazol-1-yl | CH₂ | O | CH₂CH₂ | CH₂CH₂ | H | CH=CH (E) | Bindung | 3-(Trifluormethyl)phenyl | 4,46^{[b]} |
| I-95 | 1,3-Benzodioxol-5-yl | CH₂ | O | CH₂CH₂ | CH₂CH₂ | H | CH₂CH₂ | Bindung | Phenyl | |
| I-96 | 3,5-Dimethyl-1H-pyrazol-1-yl | CH₂ | O | CH₂CH₂ | CH₂CH₂ | H | CH=CH (E) | Bindung | 3-(Trifluormethyl)phenyl | 3,56^{[b]} |
| I-97 | 2,3,6-Trifluorphenyl | CH₂ | O | CH₂CH₂ | CH₂CH₂ | H | C≡C | Bindung | Naphthalen-1-yl | 4,71^{[b]} |
| I-98* | 5-Methyl-3-(trifluormethyl)-1H-pyrazol-1-yl | CH₂ | O | CH₂CH₂ | CH₂CH₂ | H | C≡C | Bindung | Trimethylsilyl | 4,28^{[b]} |
| I-99 | 5-Methyl-3-(trifluormethyl)-1H-pyrazol-1-yl | CH₂ | O | CH₂CH₂ | CH₂CH₂ | H | C≡C | Bindung | Phenyl | 3,82^{[b]} |
| I-100 | 5-Methyl-3-(trifluormethyl)-1H-pyrazol-1-yl | CH₂ | O | CH₂CH₂ | CH₂CH₂ | H | C≡C | Bindung | 4-Methoxyphenyl | 3,79^{[b]} |
| I-101 | 5-Methyl-3-(trifluormethyl)-1H-pyrazol-1-yl | CH₂ | O | CH₂CH₂ | CH₂CH₂ | H | C≡C | Bindung | 2-Chlorphenyl | 4,12^{[b]} |
| I-102 | 5-Methyl-3-(trifluormethyl)-1H-pyrazol-1-yl | CH₂ | O | CH₂CH₂ | CH₂CH₂ | H | C≡C | Bindung | 4-Chlorphenyl | 4,37^{[b]} |
| I-103 | 5-Methyl-3-(trifluormethyl)-1H-pyrazol-1-yl | CH₂ | O | CH₂CH₂ | CH₂CH₂ | H | C≡C | Bindung | 2-Methoxyphenyl | 3,67^{[b]} |
| I-104 | 5-Methyl-3-(trifluormethyl)-1H-pyrazol-1-yl | CH₂ | O | CH₂CH₂ | CH₂CH₂ | H | C≡C | Bindung | 2,4-Dimethoxyphenyl | 3,67^{[b]} |
| I-105 | 5-Methyl-3-(trifluormethyl)-1H-pyrazol-1-yl | CH₂ | O | CH₂CH₂ | CH₂CH₂ | H | C≡C | Bindung | 2,4-Dichlorphenyl | 4,72^{[b]} |
| I-106 | 5-Methyl-3-(trifluormethyl)-1H-pyrazol-1-yl | CH₂ | O | CH₂CH₂ | CH₂CH₂ | H | C≡C | Bindung | 2,5-Dimethoxyphenyl | 3,65^{[b]} |
| I-107 | 3,5-Diethyl-1H-pyrazol-1-yl | CH₂ | O | CH₂CH₂ | CH₂CH₂ | H | CH₂CH₂ | Bindung | Naphthalen-1-yl | 4,04^{[b]} |
| I-108 | 5-Ethyl-3-(trifluormethyl)-1H-pyrazol-1-yl | CH₂ | O | CH₂CH₂ | CH₂CH₂ | H | CH₂CH₂ | Bindung | Naphthalen-1-yl | 4,65^{[b]} |
| I-109 | 3,5-Bis(difluormethyl)-1H-pyrazol-1-yl | CH₂ | O | CH₂CH₂ | CH₂CH₂ | H | CH₂CH₂ | Bindung | Naphthalen-1-yl | 4,25^{[b]} |
| I-110 | 3-tert-Butyl-5-(trifluormethyl)-1H-pyrazol-1-yl | CH₂ | O | CH₂CH₂ | CH₂CH₂ | H | CH₂CH₂ | Bindung | Naphthalen-1-yl | 5,36^{[b]} |
| I-111 | 5-tert-Butyl-3-(trifluormethyl)-1H-pyrazol-1-yl | CH₂ | O | CH₂CH₂ | CH₂CH₂ | H | CH₂CH₂ | Bindung | Naphthalen-1-yl | 5,14^{[b]} |
| I-112 | 3-tert-Butyl-5-(pentafluorethyl)-1H-pyrazol-1-yl | CH₂ | O | CH₂CH₂ | CH₂CH₂ | H | CH₂CH₂ | Bindung | Naphthalen-1-yl | 5,78^{[b]} |
| I-113 | 5-tert-Butyl-3-(pentafluorethyl)-1H-pyrazol-1-yl | CH₂ | O | CH₂CH₂ | CH₂CH₂ | H | CH₂CH₂ | Bindung | Naphthalen-1-yl | 5,54^{[b]} |
| I-114 | 3,5-Bis(trifluormethyl)-1H-pyrazol-1-yl | CH₂ | O | CH₂CH₂ | CH₂CH₂ | H | C≡C | Bindung | Naphthalen-1-yl | 5,03^{[b]} |
| I-115 | 3,5-Diethyl-1H-pyrazol-l-yl | CH₂ | O | CH₂CH₂ | CH₂CH₂ | H | C≡C | Bindung | Naphthalen-1-yl | 4,2^{[b]} |
| I-116 | 5-Ethyl-3-(trifluormethyl)-1H-pyrazol-1-yl | CH₂ | O | CH₂CH₂ | CH₂CH₂ | H | C≡C | Bindung | Naphthalen-1-yl | 4,8^{[b]} |
| I-117 | 3,5-Bis(difluormethyl)-1H-pyrazol-1-yl | CH₂ | O | CH₂CH₂ | CH₂CH₂ | H | C≡C | Bindung | Naphthalen-1-yl | 4,41^{[b]} |
| I-118 | 3-tert-Butyl-5-(trifluormethyl)-1H-pyrazol-1-yl | CH₂ | O | CH₂CH₂ | CH₂CH₂ | H | C≡C | Bindung | Naphthalen-1-yl | 5,48^{[b]} |
| I-119 | 5-tert-Butyl-3-(trifluormethyl)-1H-pyrazol-1-yl | CH₂ | O | CH₂CH₂ | CH₂CH₂ | H | C≡C | Bindung | Naphthalen-1-yl | 5,14^{[b]} |
| I-120 | 5-tert-Butyl-3-(pentafluorethyl)-1H-pyrazol-1-yl | CH₂ | O | CH₂CH₂ | CH₂CH₂ | H | C≡C | Bindung | Naphthalen-1-yl | 5,59^{[b]} |
| I-121 | 3-(Propan-2-yl)-5-(trifluormethyl)-1H-pyrazol-1-yl | CH₂ | O | CH₂CH₂ | CH₂CH₂ | H | CH₂CH₂ | Bindung | Naphthalen-1-yl | 4,89^{[b]} |
| I-122 | 3-(Propan-2-yl)-5-(trifluormethyl)-1H-pyrazol-1-yl | CH₂ | O | CH₂CH₂ | CH₂CH₂ | H | C≡C | Bindung | Naphthalen-1-yl | 4,99^{[b]} |
| I-123 | 5-Methyl-3-(trifluormethyl)-1H-pyrazol-1-yl | CH₂ | O | CH₂CH₂ | CH₂CH₂ | H | CH₂CH₂ | Bindung | 2,4-Dichlorphenyl | 4,77^{[c]}; 4,74^{[b]} |
| I-124 | 2,5-Dibromphenyl | CH₂ | O | CH₂CH₂ | CH₂CH₂ | H | C≡C | Bindung | Naphthalen-1-yl | 552^{[c]}; 5,47^{[b]} |
| I-125* | 2,5-Dichlorphenyl | CH₂ | O | CH₂CH₂ | CH₂CH₂ | H | C≡C | Bindung | H | 3,08^{[b]} |
| I-126 | 5-Methyl-3-(trifluormethyl)-1H-pyrazol-1-yl | CH₂ | O | CH₂CH₂ | CH₂CH₂ | H | CH₂CH₂ | Bindung | 2-Methylphenyl | 4,16^{[c]}; 4,08^{[b]} |
| I-127 | 5-Methyl-3-(trifluormethyl)-1H-pyrazol-1-yl | CH₂ | O | CH₂CH₂ | CH₂CH₂ | H | CH₂CH₂ | Bindung | 2-Bromphenyl | |
| I-128 | 5-Methyl-3-(trifluormethyl)-1H-pyrazol-1-yl | CH₂ | O | CH₂CH₂ | CH₂CH₂ | H | CH₂CH₂ | Bindung | 2,6-Difluorphenyl | 3,97^{[c]}; 3,9^{[b]} |
| I-129 | 5-Methyl-3-(trifluormethyl)-1H-pyrazol-1-yl | CH₂ | O | CH₂CH₂ | CH₂CH₂ | H | CH₂CH₂ | Bindung | Chinolin-8-yl | |
| I-130 | 5-Methyl-3-(trifluormethyl)-1H-pyrazol-1-yl | CH₂ | O | CH₂CH₂ | CH₂CH₂ | H | CH₂CH₂ | Bindung | 3-Methylpyridin-2-yl | |
| I-131 | 5-Methyl-3-(trifluormethyl)-1H-pyrazol-1-yl | CH₂ | O | CH₂CH₂ | CH₂CH₂ | H | CH₂CH₂ | Bindung | 3,5-Dimethylisoxazol-4-yl | |
| I-132 | 5-Methyl-3-(trifluormethyl)-1H-pyrazol-1-yl | CH₂ | O | CH₂CH₂ | CH₂CH₂ | H | CH₂CH₂ | Bindung | Cyclohexyl | 5,01^{[b]} |
| I-133 | 5-Methyl-3-(trifluormethyl)-1H-pyrazol-1-yl | CH₂ | O | CH₂CH₂ | CH₂CH₂ | H | CH₂CH₂ | Bindung | 1,2,3,4-Tetrahydronaphthalen -1-yl | 4,95^{[b]} |
| I-134 | 3,5-Bis(trifluormethyl)-1H-pyrazol-1-yl | CH₂ | O | CH₂CH₂ | CH₂CH₂ | H | CH₂CH₂ | Bindung | 2-Methylphenyl | |
| I-135 | 3,5-Bis(trifluormethyl)-1H-pyrazol-1-yl | CH₂ | O | CH₂CH₂ | CH₂CH₂ | H | CH₂CH₂ | Bindung | 2-Bromphenyl | |
| I-136 | 3,5-Bis(trifluormethyl)-1H-pyrazol-1-yl | CH₂ | O | CH₂CH₂ | CH₂CH₂ | H | CH₂CH₂ | Bindung | 2,6-Difluorphenyl | |
| I-137 | 3,5-Bis(trifluormethyl)-1H-pyrazol-1-yl | CH₂ | O | CH₂CH₂ | CH₂CH₂ | H | CH₂CH₂ | Bindung | Chinolin-8-yl | |
| I-138 | 3,5-Bis(trifluormethyl)-1H-pyrazol-1-yl | CH₂ | O | CH₂CH₂ | CH₂CH₂ | H | CH₂CH₂ | Bindung | 3-Methylpyridin-2-yl | |
| I-139 | 3,5-Bis(trifluormethyl)-1H-pyrazol-1-yl | CH₂ | O | CH₂CH₂ | CH₂CH₂ | H | CH₂CH₂ | Bindung | 3,5-Dimethylisoxazol-4-yl | |
| I-140 | 3,5-Bis(trifluormethyl)-1H-pyrazol-1-yl | CH₂ | O | CH₂CH₂ | CH₂CH₂ | H | CH₂CH₂ | Bindung | Cyclohexyl | 5,76^{[b]} |
| I-141 | 3,5-Bis(trifluormethyl)-1H-pyrazol-1-yl | CH₂ | O | CH₂CH₂ | CH₂CH₂ | H | CH₂CH₂ | Bindung | 1,2,3,4-Tetrahydronaphthalen -1-yl | |
| I-142 | 3,5-Bis(difluormethyl)-1H-pyrazol-1-yl | CH₂ | O | CH₂CH₂ | CH₂CH₂ | H | CH₂CH₂ | Bindung | 2-Methylphenyl | |
| I-143 | 3,5-Bis(difluormethyl)-1H-pyrazol-1-yl | CH₂ | O | CH₂CH₂ | CH₂CH₂ | H | CH₂CH₂ | Bindung | 2-Bromphenyl | |
| I-144 | 3,5-Bis(difluormethyl)-1H-pyrazol-1-yl | CH₂ | O | CH₂CH₂ | CH₂CH₂ | H | CH₂CH₂ | Bindung | 2,6-Difluorphenyl | |
| I-145 | 3,5-Bis(difluormethyl)-1H-pyrazol-1-yl | CH₂ | O | CH₂CH₂ | CH₂CH₂ | H | CH₂CH₂ | Bindung | Chinolin-8-yl | |
| I-146 | 3,5-Bis(difluormethyl)-1 H-pyrazol-1-yl | CH₂ | O | CH₂CH₂ | CH₂CH₂ | H | CH₂CH₂ | Bindung | 3-Methylpyridin-2-yl | |
| I-147 | 3,5-Bis(difluormethyl)-1H-pyrazol-1-yl | CH₂ | O | CH₂CH₂ | CH₂CH₂ | H | CH₂CH₂ | Bindung | 3,5-Dimethylisoxazol-4-yl | |
| I-148 | 3,5-Bis(difluormethyl)-1H-pyrazol-1-yl | CH₂ | O | CH₂CH₂ | CH₂CH₂ | H | CH₂CH₂ | Bindung | Cyclohexyl | 4,87^{[b]} |
| I-149 | 3,5-Bis(difluormethyl)-1H-pyrazol-1-yl | CH₂ | O | CH₂CH₂ | CH₂CH₂ | H | CH₂CH₂ | Bindung | 1,2,3,4-Tetrahydronaphthalen -1-yl | 4,78^{[b]} |
| I-150 | 2,5-Dichlorphenyl | CH₂ | O | CH₂CH₂ | CH₂CH₂ | H | CH₂CH₂ | Bindung | 2-Methylphenyl | |
| I-151 | 2,5-Dichlorphenyl | CH₂ | O | CH₂CH₂ | CH₂CH₂ | H | CH₂CH₂ | Bindung | 2-Bromphenyl | |
| I-152 | 2,5-Dichlorphenyl | CH₂ | O | CH₂CH₂ | CH₂CH₂ | H | CH₂CH₂ | Bindung | 2,6-Difluorphenyl | |
| I-153 | 2,5-Dichlorphenyl | CH₂ | O | CH₂CH₂ | CH₂CH₂ | H | CH₂CH₂ | Bindung | Chinolin-8-yl | |
| I-154 | 2,5-Dichlorphenyl | CH₂ | O | CH₂CH₂ | CH₂CH₂ | H | CH₂CH₂ | Bindung | 3-Methylpyridin-2-yl | |
| I-155 | 2,5-Dichlorphenyl | CH₂ | O | CH₂CH₂ | CH₂CH2 | H | CH₂CH₂ | Bindung | 3,5-Dimethylisoxazol-4-yl | |
| I-156 | 2,5-Dichlorphenyl | CH₂ | O | CH₂CH₂ | CH₂CH₂ | H | CH₂CH₂ | Bindung | Cyclohexyl | |
| I-157 | 2,5-Dichlorphenyl | CH₂ | O | CH₂CH₂ | CH₂CH₂ | H | CH₂CH₂ | Bindung | 1,2,3,4-Tetrahydronaphthalen -1-yl | |
| I-158 | 2,5-Dimethylphenyl | CH₂ | O | CH₂CH₂ | CH₂CH₂ | H | CH₂CH₂ | Bindung | 2-Methylphenyl | 4,77^{[c]} |
| | | | | | | | | | | 4,67^{[b]} |
| I-159 | 2,5-Dimethylphenyl | CH₂ | O | CH₂CH₂ | CH₂CH₂ | H | CH₂CH₂ | Bindung | 2-Bromphenyl | |
| I-160 | 2,5-Dimethylphenyl | CH₂ | O | CH₂CH₂ | CH₂CH₂ | H | CH₂CH₂ | Bindung | 2,6-Difluorphenyl | 4,55^{[c]} |
| | | | | | | | | | | 4,48^{[b]} |
| I-161 | 2,5-Dimethylphenyl | CH₂ | O | CH₂CH₂ | CH₂CH₂ | H | CH₂CH₂ | Bindung | Chinolin-8-yl | |
| I-162 | 2,5-Dimethylphenyl | CH₂ | O | CH₂CH₂ | CH₂CH₂ | H | CH₂CH₂ | Bindung | 3-Methylpyridin-2-yl | |
| I-163 | 2,5-Dimethylphenyl | CH₂ | O | CH₂CH₂ | CH₂CH₂ | H | CH₂CH₂ | Bindung | 3,5-Dimethylisoxazol-4-yl | |
| I-164 | 2,5-Dimethylphenyl | CH₂ | O | CH₂CH₂ | CH₂CH₂ | H | CH₂CH₂ | Bindung | Cyclohexyl | |
| I-165 | 2,5-Dimethylphenyl | CH₂ | O | CH₂CH₂ | CH₂CH₂ | H | CH₂CH₂ | Bindung | 1,2,3,4-Tetrahydronaphthalen-1-yl | |
| I-166 | 5-Methyl-3-(trifluormethyl)-1H-pyrazol-1-yl | CH₂ | O | CH₂CH₂ | CH₂CH₂ | H | CH=CH (Mischung von (Z)- und (E)-Isomeren) | Bindung | 2-Methylphenyl | 4,23^{[b]} |
| I-167 | 5-Methyl-3-(trifluormethyl)-1H-pyrazol-1-yl | CH₂ | O | CH₂CH₂ | CH₂CH₂ | H | CH=CH (Mischung von (Z)- und (E)-Isomeren) | Bindung | 2-Bromphenyl | 4,39 und 4,44^{[b]} |
| I-168 | 5-Methyl-3-(trifluormethyl)-1H-pyrazol-1-yl | CH₂ | O | CH₂CH₂ | CH₂CH₂ | H | CH=CH (Mischung von (Z)- und (E)-Isomeren) | Bindung | 2,6-Difluorphenyl | 3,90 und 4,21^{[b]} [bI |
| I-169 | 5-Methyl-3-(trifluormethyl)-1H-pyrazol-1-yl | CH₂ | O | CH₂CH₂ | CH₂CH₂ | H | CH=CH (Mischung von (Z)- und (E)-Isomeren) | Bindung | Chinolin-8-yl | |
| I-170 | 5-Methyl-3-(trifluormethyl)-1H-pyrazol-1-yl | CH₂ | O | CH₂CH₂ | CH₂CH₂ | H | CH=CH (Mischung von (Z)- und (E)-Isomeren) | Bindung | 3-Methylpyridin-2-yl | |
| I-171 | 5-Methyl-3-(trifluormethyl)-1H-pyrazol-1-yl | CH₂ | O | CH₂CH₂ | CH₂CH₂ | H | CH=CH (Mischung von (Z)- und (E)-Isomeren) | Bindung | 3,5-Dimethylisoxazol-4-yl | |
| I-172 | 5-Methyl-3-(trifluormethyl)-1H-pyrazol-1-yl | CH₂ | O | CH₂CH₂ | CH₂CH₂ | H | CH=CH (Mischung von (Z)- und (E)-Isomeren) | Bindung | Cyclohexyl | 4,99 und 5,07^{[b]} |
| I-173 | 5-Methyl-3-(trifluormethyl)-1H-pyrazol-1-yl | CH₂ | O | CH₂CH₂ | CH₂CH₂ | H | CH=CH (Mischung von (Z)- und (E)-Isomeren) | Bindung | 1,2,3,4-Tetrahydronaphthalen-1-yl | 4,02 und 4,10^{[b]} |
| I-174 | 3,5-Bis(trifluormethyl)-1H-pyrazol-1-yl | CH₂ | O | CH₂CH₂ | CH₂CH₂ | H | CH=CH (Mischung von (Z)- und (E)-Isomeren) | Bindung | 2-Methylphenyl | 4,93^{[b]} |
| I-175 | 3,5-Bis(trifluormethyl)-1H-pyrazol-1-yl | CH₂ | O | CH₂CH₂ | CH₂CH₂ | H | CH=CH (Mischung von (Z)- und (E)-Isomeren) | Bindung | 2-Bromphenyl | 4,99^{[b]} |
| I-176 | 3,5-Bis(trifluormethyl)-1H-pyrazol-1-yl | CH₂ | O | CH₂CH₂ | CH₂CH₂ | H | CH=CH (Mischung von (Z)- und (E)-Isomeren) | Bindung | 2,6-Difluorphenyl | 4,88^{[b]} |
| I-177 | 3,5-Bis(trifluormethyl)-1H-pyrazol-1-yl | CH₂ | O | CH₂CH₂ | CH₂CH₂ | H | CH=CH (Mischung von (Z)- und (E)-Isomeren) | Bindung | Chinolin-8-yl | |
| I-178 | 3,5-Bis(trifluormethyl)-1H-pyrazol-1-yl | CH₂ | O | CH₂CH₂ | CH₂CH₂ | H | CH=CH (Mischung von (Z)- und (E)-Isomeren) | Bindung | 3-Methylpyridin-2-yl | |
| I-179 | 3,5-Bis(trifluormethyl)-1H-pyrazol-1-yl | CH₂ | O | CH₂CH₂ | CH₂CH₂ | H | CH=CH (Mischung von (Z)- und (E)-Isomeren) | Bindung | 3,5-Dimethylisoxazol-4-yl | |
| I-180 | 3,5-Bis(trifluormethyl)-1 H-pyrazol-1-yl | CH₂ | O | CH₂CH₂ | CH₂CH₂ | H | CH=CH (Mischung von (Z)- und (E)-Isomeren) | Bindung | Cyclohexyl | 5,59 und 5,68^{[b]} |
| I-181 | 3,5-Bis(trifluormethyl)-1 H-pyrazol-1-yl | CH₂ | O | CH₂CH₂ | CH₂CH₂ | H | CH=CH (Mischung von (Z)- und (E)-Isomeren) | Bindung | 1,2,3,4-Tetrahydronaphthalen-1-yl | 5,60 und 5,78^{[b]} |
| I-182 | 3,5-Bis(difluormethyl)-1H-pyrazol-1-yl | CH₂ | O | CH₂CH₂ | CH₂CH₂ | H | CH=CH (Mischung von (Z)- und (E)-Isomeren) | Bindung | 2-Methylphenyl | |
| I-183 | 3,5-Bis(difluormethyl)-1H-pyrazol-1-yl | CH₂ | O | CH₂CH₂ | CH₂CH₂ | H | CH=CH (Mischung von (Z)- und (E)-Isomeren) | Bindung | 2-Bromphenyl | 4,25 und 4,29^{[b]} |
| I-184 | 3,5-Bis(difluormethyl)-1H-pyrazol-1-yl | CH₂ | O | CH₂CH₂ | CH₂CH₂ | H | CH=CH (Mischung von (Z)- und (E)-Isomeren) | Bindung | 2,6-Difluorphenyl | 3,74 und 4,05^{[b]} |
| I-185 | 3,5-Bis(difluormethyl)-1H-pyrazol-1-yl | CH₂ | O | CH₂CH₂ | CH₂CH₂ | H | CH=CH (Mischung von (Z)- und (E)-Isomeren) | Bindung | Chinolin-8-yl | |
| I-186 | 3,5-Bis(difluormethyl)-1H-pyrazol-1-yl | CH₂ | O | CH₂CH₂ | CH₂CH₂ | H | CH=CH (Mischung von (Z)- und (E)-Isomeren) | Bindung | 3-Methylpyridin-2-yl | |
| I-187 | 3,5-Bis(difluormethyl)-1 H-pyrazol-1-yl | CH₂ | O | CH₂CH₂ | CH₂CH₂ | H | CH=CH (Mischung von (Z)- und (E)-Isomeren) | Bindung | 3,5-Dimethylisoxazol-4-yl | |
| I-188 | 3,5-Bis(difluormethyl)-1H-pyrazol-1-yl | CH₂ | O | CH₂CH₂ | CH₂CH₂ | H | CH=CH (Mischung von (Z)- und (E)-Isomeren) | Bindung | Cyclohexyl | 4,79 und 4,85^{[b]} |
| I-189 | 3,5-Bis(difluormethyl)-1H-pyrazol-1-yl | CH₂ | O | CH₂CH₂ | CH₂CH₂ | H | CH=CH (Mischung von (Z)- und (E)-Isomeren) | Bindung | 1,2,3,4-Tetrahydronaphthalen-1-yl | 4,81 und 4,97^{[b]} |
| I-190 | 2,5-Dichlorphenyl | CH₂ | O | CH₂CH₂ | CH₂CH₂ | H | CH=CH (Mischung von (Z)- und (E)-Isomeren) | Bindung | 2-Methylphenyl | |
| I-191 | 2,5-Dichlorphenyl | CH₂ | O | CH₂CH₂ | CH₂CH₂ | H | CH=CH (Mischung von (Z)- und (E)-Isomeren) | Bindung | 2-Bromphenyl | |
| I-192 | 2,5-Dichlorphenyl | CH₂ | O | CH₂CH₂ | CH₂CH₂ | H | CH=CH (Mischung von (Z)- und (E)-Isomeren) | Bindung | 2,6-Difluorphenyl | |
| I-193 | 2,5-Dichlorphenyl | CH₂ | O | CH₂CH₂ | CH₂CH₂ | H | CH=CH (Mischung von (Z)- und (E)-Isomeren) | Bindung | Chinolin-8-yl | |
| I-194 | 2,5-Dichlorphenyl | CH₂ | O | CH₂CH₂ | CH₂CH₂ | H | CH=CH (Mischung von (Z)- und (E)-Isomeren) | Bindung | 3-Methylpyridin-2-yl | |
| I-195 | 2,5-Dichlorphenyl | CH₂ | O | CH₂CH₂ | CH₂CH₂ | H | CH=CH (Mischung von (Z)- und (E)-Isomeren) | Bindung | 3,5-Dimethylisoxazol-4-yl | |
| I-196 | 2,5-Dichlorphenyl | CH₂ | O | CH₂CH₂ | CH₂CH₂ | H | CH=CH (Mischung von (Z)- und (E)-Isomeren) | Bindung | Cyclohexyl | |
| I-197 | 2,5-Dichlorphenyl | CH₂ | O | CH₂CH₂ | CH₂CH₂ | H | CH=CH (Mischung von (Z)- und (E)-Isomeren) | Bindung | 1,2,3,4-Tetrahydronaphthalen-1-yl | |
| I-198 | 2,5-Dimethylphenyl | CH₂ | O | CH₂CH₂ | CH₂CH₂ | H | CH=CH (Mischung von (Z)- und (E)-Isomeren) | Bindung | 2-Methylphenyl | |
| I-199 | 2,5-Dimethylphenyl | CH₂ | O | CH₂CH₂ | CH₂CH₂ | H | CH=CH (Mischung von (Z)- und (E)-Isomeren) | Bindung | 2-Bromphenyl | |
| I-200 | 2,5-Dimethylphenyl | CH₂ | O | CH₂CH₂ | CH₂CH₂ | H | CH=CH (Mischung von (Z)- und (E)-Isomeren) | Bindung | 2,6-Difluorphenyl | |
| I-201 | 2,5-Dimethylphenyl | CH₂ | O | CH₂CH₂ | CH₂CH₂ | H | CH=CH (Mischung von (Z)- und (E)-Isomeren) | Bindung | Chinolin-8-yl | |
| I-202 | 2,5-Dimethylphenyl | CH₂ | O | CH₂CH₂ | CH₂CH₂ | H | CH=CH (Mischung von (Z)- und (E)-Isomeren) | Bindung | 3-Methylpyridin-2-yl | |
| I-203 | 2,5-Dimethylphenyl | CH₂ | O | CH₂CH₂ | CH₂CH₂ | H | CH=CH (Mischung von (Z)- und (E)-Isomeren) | Bindung | 3,5-Dimethylisoxazol-4-yl | |
| I-204 | 2,5-Dimethylphenyl | CH₂ | O | CH₂CH₂ | CH₂CH₂ | H | CH=CH (Mischung von (Z)- und (E)-Isomeren) | Bindung | Cyclohexyl | |
| I-205 | 2,5-Dimethylphenyl | CH₂ | O | CH₂CH₂ | CH₂CH₂ | H | CH=CH (Mischung von (Z)- und (E)-Isomeren) | Bindung | 1,2,3,4-Tetrahydronaphthalen-1-yl | |
| I-206 | 5-Methyl-3-(trifluormethyl)-1H-pyrazol-1-yl | CH₂ | O | CH₂CH₂ | CH₂CH₂ | H | C≡C | Bindung | 2-Methylphenyl | 4,15^{[c]}; 4,15^{[b]} |
| I-207 | 5-Methyl-3-(trifluormethyl)-1H-pyrazol-1-yl | CH₂ | O | CH₂CH₂ | CH₂CH₂ | H | C≡C | Bindung | 2-Bromphenyl | 4,19^{[c]}; 4,16^{[b]} |
| I-208 | 5-Methyl-3-(trifluormethyl)-1H-pyrazol-1-yl | CH₂ | O | CH₂CH₂ | CH₂CH₂ | H | C≡C | Bindung | 2,6-Difluorphenyl | 3,86^{[c]}; 3,83^{[b]} |
| I-209 | 5-Methyl-3-(trifluormethyl)-1H-pyrazol-1-yl | CH₂ | O | CH₂CH₂ | CH₂CH₂ | H | C≡C | Bindung | Chinolin-8-yl | |
| I-210 | 5-Methyl-3-(trifluormethyl)-1H-pyrazol-1-yl | CH₂ | O | CH₂CH₂ | CH₂CH₂ | H | C≡C | Bindung | 3-Methylpyridin-2-yl | 2,95^{[c]}; 2,72^{[b]} |
| I-211 | 5-Methyl-3-(trifluormethyl)-1H-pyrazol-1-yl | CH₂ | O | CH₂CH₂ | CH₂CH₂ | H | C≡C | Bindung | 3,5-Dimethylisoxazol-4-yl | |
| I-212 | 3,5-Bis(trifluormethyl)-1H-pyrazol-1-yl | CH₂ | O | CH₂CH₂ | CH₂CH₂ | H | C≡C | Bindung | 2-Methylphenyl | 4,73^{[b]} |
| I-213 | 3,5-Bis(trifluormethyl)-1H-pyrazol-1-yl | CH₂ | O | CH₂CH₂ | CH₂CH₂ | H | C≡C | Bindung | 2-Bromphenyl | 4,74^{[b]} |
| I-214 | 3,5-Bis(trifluormethyl)-1H-pyrazol-1-yl | CH₂ | O | CH₂CH₂ | CH₂CH₂ | H | C≡C | Bindung | 2,6-Difluorphenyl | 4,4^{[b]} |
| I-215 | 3,5-Bis(trifluormethyl)-1H-pyrazol-1-yl | CH₂ | O | CH₂CH₂ | CH₂CH₂ | H | C≡C | Bindung | Chinolin-8-yl | |
| I-216 | 3,5-Bis(trifluormethyl)-1H-pyrazol-1-yl | CH₂ | O | CH₂CH₂ | CH₂CH₂ | H | C≡C | Bindung | 3-Methylpyridin-2-yl | 3,37^{[b]} |
| I-217 | 3,5-Bis(trifluormethyl)-1H-pyrazol-1-yl | CH₂ | O | CH₂CH₂ | CH₂CH₂ | H | C≡C | Bindung | 3,5-Dimethylisoxazol-4-yl | |
| I-218 | 3,5-Bis(difluormethyl)-1H-pyrazol-1-yl | CH₂ | O | CH₂CH₂ | CH₂CH₂ | H | C≡C | Bindung | 2-Methylphenyl | 4,02^{[c]}; 3,99^{[b]} |
| I-219 | 3,5-Bis(difluormethyl)-1H-pyrazol-1-yl | CH₂ | O | CH₂CH₂ | CH₂CH₂ | H | C≡C | Bindung | 2-Bromphenyl | 404^{[c]}; 4,04^{[b]} |
| I-220 | 3,5-Bis(difluormethyl)-1H-pyrazol-1-yl | CH₂ | O | CH₂CH₂ | CH₂CH₂ | H | C≡C | Bindung | 2,6-Difluorphenyl | 3,73^{[c]}; 3,7^{[b]} |
| I-221 | 3,5-Bis(difluormethyl)-1H-pyrazol-1-yl | CH₂ | O | CH₂CH₂ | CH₂CH₂ | H | C≡C | Bindung | Chinolin-8-yl | |
| I-222 | 3,5-Bis(difluormethyl)-1H-pyrazol-1-yl | CH₂ | O | CH₂CH₂ | CH₂CH₂ | H | C≡C | Bindung | 3-Methylpyridin-2-yl | 2,86^{[c]}; 2,68^{[b]} |
| I-223 | 3,5-Bis(difluormethyl)-1H-pyrazol-1-yl | CH₂ | O | CH₂CH₂ | CH₂CH₂ | H | C≡C | Bindung | 3,5-Dimethylisoxazol-4-yl | 3,09^{[b]} |
| I-224 | 2,5-Dichlorphenyl | CH₂ | O | CH₂CH₂ | CH₂CH₂ | H | C≡C | Bindung | 2-Methylphenyl | 4,91^{[b]} |
| I-225 | 2,5-Dichlorphenyl | CH₂ | O | CH₂CH₂ | CH₂CH₂ | H | C≡C | Bindung | 2-Bromphenyl | 4,91^{[b]} |
| I-226 | 2,5-Dichlorphenyl | CH₂ | O | CH₂CH₂ | CH₂CH₂ | H | C≡C | Bindung | 2,6-Difluorphenyl | 4,52^{[b]} |
| I-227 | 2,5-Dichlorphenyl | CH₂ | O | CH₂CH₂ | CH₂CH₂ | H | C≡C | Bindung | Chinolin-8-yl | |
| I-228 | 2,5-Dichlorphenyl | CH₂ | O | CH₂CH₂ | CH₂CH₂ | H | C≡C | Bindung | 3-Methylpyridin-2-yl | 3,55^{[c]} 3,35^{[b]} |
| I-229 | 2,5-Dichlorphenyl | CH₂ | O | CH₂CH₂ | CH₂CH₂ | H | C≡C | Bindung | 3,5-Dimethylisoxazol-4-yl | 3,86^{[b]} |
| I-230 | 2,5-Dimethylphenyl | CH₂ | O | CH₂CH₂ | CH₂CH₂ | H | C≡C | Bindung | 2-Methylphenyl | 478^{[c]} 4,75^{[b]} |
| I-231 | 2,5-Dimethylphenyl | CH₂ | O | CH₂CH₂ | CH₂CH₂ | H | C≡C | Bindung | 2-Bromphenyl | 4,8^{[c]}; 4,77^{[b]} |
| I-232 | 2,5-Dimethylphenyl | CH₂ | O | CH₂CH₂ | CH₂CH₂ | H | C≡C | Bindung | 2,6-Difluorphenyl | 4,39^{[c]}; 4,36^{[b]} |
| I-233 | 2,5-Dimethylphenyl | CH₂ | O | CH₂CH₂ | CH₂CH₂ | H | C≡C | Bindung | Chinolin-8-yl | |
| I-234 | 2,5-Dimethylphenyl | CH₂ | O | CH₂CH₂ | CH₂CH₂ | H | C≡C | Bindung | 3-Methylpyridin-2-yl | |
| I-235 | 2,5-Dimethylphenyl | CH₂ | O | CH₂CH₂ | CH₂CH₂ | H | C≡C | Bindung | 3,5-Dimethylisoxazol-4-yl | 3,71^{[c]}; 3,67^{[b]} |
| I-236 | 3,5-Bis(trifluormethyl)-1H-pyrazol-1-yl | CH₂ | O | CH₂CH₂ | CH₂CH₂ | H | CH=CH (Z) | Bindung | 3-Methylpyridin-2-yl | 2,46^{[b]} |
| I-237 | 2,5-Dimethylphenyl | CH₂ | O | CH₂CH₂ | CH₂CH₂ | H | CH=CH (Z) | Bindung | 2-Methylphenyl | 4,95^{[c]}; 4,86^{[b]} |
| I-238* | 2,5-Dimethylphenyl | CH₂ | O | CH₂CH₂ | CH₂CH₂ | H | C≡C | Bindung | H | 2,93 [b]; 2,95[c] |
| I-239* | 3,5-Bis(difluormethyl)-1H-pyrazol-1-yl | CH₂ | O | CH₂CH₂ | CH₂CH₂ | H | C≡C | Bindung | H | 2,45^{[b]} |
| I-240 | 3,5-Bis(trifluormethyl)-1H-pyrazol-1-yl | CH₂ | O | CH₂CH₂ | CH₂CH₂ | H | CH=CH (Mischung von (Z)- und (E)-Isomeren) | Bindung | 2-Chlor-6-fluorphenyl | 4,85 und 5,09^{[b]} |
| I-241 | 3,5-Bis(difluormethyl)-1H-pyrazol-1-yl | CH₂ | O | CH₂CH₂ | CH₂CH₂ | H | CH=CH (Mischung von (Z)- und (E)-Isomeren) | Bindung | 2-Chlor-6-fluorphenyl | 4,09 und 4,33^{[b]} |
| I-242 | 5-Methyl-3-(trifluormethyl)-1H-pyrazol-1-yl | CH₂ | O | CH₂CH₂ | CH₂CH₂ | H | CH=CH (Mischung von (Z)- und (E)-Isomeren) | Bindung | 2-Chlor-6-fluorphenyl | 4,48^{[b]} |

| | | | | | | | | | | |
|---|---|---|---|---|---|---|---|---|---|---|
| Die Bestimmung der logP Werte erfolgte gemäß EEC Directive 79/831 Annex V.A8 durch HPLC (High Performance Liquid Chromatography) an reversed-phase Säulen (C 18), mit nachfolgenden Methoden: ^{[a]} Die Bestimmung erfolgt im sauren Bereich bei pH 2.3 mit 0,1% wässriger Phosphorsäure und Acetonitril als Eluenten; linearer Gradient von 10% Acetonitril bis 95% Acetonitril. ^{[b]} Die Bestimmung mit der LC-MS im sauren Bereich erfolgt bei pH 2,7 mit 0,1 % wässriger Ameisensäure und Acetonitril (enthält 0,1% Ameisensäure) als Eluenten; linearer Gradient von 10% Acetonitril bis 95% Acetonitril ^{[c]} Die Bestimmung mit der LC-MS im neutralen Bereich erfolgt bei pH 7.8 mit 0,001 molarer wässriger Ammoniumhydrogencarbonat-Lösung und Acetonitril als Eluenten; linearer Gradient von 10 % Acetonitril bis 95 % Acetonitril. | | | | | | | | | | |

Die Eichung erfolgt mit unverzweigten Alkan-2-onen (mit 3 bis 16 Kohlenstoffatomen), deren logP-Werte bekannt sind (Bestimmung der logP-Werte anhand der Retentionszeiten durch lineare Interpolation zwischen zwei aufeinander folgenden Alkanonen).

Die lambda-maX Werte wurden an Hand der UV-Spektren von 200 nm bis 400 nm in den Maxima der chromatographischen Signale ermittelt.

### NMR-Daten ausgewählter Beispiele

| **Bsp** | **NMR**-**Daten** |
|---|---|
| I-1 | ¹HNMR δ= 7.46 (m, 2H), 7.31-7.18 (m, 4H), 6.62 (d, 1H), 6.57 (d, 1H), 6.44 (s, 1H), 5.19 (bs, 2H), 4.20 (bs, 1H), 3.90 (bs, 1H), 3.26 (m, 2H), 2.90 (m, 1H), 2.21 (s, 3H), 2.05(m, 2H), 1.80-1.50 (m, 2H) ppm |
| I-2 | ¹HNMR δ= 7.95-7.92 (m, 2H), 7.87-7.84 (m, 1H), 7.52-7.43 (m, 4H), 7.11 (d, 1H), 6.93 (d, 1H), 6.88 (s, 1H), 6.45 (s, 1H), 5.14 (s, 2H), 4.05 (bs, 1H), 3.74 (bs, 1H), 3.20-3.05 (m, 2H), 2.88 (bs, 1H), 2.24 (s, 3H), 1.90-1.78 (bs, 2H), 1.50 (bs, 1H), 1.35 (bs, 1H) ppm |
| I-3 | ¹HNMR δ= 7.32 (s, 1H), 7.29-7.22 (m, 4H), 7.15 (m, 1H), 6.44 (s, 1H), 6.35 (d, 1H), 5.60 (m, 1H), 5.20 (bs, 2H), 4.30 (bs, 1H), 3.98 (bs, 1H), 3.38-3.25 (m, 2H), 3.05-2.84 (m, 4H), 2.22 (s, 3H), 2.17-2.05 (m, 2H), 1.85-1.56 (m, 2H), 1.26 (d, 3H) ppm |
| I-26 | ¹HNMR δ= 7.56 (d, 2H), 7.50 (s, 1H), 7.39-7.34 (m, 3H), 7.28-7.18 (m, 2H), 6.45 (s, 1H), 5.23 (bs, 2H), 4.37 (bs, 1H), 4.01 (bs, 1H), 3.35 (m, 1H), 3.32 (bs, 1H), 2.90 (bs, 1H), 2.23 (s, 3H), 2.18-2.10 (m, 2H), 1.80 (bs, 1H), 1.63 (bs, 1H) ppm |
| I-27 | ¹HNMR δ= 8.23 (d, 1H), 8.16 (d, 1H), 7.95 (d, 1H), 7.88 (d, 1H), 7.83 (d, 1H), 7.64 (s, 1H), 7.63-7.51 (m, 3H), 7.27 (d, 1H), 6.46 (s, 1H), 5.24 (bs, 2H), 4.38 (bs, 1H), 4.03 (bs, 1H), 3.40 (m, 1H), 3.32 (bs, 1H), 2.94 (bs, 1H), 2.24 (s, 3H), 2.23-2.12 (m, 2H), 1.85 (bs, 1H), 1.70 (bs, 1H) ppm |
| I-50 | ¹HNMR (CD₃CN) δ= 8.39 (d, 1H), 7.96 (d, 2H), 7.80 (d, 1H), 7.72 (s, 1H), 7.67-7.50 (m, 3H), 6.37 (s, 1H), 5.05 (s, 2H), 4.45 (bs, 1H), 3.98 (bs, 1H), 3.40-3.20 (m, 2H), 2.93 (bs, 1H), 2.25 (s, 3H), 2.25-2.15 (m, 2H), 1.90-1.75 (m, 2H) ppm |
| I-51 | ¹HNMR (CD₃CN) δ= 8.74 (dd, 1H), 8.59 (dd, 1H), 7.99 (s, 1H), 7.96 (m, 1H), 7.45 (dd, 1H), 6.45 (s, 1H), 5.22 (bs, 2H), 4.35 (bs, 1H), 4.00 (bs, 1H), 3.40-3.20 (m, 3H), 2.22 (s, 3H), 2.17-2.10 (m, 2H), 1.90-1.55 (m, 2H) ppm |
| I-52 | ¹HNMR (CD₃CN) δ= 7.46 (s, 1H), 7.42-7.34 (m, 4H), 7.30-7.25 (m, 1H), 6.39 (s, 1H), 5.09 (d, 1H), 5.02 (d, 1H), 4.45 (d, 1H), 3.91 (d, 1H), 3.84 (s, 2H), 3.25 (m, 2H), 2.82 (t, 1H), 2.23 (s, 3H), 2.18-2.05 (m, 2H), 1.82 (qd, 1H), 1.66 (qd, 1H) ppm |
| I-53 | ¹HNMR δ= 7.28-7.26 (m, 2H), 7.22-7.21 (m, 2H), 7.19-7.15 (m, 2H), 6.51 (s, 1H), 5.33 (d, 1H), 5.25 (d, 1H), 4.36 (d, 1H), 3.95 (d, 1H), 3.35-3.20 (m, 2H), 2.96 (s, 4H), 2.82 (t, 1H), 2.21 (s, 3H), 2.09 (d, 1H), 2.07 (d, 1H), 1.78 (m, 1H), 1.53 (m, 1H) ppm |
| I-54 | ¹HNMR δ= 7.26-7.13 (m, 5H), 7.06 (s, 1H), 5.77 (s, 1H), 4.91 (bs, 2H), 4.28 (m, 1H), 4.03 (m, 1H), 3.25 (m, 1H), 3.16 (m, 1H), 2.97 (m, 4H), 2.85 (m, 1H), 2.11 (s, 3H), 2.10-1.97 (m+s, 5H), 1.61 (m, 2H) ppm |
| I-55 | ¹HNMR δ= 7.85 (d, 1H), 7.26-7.13 (m, 5H), 7.06 (s, 1H), 6.66 (d, 1H), 5.27 (bs, 2H), 4.30 (bs, 1H), 3.95 (bs, 1H), 3.26 (m, 1H), 3.20-2.80 (m+s, 6H), 2.07 (m, 2H), 1.80-1.50 (m, 2H) ppm |
| I-57 | ¹HNMR δ= 7.34-7.12 (m, 9H), 7.04 (s, 1H), 4.38 (bs, 1H), 4.00 (bs, 1H), 3.75 (s, 2H), 3.27-2.98 (m, 2H), 2.96 (m, 4H), 2,80 (bs, 1H), 2.02 (m, 2H), 1.51 (m, 2H) ppm |
| I-58 | ¹HNMR δ= 7.27-7.21 (m, 2H), 7.20-7.14 (m, 3H), 7.05 (s, 1H), 4.37 (bs, 1H), 3.95 (bs, 1H), 3.26-3.18 (m, 2H), 2.96 (s, 4H), 2.80 (bs, 1H), 2.33 (t, 2H), 2.04 (d, 2H), 1.55 (bs, 2H), 1.50 (tt, 2H), 1.33 (tq, 2H), 0.90 (t, 3H) ppm |
| I-59 | ¹HNMR δ= 7.47 (s, 1H), 7.26-7.13 (m, 5H), 7.07 (s, 1H), 5.46 (m, 2H), 4.27 (bs, 1H), 3.93 (bs, 1H), 3.30 (m, 1H), 3.20-2.85 (m+s, 6H), 2.05 (m, 2H), 1.79 (bs, 1H), 1.57 (bs, 1H) ppm |
| I-60 | ¹HNMR δ= 7.26-7.13 (m, 5H), 7.07 (s, 1H), 5.29 (bs, 2H), 4.30 (bs, 1H), 3.92 (bs, 1H), 3.28 (m, 1H), 3.20 (m, 1H), 3.00-2.80 (m, 5H), 2.20 (s, 3H), 2.07 (m, 2H), 1.80 (bs, 1H), 1.60 (bs, 1H) ppm |
| I-61 | ¹HNMR δ= 8.07-7.90 (m, 3H), 7.26-7.21 (m, 2H), 7.20-7.14 (m, 3H), 7.08 (s, 1H), 4.50 (d, 1H), 3.35-3.23 (m, 4H), 2.97 (s, 4H), 2.22-2.10 (m, 1H), 2.00-1.87 (m, 2H), 1.87-1.45 (m, 2H) ppm |
| I-63 | ¹HNMR δ= 7.44-7.43 (m, 1H), 7.28-7.12 (m, 6H), 7.05 (s, 1H), 6.99 (d, 1H), 4.38 (bs, 1H), 3.99 (bs, 1H), 3.72 (s, 2H), 3.22-3.15 (m, 2H), 2.96 (s, 4H), 2.81 (bs, 1H), 2.00 (d, 2H), 1.52 (m, 2H) ppm |
| I-64 | ¹HNMR δ= 7.26-7.21 (m, 2H), 7.20-7.13 (m, 3H), 7.06 (s, 1H), 5.70 (m, 1H), 4.30 (bs, 1H), 4.00 (bs, 1H), 3.30-3.19 (m, 2H), 2.96 (s, 5H), 2.26 (d, 3H), 2.08-1.96 (m, 2H), 1.64-1.54 (m, 5H) ppm |
| I-65 | ¹HNMR δ= 7.40 (s, 1H), 7.26-7.15 (m, 5H), 7.06 (s, 1H), 4.35 (bs, 1H), 4.05 (bs, 1H), 3.76 (s, 3H), 3.49 (s, 2H), 3.30-3.19 (m, 2H), 2.99 (s, 4H), 2.82 (bs, 1H), 2.08-2.00 (m, 2H), 1.58 (bs, 2H) ppm |
| I-66 | ¹HNMR δ= 7.26-7.21 (m, 2H), 7.20-7.12 (m, 3H), 7.05 (s, 1H), 6.85-6.80 (m, 3H), 6.75-6.71 (dd, 1H), 3.93 (bs, 1H), 3.74 (s, 3H), 3.69 (s, 3H), 3.20 (m, 1H), 2.96 (s, 4H), 2.80-2.74 (m, 2H), 2.65-2.55 (bs, 2H), 2.04-1.97 (m, 2H), 1.58-1.42 (m, 2H), 1.28 (t, 2H) ppm |
| I-67 | ¹HNMR δ= 7.71-7.58 (m, 3H), 7.27-7.12 (m, 5H), 7.07 (s, 1H), 4.37 (bs, 1H), 4.05 (bs, 1H), 3.97 (s, 2H), 3.35-3.23 (m, 2H), 2.97 (s, 4H), 2.88 (bs, 1H), 2.07 (d, 2H), 1.65 (bs, 2H) ppm |
| I-68 | ¹HNMR δ= 7.99 (d, 1H), 7.26-7.12 (m, 5H), 7.05 (s, 1H), 6.69 (d, 1H), 5.74 (q, 1H), 4.40-3.95 (m, 2H), 3.24 (m, 1H), 3.20-2.75 (m+s, 6H), 2.05 (m, 2H), 1.30-1.70 (m+d, 5H) ppm |
| I-70 | ¹HNMR (CD₃CN) δ= 8.10 (s, 1H), 7.71 (d, 1H), 7.60 (d, 1H), 7.29-7.15 (m, 6H), 7.07 (dd, 1H), 6.87 (s, 1H), 5.45 (d, 1H), 5.36 (d, 1H), 4.48 (d, 1H), 4.00 (d, 1H), 3.35-3.23 (m, 2H), 3.00 (s, 4H), 2.86 (t, 1H), 2.18-2.06 (m, 2H), 1.80 (qd, 1H), 1.66 (qd, 1H) ppm |
| I-71 | ¹HNMR δ= 8.03 (d, 1H), 7.93 (d, 1H), 7.47 (t, 1H), 7.40 (t, 1H), 7.25-7.16 (m, 5H), 7.06 (s, 1H), 4.40 (bs, 1H), 4.34 (s, 2H), 4.09 (bs, 1H), 3.40-3.15 (m, 3H), 2.96 (s, 4H), 2.08-2.02 (m, 2H), 1.65 (bs, 2H) ppm |
| I-74 | ¹HNMR δ= 8.31 (d, 1 H), 8.02 (d, 2H), 7.82 (d, 1H), 7.69 (t, 1H), 7.63 - 7.51 (m, 3H), 5.79 (s, 1H), 4.95 (s, 2H), 3.41 - 3.32 (m, 2H), 2.19 - 2.09 (m, 3H), 2.12 (s, 3H), 2.07 (s, 3H), 1.81 - 1.53 (m, 4H) ppm |
| I-76 | ¹HNMR δ= 8.21 (d, 1H), 8.13 (d, 1H), 7.95 (d, 1H), 7.89 (d, 1H), 7.84 (d, 1H), 7.62 - 7.51 (m, 4H), 7.28 (d, 1H), 5.79 (s, 1H), 4.92 (s, 1H), 3.42 - 3.31 (m, 2H), 2.21 - 2.09 (m, 3H), 2.12 (s, 3H), 2.07 (s, 3H), 1.85 - 1.59 (m, 4H) ppm |
| I-77 | ¹HNMR δ= 7.29 (dd, 2H), 7.85 (dd, 1H), 7.55 - 7.41 (m, 4H), 7.11 (d, 1H), 6.90 (t, 2H), 5.79 (s, 1H), 4.86 (s, 2H), 3.10 (m, 1H), 2.51 (m, 2H), 2.11 (s, 3H), 2.06 (s, 3H), 2.88 - 2.72 (m, 2H), 1.49 - 1.31 (m, 4H) ppm |
| I-78 | ¹HNMR δ= 7.27-7.22 (m, 2H), 7.18-7.13 (m, 3H), 6.45 (s, 1H), 5.22 (bs, 2H), 4.38 (bs, 1H), 3.95 (bs, 1H), 3.33-3.20 (m, 2H), 2.94 (s, 4H), 2.93 (bs, 1H), 2.22 (s, 3H), 2.09-2.02 (m, 2H), 1.80-1.50 (m, 2H) ppm |
| I-79 | ¹HNMR δ= 8.09 (d, 1H), 7.89 (d, 1H), 7.76 (d, 1H), 7.56 - 7.43 (m, 2H), 7.41 - 7.32 (m, 2H), 7.11 (s, 1H), 5.78 (s, 1H), 4.92 (s, 2H), 3.47 - 3.41 (m, 4H), 3.46 - 3.22 (m, 3H), 2.12 (s, 3H), 2.09 - 2.02 (m, 2H), 2.06 (s, 3H), 1.77 - 1.49 (m, 4H) ppm |
| I-80 | ¹HNMR δ= 7.28-7.23 (m, 2H), 7.19-7.14 (m, 3H), 7.02 (s, 1H), 6.44 (s, 1H), 5.20 (bs, 2H), 4.30 (bs, 1H), 3.95 (bs, 1H), 3.30-3.20 (m, 2H), 2.87 (bs, 1H), 2.72 (m, 1H), 2.67-2.62 (m, 2H), 2.22 (s, 3H), 2.07-2.02 (m, 2H), 1.72 (bs, 1H), 1.63-1.47 (m, 5H), 1.19 (d, 3H) ppm |
| I-81 | ¹HNMR δ= 8.09 (d, 1H), 7.90 (d, 1H), 7.75 (d, 1H), 7.56-7.46 (m, 2H), 7.42-7.33 (m, 2H), 7.12 (s, 1H), 6.45 (s, 1H), 5.22 (bs, 2H), 4.31 (bs, 1H), 3.98 (bs, 1H), 3.44 (t, 2H), 3.35-3.26 (m, 2H), 3.15-3.05 (m, 2H), 2.90 (bs, 1H), 2.23 (s, 3H), 2.15-2.05 (m, 2H), 1.75 (bs, 1H), 1.62 (bs, 1H) ppm |
| I-83 | ¹HNMR δ= 7.32-7.21 (m, 2H), 7.19-7.11 (m, 4H), 7.02 (s, 1H), 5.75 (s, 1H), 4.87 (s, 1H), 3.26 - 3.12 (m, 2H), 2.72 - 2.68 (m, 2H), 2.67 - 2.59 (m, 4H), 2.11 (s, 3H), 2.05 (s, 3H), 2.02 - 1.91 (m, 4H), 1.61 - 1.42 (m, 4H), 1.19 (d, 3H) ppm |
| I-84 | ¹HNMR δ= 7.56 (d, 2H), 7.50 (s, 1H), 7.37 (d, 2H), 7.34 (s, 1H), 7.30 - 7.16 (m, 2H), 5.79 (s, 1H), 4.93 (s, 2H), 3.41 - 3.26 (m, 2H), 2.93 - 2.76 (m, 1H), 2.12 (s, 3H), 2.04 (s, 3H), 2.11 - 2.03 (m, 2H), 1.78 - 1.54 (m, 4H) ppm |
| I-85 | ¹HNMR δ= 7.31 - 7.21 (m, 5H), 7.19 - 7.12 (m, 1H), 6.33 (d, 1H), 5.79 (s, 1H), 5.62 - 5.52 (m, 1H), 4.92 (s, 2H), 4.38 - 4.12 (m, 1H), 4.12 - 3.92 (m, 1H), 3.33 - 3.22 (m, 2H), 2.96 - 2.81 (m, 4H), 2.10 (s, 3H), 2.03 (s, 3H), 1.72 - 1.53 (m, 4H), 1.21 (d, 3H) ppm |
| I-86 | ¹HNMR δ= 7.86 (s, 1H), 6.44 (s, 1H), 5.20 (bs, 2H), 4.31 (bs, 1H), 4.08 (s, 1H), 3.98 (bs, 1H), 3.31 (m, 1H), 3.28 (bs, 1H), 2.88 (bs, 1H), 2.22 (s, 3H), 2.13-2.05 (m, 2H), 1.78 (bs, 1H), 1.61 (bs, 1H) ppm |
| I-93 | ¹HNMR (CD₃CN) δ= 7.55 (d, 2H), 7.43 (d, 1H), 7.37 (t, 2H), 7.30-7.28 (m, 2H), 7.18 (d, 1H), 6.39 (s, 1H), 5.41 (d, 1H), 5.26 (s, 2H), 4.42 (d, 1H), 3.54 (t, 1H), 3.45 (m, 1H), 3.38 2.31 (s, 2.30-2.23 (m, 2H), 1.95-1.80 (m, 2H) ppm |
| I-94 | ¹HNMR δ= 7.91 (s, 1H), 7.72 (d, 1H), 7.61 - 7.49 (m, 2H), 7.47 (s, 1H), 6.65 (s, 1H), 6.44 (s, 1H), 5.12 (s, 1H), 3.29 - 3.18 (m, 2H), 2.89 - 2.74 (m, 1H), 2.23 - 2.17 (m, 2H), 2.20 (s, 3H), 2.01 (d, 2H), 1.72 - 1.61 (m, 2H), 1.61 - 1.49 (m, 2H) ppm |
| I-96 | ¹HNMR δ= 7.92 (s, 1H), 7.72 (d, 1H), 7.61 - 7.51 (m, 2H), 7.45 (s, 1H), 6.64 (s, 1H), 5.78 (s, 1H), 4.85 (s, 1H), 3.28 - 3.12 (m, 2H), 3.11 - 3.05 (m, 1H), 2.12 - 2.09 (m, 2H), 2.10 (s, 3H), 2.05 (s, 3H), 1.98 (d, 2H), 1.63 - 1.48 (m, 4H) ppm |
| I-97 | ¹HNMR δ= 8.34 (d, 1H), 8.08 (s, 1H), 8.01 (d, 2H), 7.83 (dd, 1H), 7.68 (m, 1H), 7.62 (m, 1H), 7.57 (dd, 1H), 7.37 (m, 1H), 7.07 (m, 1H), 4.37 (bs, 1H), 4.15 (bs, 1H), 3.86 (bs, 2H), 3.40 (m, 1H), 3.34 (bs, 1H), 2.88 (bs, 1H), 2.16 (bs, 2H), 1.78 (bs, 1H), 1.64 (bs, 1H) ppm |
| I-98 | ¹HNMR δ= 7.70 (s, 1H), 6.27 (s, 1H), 5.08 (d, 1H), 5.01 (d, 1H), 4.12 (bd, 1H), 3.76 (bd, 1H), 3.05 (t, 1H), 2.59 (t, 1H), 1.97 (s, 3H), 1.88-1.77 (m, 2H), 1.56 (tdd, 1H), 1.30 (tdd, 1H), 0.00 (s, 9H) ppm |
| I-99 | ¹HNMR δ= 7.92 (s, 1H), 7.57-7.54 (m, 2H), 7.45-7.41 (m, 3H), 6.45 (s, 1H), 5.23 (bs, 2H), 4.33 (bs, 1H), 3.98 (bs, 1H), 3.36 (m, 1H), 3.28 (bs, 1H), 2.89 (bs, 1H), 2.23 (s, 3H), 2.16-2.08 (m, 2H), 1.80 (bs, 1H), 1.63 (bs, 1H) ppm |
| I-100 | ¹HNMR δ= 7.84 (s, 1H), 7.49 (d, 2H), 6.98 (d, 2H), 6.45 (s, 1H), 5.22 (bs, 2H), 4.33 (bs, 1H), 3.98 (bs, 1H), 3.80 (s, 3H), 3.34 (m, 1H), 3.28 (bs, 1H), 2.89 (bs, 1H), 2.22 (s, 3H), 2.15-2.09 (m, 2H), 1.80 (bs, 1H), 1.63 (bs, 1H) ppm |
| I-101 | ¹HNMR δ= 7.99 (s, 1H), 7.67 (dd, 1H), 7.56 (dd, 1H), 7.45 (td, 1H), 7.39 (td, 1H), 6.45 (s, 1H), 5.23 (bs, 2H), 4.33 (bs, 1H), 3.98 (bs, 1H), 3.37 (m, 1H), 3.28 (bs, 1H), 2.89 (bs, 1H), 2.23 (s, 3H), 2.15-2.09 (m, 2H), 1.80 (bs, 1H), 1.63 (bs, 1H) ppm |
| I-102 | ¹HNMR δ= 7.95 (s, 1H), 7.60-7.55 (m, 2H), 7.52-7.46 (m, 2H), 6.45 (s, 1H), 5.22 (bs, 2H), 4.33 (bs, 1H), 3.98 (bs, 1H), 3.35 (m, 1H), 3.28 (bs, 1H), 2.89 (bs, 1H), 2.22 (s, 3H), 2.15-2.08 (m, 2H), 1.80 (bs, 1H), 1.63 (bs, 1H) ppm |
| I-103 | ¹HNMR δ= 7.86 (s, 1H), 7.47 (dd, 1H), 7.40 (td, 1H), 7.09 (d, 1H), 6.98 (td, 1H), 6.45 (s, 1H), 5.23 (bs, 2H), 4.33 (bs, 1H), 3.98 (bs, 1H), 3.86 (s, 3H), 3.35 (m, 1H), 3.29 (bs, 1H), 2.89 (bs, 1H), 2.23 (s, 3H), 2.15-2.08 (m, 2H), 1.80 (bs, 1H), 1.63 (bs, 1H) ppm |
| I-104 | ¹HNMR δ= 7.81 (s, 1H), 7.38 (d, 1H), 6.63 (d, 1H), 6.56 (dd, 1H), 6.45 (s, 1H), 5.22 (bs, 2H), 4.33 (bs, 1H), 3.98 (bs, 1H), 3.85 (s, 3H), 3.81 (s, 3H), 3.34 (m, 1H), 3.29 (bs, 1H), 2.89 (bs, 1H), 2.22 (s, 3H), 2.15-2.07 (m, 2H), 1.80 (bs, 1H), 1.64 (bs, 1H) ppm |
| I-105 | ¹HNMR (CD₃CN) δ= 7.67 (s, 1H), 7.58 (d, 1H), 7.57 (d, 1H), 7.38 (dd, 1H), 6.37 (s, 1H), 5.04 (bs, 2H), 4.46 (bs, 1H), 3.95 (bs, 1H), 3.33 (m, 1H), 3.29 (bs, 1H), 2.90 (bs, 1H), 2.24 (s, 3H), 2.20-2.13 (m, 2H), 1.90-1.66 (m, 2H) ppm |
| I-106 | ¹HNMR δ= 7.87 (s, 1H), 7.04-6.95 (m, 3H), 6.45 (s, 1H), 5.22 (bs, 2H), 4.33 (bs, 1H), 3.98 (bs, 1H), 3.81 (s, 3H), 3.74 (s, 3H), 3.34 (m, 1H), 3.29 (bs, 1H), 2.89 (bs, 1H), 2.23 (s, 3H), 2.15-2.08 (m, 2H), 1.80 (bs, 1H), 1.63 (bs, 1H) ppm |
| I-123 | ¹HNMR (CD₃CN) δ= 7.44 (d, 1H), 7.22 (dd, 1H), 7.18 (d, 1H), 6.88 (s, 1H), 6.40 (s, 1H), 5.11 (d, 1H), 5.04 (d, 1H), 4.45 (bd, 1H), 3.91 (bd, 1H), 3.27 (m, 1H), 3.09 (t, 2H), 3.00 (t, 2H), 2.84 (td, 1H), 2.23 (s, 3H), 2.15 (bd, 1H), 2.10 (bd, 1H), 1.81 (tdd, 1H), 1.63 (tdd, 1H) ppm |
| I-124 | ¹HNMR (CD₃CN) δ= 8.40 (d, 1H), 7.98 (d, 2H), 7.82 (dd, 1H), 7.76 (s, 1H), 7.68 (m, 1H), 7.62 (m, 1H), 7.56-7.45 (m, 3H), 7.33 (dd, 1H), 4.45 (bd, 1H), 4.06 (bd, 1H), 3.88 (d, 1H), 3.81 (d, 1H), 3.36 (m, 1H), 3.30 (t, 1H), 2.84 (t, 1H), 2.25-2.10 (m, 2H), 1.84 (m, 1H), 1.70 (m, 1H) ppm |
| I-125 | ¹HNMR δ= 7.93 (s, 1H), 7.46-7.40 (m, 2H), 7.36 (d, 1H), 4.45-4.35 (m, 1H), 4.23 (s, 1H), 4.11-4.02 (m, 1H), 3.87 (d, 2H), 2.88-2.72 (m, 1H), 2.15-2.00 (m, 2H), 1.79-1.66 (m, 1H), 1.60-1.46 (m, 1H) |
| I-126 | ¹HNMR (CD₃CN) δ= 7.16-7.05 (m, 4H), 6.87 (s, 1H), 6.39 (s, 1H), 5.11 (d, 1H), 5.03 (d, 1H), 4.46 (d, 1H), 3.91 (d, 1H), 3.32-3.23 (m, 2H), 3.01-2.93 (m, 4H), 2.84 (t, 1H), 2.27 (s, 3H), 2.23 (s, 3H), 2.18-2.07 (m, 2H), 1.82 (tdd, 1H), 1.65 (tdd, 1H) |
| I-128 | ¹HNMR (CD₃CN) δ= 7.28-7.19 (m, 1H), 6.96-6.88 (m, 3H), 6.39 (s, 1H), 5.10 (d, 1H), 5.03 (d, 1H), 4.43 (d, 1H), 3.90 (d, 1H), 3.32-3.19 (m, 2H), 3.07-2.94 (m, 4H), 2.88 (td, 1H), 2.23 (s, 3H), 2.16-2.03 (m, 2H), 1.79 (tdd, 1H), 1.62 (tdd, 1H) |
| I-132 | ²HNMR δ= 7.12 (s, 1H), 6.49 (s, 1H), 5.31 (d, 1H), 5.22 (d, 1H), 4.35 (d, 1H), 3.95 (d, 1H), 3.45-3.20 (m, 2H), 2.82 (dd, 1H), 2.67 (dd, 2H), 2.21 (s, 3H), 2.06 (dd, 2H), 1.80-1.49 (m, 8H), 1.30-1.09 (m, 5H), 0.98-0.85 (m, 2H) ppm |
| I-133 | ¹HNMR (CD₃CN) δ= 7.16-7.12 (m, 1H), 7.11-7.02 (m, 3H), 6.93 (s, 1H), 6.38 (s, 1H), 5.08 (d, 1H), 5.01 (d, 1H), 4.45 (bd, 1H), 3.99 (bd, 1H), 3.28-3.18 (m, 2H), 2.86-2.65 (m, 5H), 2.21 (s, 3H), 2.17-2.03 (m, 3H), 1.94-1.57 (m, 8H) ppm |
| I-134 | ¹HNMR (CD₃CN) δ= 7.17 (s, 1H), 7.14-7.07 (m, 4H), 6.88 (s, 1H), 5.35 (d, 1H), 5.28 (d, 1H), 4.43 (d, 1H), 3.86 (d, 1H), 3.31-3.24 (m, 2H), 3.00-2.95 (m, 4H), 2.86 (ddd, 1H), 2.27 (s, 3H), 2.16 (d, 1H), 2.10 (d, 1H), 1.82 (tdd, 1H), 1.65 (tdd, 1H) ppm |
| I-136 | ¹HNMR (CD₃CN) δ= 7.21 (m, 1H), 7.17 (s, 1H), 6.94-6.86 (m, 3H), 5.34 (d, 1H), 5.28 (d, 1H), 4.39 (d, 1H), 3.84 (d, 1H), 3.30-3.22 (m, 2H), 3.02 (t, 2H), 2.97 (t, 2H), 2.87 (ddd, 1H), 2.13 (d, 1H), 2.06 (d, 1H), 1.79 (tdd, 1H), 1.61 (tdd, 1H) ppm |
| I-138 | ¹HNMR (CD₃CN) δ= 8.32 (d, 1H), 7.44 (d, 1H), 7.17 (s, 1H), 7.06 (dd, 1H), 6.89 (s, 1H), 5.34 (d, 1H), 5.28 (d, 1H), 4.42 (d, 1H), 3.85 (d, 1H), 3.30-3.23 (m, 2H), 3.12 (s, 4H), 2.86 (ddd, 1H), 2.23 (s, 3H), 2.15 (d, 1H), 2.09 (d, 1H), 1.80 (tdd, 1H), 1.62 (tdd, 1H) ppm |
| I-140 | ¹HNMR (CD₃CN) δ= 7.17 (s, 1H), 6.90 (s, 1H), 5.35 (d, 1H), 5.27 (d, 1H), 4.42 (d, 1H), 3.86 (d, 1H), 3.32-3.21 (m, 2H), 2.86 (ddd, 1H), 2.75-2.67 (m, 2H), 2.20-2.04 (m, 2H), 1.85-1.50 (m, 9H), 1.42-1.12 (m, 4H), 1.00-0.88 (m, 2H) ppm |
| I-148 | ¹HNMR δ= 7.18 (t, 1H), 7.13 (t, 1H), 7.03 (t, 1H), 6.91 (s, 1H), 5.43 (d, 1H), 5.35 (s, 1H), 4.32 (d, 1H), 3.94 (d, 1H), 3.30-3.21 (m, 2H), 2.82 (dd, 1H), 2.67 (dd, 2H), 2.10-2.00 (m, 2H), 1.83-1.48 (m, 8H), 1.35-1.10 (m, 5H), 0.95-0.85 (m, 2H) ppm |
| I-149 | ¹HNMR (CD₃CN) δ= 7.17-7.10 (m, 1H), 7.11-7.03 (m, 3H), 6.95 (s, 1H), 6.89 (t, 1H), 6.82 (s, 1H), 6.78 (t, 1H), 5.23 (d, 1H), 5.17 (d, 1H), 4.44 (bd, 1H), 3.88 (bd, 1H), 3.30-3.21 (m, 2H), 2.98-2.67 (m, 5H), 2.15-2.03 (m, 3H), 1.94-1.58 (m, 8H) ppm |
| I-I58 | ¹HNMR δ= 7.18-7.05 (m, 5H), 7.03 (d, 1H), 6.93 (d, 1H), 6.90 (s, 1H), 4.47 (d, 1H), 3.94 (d, 1H), 3.70 (d, 1H), 3.62 (d, 1H), 3.35-3.22 (m, 2H), 2.95-2.85 (m, 4H), 2.78 (t, 1H), 2.25 (s, 3H), 2.23 (s, 3H), 2.15 (s, 3H), 2.10-1.94 (m, 2H), 1.61-1.45 (m, 2H) |
| I-160 | ¹HNMR (CD₃CN) δ= 7.28-7.17 (m, 1H), 7.05 (d, 1H), 6.96 (d, 1H), 6.95-6.85 (m, 4H), 4.52 (d, 1H), 3.93 (d, 1H), 3.68 (d, 1H), 3.62 (d, 1H), 3.26-3.11 (m, 2H), 3.06-2.92 (m, 4H), 2.79 (td, 1H), 2.26 (s, 3H), 2.18 (s, 3H), 2.09-1.97 (m, 2H), 1.60 (qd, 2H) |
| I-166 | ¹HNMR δ= 7.65-7.54 (m, 2H), 7.25-7.10 (m, 4H), 6.88 und 6.74 (s, 1H), 6.50 (s, 1H), 5.37-5.18 (m, 2H), 4.40 und 4.20 (d, 1H), 4.00 und 3.85 (d, 1H), 3.42-3.18 (m, 2H), 2.85 (dd, 1H), 2.38 und 2.20 (s, 3H), 2.22 und 2.20 (s, 3H), 2.19-2.10 und 2.05-1.93 (m, 2H), 1.83 und 1.67 (tdd, 1H), 1.59 und 1.48 (tdd, 1H) ppm |
| I-167 | ¹HNMR δ= 7.86 (dd, 1H), 7.67-7.62 (m, 2H), 7.56 (dd, 1H), 7.43 (dd, 1H), 7.27 (d, 1H), 7.23 (dd, 1H), 6.50 (s, 1H), 5.34 (d, 1H), 5.24 (d, 1H), 4.40 (d, 1H), 3.99 (d, 1H), 3.45-3.20 (m, 2H), 2.83 (dd, 1H), 2.20 (s, 3H), 2.18-2.08 (m, 2H), 1.82 (tdd, 1H), 1.58 (tdd, 1H) ppm |
| I-172 | ¹HNMR δ= 7.37 und 7.30 (s, 1H), 6.49 (s, 1H), 6.44 und 5.49 (dd, 1H), 6.35 und 6.24 (d, 1H), 5.35-5.18 (m, 2H), 4.37 und 4.29 (d, 1H), 3.95 (bs, 1H), 3.35-3.13 (m, 3H), 2.93 und 2.82 (dd, 1H), 2.21 (s, 3H), 2.18-2.02 (m, 2H), 1.85-1.48 (m, 6H), 1.35-1.05 (m, 6H) ppm |
| I-173 | ¹HNMR δ= 7.51 und 7.34 (s, 1H), 7.13-7.04 (m, 4H), 6.54 und 5.67 (dd, 1H), 6.48 und 6.47 (s, 1H), 6.45 und 6.38 (d, 1H), 5.30 und 5.29 (d, 1H), 5.21 und 5.19 (d, 1H), 4.94 und 3.62 (m, 1H), 4.32 und 4.29 (d, 1H), 3.94 (m, 1H), 3.36-3.20 (m, 2H), 2.92-2.73 (m, 3H), 2.20 und 2.18 (s, 3H), 2.16-1.63 (m, 7H), 1.58 (m, 1H) ppm |
| I-174 | ¹HNMR δ= 7.65-7.55 (m, 3H), 7.24-7.17 (m, 4H), 7.15 und 7.11 (s, 1H), 6.89 und 6.72 (s, 1H), 5.65-5.46 (m, 2H), 4.37 und 4.16 (d, 1H), 3.98 und 3.82 (d, 1H), 3.43-3.19 (m, 2H), 2.87 (dd, 1H), 2.38 und 2.20 (s, 3H), 2.18-2.10 umd 2.05-1.92 (m, 2H), 1.82 und 1.66 (tdd, 1H), 1.58 und 1.43 (tdd, 1H) ppm |
| I-175 | ¹HNMR δ= 7.84 (dd, 1H), 7.67-7.56 (m, 4H), 7.41 (t, 1H), 7.27 (d, 1H), 7.23 (td, 1H), 5.62 (d, 1H), 5.50 (d, 1H), 4.37 (d, 1H), 3.98 (d, 1H), 3.45-3.15 (m, 2H), 2.87 (dd, 1H), 2.18-2.09 (m, 2H), 1.82 (tdd, 1H), 1.56 (tdd, 1H) ppm |
| I-180 | ¹HNMR (CD₃CN) δ= 7.17 (s, 1H), 7.13 und 7.02 (s, 1H), 6.49 und 5.55 (dd, 1H), 6.37 und 6.23 (d, 1H), 5.34 (d, 1H), 5.31 (d, 1H), 4.43 und 4.37 (d, 1H), 3.86 (d, 1H), 3.37-3.18 (m, 3H), 2.96 und 2.87 (ddd, 1H), 2.22-2.07 (m, 2H), 1.95-1.52 (m, 6H), 1.42-1.08 (m, 6H) ppm |
| I-181 | ¹HNMR (CD₃CN) δ= 7.25-7.03 (m, 6H), 6.58 und 5.71 (dd, 1H), 6.46 und 6.39 (d, 1H), 5.35-5.23 (m, 2H), 4.98 und 4.67 (m, 1H), 4.42 und 4.34 (d, 1H), 3.84 und 3.63 (m, 1H), 3.34-3.23 (m, 2H), 2.96-2.65 (m, 3H), 2.20-2.04 (m, 2H), 2.00-1.58 (m, 6H) ppm |
| I-182 | ¹HNMR δ= 7.65-7.57 (m, 2H), 7.33-6.70 (m, 8H), 5.43-5.33 (m, 2H), 4.38 und 4.17 (bd, 1H), 3.99 und 3.84 (bd, 1H), 3.43-3.18 (m, 2H), 2.85 (t, 1H), 2.3 und 2.20 (s, 3H), 2.17-1.90 (m, 2H), 1.88-1.42 (m, 2H) ppm |
| I-I83 | ¹HNMR δ= 7.86 (ddd, 1H), 7.68-7.62 (m, 2H), 7.55 (dd, 1H), 7.45-7.38 (m, 1H), 7.27 (d, 1H), 7.23 (dd, 1H), 7.18 (t, 1H), 7.03 (t, 1H), 6.90 (s, 1H), 5.50-5.32 (m, 2H), 4.38 und 4.12 (d, 1H), 3.99 und 3.80 (d, 1H), 3.45-3.15 (m, 2H), 2.86 (dd, 1H), 2.18-2.08 (m, 2H), 1.95 und 1.83 (tdd, 1H), 1.59 und 1.43 (tdd, 1H) ppm |
| I-188 | ¹HNMR δ= 7.37 und 7.30 (s, 1H), 7.18 und 7.16 (t, 1H), 7.03 und 7.02 (t, 1H), 6.90 (s, 1H), 6.44 und 5.49 (dd, 1H), 6.35 und 6.24 (d, 1H), 5.45-5.30 (m, 2H), 4.35 und 4.28 (d, 1H), 3.94 (d, 1H), 3.40-3.15 (m, 3H), 2.93 und 2.82 (dd, 1H), 2.20-2.02 (m, 2H), 1.85-1.48 (m, 6H), 1.37-1.05 (m, 6H) ppm |
| I-189 | ¹HNMR δ= 7.52 und 7.38 (s, 1H), 7.17 und 7.15 (t, 1H), 7.13-7.03 (m, 4H), 7.03 und 7.02 (t, 1H), 6.90 (s, 1H), 6.54 und 5.67 (dd, 1H), 6.45 und 6.38 (d, 1H), 5.45-5.32 (m, 2H), 4.95 und 3.63 (dd, 1H), 4.33 und 4.28 (d, 1H), 3.96-3.90 (m, 1H), 3.40-3.20 (m, 2H), 2.85 und 2.80 (dd, 1H), 2.82-2.70 (m, 2H), 2.16-1.68 (m, 6H), 1.60-1.49 (m, 2H) ppm |
| I-206 | ¹HNMR (CD₃CN) δ= 7.62 (s, 1H), 7.50 (d, 1H), 7.33-7.26 (m, 2H), 7.25-7.17 (m, 1H), 6.39 (s, 1H), 5.10 (d, 1H), 5.03 (d, 1H), 4.48 (d, 1H), 3.92 (d, 1H), 3.36-3.21 (m, 2H), td (2.84, 1H), 2.48 (s, 3H), 2.22 (s, 3H), 2.20-2.09 (m, 2H), 1.85 (tdd,1 H), 1.68 (tdd, 1H) |
| I-207 | ¹HNMR δ= 8.05 (s, 1H), 7.76 (dd, 1H), 7.68 (dd, 1H), 7.46 (td, 1H), 7.38 (td, 1H), 6.49 (s, 1H), 5.32 (d, 1H), 5.23 (d, 1H), 4.37 (d, 1H), 3.98 (d, 1H), 3.43-3.20 (m, 2H), 2.84 (t, 1H), 2.20 (s, 3H), 2.09 (t, 2H), 1.82 (tdd, 1H), 1.58 (tdd, 1H) |
| I-208 | ¹HNMR δ= 8.14 (s, 1H), 7.62-7.51 (m, 1H), 7.28 (d, 1H), 7.26 (d, 1H), 6.49 (s, 1H), 5.32 (d, 1H), 5.23 (d, 1H), 4.37 (d, 1H), 3.97 (d, 1H), 3.44-3.20 (m, 2H), 2.85 (t, 1H), 2.21 (s, 3H), 2.12 (t, 2H), 1.89-1.76 (m, 1H), 1.65-1.50 (m, 1H) |
| I-210 | ¹HNMR δ= 8.44 (d, 1H), 8.12 (s, 1H), 7.75 (d, 1H), 7.35 (dd, 1H), 6.49 (s, 1H), 5.32 (d, 1H), 5.23 (d, 1H), 4.37 (d, 1H), 3.98 (d, 1H), 3.44-3.21 (m, 2H), 2.85 (t, 1H), 2.46 (s, 3H), 2.21 (s, 3H), 2.08-1.96 (m, 2H), 1.88-1.65 (m, 1H), 1.65-1.51 (m, 1H) |
| I-212 | ¹HNMR δ= 7.98 (s, 1H), 7.58 (s, 1H), 7.51 (d, 1H), 7.33 (m, 2H), 7.24 (m, 1H), 5.60 (d, 1H), 5.50 (d, 1H), 4.34 (m, 1H), 3.96 (m, 1H), 3.40-3.25 (m, 2H), 2.86 (m, 1H), 2.45 (s, 3H), 2.09 (m, 2H), 1.80 (m, 1H), 1.55 (m, 1H) ppm |
| I-213 | ¹HNMR δ= 8.05 (s, 1H), 7.76 (dd, 1H), 7.68 (dd, 1H), 7.58 (s, 1H), 7.45 (td, 1H), 7.36 (td, 1H), 5.60 (d, 1H), 5.50 (d, 1H), 4.34 (m, 1H), 3.95 (m, 1H), 3.25-3.39 (m, 2H), 2.86 (m, 1H), 2.10 (m, 2H), 1.80 (m, 1H), 1.5 (m, 1H) ppm |
| I-214 | ¹HNMR δ= 8.14 (s, 1H), 7.60-7.52 (m, 2H), 7.31-7.25 (m, 2H), 5.59 (d, 1H), 5.50 (d, 1H), 4.33 (m, 1H), 3.96 (m, 1H), 3.49-3.15 (m, 2H), 2.86 (m, 1H), 2.11 (m, 2H), 1.80 (m, 1H), 1.54 (m, 1H) ppm |
| I-216 | ¹HNMR δ= 8.44 (dd, 1H), 8.12 (s, 1H), 7.75 (d, 1H), 7.58 (s, 1H), 7.34 (dd, 1H), 5.60 (d, 1H), 5.50 (d, 1H), 4.34 (m, 1H), 3.97 (m, 1H), 3.42-3.26 (m, 2H), 2.87 (m, 1H), 2.46 (s, 3H), 2.12 (m, 2H), 1.81 (m, 1H), 1.56 (m, 1H) ppm |
| I-218 | ¹HNMR δ= 7.98 (s, 1H), 7.60-7.50 (m, 1H), 7.36-7.30 (m, 2H), 7.30-7.21 (m, 1H), 7.18 (t, 1H), 7.02 (t, 1H), 6.90 (s, 1H), 5.43 (d, 2H), 4.41-4.32 (m, 1H), 4.02-3.91 (m, 1H), 2.90-2.80 (m, 1H), 2.46 (s, 3H), 2.19-2.05 (m, 2H), 1.90-1.73 (m, 1H), 1.65-1.52 (m, 1H) |
| I-219 | ¹HNMR 8= 8.05 (s, 1H), 7.76 (d, 1H), 7.69 (d, 1H), 7.46 (t, 1H), 7.39 (t, 1H), 7.18 (t, 1H), 7.02 (t, 1H), 6.90 (s, 1H), 5.39 (d, 2H), 4.40-4.29 (m, 1H), 4.01-3.91 (m, 1H), 2.92-2.81 (m, 1H), 2.19-2.06 (m, 2H), 1.89-1.74 (m, 1H), 1.65-1.50 (m, 1H) |
| I-220 | ¹HNMR δ= 8.14 (s, 1H), 7.65-7.52 (m, 1H), 7.30-7.21 (m, 2H), 7.18 (t, 1H), 7.02 (t, 1H), 6.90 (s, 1H), 5.39 (d, 2H), 4.40-4.29 (m, 1H), 4.02-3.91 (m, 1H), 2.90-2.77 (m, 1H), 2.18-2.02 (m, 2H), 1.90-1.76 (m, 1H), 1.68-1.51 (m, 1H) |
| I-222 | ¹HNMR δ= 8.45 (bs, 1H), 8.12 (s, 1H), 7.75 (d, 1H), 7.38-7.33 (m, 1H), 7.18 (t, 1H), 7.02 (t, 1H), 6.90 (s, 1H), 5.39 (d, 2H), 4.39-4.31 (m, 1H), 4.01-3.92 (m, 1H), 3.00-2.78 (m, 1H), 2.46 (s, 3H), 2.18-2.03 (m, 2H), 1.89-1.76 (m, 1H), 1.65-1.52 (m, 1H) |
| I-223 | ¹HNMR δ= 8.02 (s, 1H), 7.15 (t, 1H), 7.02 (t, 1H), 6.90 (s, 1H), 5.38 (d, 2H), 4.39-4.30 (m, 1H), 4.04-3.91 (m, 1H), 2.87-2.79 (m, 1H), 2.28 (s, 3H), 2.16-2.04 (m, 2H), 1.90-1.73 (m, 1H), 1.62-1.49 (m, 1H) |
| I-224 | ¹HNMR δ= 7.98 (s, 1H), 7.50 (d, 1H), 7.47-7.42 (m, 2H), 7.39-7.32 (m, 3H), 7.27-7.20 (m, 1H), 4.46-4.38 (m, 1H), 4.12-4.04 (m, 1H), 3.88 (d, 2H), 2.85-2.76 (m, 1H), 2.46 (s, 3H), 2.17-2.05 (m, 2H), 1.79-1.67 (m, 1H), 1.62-1.48 (m, 1H) |
| I-225 | ¹HNMR δ= 8.05 (s, 1H), 7.76 (d, 1H), 7.68 (d, 1H), 7.50-7.44 (m, 3H), 7.41-7.30 (m, 2H), 4.46-4.36 (m, 1H), 4.11-4.02 (m, 1H), 3.87 (d, 2H), 2.86-2.74 (m, 1H), 2.17-2.05 (m, 2H), 1.80-1.68 (m, 1H), 1.64-1.50 (m, 1H) |
| I-226 | ¹HNMR δ= 8.13 (s, 1H), 7.65-7.52 (m, 2H), 7.49-7.43 (m, 2H), 7.35 (d, 1H), 7.25 (t, 1H), 4.46-4.37 (m, 1H), 4.13-4.04 (m, 1H), 3.88 (d, 2H), 2.86-2.75 (m, 1H), 2.18-2.04 (m, 2H), 1.80-1.69 (m, 1H), 1.62-1.50 (m, 1H) |
| I-228 | ¹HNMR δ= 8.46 (bs, 1H), 8.12 (s, 1H), 7.76 (d, 1H), 7.68-7.52 (m, 1H), 7.48-7.42 (m, 2H), 7.39-7.32 (m, 1H), 4.46-4.39 (m, 1H), 4.13-4.04 (m, 1H), 3.87 (d, 2H), 2.88-2.75 (m, 1H), 2.47 (s, 3H), 2.18-2.05 (m, 2H), 1.81-1.69 (m, 1H), 1.62-1.50 (m, 1H) |
| I-229 | ¹HNMR δ= 8.01 (s, 1H), 7.47-7.41 (m, 2H), 7.34 (d, 1H), 4.44-4.35 (m, 1H), 4.12-4.04 (m, 1H), 3.87 (d, 2H), 2.86-2.75 (m, 1H), 2.28 (s, 3H), 2.17-2.04 (m, 2H), 1.80-1.68 (m, 1H), 1.61-1.50 (m, 1H) |
| I-230 | ¹HNMR δ= 7.97 (s, 1H), 7.51 (d, 1H), 7.34 (d, 2H), 7.28-7.20 (m, 1H), 7.03 (d, 1H), 6.93 (d, 1H), 6.90 (s, 1H), 4.48 (d, 1H), 3.97 (d, 1H), 3.69 (d, 1H), 3.64 (d, 1H), 3.40-3.25 (m, 1H), 3.20 (t, 1H), 2.78 (t, 1H), 2.45 (s, 3H), 2.24 (s, 3H), 2.15 (s, 3H), 2.02 (t, 2H), 1.65-1.47 (m, 2H) |
| I-231 | ¹HNMR δ= 8.03 (s, 1H), 7.76 (dd, 1H), 7.68 (dd, 1H), 7.45 (td, 1H), 7.38 (td, 1H), 7.03 (d, 1H), 6.93 (d, 1H), 6.88 (s, 1H), 4.48 (d, 1H), 3.98 (d, 1H), 3.70 (d, 1H), 3.64 (d, 1H), 3.40-3.26 (m, 1H), 3.20 (t, 1H), 2.79 (t, 1H), 2.23 (s, 3H), 2.15 (s, 3H), 2.03 (t, 2H), 1.68-1.47 (m, 2H) |
| I-232 | ¹HNMR δ= 8.12 (s, 1H), 7.62-7.51 (m, 1H), 7.27 (t, 2H), 7.03 (d, 1H), 6.93 (d, 1H), 6.90 (s, 1H), 4.53-4.45 (m, 1H), 4.01-3.93 (m, 1H), 3.69 (d, 1H), 3.63 (d, 1H), 3.40-3.15 'm, 2H), 2.78 (t, 1H), 2.23 (s, 3H), 2.15 (s, 3H), 2.14-2.00 (m, 2H), 1.68-1.48 (m, 2H) |
| I-235 | ¹HNMR δ= 8.00 (s, 1H), 7.03 (d, 1H), 6.93 (d, 1H), 6.89 (s, 1H), 4.48 (d, 1H), 3.96 (d, 1H), 3.68 (d, 1H), 3.63 (d, 1H), 3.40-3.15 (m, 2H), 2.77 (t, 1H), 2.28 (s, 3H), 2.23 (s, 3H), 2.15 (s, 3H), 2.04 (t, 2H), 1.65-1.48 (m, 2H) |
| I-236 | ¹HNMR δ= 8.40 (dd, 1H), 7.74-7.56 (m, 5H), 7.17 (dd, 1H), 5.62 (d, 1H), 5.50 (d, 1H), 4.37 (m, 1H), 3.97 (m, 1H), 3.50-3.20 (m, 2H), 2.87 (m, 1H), 2.40 (s, 3H), 2.12 (m, 2H), 1.85 (m, 1H), 1.57 (m, 1H) ppm |
| I-237 | ¹HNMR δ= 7.68-7.50 (m, 3H), 7.25-7.15 (m, 3H), 7.11 (d, 1H), 7.03 (d, 1H), 6.93 (d, 1H), 6.90 (s, 1H), 4.51 (d, 1H), 3.97 (d, 1H), 3.71 (d, 1H), 3.63 (d, 1H), 3.40-3.15 (m, 2H), 2.79 (t, 1H), 2.38 (s, 3H), 2.24 (s, 3H), 2.14 (s, 3H), 2.12-2.00 (m, 2H), 1.65-1.48 (m, 2H) |
| I-238 | ¹HNMR δ= 7.92 (s, 1H), 7.02 (d, 1H), 6.93 (d, 1H), 6.89 (s, 1H), 4.46 (d, 1H), 4.22 (s, 1H), 3.95 (d, 1H), 3.68 (d, 1H), 3.62 (d, 1H), 3.34-3.23 (m, 1H), 3.18 (t, 1H), 2.77 (t, 1H), 2.23 (s, 3H), 2.14 (s, 3H), 2.02 (t, 2H), 1.63-1.44 (m, 2H) |
| I-239 | ¹HNMR δ= 7.93 (s, 1H), 7.17 (t, 1H), 7.02 (t, 1H), 6.90 (s, 1H), 5.38 (d, 2H), 4.37-4.30 (m, 1H), 4.23 (s, 1H), 4.01-3.89 (m, 1H), 2.89-2.77 (m, 1H), 2.15-2.00 (m, 2H), 1.84-1.70 (m, 1H), 1.61-1.49 (m, 1H) |
| I-240 | ¹HNMR δ= 7.73 (s, 1H), 7.59 (s, 1H), 7.54 (d, 1H), 7.42-7.27 (m, 4H), 5.63 (d, 1H), 5.50 (d, 1H), 4.37 (d, 1H), 3.98 (d, 1H), 3.45-3.20 (m, 2H), 2.87 (dd, 1H), 2.18-2.08 (m, 2H), 1.83 (tdd, 1H), 1.58 (tdd, 1H) ppm |
| I-241 | ¹HNMR δ= 7.73 (s, 1H), 7.54 (d, 1H), 7.42-7.27 (m, 4H), 7.19 (t, 1H), 7.03 (t, 1H), 6.91 (s, 1H), 5.45 (d, 1H), 5.36 (d, 1H), 4.38 (d, 1H), 4.01 (d, 1H), 3.45-3.22 (m, 2H), 2.86 (dd, 1H), 2.20-2.09 (m, 2H), 1.83 (tdd, 1H), 1.60 (tdd, 1H) ppm |
| I-242 | ¹HNMR δ= 7.92 (d, 1H), 7.73 (s, 1H), 7.58-7.53 (dd, 1H), 7.45-7.28 (m, 3H), 6.50 (s, 1H), 5.34 (d, 1H), 5.23 (d, 1H), 4.40 (d, 1H), 3.99 (d, 1H), 3.43-3.20 (m, 2H), 2.85 (dd, 1H), 2.21 (s, 3H), 2.18-2.09 (m, 2H), 1.84 (tdd, 1H), 1.59 (tdd, 1H) ppm |

Die chemischen NMR-Verschiebungen in ppm wurden bei 400 MHz falls nicht anders angegeben im Lösungsmittel DMSO-d₆ mit Tetramethylsilan als internem Standard gemessen.

Folgende Abbkürzungen beschreiben die Signalaufspaltung:
b = Breite, s = Singulett, d = Dublett, t = Triplett, q = Quadruplett, m = Multiplett

### Verwendungsbeispiele

### Beispiel A

### Phytophthora-Test (Tomate) / protektiv

| | |
|---|---|
| Lösungsmittel: | 49 Gewichtsteile N, N - Dimethylformamid |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Tomatenpflanzen mit der Wirkstoffzubereitung in der angegebenen Aufwandmenge besprüht. 1 Tag nach der Behandlung werden die Pflanzen mit einer Sporensuspension von ***Phytophthora infestans*** inokuliert und stehen dann 24h bei 100 rel. Feuchte und 22°C. Anschließend werden die Pflanzen in einer Klimazelle bei ca. 96% relativer Luftfeuchtigkeit und einer Temperatur von ca. 20°C aufgestellt.

7 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, daß kein Befall beobachtet wird.

In diesem Test zeigen die erfindungsgemäßen Verbindungen I-1, I-2, I-3, I-9, I-15, I-26, I-27, I-35, I-43, I-50, I-51, I-52, I-53, I-59, I-74, I-77, I-79, I-80, I-81, I-83, I-93, I-96, I-98*, I-99, I-100, I-101, I-102, I-103, I-104, I-105, I-107, I-108, I-109, I-114, I-115, I-116, I-117, I-121, I-122 und I-123 bei einer Konzentration an Wirkstoff von 500ppm einen Wirkungsgrad von 70% oder mehr.

### Beispiel B

### Plasmopara-Test (Rebe) / protektiv

| | |
|---|---|
| Lösungsmittel: | 24,5 Gewichtsteile Aceton |
| | 24,5 Gewichtsteile Dimethylacetamid |
| Emulgator: | 1 Gewichtsteil Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wäßrigen Sporensuspension von ***Plasmopara viticola*** inokuliert und verbleiben dann 1 Tag in einer Inkubationskabine bei ca. 20°C und 100% relativer Luftfeuchtigkeit. Anschließend werden die Pflanzen 4 Tage im Gewächshaus bei ca. 21°C und ca. 90% Luftfeuchtigkeit aufgestellt. Die Pflanzen werden dann angefeuchtet und 1 Tag in eine Inkubationskabine gestellt.

6 Tage nach der Inokulation erfolgt die Auswertung. Dabei bedeutet 0% ein Wirkungsgrad, der demjenigen der Kontrolle entspricht, während ein Wirkungsgrad von 100% bedeutet, daß kein Befall beobachtet wird.

In diesem Test zeigen die erfindungsgemäßen Verbindungen I-1, I-9, I-35, I-43, I-50, I-51, I-53, I-59, I-74, I-80, I-81, I-93, I-96, I-98*, I-99, I-100, I-101, I-102, I-103, I-104, I-105, I-108, I-109, I-114, I-115, I-116 und I-117 bei einer Konzentration an Wirkstoff von 100ppm einen Wirkungsgrad von 70% oder mehr.

## Patentansprüche

1. Verbindungen der Formel **(I)**, 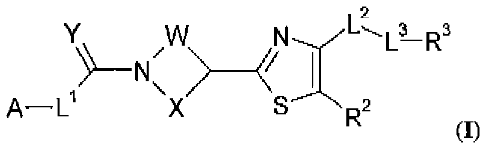 in welcher die Symbole folgende Bedeutungen haben:
A steht für unsubstituiertes oder substituiertes Phenyl,
wobei die Substituenten unabhängig voneinander ausgewählt sind aus folgender Liste
Cyano, Nitro, Halogen, C₁-C₆-Alkyl, C₁-C₃-Halogenalkyl, C₃-C₆-Cycloalkyl, C₃-C₆-Halogencycloalkyl, C₄-C₁₀-Cycloalkylalkyl, C₄-C₁₀-Alkylcycloalkyl, C₅-C₁₀-Alkylcycloalkylalkyl, C₂-C₆-Alkenyl, C₂-C₆-Halogenalkenyl, C₂-C₆-Alkinyl, C₂-C₆-Halogenalkjnyl, Tri(C₁-C₂-alkyl)silyl, Benzyl, Phenyl, Hydroxy, C₁-C₄-Alkoxy, C₁-C₄-Halogenalkoxy, OCH₂OCH₃, SH, C₁-C₄-Alkylthio, C₁-C₆-Halogenalkylthio, CHO, COOH, (C₁-C₄-Alkoxy)carbonyl, CONR⁶R⁷, CR⁶=NOR⁷, (C₁-C₄-Alkyl)carbonyl, (C₁-C₄-Halogenalkyl)carbonyl, (C₁-C₄-Alkyl)carbonyloxy, (C₁-C₄-Alkyl)carbonylthio, C₁-C₄-Alkylsulfinyl, C₁-C₄-Halogenalkylsulfinyl, C₁-C₄-Alkylsulfonyl, C₁-C₄-Halogenalkylsulfonyl, NR⁶R⁷, NR⁶COR⁷, SF₅, SO₂NR⁶R⁷, C₂-C₄-Alkoxyalkyl, oder 1-Methoxycyclopropyl
oder
A ist ein gegebenenfalls benzokondensiertes, unsubstituiertes oder substituiertes 5- oder 6-gliedriges Heteroaryl, wobei die Substituenten unabhängig voneinander ausgewählt sind aus folgender Liste
Substituenten am Kohlenstoff:
Cyano, Nitro, Halogen, C₁-C₆-Alkyl, C₁-C₃-Halogenalkyl, C₃-C₆-Cycloalkyl, C₃-C₆-Halogencycloalkyl C₄-C₁₀-Cycloalkylalkyl, C₄-C₁₀-Alkylcycloalkyl, C₅-C₁₀-Alkylcycloalkylalkyl, C₂-C₆-Alkenyl, C₂-C₆-Halogenalkenyl, C₂-C₆-Alkinyl, C₂-C₆-Halogenalkinyl, Tri(C₁-C₂-alkyl)silyl, Benzyl, Phenyl, Hydroxy, C₁-C₄-Alkoxy, C₁-C₄-Halogenalkoxy, OCH₂OCH₃, SH, C₁-C₄-Alkylthio, C₁-C₆-Halogenalkylthio, CHO, COOH, (C₁-C₄-Alkoxy)carbonyl, CONR⁶R⁷, CR⁶=NOR⁷, (C₁-C₄-Alkyl)carbonyl, (C₁-C₄-Halogenalkyl)carbonyl, (C₁-C₄-Alkyl)carbonyloxy, (C₁-C₄-Alkyl)carbonylthio, C₁-C₄-Alkylsulfnyl, C₁-C₄-Halogenalkylsulfinyl, C₁-C₄-Alkylsulfonyl, C₁-C₄-Halogenalkylsulfonyl, NR⁶R⁷, NR⁶COR⁷, SF₅, SO₂NR⁶R⁷, C₂-C₄-Alkoxyalkyl oder 1-Methoxycyclopropyl
Substituenten am Stickstoff:
Hydroxy, NR⁶R⁷, C₁-C₆-Alkyl, C₁-C₃-Halogenalkyl, C₃-C₆-Cycloalkyl, C₃-C₆-Halogencycloalkyl, C₄-C₁₀-Alkylcycloalkyl, C₂-C₆-Alkenyl, C₂-C₆-Halogenalkenyl, C₂-C₆-Alkinyl, oder C₂-C₆-Halogenalkinyl
L¹ ist (C(R¹)₂)ₙ
mit n = 0 bis 3
R¹ ist gleich oder verschieden unabhängig voneinander Wasserstoff, Halogen, C₁-C₂-Alkyl, C₁-C₂-Halogenalkyl oder Cyano,
unter der Voraussetzung, dass L¹ maximal zwei R¹ enthalten kann, die von Wasserstoff verschieden sind
Y steht für Schwefel oder Sauerstoff,
W ist eine unsubstituierte oder einfach substituierte C₁- bis C₃-Kohlenstoffkette, wobei der Substituent ausgewählt ist aus Oxo, Hydroxy, Cyano oder C₁-C₄-Alkyl
X ist eine unsubstituierte oder einfach substituierte C₁- bis C₂-Kohlenstoffkette, wobei der Substituent ausgewählt ist aus Oxo, Hydroxy, Cyano oder C₁-C₄-Alkyl
R² steht für Wasserstoff, C₁-C₂-Alkyl, C₁-C₂-Halogenalkyl oder Halogen
L² steht für -C(R⁸)₂-C(R⁸)₂- oder -CR⁹=CR⁹- oder -C≡C-
L³ steht für eine direkte Bindung
oder
L³ steht für eine C₁- bis C₄-Kohlenstoffkette, die bis zu zwei Substituenten enthalten kann, wobei die Substituenten unabhängig voneinander ausgewählt sind aus folgender Liste:
Halogen, C₁-C₂-Alkyl, C₁-C₂-Alkoxy, C₁-C₂-Halogenalkyl oder C₃-C₆-Cycloalkyl
R³ steht für unsubstituiertes oder einfach substituiertes C₃-C₁₀-Cycloalkyl, wobei der Substituent ausgewählt ist aus folgender Liste: Cyano, Halogen, C₁-C₆-Alkyl, C₁-C₃-Halogenalkyl, C₃-C₆-Cycloalkyl, C₂-C₆-Alkenyl, C₂-C₆-Halogenalkenyl, C₂-C₆-Alkinyl, C₂-C₆-Halogenalkinyl, Tri(C₁-C₂-alkyl)silyl, Phenyl, Hydroxy, Oxo, C₁-C₆-Alkoxy, C₁-C₆-Halogenalkoxy, C₂-C₆-Alkenyloxy, C₂-C₆-Alkinyloxy, C₁-C₆-Alkylthio, oder C₁-C₆-Halogenalkylthio,
oder
R³ steht für unsubstituiertes oder substituiertes Phenyl,
wobei die Substituenten unabhängig voneinander ausgewählt sind aus folgender Liste:
Cyano, Nitro, Halogen, C₁-C₆-Alkyl, C₁-C₃-Halogenalkyl, C₃-C₆-Cycloalkyl, C₃-C₆-Halogencycloalkyl, C₄-C₁₀-Cycloalkylalkyl, C₄-C₁₀-Halogencycloalkylalkyl, C₄-C₁₀-Alkylcycloalkyl, C₅-C₁₀-Alkylcycloalkylalkyl, C₄-C₁₀-Cycloalkoxyalkyl, C₂-C₆-Alkenyl, C₂-C₆-Halogenalkenyl, C₃-C₈-Cycloalkenyl, C₃-C₈-Halocycloalkenyl, C₂-C₆-Alkinyl, C₂-C₆-Halogenalkinyl, C₂-C₆-Alkoxyalkyl, C₂-C₆-Halogenalkoxyalkyl, C₃-C₈-Alkoxyalkoxyalkyl, Tri(C₁-C₂-alkyl)silyl, Benzyl, Phenyl, Hydroxy, C₁-C₆-Alkoxy, C₂-C₆-Alkoxyalkoxy, C₁-C₆-Halogenalkoxy, C₂-C₆-Alkenyloxy, C₂-C₆-Halogenalkenyloxy, C₂-C₆-Alkinyloxy, C₂-C₆-Halogenalkinyloxy, C₃-C₆-Cycloalkoxy, C₃-C₆-Cycloalkyloxy, C₃-C₆-Halocycloalkoxy, C₄-C₁₀-Cycloalkylalkyloxy, NR⁶R⁷, SH, SF₅, C₁-C₆-Alkylthio, C₁-C₆-Halogenalkylthio, C₂-C₆-AlkylAlkylthio, C₃-C₆-Cycloalkylthio, CHO, COOH, (C₁-C₆-Alkoxy)carbonyl, CONR⁶R⁷, CR⁶=NOR⁷, (C₁-C₆-Alkyl)carbonyl, (C₁-C₆-Halogenalkyl)carbonyl, (C₁-C₆-Alkyl)carbonyloxy, (C₁-C₆-Halogenalkyl)carbonyloxy, (C₁-C₆-Alkyl)carbonylthio, C₁-C₆-Alkytsulfinyl, C₁-C₆-Halogenalkylsulfinyl, C₁-C₆-Alkylsulfanyl, C₁-C₆-Halogenalkylsulfonyl,
NR⁶COR¹ oder SO₂NR⁶R⁷
oder
R³ steht für gesättigtes oder teilweise oder vollständig ungesättigtes Naphthyl oder Indenyl,
das bis zu zwei Substituenten enthalten kann, wobei die Substituenten unabhängig voneinander ausgewählt sind aus folgender Liste:
Cyano, Halogen, C₁-C₆-Alkyl, C₁-C₃-Halogenalkyl, C₃-C₆-Cycloalkyl, C₂-C₆-Alkenyl, C₂-C₆-Halogenalkenyl, C₂-C₆-Alkinyl, C₂-C₆-Halogenalkinyl, Tri(C₁-C₂-alkyl)silyl, Phenyl, Hydroxy, C₁-C₆-Alkoxy, C₁-C₆-Halogenalkoxy, C₂-C₆-Alkenyloxy, C₂-C₆-Alkinyloxy, C₁-C₆-Alkylthio oder C₁-C₆-Halogenalkylthio,
oder
R³ steht für einen unsubstituierten oder substituierten 5-oder 6-gliedrigen Heteroarylrest,
wobei die Substituenten unabhängig voneinander ausgewählt sind aus folgender Liste:
Substituenten am Kohlenstoff: Cyano, Nitro, Halogen, C₁-C₆-Alkyl, C₁-C₃-Halogenalkyl, C₃-C₆-Cycloalkyl, C₃-C₆-Halogencycloalkyl C₄-C₁₀-Cycloaklalkyl, C₄-C₁₀-Alkylcycloalkyl, C₅-C₁₀-Alkylcycloalkylalkyl, C₂-C₆-Alkenyl, C₂-C₆-Halogenalkenyl, C₂-C₆-Alkinyl, C₂-C₆-Halogenalkinyl, Tri(C₁-C₂-alkyl)silyl, Benzyl, Phenyl, Hydroxy, C₁-C₄-Alkoxy, C₁-C₄-Halogenalkoxy, OCH₂OCH₃, SH, C₁-C₄-Alkylthio, C₁-C₆-Halogenalkylthio, COOH, (C₁-C₄-Alkoxy)carbonyl, CONR⁶R⁷, (C₁-C₄-Alkyl)carbonyl, (C₁-C₄-Halogenalkyl)carbonyl, (C₁-C₄-Alkyl)carbonyloxy, (C₁-C₄-Alkyl)carbonylthio, C₁-C₄-Alkylsulfinyl, C₁-C₄-Halogenalkylsulfinyl, C₁-C₄-Alkylsulfonyl, C₁-C₄-Halogenalkylsulfonyl, NR⁶R⁷, NR⁶COR⁷, SF₅, SO₂NR⁶R⁷, C₂-C₄-Alkoxyalkyl oder 1-Methoxycyclopropyl
Substituenten am Stickstoff: Hydroxy, C₁-C₆-Alkyl C₁-C₃-Halogenalkyl, C₃-C₆-Cycloalkyl, C₃-C₆-Halogencycloalkyl, C₄-C₁₀-Alkylcycloalkyl, C₂-C₆-Alkenyl, C₂-C₆-Halogenalkinyl, C₂-C₆-Alklnyl, C₂-C₆-Halogenalkinyl oder Phenyl
oder
R³ steht für benzokondensiertes unsubstituiertes oder substituiertes 5- oder 6-gliedriges Heteroaryl, das bis zu zwei Substituenten enthalten kann, wobei die Substituenten unabhängig voneinander ausgewählt sind aus folgender Liste:
Substituenten am Kohlenstoff: Cyano, Nitro, Halogen, C₁-C₆-Alkyl, C₁-C₃-Halogenalkyl, C₃-C₆-Cycloalkyl, C₃-C₆-Halogencycloalkyl C₄-C₁₀-Cycloalkylalkyl, C₄-C₁₀-Alkylcycloalkyl, C₅-C₁₀-Alkylcycloalkylalkyl, C₂-C₆-Alkenyl, C₂-C₆-Halogenalkenyl, C₂-C₆-Alkinyl, C₂-C₆-Halogenalkinyl, Tri(C₁-C₂-alkyl)silyl, Benzyl, Phenyl, Hydroxy, C₁-C₄-Alkoxy, C₁-C₄-Halogenalkoxy, OCH₂OCH₃, SH, C₁-C₄-Alkylthio, C₁-C₆-Halogenalkylthio, COOH, (C₁-C₄-Alkoxy)carbonyl, CONR⁶R⁷, (C₁-C₄-Alkyl)carbonyl, (C₁-C₄-Halogenalkyl)carbonyl, (C₁-C₄-Alkyl)carbonyloxy, (C₁-C₄-Alkyl)carbonylthio, C₁-C₄-Alkylsulfinyl, C₁-C₄-Halogenalkylsulfinyl, C₁-C₄-Alkylsulfonyl, C₁-C₄-Halogenalkylsulfonyl, NR⁶R⁷, NR⁶COR⁷, SF₅, SO₂NR⁶R⁷, C₂-C₄-Alkoxyalkyl, oder 1-Methoxycyclopropyl
Substituenten arm Stickstoff: C₁-C₆-Alkyl, C₁-C₃-Halogenalkyl, C₃-C₆-Cycloalkyl, C₃-C₆-Halogencycloalkyl, C₄-C₁₀-Alkylcycloalkyl, C₂-C₆-Alkenyl, C₂-C₆-Halogenalkenyl, C₂-C₆-Alkinyl, C₂-C₆-Halogenalkinyl oder Phenyl
oder
R³ ist ein über ein Kohlenstoffatom gebundener, unsubstituierter oder einfach substituierter 5-bis 15-gliedriger Heterocyclylrest, der bis zu zwei weitere Heteroatome, ausgewählt aus Stickstoff, Sauerstoff und Schwefel enthalten kann, wobei der Substituent ausgewählt ist aus folgender Liste:
Substituenten am Kohlenstoff: Cyano, Halogen, C₁-C₆-Alkyl, C₁-C₃-Halogenalkyl, C₃-C₆-Cycloalkyl, C₂-C₆-Alkenyl, C₂-C₆-Halogenalkenyl, C₇-C₆-Alkinyl, C₂-C₆-Halogenalkinyl, Tri(C₁-C₂-alkyl)silyl, Phenyl, Hydroxy, C₁-C₆-Alkoxy, C₁-C₆-Halogenalkoxy, C₂-C₆-Alkenyloxy, C₂-C₆-Alkinyloxy, C₁-C₆-Alkylthio, oder C₁-C₆-Halogenalkylthio
Substituenten am Stickstoff: C₁-C₆-Alkyl, C₁-C₃-Halogenalkyl, C₃-C₆-Cycloalkyl, C₂-C₆-Alkenyl, C₂-C₆-Halogenalkenyl, C₂-C₆-Alkinyl, C₂-C₆-Halogenalkinyl, Tri(C₁-C₂-alkyl)silyl oder Phenyl
R⁶, R⁷ stehen unabhängig voneinander für Wasserstoff, C₁-C₄-Alkyl, C₁-C₃-Halogenalkyl, C₃-C₆-Cycloalkyl
R⁸ ist gleich oder verschieden unabhängig voneinander Wasserstoff, C₁-C₄-Alkyl,
R⁹ ist gleich oder verschieden unabhängig voneinander Wasserstoff, C₁-C₄-Alkyl,
sowie agrochemisch wirksame Salze davon.

2. Verbindungen der Formel **(I)** nach Anspruch 1,
in welcher die Symbole folgende Bedeutungen haben,
A steht für Phenyl, das bis zu zwei Substituenten enthalten kann, wobei die Substituenten unabhängig voneinander ausgewählt sind aus folgender Liste :
Cyano, Nitro, Halogen, C₁-C₄-Alkyl, C₁-C₃-Halogenalkyl, C₃-C₆-Cycloalkyl, C₂-C₄-Alkenyl, C₂-C₄-Alkinyl, Hydroxy, C₁-C₄-Alkoxy, C₁-C₄-Halogenalkoxy, C₁-C₄-Alkylthio, C₁-C₄-Halogenalkylthio, (C₁-C₄-Alkoxy)carbonyl, (C₁-C₄-Alkyl)carbonyl, (C₁-C₄-Halogenalkyl)carbonyl, C₁-C₄-Alkylsulfinyl, C₁-C₄-Halogenalkylsulfinyl, C₁-C₄-Alkylsulfonyl oder C₁-C₄-Halogenalkylsulfonyl,
oder
A steht für einen heteroaromatischen Rest ausgewählt aus folgender Gruppe: ein Furan-2-yl, Furan-3-yl, Thiophen-2-yl, Thiophen-3-yl, Isoxazol-3-yl, Isoxazol-4-yl, Isoxazol-5-yl, 1H-Pyrrol-1-yl, 1H-Pyrrol-2-yl, 1H-Pyrrol-3-yl, Oxazol-2-yl, Oxazol-4-yl, Oxazol-5-yl, Thiazol-2-yl, Thiazol-4-y1, Thiazol-5-yl, Isothiazol-3-yl, Isothiazol-4-yl, Isothiazol-5-yl, Pyrazol-1-yl, Pyrazol-3-yl, Pyrazol-4-yl, Imidazol-1-yl, Imidazol-2-yl, Imidazol-4-yl, 1,2,4-Oxadiazol-3-yl, 1,2,4-Oxadiazol-5-yl, 1,3,4-Oxadiazol-2-yl, 1,2,4-Thiadiazol-3-yl, 1,2,4-Thiadiazol-5-yl, 1,3,4-Thiadiazol-2-yl, 1,2,3-Triazol-1-yl, 1,2,3-Triazol-2-yl, 1,2,3-Triazol-4-yl, 1,2,4-Triazol-1-yl, 1,2,4-Triazol-3-yl, Pyridin-2-yl, Pyridin-3-yl, Pyridin-4-yl, Pyridazin-3-yl, Pyridazin-4-yl, Pyrimidin-2-yl, Pyrimidin-4-yl, Pyrimidin-5-yl, Pyrazin-2-yl, 1,3,5-Triazin-2-yl, 1,2,4-Triaain-3-yl, 1H-Indol-1-yl, 1H-Indol-2-yl, 1H-Indol-3-yl, 1H-Indol-4-yl, 1H-Indol-5-yl, 1H-Indol-6-yl, 1H-Indo1-7-yl, 1H-Benzimidazol-1-yl, 1H-Benzimidazol-2-yl, 1H-Benzimidazol-4-yl, Benzimidazol-5-yl, 1H-Indazol-1-yl, 1H-Indazol-3-yl, 1H-Indazol-4-yl, 1H-Indazol-5-yl, 1H-Indazol-6-yl, 1H-Indazol-7-yl, 2H-Indazol-2-yl, 1-benzofuran-2-yl, 1-benzofuran-3-yl, 1-benzofuran-4-yl, 1-benzofuran-5-yl, 1-benzofaran-6-yl, 1-benzofuran-7-yl, 1-benzothiophen-2-yl, 1-benzothiophen-3-yl, 1-benzothiophen-4-yl, 1-benzothiophen-5-yl, 1-benzothiophen-6-yl, 1-benzothiophen-7-yl, 1,3-Benzothiozol-2-yl, 1,3-Benzoxazol-2-yl, Quinolin-2-yl, Quinolin-3-yl, Quinolin-4-yl, Quinolin-5-yl, Quinolin-6-yl, Quinolin-7-yl, Quinolin-8-yl, Isoquinolin-1-yl, Isoquinolin-3-yl, Isoquinolin-4-yl, Isaquinalin-5-yl, Isoquinolin-6-yl, Isoquinolin-7-yl, oder Isoquinolin-8-yl,
der bis zu zwei Substituenten enthalten kann, wobei die Substituenten unabhängig voneinander ausgewählt sind aus folgender Liste :
Substituenten am Kohlenstoff: Cyano, Halogen, C₁-C₄-Alkyl, C₁-C₃-Halogenalkyl, Hydroxy, C₁-C₄-Alkoxy, C₁-C₄-Halogenalkoxy, C₁-C₄-Alkylthio oder C₁-C₄-Halogenalkylthio,
Substituenten am Stickstoff: C₁-C₄-Alkyl, C₁-C₃-Halogenalkyl, Cyclopropyl, C₂-C₄-Alkenyl oder C₂-C₄-Alkinyl,
L¹ ist (C(R¹)₂)ₙ
mit n = 0 bis 3
R¹ ist gleich oder verschieden unabhängig voneinander Wasserstoff, Chlor, Fluor, Methyl, CF₃ oder Cyano,
unter der Voraussetzung, dass L¹ maximal zwei R¹ enthalten kann, die von Wasserstoff verschieden sind,
Y steht für Schwefel oder Sauerstoff,
W ist eine unsubstituierte oder einfach substituiertes C₁- bis C₂-Kohlenstoffkette, wobei der Substituent ausgewählt ist aus Cyano, oder C₁-C₂-Alkyl
X ist eine unsubstituierte oder einfach substituiertes C₁- bis C₂-Kohlenstoffkette, wobei der Substituent ausgewählt ist aus Cyano, oder C₁-C₂-Alkyl
R² steht für Wasserstoff, C₁-C₂-Alkyl oder Halogen,
L² steht für -C(R⁸)₂-C(R⁸)₂- oder -CR⁹=CR⁹- oder -C≡C-,
L³ steht für eine direkte Bindung,
oder
L³ steht für eine C₁- bis C₄-Kohlenstoffkette, die bis zu zwei Substituenten enthalten kann, wobei die Substituenten unabhängig voneinander ausgewählt sind aus folgender Liste:
C₁-C₂-Alkyl, C₁-C₂-Alkoxy, C₁-C₂-Halogenalkyl oder Cyclopropyl,
R³ steht für unsubstituiertes oder einfach substituiertes C₃-C₁₀-Cycloalkyl, wobei der Substituent ausgewählt ist aus folgender Liste:
Halogen, C₁-C₆-Alkyl, C₃-C₆-Cycloalkyl, C₂-C₆-Alkenyl, C₂-C₆-Alkinyl, Tri(C₁-C₂-alkyl)silyl, Phenyl oder Oxo,
oder
R³ steht für Phenyl, das bis zu drei Substituenten enthalten kann, wobei die Substituenten unabhängig voneinander ausgewählt sind aus folgender Liste :
Cyano, Nitro, Halogen, C₁-C₆-Alkyl, C₁-C₃-Halogenalkyl, C₃-C₆-Cycloalkyl, C₃-C₆-Halogencycloalkyl, C₂-C₆-Alkenyl, C₂-C₆-Halogenalkenyl, C₂-C₆-Alkinyl, C₂-C₆-Halogenalkinyl, Tri(C₁-C₂-alkyl)silyl, Phenyl, Hydroxy, C₁-C₆-Alkoxy, C₁-C₆-Halogenalkoxy, C₁-C₆-Alkylthio oder C₁-C₆-Halogenalkylthio,
oder
R³ steht für Naphthalen-1-yl, Naphthalen-2-yl, 1,2,3,4-Tetrahydronaphthalen-1-yl, 1,2,3,4-Tetrahydronaphthalen-2-yl, 5,6,7,8-Tetrahydronaphthalen-1-yl, 5,6,7,8-Tetrahydronaphthalen-2-yl, Decalin-1-yl, Decalin-2-yl, 1H-Inden-1-yl, 1H-Inden-2-yl, 1H-Inden-3-yl, 1H-Inden-4-yl, 1H-Inden-5-yl, 1H-Inden-6-yl, 1H-Inden-7-yl, 2,3-Dihydro-1H-inden-1-yl, 2,3-Dihydro-1H-inden-2-yl, 2,3-Dihydro-1H-Inden-4-yl oder 2,3-Dihydro-1H-inden-5-yl,
das bis zu zwei Substituenten enthalten kann, wobei die Substituenten unabhängig voneinander ausgewählt sind aus folgender Liste :
Cyano, Halogen, C₁-C₆-Alkyl, C₁-C₃-Halogenalkyl, C₃-C₆-Cycloalkyl, Phenyl, Hydroxy, C₁-C₆-Alkoxy, C₁-C₃-Halogenalkoxy, C₁-C₆-Alkylthio oder C₁-C₃-Halogenalkylthio,
oder
R³ steht für Furan-2-yl, Furan-3-yl, Thiophen-2-yl, Thiophen-3-yl, Isoxazol-3-yl, Isoxazol-4-yl, Isoxazol-5-yl, 1H-Pyrrol-1-yl, 1H-Pyrrol-2-yl, 1H-Pyrrol-3-yl, Oxazol-2-yl, Oxazol-4-yl, Oxazol-5-yl, Thiazol-2-yl, Thiazol-4-yl, Thiazol-5-yl, Isothiazol-3-yl, Isothiazol-4-yl, Isothiazol-5-yl, Pyrazol-1-yl, Pyrazol-3-yl, Pyrazol-4-yl, Imidazol-1-yl, Imidazol-2-yl, Imidazol-4-yl, 1,2,4-Oxadiazol-3-yl, 1,2,4-Oxadiazol-5-yl, 1,3,4-Oxadiazol-2-yl, 1,2,4-Thiadiazol-3-yl, 1,2,4-Thiadiazol-5-yl, 1,3,4-Thiadiazol-2-yl, 1,2,3-Triazol-1-yl, 1,2,3-Triazol-2-y1, 1,2,3-Triazol-4-yl, 1,2,4-Triazol-1-yl, 1,2,4-Triazol-3-yl, Pyridin-2-yl, Pyridin-3-yl, Pyridin-4-yl, Pyridazin-3-yl, Pyridazin-4-yl, Pyrimidin-2-yl, Pyrimidin-4-yl, Pyrimidin-5-yl, Pyrazin-2-yl, 1,3,5-Triazin-2-yl, 1,2,4-Triazin-3-yl,
das bis zu zwei Substituenten enthalten kann, wobei die Substituenten unabhängig voneinander ausgewählt sind aus folgender Leiste :
Substituenten am Kohlenstoff: Cyano, Halogen, C₁-C₆-Alkyl, C₁-C₃-Halogenalkyl, C₃-C₆-Cycloalkyl, Phenyl, Hydroxy, C₁-C₄-Alkoxy, C₁-C₄-Halogenalkoxy, C₁-C₄-Alkylthio oder C₁-C₄-Halogenalkylthio,
Substituenten am Stickstoff: C₁-C₆-Alkyl, C₁-C₃-Halogenalkyl, C₃-C₆-Cycloalkyl, C₂-C₆-Alkenyl, C₂-C₆-Alkinyl oder Phenyl,
oder
R³ steht für 1H-Indol-1-yl, 1H-Indol-2-yl, 1H-Indol-3-yl, 1H-Indol-4-yl, 1H-Indol-5-yl, 1H-Indol-6-yl, 1H-Indol-7-yl, 1H-Benzimidazol-1-yl, 1H-Benzimidazol-2-yl, 1H-Benzimidazol-4-yl, Benzimidazol-5-yl, 1H-Indazol-1-yl, 1H-Indazol-3-yl, 1H-Indazol-4-yl, 1H-Indazol-5-yl, 1H-Indazol-6-yl, 1H-Indazol-7-y1, 2H-Indazol-2-yl, 1-benzofuran-2-yl, 1-benzofuran-3-yl, 1-benzofuran-4-yl, 1-benzofuran-5-yl, 1-benzofuran-6-yl, 1-benzofuran-7-yl, 1-benzothiophen-2-yl, 1-benzothiophen-3-yl, 1-benzothiophen-4-yl, 1-benzothiophen-5-yl, 1-benzothiophen-6-yl, 1-benzothiophen-7-yl, Quinolin-2-yl, Quinolin-3-yl, Quinolin-4-yl, Quinolin-5-yl, Quinolin-6-yl, Quinolin-7-yl, Quinolin-8-yl, Isoquinolin-1-yl, Isoquinolin-3-yl, Isoquinolin-4-yl, Isoquinolin-5-yl, Isoquinolin-6-yl, Isoquinolin-7-yl, oder Isoquinolin-8-yl,
das bis zu zwei Substituenten enthalten kann, wobei die Substituenten unabhängig voneinander ausgewählt sind aus folgender Liste :
Substituenten am Kohlenstoff: Cyano, Halogen, C₁-C₆-Alkyl, C₁-C₃-Halogenalkyl, C₃-C₆-Cycloalkyl, phenyl, Hydroxy, C₁-C₄-Alkoxy, C₁-C₄-Halogenalkoxy, C₁-C₄-Alkylthio oder C₁-C₄-Halogenalkylthio,
Substituenten am Stickstoff: C₁-C₆-Alkyl, C₁-C₃-Halogenalkyl, C₃-C₆-Cycloalkyl, C₂-C₆-Alkenyl, C₂-C₆-Alkinyl oder Phenyl,
oder
R³ steht tür einen über ein Kohlenstoffatom gebundenen, unsubstituierten oder einfach substituierten 5-bis 6-gliedrigen Heterocyclylrest, der bis zu zwei weitere Heteroatome, ausgewählt aus Stickstoff, Sauerstoff und Schwefel enthalten kann, wobei der Substituent ausgewählt ist aus folgender Liste:
Substituenten am Kohlenstoff: C₁-C₆-Alkyl, C₁-C₃-Halogenalkyl, C₃-C₆-Cycloalkyl, C₂-C₆-Alkenyl, C₂-C₆-Alkinyl. Tri(C₁-C₂-alkyl)silyl oder Phenyl,
Substituenten am Stickstoff: C₁-C₆-Alkyl, C₃-C₆-Cycloalkyl, C₂-C₆-Alkenyl, C₂-C₆-Alkinyl, Tri(C₁-C₂-alkyl)silyl oder Phenyl,
R⁸ ist gleich oder verschieden unabhängig voneinander Wasserstoff, C₁-C₂-Alkyl, R⁹ ist gleich oder verschieden unabhängig voneinander Wasserstoff, C₁-C₂-Alkyl, sowie agrochemisch wirksame Salze davon.

3. Verbindungen der Formel (**I**) nach einem oder mehreren der Ansprüche 1 bis 2,
in welcher die Symbole folgende Bedeutungen haben,
A steht für Phenyl, das bis zu zwei Substituenten enthalten kann, wobei die Substituenten unabhängig voneinander ausgewählt sind aus folgender Liste: Cyano, Nitro, Halogen, C₁-C₂-Alkyl, C₁-C₂-Halogenalkyl, Hydroxy, C₁-C₂-Alkoxy oder C₁-C₂-Halogenalkoxy,
oder
A steht für einen heteroaromatischen Rest ausgewählt aus folgender Gruppe: Furan-2-yl, Furan-3-yl, Thiophen-2-yl, Thiophen-3-yl, Oxaxol-2-yl, Oxazol-4-yl, Oxazol-5-yl, Thiazol-2-yl, Thiazol-4-yl, Thiazol-5-yl, Pyrazol-1-yl, Pyrazol-3-yl, Pyrazol-4-yl, Imidazol-1-yl, Imidazol-2-yl, Imidazol-4-yl, 1,2,4-Triazol-1-yl, Pyridin-2-yl, Pyridin-3-yl, Pyridin-4-yl, 1,2,4-Triazin-3-yl, 1H-Benzimidazol-2-yl oder 1,3-Benzothiazol-2-yl,
der bis zu zwei Substitutenten enthalten kann, wobei die Substituenten unabhängig voneinander ausgewählt sind aus folgender Liste:
Substituenten am Kohlenstoff: Cyano, Halogen, C₁-C₂-Alkyl, C₁-C₂-Halogenalkyl, Hydroxy, C₁-C₂-Alkoxy oder C₁-C₂-Halogenalkoxy,
Substituenten am Stickstoff: C₁-C₂-Alkyl oder C₁-C₂-Halogenalkyl,
L¹ ist (C(R¹)₂)ₙ
mit n = 0 bis 3,
R¹ ist gleich oder verschieden unabhängig voneinander Wasserstoff oder Methyl,
unter der Voraussetzung, dass L¹ maximal zwei Methylsubstituenten enthalten kann,
Y steht für Schwefel oder Sauerstoff,
W steht für -CH₂CH₂-,
X steht für -CH₂CH₂-,
R² steht für Wasserstoff, Methyl oder Halogen,
L² steht für -C(R⁸)₂-C(R⁸)₂- oder -CR⁹=CR⁹- oder -C≡C-,
L³ steht für eine direkte Bindung,
oder
L³ steht für eine C₁- bis C₄-Kohlenstoffkette, die bis zu zwei Substituenten enthalten kann, wobei die Substituenten unabhängig voneinander ausgewählt sind aus folgender Liste:
Methyl, Methoxy oder CF₃.
R³ steht für unsubstituiertes oder einfach substituiertes C₃-C₁₀-Cycloalkyl, wobei der Substituent ausgewählt ist aus folgender Liste:
Fluor, Chlor, Methyl, Ethyl, Cyclopropyl, Cyclopentyl oder Cyclohexyl,
oder
R³ steht für Phenyl, das bis zu drei Substituenten enthalten kann, wobei die Substituenten unabhängig voneinander ausgewählt sind aus folgender Liste :
Cyano, Halogen, C₁-C₆-Alkyl, C₁-C₃-Halogenalkyl, Phenyl, Hydroxy, C₁-C₆-Alkoxy, C₁-C₆-Halogenalkoxy, C₁-C₆-Alkylthio oder C₁-C₆-Halogenalkylthio,
oder
R³ steht für Naphthalen-1-yl, Naphthalen-2-yl, 1,2,3,4-Tetrahydronaphthalen-1-yl, 1,2,3,4-Tetrahydronaphthalen-2-yl, 5,6,7,8-Tetrahydronaphthalen-1-yl, 5,6,7,8-Tetrahydronaphthalen-2-yl, Decalin-1-yl, Decalin-2-yl, 1H-Inden-1-yl, 1H-Inden-2-yl, 1H-Inden-3-yl, 1H-Inden-4-yl, 1H-Inden-5-yl, 1H-Inden-6-yl, 1H-Inden-7-yl, 2,3-Dihydro-1H-inden-1-yl, 2,3-Dihydro-1H-inden-2-yl, 2,3-Dihydro-1H-Inden-4-yl oder 2,3-Dihydro-1H-inden-5-yl,
das bis zu zwei Substituenten enthalten kann, wobei die Substituenten unabhängig voneinander ausgewählt sind aus folgender Liste:
Fluor, Chlor, Brom, Jod, Methyl, Ethyl, CF₃, CHF₂, Cyclopropyl, Phenyl, Hydroxy, OMe, OEt, OCF₃, OCHF₂, OC₂F₅, SMe oder SCF₃,
oder
R³ steht für Furan-2-yl, Furan-3-yl, Thiophen-2-yl, Thiophen-3-yl, Isoxazol-3-yl, Isoxazol-4-yl, lsoxazol-5-yl, 1H-Pyrrol-1-yl, 1H-Pyrrol-2-yl, 1H-Pyrrol-3-yl, Oxazol-2-yl, Oxazol-4-yl, Oxazol-5-yl, Thiazol-2-yl, Thiazol-4-yl, Thiazol-5-yl, Isothiazol-3-yl, Isothiazol-4-yl, Isothiazol-5-yl, Pyrazol-1-yl, Pyrazol-3-yl, Pyrazol-4-yl, Imidazol-1-yl, Imidazol-2-yl, Imidazol-4-yl, 1,2,4-Oxadiazol-3-yl, 1,2,4-Oxadiazol-5-yl, 1,3,4-Oxadiazol-2-yl, 1,2,4-Thiadiazol-3-yl, 1,2,4-Thiadiazol-5-yl, 1,3,4-Thiadiazol-2-yl, 1,2,3-Triazol-1-yl, 1,2,3-Triazol-2-yl, 1,2,3-Triazol-4-yl, 1,2,4-Triazol-yl, 1,2,4-Triazol-3-yl, Pyridin-2-yl, Pyridin-3-yl, Pyridin-4-yl, Pyridazin-3-yl, Pyridazin-4-yl, Pyrimidin-2-yl, Pyrimidin-4-yl, Pyrimidin-5-yl, Pyrazin-2-yl, 1,3,5-Triazin-2-yl oder 1,2,4-Triazin-3-yl,
das bis zu zwei Substituenten enthalten kann, wobei die Substituenten unabhängig voneinander ausgewählt sind aus folgender Liste :
Substituenten am Kohlenstoff: Cyano, Fluor, Chlor, Brom, Jod, Methyl, Ethyl, 1-Methylethyl, 1,1-Dimethylethyl, CF₃, CHF₂, C₂F₅, CCl₃, Cyclopropyl, Phenyl, Hydroxy, OMe, OEt, O*iso*Pr, OCF₃, OCHF₂, OC₂F₅, SMe oder SCF₃,
Substituenten am Stickstoff: Methyl, Ethyl, 1-Methylethyl, 1,1-Dimethylethyl, Cyclopropyl oder Phenyl,
oder
R³ steht für 1H-Indol-1-yl, 1H-Indol-2-yl, 1H-Indol-3-yl, 1H-Indol-4-yl, 1H-Indol-5-yl, 1H-Indol-6-yl, 1H-Indol-7-yl, 1H-Benzimidazol-1-yl, 1H-Benzimidazol-2-yl, 1H-Benzimidazol-4-yl, Benzimidazol-5-yl, 1H-Indazol-1-yl, 1H-Indazol-3-yl, 1H-Indazol-4-yl, 1H-Indazol-5-yl, 1H-Indazol-6-yl, 1H-Indazol-7-yl, 2H-Indazol-2-yl, 1-benzofuran-2-yl, 1-benzofuran-3-yl, 1-benzofuran-4-yl, 1-benzofuran-5-yl, 1-benzofuran-6-yl, 1-benzofuran-7-yl, 1-benzothiophen-2-yl, 1-benzothiophen-3-yl, 1-benzothiophen-4-yl, 1-benzothiophen-5-yl, 1-benzothiophen-6-yl, 1-benzothiophen-7-yl, Quinolin-2-yl, Quinolin-3-yl, Quinolin-4-yl, Quinolin-5-yl, Quinolin-6-yl, Quinolin-7-yl, Quinolin-8-yl, Isoquinolin-1-yl, Isoquinolin-3-yl, Isoquinolin-4-yl, Isoquinolin-5-yl, Isoquinolin-6-yl, Isoquinolin-7-yl, oder Isoquinolin-8-yl,
das bis zu zwei Substituenten enthalten kann, wobei die Substituenten unabhängig voneinander ausgewählt sind aus folgender Liste :
Substituenten am Kohlenstoff: Cyano, Fluor, Chlor, Brom, Jod, Methyl, Ethyl, 1-Methylethyl, 1,1-Dimethylethyl, CF₃, CHF₂, C₂F₅, CCl₃, Cyclopropyl, Phenyl, Hydroxy, OMe, OEt, O*iso*Pr*,* OCF₃, OCHF₂, OC₂F₅, SMe oder SCF₃,
Substituenten am Stickstoff: Methyl, Ethyl, 1-Methylethyl, 1,1-Dimethylethyl, Cyclopropyl oder Phenyl,
oder
R³ steht für unsubstituiertes oder einfach substituiertes Pyrrolidin-2-yl, Pyrrolidin-3-yl, Morpholin-3-yl, Morpholin-2-yl, Piperidin-2-yl, Piperidin-3-yl oder Piperazin-2-yl, wobei der Substituent ausgewählt ist aus folgender Liste;
Substituenten am Kohlenstoff: Methyl, Ethyl, CF₃, Cyclopropyl oder Phenyl,
Substituenten am Stickstoff: Ethyl, Methyl, Cyclopropyl oder Phenyl,
R⁸ ist gleich oder verschieden unabhängig voneinander Wasserstoff, Methyl, Ethyl,
R⁹ ist gleich oder verschieden unabhängig voneinander Wasserstoff, Methyl oder Ethyl,
sowie agrochemisch wirksame Salze davon.

4. Verbindungen der Formel (I) nach einem oder mehreren der Ansprüche 1 bis 3,
in welcher die Symbole folgende Bedeutungen haben,
A steht für Phenyl, das bis zu zwei Substituenten enthalten kann, wobei die Substituenten unabhängig voneinander ausgewählt sind aus folgender Liste:
Cyano, Fluor, Chlor, Brom, Jod, Methyl, Ethyl, CF₃, CHF₂, C₂F₅, CCl₃, Hydroxy, OMe, OCF₃, OCHF₂, OCH₂CF₃ oder OC₂F₅.
oder
A steht für einen heteroaromatischen Rest ausgewählt aus folgender Gruppe: Thiophen-3-yl, Pyrazol-1-yl, Pyrazol-4-yl, Pyridin-4-yl, 1H-Benzimidazol-2-yl oder 1,3-Benzothiazol-2-yl,
der bis zu zwei Substituenten enthalten kann, wobei die Substituenten unabhängig voneinander ausgewählt sind aus folgender Liste:
Substituenten am Kohlenstoff: Cyano, Fluor, Chlor, Brom, Jod, Methyl, Ethyl, CF₃, CHF₂, C₂F₅, CCl₃, Hydroxy, OMe, OCF₃, OCHF₂, OCH₂CF₃, oder OC₂F₅.
Substituenten am Stickstoff: Methyl, Ethyl oder CF₃.
L¹ ist (C(R¹)₂)ₙ
mit n = 0 bis 3,
R¹ ist gleich oder verschieden unabhängig voneinander Wasserstoff oder Methyl,
unter der Voraussetzung, dass L¹ maximal einen Methylsubstituenten enthalten kann,
Y steht für Schwefel oder Sauerstoff,
W steht für -CH₂CH₂-,
X steht für -CH₂CH₂-,
R² steht für Wasserstoff, Methyl, Chlor oder Brom,
L² steht für -C(R⁸)₂-C(R⁸)₂- oder -CR⁹=CR⁹- oder -C=C-,
L³ steht für eine direkte Bindung,
oder
L³ steht für eine C₁- bis C₄-Kohlenstoffkette,
R³ steht für Cyclopropyl, Cyclobutyl, Cyclopentyl, Cyclohexyl, Cycloheptyl oder Cyclooctyl,
oder
R³ steht für Phenyl, das bis zu zwei Substituenten enthalten kann, wobei die Substituenten unabhängig voneinander ausgewählt sind aus folgender Liste :
Cyano, Fluor, Chlor, Brom, Jod, Methyl, Ethyl, 1-Methylethyl, 1,1-Dimethylethyl, CF₃, CHF₂, C₂F₅, CCl₃, Phenyl, Hydroxy, OMe, OEt, O*iso*Pr, OCF₃, OCHF₂, OC₂F₅, SMe oder SCF₃,
oder
R³ steht für Naphthalen-1-yl, Naphthaten-2-yl, 1,2,3,4-Tetrahydronaphthalen-1-yl, 1,2,3,4-Tetrahydronaphthalen-2-yl, 5,6,7,8-Tetrahydronaphthalen-1-yl, 5,6,7,8-Tetrahydroriaphthalen-2-yl, Decalin-1-yl, Decalin-2-yl, 1H-Inden-1-yl, 1H-Inden-2-yl, 1H-Inden-3-yl, 1H-Inden-4-yl, 1H-Inden-5-yl, 1H-Inden-6-yl, 1H-Inden-7-yl, 2,3-Dihydro-1H-inden-1-yl, 2,3-Dihydro-1H-inden-2-yl, 2,3-Dihydro-1H-Inden-4-yl oder 2,3-Dihydro-1H-inden-5-yl,
oder
R³ steht für Furan-2-yl, Furan-3-yl, Thiophen-2-yl, Thiophen-3-yl, Isoxazol-3-yl, Isoxazol-4-yl, Isoxazol-5-yl, 1H-Pyrrol-1-yl, 1H-Pyrrol-2-yl, 1H-Pyrrol-3-yl, Oxazol-2-yl, Oxazol-4-yl, Oxazol-5-yl, Thiazol-2-yl, Thiazol-4-yl, Thiazol-5-yl, Isothiazol-3-yl, Isothiazol-4-yl, Isothiazol-5-yl, Pyrazol-1-yl, Pyrazal-3-yl, Pyrazol-4-yl, Imidazol-1-yl, Imidazol-2-yl, Imidazol-4-yl, 1,2,4-Oxadiazol-3-yl, 1,2,4-Oxadiazol-5-yl, 1,3,4-Oxadiazol-2-yl, 1,2,4-Thiadiaxal-3-yl, 1,2,4-Thiadiazol-5-yl, 1,3,4-Thiadiazol-2-yl, 1,2,3-Triazol-1-yl, 1,2,3-Triazol-2-yl, 1,2,3-Triazol-4-yl, 1,2,4-Triazol-1-yl, 1,2,4-Triazol-3-yl, Pyridin-2-yl, Pyridin-3-yl, Pyridin-4-yl, Pyridazin-3-yl, Pyridazin-4-yl, Pyrimidin-2-yl, Pyrimidin-4-yl, Pyrimidin-5-yl, Pyrazin-2-yl, 1,3,5-Triazin-2-yl oder 1,2,4-Triazin-3-yl,
oder
R³ steht für 1H-Indol-1-yl, 1H-Indol-2-yl, 1H-Indol-3-yl, 1H-Indol-4-yl, 1H-Indol-5-yl, 1H-Indol-6-yl, 1H-Indol-7-yl, 1H-Benzimidazol-1-yl, 1H-Benzimidazol-2-yl, 1H-Benzimidazol-4-yl, Benzimidazol-5-yl, 1H-Indazol-1-yl, 1H-Indazol-3-yl, 1H-Indazol-4-yl, 1H-Indazol-5-yl, 1H-Indazol-6-yl, 1H-Indazol-7-yl, 2H-Indazol-2-yl, 1-benzofuran-2-yl, 1-benzofuran-3-yl, 1-benzofuran-4-yl, 1-benzofuran-5-yl, 1-benzofuran-6-yl, 1-benzofuran-7-yl, 1-benzothiophen-2-yl, 1-benzothiophen-3-yl, 1-benzothiophen-4-yl, 1-benzothiophen-5-yl, 1-benzothiophen-6-yl, 1-benzothiophen-7-yl, Quinolin-2-yl, Quinolin-3-yl, Quinolin-4-yl, Quinolin-5-yl, Quinolin-6-yl, Quinolin-7-yl, Quinolin-8-yl, Isoquinolin-1-yl, Isoquinolin-3-yl, Isoquinolin-4-yl, Isoquinolin-5-yl, Isoquinolin-6-yl, Isoquinolin-7-yl oder Isoquinolin-8-yl,
oder
R³ steht für Pyrrolidin-2-yl, Pyrrolidin-3-yl, Morpholin-3-yl, Morpholin-2-yl, Piperidin-2-yl, Piperidin-3-yl, oder Piperazin-2-yl,
R⁸ ist gleich oder verschieden unabhängig voneinander Wasserstoff, Methyl oder Ethyl,
R⁹ ist gleich oder verschieden unabhängig voneinander Wasserstoff, Methyl oder Ethyl,
sowie agrochemisch wirksame Salze davon.

5. Verfahren zur Bekämpfung von pflanzenpathogenen Schadpilzen in und/oder auf Pflanzen oder in und/oder auf Saatgut von Pflanzen, **dadurch gekennzeichnet, dass** man Verbindungen der Formel (I) gemäß einem oder mehreren der Ansprüche 1 bis 4, auf die pflanzenpathogenen Schadpilze und/oder deren Lebensraum ausbringt.

6. Mittel zur Bekämpfung von pflanzenpathogenen Schadpilzen in und/oder auf Pflanzen oder in und/oder auf Saatgut von Pflanzen, **gekennzeichnet durch** einen Gehalt an mindestens einem gemäß einem oder mehreren der Ansprüche 1 bis 4, neben Streckmitteln und/oder oberflächenaktiven Stoffen.

7. Verwendung von heterocyclyl substituierten Thiazole der Formel **(I)**, gemäß einem oder mehreren der Ansprüche 1 bis 4 zur Bekämpfung von pflanzenpathogenen Schadpilzen in und/oder auf Pflanzen oder in und/oder auf Saatgut von Pflanzen.

8. Verfahren zur Herstellung von Mitteln zur Bekämpfung von pflanzenpathogenen Schadpilzen, **dadurch gekennzeichnet, dass** man heterocyclyl substituierte Thiazole der Formel (**I**) gemäß einem oder mehreren der Ansprüche 1 bis 4 mit Streckmitteln und/oder oberflächenaktiven Stoffen vermischt.

9. Verfahren zum Herstellen der Verbindungen der Formel (**I**) umfassend wenigstens einen der folgenden Schritte (a) bis (n):
(a) Umsetzung von Verbindungen der Formel (**VII**) zu Verbindungen der Formel (**VIII**) in Gegenwart einer metallorganischen Verbindung R⁹-M und gegebenenfalls in Gegenwart eines Lösungsmittels gemäß dem nachfolgenden Reaktionsschema (Schema 1): 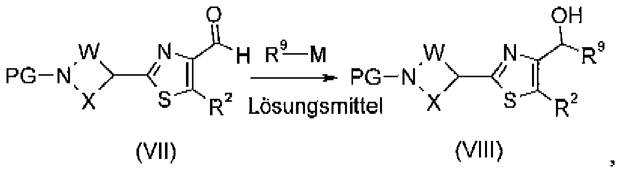 wobei
PG = unsubstituiertes oder substituiertes Phenylmethyl (wobei die Substituenten unabhängig voneinenader ausgewählt sind aus Methyl, Methoxy, Nitro, Dioxolano), Acetyl, C₁-C₄-Alkoxycarbonyl, oder Benzyloxycarbonyl,
M = z.B. MgCl, MgBr, MgI, Li,
R⁹= C₁-C₄-Alkyl oder C₃-C₆-Cycloalkyl,
W, X, und R² wie für Formel (**I**) in Anspruch 1 definiert.
(b) Umsetzung von Alkoholen der Formel (**VIII**) mit einem Oxidationsmittel in Gegenwart eines Lösungsmittels zu Ketonen der Formel (**IX**) gemäß dem nachfolgenden Reaktionsschema (Schema 2): 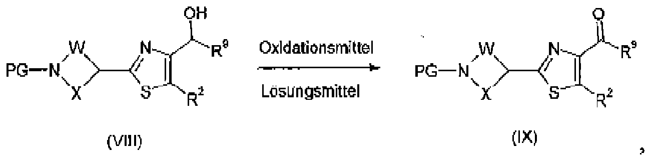 wobei
PG = unsubstituiertes oder substituiertes Phenylmethyl (wobei die Substituenten unabhängig voneinenader ausgewählt sind aus Methyl, Methoxy, Nitro, Dioxolano), Acetyl, oder C₁-C₄-Alkoxycarbonyl,
R⁹ = C₁-C₄-Alkyl oder C₃-C₆-Cycloalkyl,
W, X, und R² wie für Formel (**I**) in Anspruch 1 definiert.
(c) Umsetzung von Ketonen der Formel (**IX**) zu Verbindungen der Formel (**IVb**) in Gegenwart einer Base oder einer Säure und gegebenenfalls eines Lösungsmittels gemäß dem nachfolgenden Reaktionsschema (Schema 3): 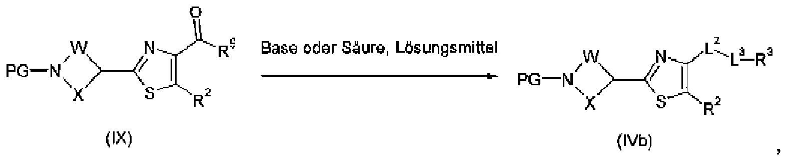 wobei
PG = unsubstituiertes oder substituiertes Phenylmethyl (wobei die Substituenten unabhängig voneinenader ausgewählt sind aus Methyl, Methoxy, Nitro, Dioxolano), Acetyl, oder C₁-C₄-Alkoxycarbonyl,
L²= -CR⁹=CR⁹-,
W, X, L³, R², R³ und R⁹ wie für Formel (**I**) in Anspruch 1 definiert.
(d) Umsetzung von Aldehyden der Formel (**VII**) zu Verbindungen der Formel (**IVb**) in Gegenwart einer Base oder einer Säure und gegebenenfalls eines Lösungsmittels gemäß dem nachfolgenden Reaktionsschema (Schema 4): 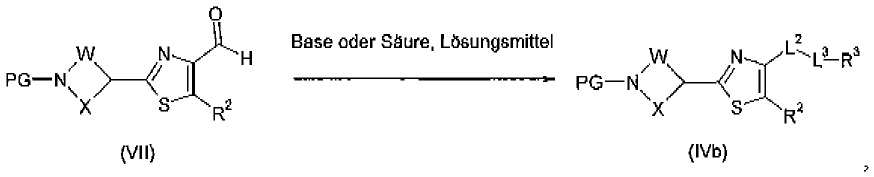 wobei
PG = unsubstituiertes oder substituiertes Phenylmethyl (wobei die Substituenten unabhängig voneinenader ausgewählt sind aus Methyl, Methoxy, Nitro, Dioxolano), Acetyl, oder C₁-C₄-Alkoxycarbonyl,
L²= -CR⁹=CR⁹-,
W, X, L³, R², R³ und R⁹ wie für Formel (**I**) in Anspruch 1 definiert.
(e) Umsetzung von Aldehyden der Formel (**VII**) zu Alkinen der Formel (**X**) in Gegenwart einer Base und gegebenenfalls eines Lösungsmittels gemäß dem nachfolgenden Reaktionsschema (Schema 5): 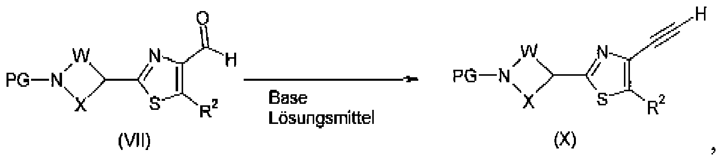 wobei
PG = unsubstituiertes oder substituiertes Phenylmethyl (wobei die Substituenten unabhängig voneinenader ausgewählt sind aus Methyl, Methoxy, Nitro, Dioxolano), Acetyl, oder C₁-C₄-Alkoxycarbonyl,
W, X and R² wie für Formel (**I**) in Anspruch 1 definiert.
(f) Umsetzung von Alkinen der Formel (**X**) mit einem R³-L³-Halogenid in einer ggf. katalysierten C-C-Kupplungsreaktion in Gegenwart einer Base und gegebenenfalls eines Lösungsmittels zu Verbindungen der Formel (**IVc**) gemäß dem nachfolgenden Reaktionsschema (Schema 6): 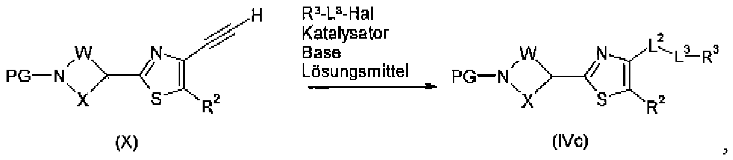 wobei
PG = unsubstituiertes oder substituiertes Phenylmethyl (wobei die Substituenten unabhängig voneinenader ausgewählt sind aus Methyl, Methoxy, Nitro, Dioxolano), Acetyl, oder C₁-C₄-Alkoxycarbonyl,
Hal = Cl, Br oder I,
L² = -C≡C-,
W, X, L³, R² und R³ wie für Formel (**I**) in Anspruch 1 definiert.
(g) Umsetzung von Alkinen der Formel (**X**) mit ein Trialkylsilylchlorid (R¹⁰R¹¹R¹²)SiCl oder -triflat (R¹⁰R¹¹R¹²)SiOSO₂CF₃, oder anderen bekannten Silylierungsreagenzien in Gegenwart einer Base und gegebenenfalls eines Lösungsmittels zu Verbindungen der Formel (**XI**) gemäß dem nachfolgenden Reaktionsschema (Schema 7): 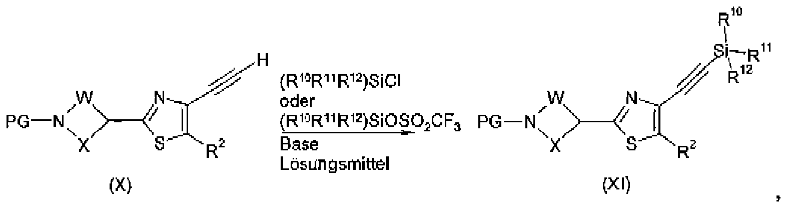 wobei
PG = unsubstituiertes oder substituiertes Phenylmethyl (wobei die Substituenten unabhängig voneinenader ausgewählt sind aus Methyl, Methoxy, Nitro, Dioxolano), Acetyl, oder C₁-C₄-Alkoxycarbonyl,
R¹⁰, R¹¹ und R¹² = C₁-C₄ alkyl oder Phenyl,
W, X und R² wie für Formel (**I**) in Anspruch 1 definiert.
(h) Umsetzung von Alkinen der Formel (**XI**) mit einer Verbindung R³-L³-Hal ggf. in Gegenwart eines Katalysators in Gegenwart einer Base und Gegebenenfalls eines Lösungsmittels zu Verbindungen der Formel (**IVc**) gemäß dem nachfolgenden Reaktionsschema, (Schema 8): 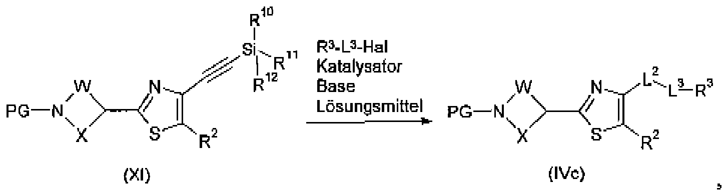 wobei
PG = unsubstituiertes oder substituiertes Phenylmethyl (wobei die Substituenten unabhängig voneinenader ausgewählt sind aus Methyl, Methoxy, Nitro, Dioxolano), Acetyl, oder C₁-C₄-Alkoxycarbonyl,
Hal = Cl, Br oder I,
R² = Wasserstoff, C₁-C₂-Alkyl oder C₁-C₂-Halogenalkyl,
L²=-C≡C-,
R¹⁰, R¹¹ und R¹² = C₁-C₄-Alkyl oder Phenyl,
W, X, L³ und R³ wie für Formel (**I**) in Anspruch 1 definiert.
(i) Umsetzung von Thioamiden der Formel (**V**) mit Verbindungen der Formel (**VIa**) oder (**VIb**) zu Verbindungen der Formeln (**IVa**) oder (**IVb**), in Gegenwart einer Base und gegebenenfalls in Gegenwart eines Lösungsmittels gemäß dem nachfolgenden Reaktionsschema (Schema 9): 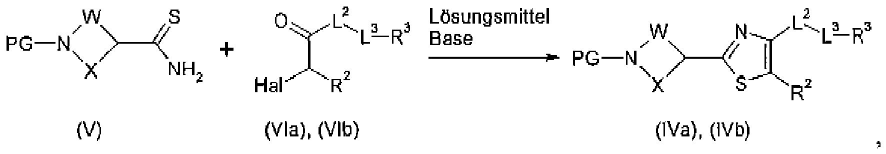 wobei
PG = unsubstituiertes oder substituiertes Phenylmethyl (wobei die Substituenten unabhängig voneinenader ausgewählt sind aus Methyl, Methoxy, Nitro, Dioxolano), Acetyl, C₁-C₄-Alkoxycarbonyl oder Benzyloxycarbonyl,
Hal = Cl, Br oder I,
L² = -C(R⁸)₂-C(R⁸)₂- für Verbindungen mit der Formel (**IVa**) und (**VIa**),
L² = -CR⁹=CR⁹ - für Verbindungen mit der Formel (**IVb**) und (**VIb**),
W, X, L³, R², R³, R⁸ und R⁹ wie für Formel (**I**) in Anspruch 1 definiert.
(j) Umsetzung von Verbindungen der Formeln (**IVb**) oder (**IVc**) zu Verbindungen der Formel (**IVa**) in Gegenwart von Wasserstoff, eines Katalysators und gegebenenfalls eines Lösungsmittels gemäß dem nachfolgenden Reaktionsschema, (Schema 10): 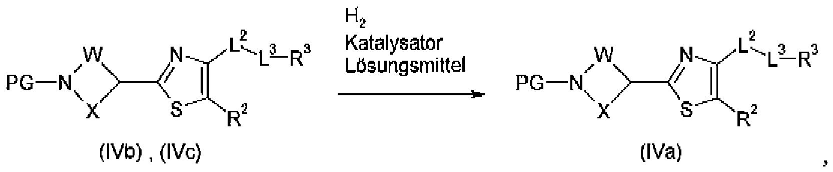 wobei
PG = Acetyl oder C₁-C₄-Alkoxycarbonyl,
L² = -C(R⁸)₂-C(R⁸)₂- für Verbindungen mit der Formel (**IVa**),
L² = -CR⁹=CR⁹- für Verbindungen mit der Formel (**IVb**),
L² = -C≡C- für Verbindungen mit der Formel (**IVc**),
W, X, L³, R², R³, R⁸ und R⁹ wie für Formel (**I**) in Anspruch 1 definiert.
(k) Umsetzung von Verbindungen der Formel (**IV**) zu Verbindungen der Formel **(II)** gegebenenfalls in Gegenwart eines Lösungsmittels sowie ggf. in Gegenwart einer Säure oder ggf. in Gegenwart einer Base oder ggf. in Gegenwart einer Wasserstoffquelle gemäß dem nachfolgenden Reaktionsschema (Schema 11): 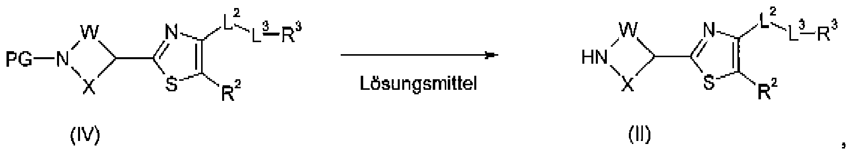 wobei
PG = Acetyl, C₁-C₄-Alkoxycarbonyl oder Benzyloxycarbonyl, W, X, L², L³, R², und R³ wie für Formel (**I**) in Anspruch 1 definiert.
(l) Umsetzung von Verbindungen der Formel (**IIa**') zu Verbindungen der Formel (**IIa**) in Gegenwart eines Halogenierungsmittels, gegebenenfalls in Gegenwart einer Säure und eines Lösungsmittels gemäß dem nachfolgenden Reaktionsschema (Schema 12): 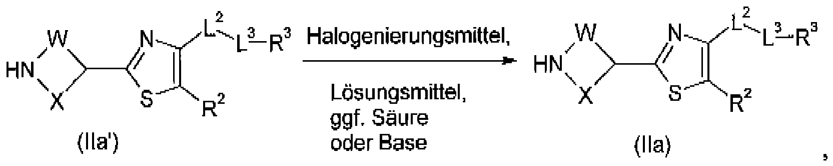 wobei
L²=C(R⁸)₂-C(R⁸)₂-,
R² = I, Br oder Cl für Verbindungen mit der Formel (**IIa**),
R² = Wasserstoff für Verbindungen mit der Formel (**IIa'**)**,**
W, X, L³, R³ und R⁸ wie für Formel (**I**) in Anspruch 1 definiert.
(m) Umsetzung von Verbindungen der Formel (**III**) mit Verbindungen der Formel (**II**) zu Verbindungen der Formel (**I**) gegebenenfalls in Gegenwart eines Kupplungsreagenzes, einer Base und eines Lösungsmittels gemäß dem nachfolgenden Reaktionsschema (Schema 13): 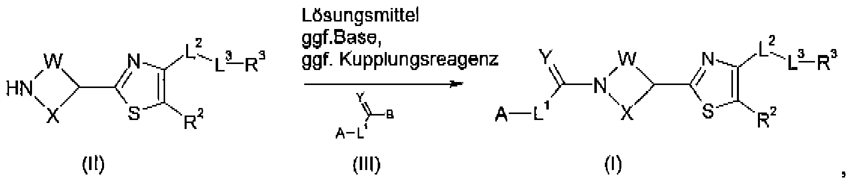 wobei
B = OH, Chlor, Brom oder Jod,
Y = Sauerstoff,
A, W, X, L¹, L², L³, R², und R³ wie für Formel (**I**) in Anspruch 1 definiert sind.
(n) Umsetzung von Verbindungen der Formel (**I**) zu Verbindungen der Formel (**I**) in Gegenwart eines Schwefelungsreagenzes und gegebenenfalls in Gegenwart eines Lösungsmittels gemäß dem nachfolgenden Reaktionsschema (Schema 14): 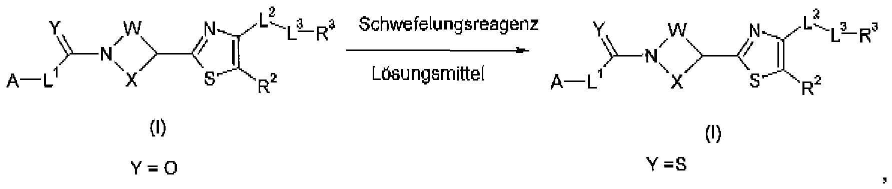 wobei
A, W, X, L¹, L², L³, R², und R³ wie für Formel (**I**) in Anspruch 1 definiert sind.

10. Verfahren zum Herstellen der Verbindungen der Formel **(I)** umfassend wenigstens einen der folgenden Schritte (o) bis (u):
(o) Umsetzung von Aldehyden der Formel (**VII**) zu Alkinen der Formel (**XIII**) in Gegenwart einer Base und gegebenenfalls eines Lösungsmittels gemäß dem nachfolgenden Reaktionsschema (Schema 17): 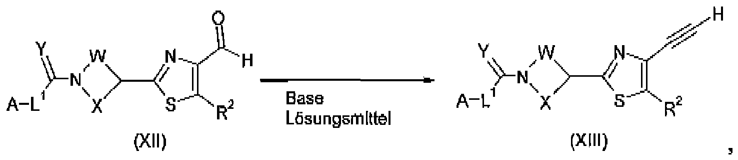 wobei
A, W, X, Y, L¹ und R² wie für Formel (**I**) oben definiert.
(p) Umsetzung von Alkinen der Formel (**XIII**) mit ein Trialkylsilylchlorid (R¹⁰R¹¹R¹²)SiCl oder -triflat (R¹⁰R¹¹R¹²)SiOSO₂CF₃, oder anderen bekannten Silylierungsreagenzien in Gegenwart einer Base und gegebenenfalls eines Lösungsmittels zu Verbindungen der Formel (**XIV**) gemäß dem nachfolgenden Reaktionsschema (Schema 18): 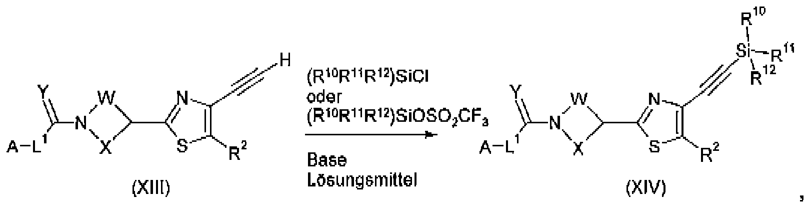 wobei
R¹⁰, R¹¹ und R¹² = C₁-C₄ alkyl oder Phenyl,
A, W, X, Y, L¹ und R² wie für Formel (**I**) oben definiert.
(q) Umsetzung von Alkanen der Formel (**XIII**) mit einem R³-L³-Halogenid in einer ggf. katalysierten C-C-Kupplungsreaktion in Gegenwart einer Base und gegebenenfalls eines Lösungsmittels zu Verbindungen der Formel (Ic) gemäß dem nachfolgenden Reaktionsschema (Schema 19): 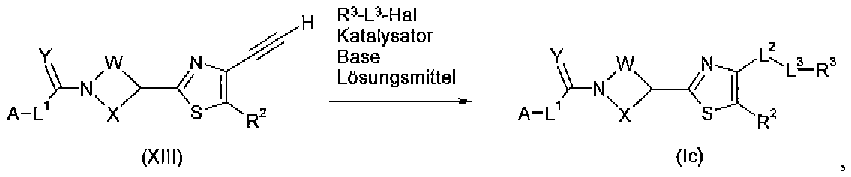 wobei
Hal = Cl, Br oder I,
L² = -C≡C-,
A, W, X, Y, L¹ L³, R² und R³ wie für Formel (**I**) oben definiert.
(r) Umsetzung von Alkinen der Formel (**XIV**) mit einer Verbindung R³-L³-Hal ggf. in Gegenwart eines Katalysators in Gegenwart einer Base und gegebenenfalls eines Lösungsmittels zu Verbindungen der Formel (**Ic**) gemäß dem nachfolgenden Reaktionsschema (Schema 20): 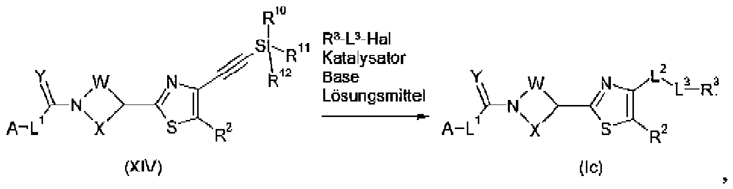 wobei
Hal = Cl, Br oder I,
L² = -C≡C-,
R¹⁰, R¹¹ und R¹² = C₁-C₄-Alkyl oder Phenyl,
A, W, X, Y, L¹ L³, R² und R³ wie für Formel (**I**) oben definiert.
(s) Umsetzung von Aldehyden der Formel (**XII**) zu Verbindungen der Formel (**Ib**) in Gegenwart einer Base oder einer Säure und gegebenenfalls eines Lösungsmittels gemäß dem nachfolgenden Reaktionsschema (Schema 21): 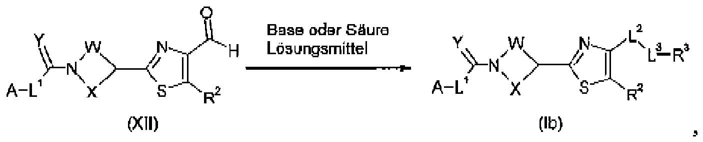 wobei
L² = -CR⁹=CR⁹-,
A, W, X, Y, L¹ L³, R², R³ und R⁹ wie für Formel (**I**) oben definiert.
(t) Umsetzung von Verbindungen der Formeln (**Ic**) zu Verbindungen der Formel (**Ib**) in Gegenwart von Wasserstoff, eines Katalysators und gegebenenfalls eines Lösungsmittels gemäß dem nachfolgenden Reaktionsschema, (Schema 22): 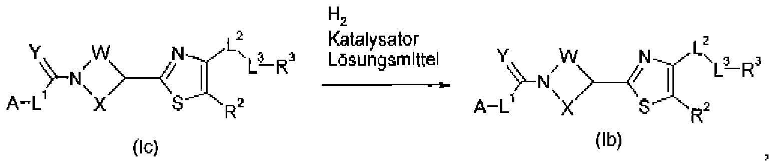 wobei
L²= -CR⁹=CR⁹- für Verbindungen mit der Formel (**Ib**),
L² = -C≡C- für Verbindungen mit der Formel (**Ic**),
A, W, X, Y, L¹ L³, R², R³ und R⁹ wie für Formel (**I**) oben definiert.
(u) Umsetzung von Verbindungen der Formeln (**Ib**) oder (**Ic**) zu Verbindungen der Formel (**Ia**) in Gegenwart von Wasserstoff, eines Katalysators und gegebenenfalls eines Lösungsmittels gemäß dem nachfolgenden Reaktionsschema, (Schema 23): 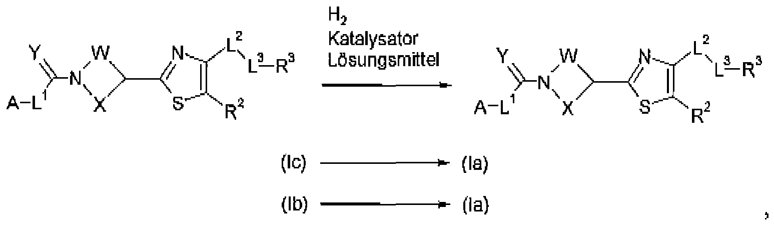 wobei
L² = -C(R⁸)₂-C(R⁸)₂- für Verbindungen mit der Formel (**Ia**),
L² = -CR⁹=CR⁹- für Verbindungen mit der Formel (**Ib**),
L² = -C≡C- für Verbindungen mit der Formel (**Ic**),
A, W, X, Y, L¹ L³, R², R³, R⁸ und R⁹ wie für Formel (**I**) oben definiert.

## Claims

1. Compounds of the formula **(I),** 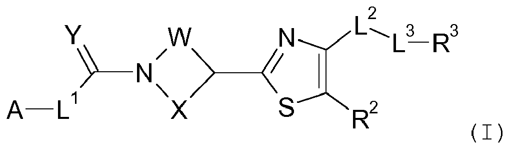 in which the symbols have the following meanings:
A represents unsubstituted or substituted phenyl,
where the substituents independently of one another are selected from the list below
cyano, nitro, halogen, C₁-C₆-alkyl, C₁-C₃-haloalkyl, C₃-C₆-cycloalkyl, C₃-C₆-halocycloalkyl, C₄-C₁₀-cycloalkylalkyl, C₄-C₁₀-alkylcycloalkyl, C₅-C₁₀-alkylcycloalkylalkyl, C₂-C₆-alkenyl, C₂-C₆-haloalkenyl, C₂-C₆-alkynyl, C₂-C₆-haloalkynyl, tri (C₁-C₂-alkyl)silyl, benzyl, phenyl, hydroxyl, C₁-C₄-alkoxy, C₁-C₄-haloalkoxy, OCH₂OCH₃, SH, C₁-C₄-alkylthio, C₁-C₆- haloalkyl thio, CHO, COOH, (C₁-C₄-alkoxy) carbonyl, CONR⁶R⁷, CR⁶=NOR⁷, (C₁-C₄-alkyl)carbonyl, (C₁-C₄-haloalkyl) carbonyl, (C₁-C₄-alkyl)carbonyloxy, (C₁-C₄-alkyl)carbonylthio, C₁-C₄-alkylsulphinyl, C₁-C₄-haloalkylsulphinyl, C₁-C₄-alkylsulphonyl, C₁-C₄-haloalkylsulphonyl, NR⁶R⁷, NR⁶COR⁷, SF₅, SO₂NR⁶R⁷, C₂-C₄-alkoxyalkyl or 1-methoxycyclopropyl
or
A is an optionally benzo-fused unsubstituted or substituted 5-or 6-membered heteroaryl, where the substituents independently of one another are selected from the list below
substituents at carbon:
cyano, nitro, halogen, C₁-C₆-alkyl, C₁-C₃-haloalkyl, C₃-C₆-cycloalkyl, C₃-C₆-halocycloalkyl C₄-C₁₀-cycloalkylalkyl, C₄-C₁₀-alkylCyCloalkyl, C₅-C₁₀-alkylcycloalkylalkyl, C₂-C₆-alkenyl, C₂-C₆-haloalkenyl, C₂-C₆-alkynyl, C₂-C₆-haloalkynyl, tri(C₁-C₂-alkyl)silyl, benzyl, phenyl, hydroxyl, C₁-C₄-alkoxy, C₁-C₄-haloalkoxy, OCH₂OCH₃, SH, C₁-C₄-alkylthio, C₁-C₆-haloalkylthio, CHO, COOH, (C₁-C₄-alkoxy)carbonyl, CONR⁶R⁷, CR⁶=NOR⁷, (C₁-C₄-alkyl)carbonyl, (C₁-C₄-haloalkyl)carbonyl, (C₁-C₄-alkyl)carbonyloxy, (C₁-C₄-alkyl) carbonylthio, C₁-C₄-alkylsulphinyl, C₁-C₄-haloalkylsulphinyl, C₁-C₄-alkylsulphonyl, C₁-C₄-haloalkylsulphonyl, NR⁶R⁷, NR⁶COR⁷, SF₅, SO₂NR⁶R⁷, C₂-C₄-alkoxyalkyl or 1-methoxycyclopropyl
substituents at nitrogen:
hydroxyl, NR⁶R⁷, C₁-C₆-alkyl, C₁-C₃-haloalkyl, C₃-C₆-cycloalkyl, C₃-C₆-halocycloalkyl, C₄-C₁₀-alkylcycloalkyl, C₂-C₆-alkenyl, C₂-C₆-haloalkenyl, C₂-C₆-alkynyl, or C₂-C₆-haloalkynyl
L¹ represents (C(R¹)₂)ₙ
where n = 0 to 3
R¹ are identical or different and independently of one another represent hydrogen, halogen, C₁-C₂-alkyl, C₁-C₂-haloalkyl or cyano,
with the provision that L¹ may contain at most two R¹ different from hydrogen
Y represents sulphur or oxygen,
W represents an unsubstituted or monosubstituted C₁- to C₃-carbon chain, where the substituent is selected from the group consisting of oxo, hydroxyl, cyano and C₁-C₄-alkyl
X represents an unsubstituted or monosubstituted C₁- to C₂-carbon chain, where the substituent is selected from the group consisting of oxo, hydroxyl, cyano and C₁-C₄-alkyl
R² represents hydrogen, C₁-C₂-alkyl, C₁-C₂-haloalkyl or halogen
L² represents -C(R⁸)₂-C(R⁸)₂- or -CR⁹=CR⁹ or -C=C-
L³ represents a direct bond
or
L³ represents a C₁- to C₄-carbon chain which may contain up to two substituents, where the substituents independently of one another are selected from the list below:
halogen, C₁-C₂-alkyl, C₁-C₂-alkoxy, C₁-C₂-haloalkyl or C₃-C₆-cycloalkyl
R³ represents unsubstituted or monosubstituted C₃-C₁₀-cycloalkyl, where the substituent is selected from the list below: cyano, halogen, C₁-C₆-alkyl, C₁-C₃-haloalkyl, C₃-C₆-cycloalkyl, C₂-C₆-alkenyl, C₂-C₆-haloalkenyl, C₂-C₆-alkynyl, C₂-C₆-haloalkynyl, tri(C₁-C₂-alkyl)silyl, phenyl, hydroxyl, oxo, C₁-C₆-alkoxy, C₁-C₆-haloalkoxy, C₂-C₆-alkenyloxy, C₂-C₆-alkynyloxy, C₁-C₆-alkylthio or C₁-C₆-haloalkylthio,
or
R³ represents unsubstituted or substituted phenyl,
where the substituents independently of one another are selected from the list below:
cyano, nitro, halogen, C₁-C₆-alkyl, C₁-C₃-haloalkyl, C₃-C₆-CyCloalkyl, C₃-C₆-halocycloalkyl, C₄-C₁₀-cycloalkylalkyl, C₄-C₁₀-halocycloalkylalkyl, C₄-C₁₀-alkylcycloalkyl, C₅-C₁₀-alkylcycloalkylalkyl, C₄-C₁₀-cycloalkoxyalkyl, C₂-C₆-alkenyl, C₂-C₆-haloalkenyl, C₃-C₈-cycloalkenyl, C₃-C₈-halocycloalkenyl, C₂-C₆-alkynyl, C₂-C₆-haloalkynyl, C₂-C₆-alkoxyalkyl, C₂-C₆-haloalkoxyalkyl, C₃-C₈-alkoxyalkoxyalkyl, tri (C₁-C₂-alkyl)silyl, benzyl, phenyl, hydroxyl, C₁-C₆-alkoxy, C₂-C₆-alkoxyalkoxy, C₁-C₆-haloalkoxy, C₂-C₆-alkenyloxy, C₂-C₆-haloalkenyloxy, C₂-C₆-alkynyloxy, C₂-C₆-haloalkynyloxy, C₃-C₆-cycloalkoxy, C₃-C₆-cycloalkyloxy, C₃-C₆-halocycloalkoxy, C₄-C₁₀-cycloalkylalkyloxy, NR⁶R⁷, SH, SF₅, C₁-C₆-alkylthio, C₁-C₆-haloalkylthio, C₂-C₆-alkylalkylthio, C₃-C₆-cycloalkylthio, CHO, COOH, (C₁-C₆-alkoxy) carbonyl, CONR⁶R⁷, CR⁶=NOR⁷, (C₁-C₆-alkyl) carbonyl, (C₁-C₆-haloalkyl)carbonyl, (C₁-C₆-alkyl)carbonyloxy, (C₁-C₆-haloalkyl)carbonyloxy, (C₁-C₆-alkyl)carbonylthio, C₁-C₆-alkylsulphinyl, C₁-C₆-haloalkylsulphinyl, C₁-C₆-alkylsulphonyl, C₁-C₆-haloalkylsulphonyl, NR⁶COR⁷ or SO₂NR⁶R⁷
or
R³ represents saturated or partially or fully unsaturated naphthyl or indenyl,
which may contain up to two substituents, where the substituents independently of one another are selected from the list below:
cyano, halogen, C₁-C₆-alkyl, C₁-C₃-haloalkyl, C₃-C₆-cycloalkyl, C₂-C₆-alkenyl, C₂-C₆-haloalkenyl, C₂-C₆-alkynyl, C₂-C₆-haloalkynyl, tri(C₁-C₂-alkyl)silyl, phenyl, hydroxyl, C₁-C₆-alkoxy, C₁-C₆-haloalkoxy, C₂-C₆-alkenyloxy, C₂-C₆-alkynyloxy, C₁-C₆-alkylthio or C₁-C₆-haloalkylthio,
or
R³ represents an unsubstituted or substituted 5- or 6-membered heteroaryl radical,
where the substituents independently of one another are selected from the list below:
substituents at carbon: cyano, nitro, halogen, C₁-C₆-alkyl, C₁-C₃-haloalkyl, C₃-C₆-cycloalkyl, C₃-C₆-halocycloalkyl C₄-C₁₀-cycloalkylalkyl, C₄-C₁₀-alkylcycloalkyl, C₅-C₁₀-alkylcycloalkylalkyl, C₂-C₆-alkenyl, C₂-C₆-haloalkenyl, C₂-C₆-alkynyl, C₂-C₆-haloalkynyl, tri(C₁-C₂-alkyl)silyl, benzyl, phenyl, hydroxyl, C₁-C₄-alkoxy, C₁-C₄-haloalkoxy, OCH₂OCH₃, SH, C₁-C₄-alkylthio, C₁-C₆-haloalkylthio, COOH, (C₁-C₄-alkoxy) carbonyl, CONR⁶R⁷, (C₁-C₄-alkyl) carbonyl, (C₁-C₄-haloalkyl)carbonyl, (C₁-C₄-alkyl)carbonyloxy, (C₁-C₄-alkyl)carbonylthio, C₁-C₄-alkylsulphinyl, C₁-C₄-haloalkylsulphinyl, C₁-C₄-alkylsulphonyl, C₁-C₄-haloalkylsulphonyl, NR⁶R⁷, NR⁶COR⁷, SF₅, SO₂NR⁶R⁷, C₂-C₄-alkoxyalkyl or 1-methoxycyclopropyl
substituents at nitrogen: hydroxyl, C₁-C₆-alkyl, C₁-C₃-haloalkyl, C₃-C₆-cycloalkyl, C₃-C₆-halocycloalkyl, C₄-C₁₀-alkylcycloalkyl, C₂-C₆-alkenyl, C₂-C₆-haloalkenyl, C₂-C₆-alkynyl, C₂-C₆-haloalkynyl or phenyl
or
R³ represents benzo-fused unsubstituted or substituted 5- or 6-membered heteroaryl which may contain up to two substituents, where the substituents independently of one another are selected from the list below:
substituents at carbon: cyano, nitro, halogen, C₁-C₆-alkyl, C₁-C₃-haloalkyl, C₃-C₆-cycloalkyl, C₃-C₆-halocycloalkyl C₄-C₁₀-cycloalkylalkyl, C₄-C₁₀-alkylcycloalkyl, C₅-C₁₀-alkylcycloalkylalkyl, C₂-C₆-alkenyl, C₂-C₆-haloalkenyl, C₂-C₆-alkynyl, C₂-C₆-haloalkynyl, tri(C₁-C₂-alkyl)silyl, benzyl, phenyl, hydroxyl, C₁-C₄-alkoxy, C₁-C₄-haloalkoxy, OCH₂OCH₃, SH, C₁-C₄-alkylthio, C₁-C₆-haloalkylthio, COOH, (C₁-C₄-alkoxy) carbonyl, CONR⁶R⁷, (C₁-C₄-alkyl)carbonyl, (C₁-C₄-haloalkyl)carbonyl, (C₁-C₄-alkyl)carbonyloxy, (C₁-C₄-alkyl)carbonylthio, C₁-C₄-alkylsulphinyl, C₁-C₄-haloalkylsulphinyl, C₁-C₄-alkylsulphonyl, C₁-C₄-haloalkylsulphonyl, NR⁶R⁷, NR⁶COR⁷, SF₅, SO₂NR⁶R⁷, C₂-C₄-alkoxyalkyl or 1-methoxycyclopropyl
substituents at nitrogen: C₁-C₆-alkyl, C₁-C₃-haloalkyl, C₃-C₆-cycloalkyl, C₃-C₆-halocycloalkyl, C₄-C₁₀-alkylcycloalkyl, C₂-C₆-alkenyl, C₂-C₆-haloalkenyl, C₂-C₆-alkynyl, C₂-C₆-haloalkynyl or phenyl
or
R³ is an unsubstituted or monosubstituted 5- to 15-membered heterocyclyl radical which is attached via a carbon atom and which may contain up to two further heteroatoms selected from the group consisting of nitrogen, oxygen and sulphur, where the substituent is selected from the list below:
substituents at carbon: cyano, halogen, C₁-C₆-alkyl, C₁-C₃-haloalkyl, C₃-C₆-cycloalkyl, C₂-C₆-alkenyl, C₂-C₆-haloalkenyl, C₂-C₆-alkynyl, C₂-C₆-haloalkynyl, tri(C₁-C₂-alkyl)silyl, phenyl, hydroxyl, C₁-C₆-alkoxy, C₁-C₆-haloalkoxy, C₂-C₆-alkenyloxy, C₂-C₆-alkynyloxy, C₁-C₆-alkylthio or C₁-C₆-haloalkylthio
substituents at nitrogen: C₁-C₆-alkyl, C₁-C₃-haloalkyl, C₃-C₆-cycloalkyl, C₂-C₆-alkenyl, C₂-C₆-haloalkenyl, C₂-C₆-alkynyl, C₂-C₆-haloalkynyl, tri(C₁-C₂-alkyl)silyl or phenyl
R⁶, R⁷ independently of one another represent hydrogen, C₁-C₄-alkyl, C₁-C₃-haloalkyl, C₃-C₆-cycloalkyl,
R⁸ are identical or different and independently of one another represent hydrogen, C₁-C₄-alkyl,
R⁹ are identical or different and independently of one another represent hydrogen, C₁-C₄-alkyl,
and also the agrochemically active salts thereof.

2. Compounds of the formula **(I)** according to Claim 1
in which the symbols have the following meanings:
A represents phenyl which may contain up to two substituents, where the substituents independently of one another are selected from the list below:
cyano, nitro, halogen, C₁-C₄-alkyl, C₁-C₃-haloalkyl, C₃-C₆-cycloalkyl, C₂-C₄-alkenyl, C₂-C₄-alkynyl, hydroxyl, C₁-C₄-alkoxy, C₁-C₄-haloalkoxy, C₁-C₄-alkylthio, C₁-C₄-haloalkylthio, (C₁-C₄-alkoxy)carbonyl, (C₁-C₄-alkyl)carbonyl, (C₁-C₄-haloalkyl)carbonyl, C₁-C₄-alkylsulphinyl, C₁-C₄-haloalkylsulphinyl, C₁-C₄-alkylsulphonyl or C₁-C₄-haloalkylsulphonyl,
or
A represents a heteroaromatic radical selected from the group below: furan-2-yl, furan-3-yl, thiophen-2-yl, thiophen-3-yl, isoxazol-3-yl, isoxazol-4-yl, isoxazol-5-yl, 1H-pyrrol-1-yl, 1H-pyrrol-2-yl, 1H-pyrrol-3-yl, oxazol-2-yl, oxazol-4-yl, oxazol-5-yl, thiazol-2-yl, thiazol-4-yl, thiazol-5-yl, isothiazol-3-yl, isothiazol-4-yl, isothiazol-5-yl, pyrazol-1-yl, pyrazol-3-yl, pyrazol-4-yl, imidazol-1-yl, imidazol-2-yl, imidazol-4-yl, 1,2,4-oxadiazol-3-yl, 1,2,4-oxadiazol-5-yl, 1,3,4-oxadiazol-2-yl, 1,2,4-thiadiazol-3-yl, 1,2,4-thiadiazol-5-yl, 1,3,4-thiadiazol-2-yl, 1,2,3-triazol-l-yl, 1,2,3-triazol-2-yl, 1.2.3-triazol-4-yl, 1,2,4-triazol-l-yl, 1.2.4-triazol-3-yl, pyridin-2-yl, pyridin-3-yl, pyridin-4-yl, pyridazin-3-yl, pyridazin-4-yl, pyrimidin-2-yl, pyrimidin-4-yl, pyrimidin-5-yl, pyrazin-2-yl, 1,3,5-triazin-2-yl, 1,2,4-triazin-3-yl, 1H-indol-1-yl, 1H-indol-2-yl, 1H-indol-3-yl, 1H-indol-4-yl, 1H-indol-5-yl, 1H-indol-6-yl, 1H-indol-7-yl, 1H- benzimidazol-1-yl, 1H-benzimidazol-2-yl, 1H-benzimidazol-4-yl, benzimidazol-5-yl, 1H-indazol-1-yl, 1H-indazol-3-yl, 1H-indazol-4-yl, 1H-indazol-5-yl, 1H-indazol-6-yl, 1H-indazol-7-yl, 2H-indazol-2-yl, 1-benzofuran-2-yl, 1-benzofuran-3-yl, 1-benzofuran-4-yl, 1-benzofuran-5-yl, 1-benzofuran-6-yl, 1-benzofuran-7-yl, 1-benzothiophen-2-yl, 1-benzothiophen-3-yl, 1-benzothiophen-4-yl, 1-benzothiophen-5-yl, 1-benzothiophen-6-yl, 1-benzothiophen-7-yl, 1,3-benzothiazol-2-yl, 1,3-benzoxazol-2-yl, quinolin-2-yl, quinolin-3-yl, quinolin-4-yl, quinolin-5-yl, quinolin-6-yl, quinolin-7-yl, quinolin-8-yl, isoquinolin-1-yl, isoquinolin-3-yl, isoquinolin-4-yl, isoquinolin-5-yl, isoquinolin-6-yl, isoquinolin-7-yl or isoquinolin-8-yl,
which may contain up to two substituents, where the substituents independently of one another are selected from the list below:
substituents at carbon: cyano, halogen, C₁-C₄-alkyl, C₁-C₃-haloalkyl, hydroxyl, C₁-C₄-alkoxy, C₁-C₄-haloalkoxy, C₁-C₄-alkylthio or C₁-C₄-haloalkylthio,
substituents at nitrogen: C₁-C₄-alkyl, C₁-C₃-haloalkyl, cyclopropyl, C₂-C₄-alkenyl or C₂-C₄-alkynyl,
L¹ represents (C(R¹)₂)ₙ
where n = 0 to 3
R¹ are identical or different and independently of one another represent hydrogen, chlorine, fluorine, methyl, CF₃ or cyano,
with the provision that L¹ may contain at most two R¹ different from hydrogen,
Y represents sulphur or oxygen,
W represents an unsubstituted or monosubstituted C₁- to C₂-carbon chain, where the substituent is selected from the group consisting of cyano and C₁-C₂-alkyl
X represents an unsubstituted or monosubstituted C₁- to C₂-carbon chain, where the substituent is selected from the group consisting of cyano and C₁-C₂-alkyl,
R² represents hydrogen, C₁-C₂-alkyl or halogen,
L² represents -C(R⁸)₂-C(R⁸)₂- or -CR⁹=CR⁹- or -C=C-,
L³ represents a direct bond,
or
L³ represents a C₁- to C₄-carbon chain which may contain up to two substituents, where the substituents independently of one another are selected from the list below:
C₁-C₂-alkyl, C₁-C₂-alkoxy, C₁-C₂-haloalkyl or cyclopropyl,
R³ represents unsubstituted or monosubstituted C₃-C₁₀-cycloalkyl,. where the substituent is selected from the list below:
halogen, C₁-C₆-alkyl, C₃-C₆-cycloalkyl, C₂-C₆-alkenyl, C₂-C₆-alkynyl, tri (C₁-C₂-alkyl) silyl, phenyl or oxo,
or
R³ represents phenyl which may contain up to three substituents, where the substituents independently of one another are selected from the list below:
cyano, nitro, halogen, C₁-C₆-alkyl, C₁-C₃-haloalkyl, C₃-C₆-cycloalkyl, C₃-C₆-halocycloalkyl, C₂-C₆-alkenyl, C₂-C₆-haloalkenyl, C₂-C₆-alkynyl, C₂-C₆-haloalkynyl, tri(C₁-C₂-alkyl)silyl, phenyl, hydroxyl, C₁-C₆-alkoxy, C₁-C₆-haloalkoxy, C₁-C₆-alkylthio or C₁-C₆-haloalkylthio,
or
R³ represents naphthalen-1-yl, naphthalen-2-yl, 1,2,3,4-tetrahydronaphthalen-1-yl, 1,2,3,4-tetrahydronaphthalen-2-yl, 5,6,7,8-tetrahydronaphthalen-1-yl, 5,6,7,8-tetrahydronaphthalen-2-yl, decalin-1-yl, decalin-2-yl, 1H-inden-1-yl, 1H-inden-2-yl, 1H-inden-3-yl, 1H-inden-4-yl, 1H-inden-5-yl, 1H-inden-6-yl, 1H-inden-7-yl, 2,3-dihydro-1H-inden-1-yl, 2,3-dihydro-1H-inden-2-yl, 2,3-dihydro-1H-inden-4-yl or 2,3-dihydro-1H-inden-5-yl,
which may contain up to two substituents, where the substituents independently of one another are selected from the list below:
cyano, halogen, C₁-C₆-alkyl, C₁-C₃-haloalkyl, C₃-C₆-cycloalkyl, phenyl, hydroxyl, C₁-C₆-alkoxy, C₁-C₃-haloalkoxy, C₁-C₆-alkylthio or C₁-C₃-haloalkylthio,
or
R³ represents furan-2-yl, furan-3-yl, thiophen-2-yl, thiophen-3-yl, isoxazol-3-yl, isoxazol-4-yl, isoxazol-5-yl, 1H-pyrrol-1-yl, 1H-pyrrol-2-yl, 1H-pyrrol-3-yl, oxazol-2-yl, oxazol-4-yl, oxazol-5-yl, thiazol-2-yl, thiazol-4-yl, thiazol-5-yl, isothiazol-3-yl, isothiazol-4-yl, isothiazol-5-yl, pyrazol-1-yl, pyrazol-3-yl, pyrazol-4-yl, imidazol-1-yl, imidazol-2-yl, imidazol-4-yl, 1,2,4-oxadiazol-3-yl, 1,2,4-oxadiazol-5-yl, 1,3,4-oxadiazol-2-yl, 1,2,4-thiadiazol-3-yl, 1,2,4-thiadiazol-5-yl, 1,3,4-thiadiazol-2-yl, 1,2,3-triazol-1-yl, 1,2,3-triazol-2-yl, 1,2,3-triazol-4-yl, 1,2,4-triazol-1-yl, 1,2,4-triazol-3-yl, pyridin-2-yl, pyridin-3-yl, pyridin-4-yl, pyridazin-3-yl, pyridazin-4-yl, pyrimidin-2-yl, pyrimidin-4-yl, pyrimidin-5-yl, pyrazin-2-yl, 1,3,5-triazin-2-yl, 1,2,4-triazin-3-yl,
which may contain up to two substituents, where the substituents independently of one another are selected from the list below:
substituents at carbon: cyano, halogen, C₁-C₆-alkyl, C₁-C₃-haloalkyl, C₃-C₆-cycloalkyl, phenyl, hydroxyl, C₁-C₄-alkoxy, C₁-C₄-haloalkoxy, C₁-C₄-alkylthio or C₁-C₄-haloalkylthio,
substituents at nitrogen: C₁-C₆-alkyl, C₁-C₃-haloalkyl, C₃-C₆-cycloalkyl, C₂-C₆-alkenyl, C₂-C₆-alkynyl or phenyl,
or
R³ represents 1H-indol-1-yl, 1H-indol-2-yl, 1H-indol-3-yl, 1H-indol-4-yl, 1H-indol-5-yl, 1H-indol-6-yl, 1H-indol-7-yl, 1H-benzimidazol-1-yl, 1H-benzimidazol-2-yl, 1H-benzimidazol-4-yl, benzimidazol-5-yl, 1H-indazol-1-yl, 1H-indazol-3-yl, 1H-indazol-4-yl, 1H-indazol-5-yl, 1H-indazol-6-yl, 1H-indazol-7-yl, 2H-indazol-2-yl, 1-benzofuran-2-yl, 1-benzofuran-3-yl, 1-benzofuran-4-yl, 1-benzofuran-5-yl, 1-benzofuran-6-yl, 1-benzofuran-7-yl, 1-benzothiophen-2-yl, 1-benzothiophen-3-yl, 1-benzothiophen-4-yl, 1-benzothiophen-5-yl, 1-benzothiophen-6-yl, 1-benzothiophen-7-yl, quinolin-2-yl, quinolin-3-yl, quinolin-4-yl, quinolin-5-yl, quinolin-6-yl, quinolin-7-yl, quinolin-8-yl, isoquinolin-1-yl, isoquinolin-3-yl, isoquinolin-4-yl, isoquinolin-5-yl, isoquinolin-6-yl, isoquinolin-7-yl or isoquinolin-8-yl,
which may contain up to two substituents, where the substituents independently of one another are selected from the list below:
substituents at carbon: cyano, halogen, C₁-C₆-alkyl, C₁-C₃-haloalkyl, C₃-C₆-cycloalkyl, phenyl, hydroxyl, C₁-C₄-alkoxy, C₁-C₄-haloalkoxy, C₁-C₄-alkylthio or C₁-C₄-haloalkylthio,
substituents at nitrogen: C₁-C₆-alkyl, C₁-C₃-haloalkyl, C₃-C₆-cycloalkyl, C₂-C₆-alkenyl, C₂-C₆-alkynyl or phenyl,
or
R³ represents an unsubstituted or monosubstituted 5- to 6-membered heterocyclyl radical which is attached via a carbon atom and which may contain up to two further heteroatoms selected from the group consisting of nitrogen, oxygen and sulphur, where the substituent is selected from the list below:
substituents at carbon: C₁-C₆-alkyl, C₁-C₃-haloalkyl, C₃-C₆-cycloalkyl, C₂-C₆-alkenyl, C₂-C₆-alkynyl, tri(C₁-C₂-alkyl)silyl or phenyl,
substituents at nitrogen: C₁-C₆-alkyl, C₃-C₆-cycloalkyl, C₂-C₆-alkenyl, C₂-C₆-alkynyl, tri(C₁-C₂-alkyl)silyl or phenyl,
R⁸ are identical or different and independently of one another represent hydrogen, C₁-C₂-alkyl,
R⁹ are identical or different and independently of one another represent hydrogen, C₁-C₂-alkyl,
and also the agrochemically active salts thereof.

3. Compounds of the formula **(I)** according to one or more of Claims 1 to 2
in which the symbols have the following meanings,
A represents phenyl which may contain up to two substituents, where the substituents independently of one another are selected from the list below:
cyano, nitro, halogen, C₁-C₂-alkyl, C₁-C₂-haloalkyl, hydroxyl, C₁-C₂-alkoxy or C₁-C₂-haloalkoxy,
or
A represents a heteroaromatic radical selected from the group below: furan-2-yl, furan-3-yl, thiophen-2-yl, thiophen-3-yl, oxazol-2-yl, oxazol-4-yl, oxazol-5-yl, thiazol-2-yl, thiazol-4-yl, thiazol-5-yl; pyrazol-1-yl, pyrazol-3-yl, pyrazol-4-yl, imidazol-1-yl, imidazol-2-yl, imidazol-4-yl, 1,2,4-triazol-1-yl, pyridin-2-yl, pyridin-3-yl, pyridin-4-yl, 1,2,4-triazin-3-yl, 1H-benzimidazol-2-yl or 1,3-benzothiazol-2-yl,
which may contain up to two substituents, where the substituents independently of one another are selected from the list below:
substituents at carbon: cyano, halogen, C₁-C₂-alkyl, C₁-C₂-haloalkyl, hydroxyl, C₁-C₂-alkoxy or C₁-C₂-haloalkoxy,
substituents at nitrogen: C₁-C₂-alkyl or C₁-C₂-haloalkyl,
L¹ represents (C(R¹)₂)ₙ
where n = 0 to 3,
R¹ are identical or different and independently of one another represent hydrogen or methyl,
with the provision that L¹ may contain at most two methyl substituents,
Y represents sulphur or oxygen,
W represents -CH₂CH₂-,
X represents -CH₂CH₂-,
R² represents hydrogen, methyl or halogen,
L² represents -C(R⁸)₂-C(R⁸)₂- or -CR⁹=CR⁹- or -C=C-,
L³ represents a direct bond,
or
L³ represents a C₁- to C₄-carbon chain which may contain up to two substituents, where the substituents independently of one another are selected from the list below:
methyl, methoxy or CF₃,
R³ represents unsubstituted or monosubstituted C₃-C₁₀-cycloalkyl, where the substituent is selected from the list below:
fluorine, chlorine, methyl, ethyl, cyclopropyl, cyclopentyl or cyclohexyl,
or
R³ represents phenyl which may contain up to three substituents, where the substituents independently of one another are selected from the list below:
cyano, halogen, C₁-C₆-alkyl, C₁-C₃-haloalkyl, phenyl, hydroxyl, C₁-C₆-alkoxy, C₁-C₆-haloalkoxy, C₁-C₆-alkylthio or C₁-C₆-haloalkylthio,
or
R³ represents naphthalen-1-yl, naphthalen-2-yl, 1,2,3,4-tetrahydronaphthalen-1-yl, 1,2,3,4-tetrahydronaphthalen-2-yl, 5,6,7,8-tetrahydronaphthalen-1-yl, 5,6,7,8-tetrahydronaphthalen-2-yl, decalin-1-yl, decalin-2-yl, 1H-inden-1-yl, 1H-inden-2-yl, 1H-inden-3-yl, 1H-inden-4-yl, 1H-inden-5-yl, 1H-inden-6-yl, 1H-inden-7-yl, 2,3-dihydro-1H-inden-1-yl, 2,3-dihydro-1H-inden-2-yl, 2,3-dihydro-1H-inden-4-yl or 2,3-dihydro-1H-inden-5-yl,
which may contain up to two substituents, where the substituents independently of one another are selected from the list below:
fluorine, chlorine, bromine, iodine, methyl, ethyl, CF₃, CHF₂, cyclopropyl, phenyl, hydroxyl, OMe, OEt, OCF₃, OCHF₂, OC₂F₅, SMe or SCF₃,
or
R³ represents furan-2-yl, furan-3-yl, thiophen-2-yl, thiophen-3-yl, isoxazol-3-yl, isoxazol-4-yl, isoxazol-5-yl, 1H-pyrrol-1-yl, 1H-pyrrol-2-yl, 1H-pyrrol-3-yl, oxazol-2-yl, oxazol-4-yl, oxazol-5-yl, thiazol-2-yl, thiazol-4-yl, thiazol-5-yl, isothiazol-3-yl, isothiazol-4-yl, isothiazol-5-yl, pyrazol-1-yl, pyrazol-3-yl, pyrazol-4-yl, imidazol-1-yl, imidazol-2-yl, imidazol-4-yl, 1,2,4-oxadiazol-3-yl, 1,2,4-oxadiazol-5-yl, 1,3,4-oxadiazol-2-yl, 1,2,4-thiadiazol-3-yl, 1,2,4-thiadiazol-5-yl, 1,3,4-thiadiazol-2-yl, 1,2,3-triazol-l-yl, 1,2,3-triazol-2-yl, 1,2,3-triazol-4-yl, 1,2,4-triazol-l-yl, 1,2,4-triazol-3-yl, pyridin-2-yl, pyridin-3-yl, pyridin-4-yl, pyridazin-3-yl, pyridazin-4-yl, pyrimidin-2-yl, pyrimidin-4-yl, pyrimidin-5-yl, pyrazin-2-yl, 1,3,5-triazin-2-yl or 1,2,4-triazin-3-yl,
which may contain up to two substituents, where the substituents independently of one another are selected from the list below:
substituents at carbon: cyano, fluorine, chlorine, bromine, iodine, methyl, ethyl, 1-methylethyl, 1, 1-dimethylethyl, CF₃, CHF₂, C₂F₅, CCl₃, cyclopropyl, phenyl, hydroxyl, OMe, OEt, O*iso*Pr, OCF₃, OCHF₂, OC₂F₅, SMe or SCF₃,
substituents at nitrogen: methyl, ethyl, 1-methylethyl, 1,1-dimethylethyl, cyclopropyl or phenyl,
or
R³ represents 1H-indol-1-yl, 1H-indol-2-yl, 1H-indol-3-yl, 1H-indol-4-yl, 1H-indol-5-yl, 1H-indol-6-yl, 1H-indol-7-yl, 1H-benzimidazol-1-yl, 1H-benzimidazol-2-yl, 1H-benzimidazol-4-yl, benzimidazol-5-yl, 1H-indazol-1-yl, 1H-indazol-3-yl, 1H-indazol-4-yl, 1H-indazol-5-yl, 1H-indazol-6-yl, 1H-indazol-7-yl, 2H-indazol-2-yl, 1-benzofuran-2-yl, 1-benzofuran-3-yl, 1-benzofuran-4-yl, 1-benzofuran-5-yl, 1-benzofuran-6-yl, 1-benzofuran-7-yl, 1-benzothiophen-2-yl, 1-benzothiophen-3-yl, 1-benzothiophen-4-yl, 1-benzothiophen-5-yl, 1-benzothiophen-6-yl, 1-benzothiophen-7-yl, quinolin-2-yl, quinolin-3-yl, quinolin-4-yl, quinolin-5-yl, quinolin-6-yl, quinolin-7-yl, quinolin-8-yl, isoquinolin-1-yl, isoquinolin-3-yl, isoquinolin-4-yl, isoquinolin-5-yl, isoquinolin-6-yl, isoquinolin-7-yl or isoquinolin-8-yl,
which may contain up to two substituents, where the substituents independently of one another are selected from the list below:
substituents at carbon: cyano, fluorine, chlorine, bromine, iodine, methyl, ethyl, 1-methylethyl, 1,1-dimethylethyl, CF₃, CHF₂, C₂F₅, CCl₃, cyclopropyl, phenyl, hydroxyl, OMe, OEt, O*iso*Pr, OCF₃. OCHF₂, OC₂F₅, SMe or SCF₃,
substituents at nitrogen: methyl, ethyl, 1-methylethyl, 1,1-dimethylethyl, cyclopropyl or phenyl,
or
R³ represents unsubstituted or monosubstituted pyrrolidin-2-yl, pyrrolidin-3-yl, morpholin-3-yl, morpholin-2-yl, piperidin-2-yl, piperidin-3-yl or piperazin-2-yl, where the substituent is selected from the list below:
substituents at carbon: methyl, ethyl, CF₃, cyclopropyl or phenyl,
substituents at nitrogen: methyl, ethyl, cyclopropyl or phenyl,
R⁸ are identical or different and independently of one another represent hydrogen, methyl, ethyl,
R⁹ are identical or different and independently of one another represent hydrogen, methyl or ethyl,
and also the agrochemically active salts thereof.

4. Compounds of the formula **(I)** according to one or more of Claims 1 to 3
in which the symbols have the following meanings,
A represents phenyl which may contain up to two substituents, where the substituents independently of one another are selected from the list below:
cyano, fluorine, chlorine, bromine, iodine, methyl, ethyl, CF₃, CHF₂, C₂F₅, CCl₃, hydroxyl, OMe, OCF₃, OCHF₂, OCH₂CF₃ or OC₂F₅,
or
A represents a heteroaromatic radical selected from the group below: thiophen-3-yl, pyrazol-1-yl, pyrazol-4-yl, pyridin-4-yl, 1H-benzimidazol-2-yl or 1,3-benzothiazol-2-yl,
which may contain up to two substituents, where the substituents independently of one another are selected from the list below:
substituents at carbon: cyano, fluorine, chlorine, bromine, iodine, methyl, ethyl, CF₃, CHF₂, C₂F₅, CCl₃, hydroxyl, OMe, OCF₃, OCHF₂, OCH₂CF₃ or OC₂F₅,
substituents at nitrogen: methyl, ethyl or CF₃,
L¹ represents (C(R¹)₂)ₙ
where n = 0 to 3,
R¹ are identical or different and independently of one another represent hydrogen or methyl,
with the provision that L¹ may contain at most one methyl substituent,
Y represents sulphur or oxygen,
W represents -CH₂CH₂-,
X represents -CH₂CH₂-,
R² represents hydrogen, methyl, chlorine or bromine,
L² represents -C(R⁸)₂-C(R⁸)₂- or -CR⁹₌CR⁹- or -C≡C-,
L³ represents a direct bond,
or
L³ represents a C₁- to C₄-carbon chain,
R³ represents cyclopropyl, cyclobutyl, cyclopentyl, cyclohexyl, cycloheptyl or cyclooctyl,
or
R³ represents phenyl which may contain up to two substituents, where the substituents independently of one another are selected from the list below:
cyano, fluorine, chlorine, bromine, iodine, methyl, ethyl, 1-methylethyl, 1,1-dimethylethyl, CF₃, CHF₂, C₂F₅, CCl₃, phenyl, hydroxyl, OMe, OEt, O*iso*Pr, OCF₃, OCHF₂, OC₂F₅, SMe or SCF₃,
or
R³ represents naphthalen-1-yl, naphthalen-2-yl, 1,2,3,4-tetrahydronaphthalen-1-yl, 1,2,3,4-tetrahydronaphthalen-2-yl, 5,6,7,8-tetrahydronaphthalen-1-yl, 5,6,7,8-tetrahydronaphthalen-2-yl, decalin-1-yl, decalin-2-yl, 1H-inden-1-yl, 1H-inden-2-yl, 1H-inden-3-yl, 1H-inden-4-yl, 1H-inden-5-yl, 1H-inden-6-yl, 1H-inden-7-yl, 2,3-dihydro-1H-inden-1-yl, 2,3-dihydro-1H-inden-2-yl, 2,3-dihydro-1H-inden-4-yl or 2,3-dihydro-1H-inden-5-yl,
or
R³ represents furan-2-yl, furan-3-yl, thiophen-2-yl, thiophen-3-yl, isoxazol-3-yl, isoxazol-4-yl, isoxazol-5-yl, 1H-pyrrol-1-yl, 1H-pyrrol-2-yl, 1H-pyrrol-3-yl, oxazol-2-yl, oxazol-4-yl, oxazol-5-yl, thiazol-2-yl, thiazol-4-yl, thiazol-5-yl, isothiazol-3-yl, isothiazol-4-yl, isothiazol-5-yl, pyrazol-1-yl, pyrazol-3-yl, pyrazol-4-yl, imidazol-1-yl, imidazol-2-yl, imidazol-4-yl, 1,2,4-oxadiazol-3-yl, 1,2,4-oxadiazol-5-yl, 1,3,4-oxadiazol-2-yl, 1,2,4-thiadiazol-3-yl, 1,2,4-thiadiazol-5-yl, 1,3,4-thiadiazol-2-yl, 1,2,3-triazol-1-yl, 1,2,3-triazol-2-yl, 1,2,3-triazol-4-yl, 1,2,4-triazol-1-yl, 1,2,4-triazol-3-yl, pyridin-2-yl, pyridin-3-yl, pyridin-4-yl, pyridazin-3-yl, pyridazin-4-yl, pyrimidin-2-yl, pyrimidin-4-yl, pyrimidin-5-yl, pyrazin-2-yl, 1,3,5-triazin-2-yl or 1,2,4-triazin-3-yl,
or
R³ represents 1H-indol-1-yl, 1H-indol-2-yl, 1H-indol-3-yl, 1H-indol-4-yl, 1H-indol-5-yl, 1H-indol-6-yl, 1H-indol-7-yl, 1H-benzimidazol-1-yl, 1H-benzimidazol-2-yl, 1H-benzimidazol-4-yl, benzimidazol-5-yl, 1H-indazol-1-yl, 1H-indazol-3-yl, 1H-indazol-4-yl, 1H-indazol-5-yl, 1H-indazol-6-yl, 1H-indazol-7-yl, 2H-indazol-2-yl, 1-benzofuran-2-yl, 1-benzofuran-3-yl, 1-benzofuran-4-yl, 1-benzofuran-5-yl, 1-benzofuran-6-yl, 1-benzofuran-7-yl, 1-benzothiophen-2-yl, 1-benzothiophen-3-yl, 1-benzothiophen-4-yl, 1-benzothiophen-5-yl, 1-benzothiophen-6-yl, 1-benzothiophen-7-yl, quinolin-2-yl, quinolin-3-yl, quinolin-4-yl, quinolin-5-yl, quinolin-6-yl, quinolin-7-yl, quinolin-8-yl, isoquinolin-1-yl, isoquinolin-3-yl, isoquinolin-4-yl, isoquinolin-5-yl, isoquinolin-6-yl, isoquinolin-7-yl or isoquinolin-8-yl,
or
R³ represents pyrrolidin-2-yl, pyrrolidin-3-yl, morpholin-3-yl, morpholin-2-yl, piperidin-2-yl, piperidin-3-yl or piperazin-2-yl,
R⁸ are identical or different and independently of one another represent hydrogen, methyl or ethyl,
R⁹ are identical or different and independently of one another represent hydrogen, methyl or ethyl,
and also the agrochemically active salts thereof.

5. Method for controlling phytopathogenic harmful fungi in and/or on plants or in and/or on plant seeds, **characterized in that** compounds of the formula (I) according to one or more of Claims 1 to 4 are applied to the phytopathogenic harmful fungi and/or their habitat.

6. Compositions for controlling phytopathogenic harmful fungi in and/or on plants or in and/or on plant seeds, **characterized in that** they comprise at least one according to one or more of Claims 1 to 4, in addition to extenders and/or surfactants.

7. Use of heterocyclyl-substituted thiazoles of the formula **(I)** according to one or more of Claims 1 to 4 for controlling phytopathogenic harmful fungi in and/or on plants or in and/or on plant seeds.

8. Process for preparing compositions for controlling phytopathogenic harmful fungi, **characterized in that** heterocyclyl-substituted thiazoles of the formula **(I)** according to one or more of Claims 1 to 4 are mixed with extenders and/or surfactants.

9. Process for preparing the compounds of the formula **(I),** comprising at least one of steps (a) to (n) below:
(a) the conversion of compounds of the formula (**VII**) in the presence of an organometallic compound R⁹-M and, if appropriate, in the presence of a solvent, into compounds of the formula (**VIII**), according to the reaction scheme below (Scheme 1) : 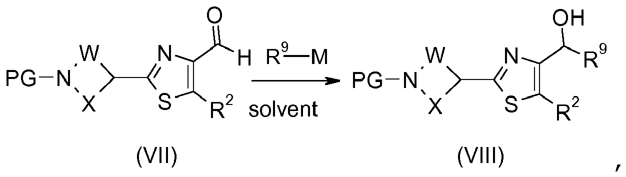 where
PG = unsubstituted or substituted phenylmethyl (where the substituents independently of one another are selected from the group consisting of methyl, methoxy, nitro, dioxolano), acetyl, C₁-C₄-alkoxycarbonyl and benzyloxycarbonyl,
M = for example MgCl, MgBr, MgI, Li,
R⁹ = C₁-C₄-alkyl or C₃-C₆-cycloalkyl,
W, X, and R² are as defined for formula (**I**) in Claim 1;
(b) the reaction of alcohols of the formula (**VIII**) with an oxidizing agent in the presence of a solvent, to give ketones of the formula **(IX**), according to the reaction scheme below (Scheme 2): 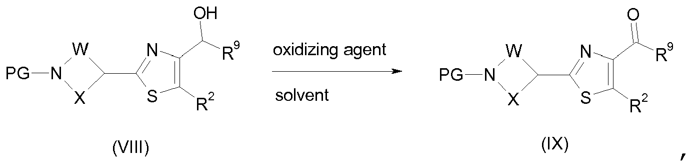 where
PG = unsubstituted or substituted phenylmethyl (where the substituents independently of one another are selected from the group consisting of methyl, methoxy, nitro, dioxolano), acetyl and C₁-C₄-alkoxycarbonyl,
R⁹ = C₁-C₄-alkyl or C₃-C₆-cycloalkyl,
W, X, and R² are as defined for formula (**I**) in Claim 1;
(c) the conversion of ketones of the formula (**IX**) in the presence of a base or an acid and, if appropriate, a solvent, into compounds of the formula (**IVb**), according to the reaction scheme below (Scheme 3): 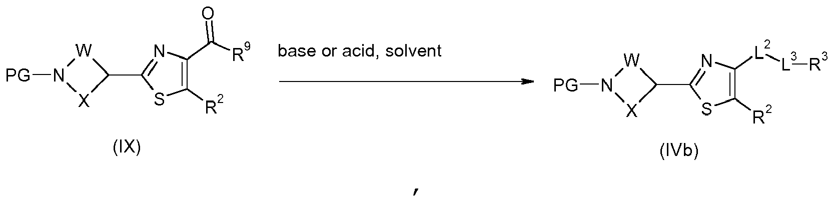 where
PG = unsubstituted or substituted phenylmethyl (where the substituents independently of one another are selected from the group consisting of methyl, methoxy, nitro, dioxolano), acetyl and C₁-C₄-alkoxycarbonyl,
L² = -CR⁹=CR⁹-,
W, X, L³, R², R³ and R⁹ are as defined for formula (**I**) in Claim 1;
(d) the conversion of aldehydes of the formula (**VII**) in the presence of a base or an acid and, if appropriate, a solvent, into compounds of the formula (**IVb**), according to the reaction scheme below (Scheme 4): 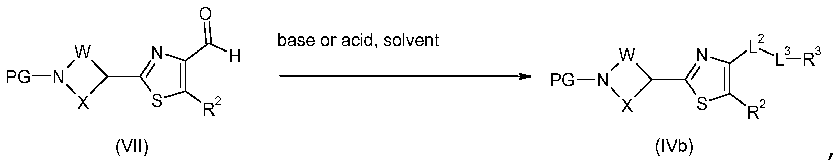 where
PG = unsubstituted or substituted phenylmethyl (where the substituents independently of one another are selected from the group consisting of methyl, methoxy, nitro, dioxolano), acetyl and C₁-C₄-alkoxycarbonyl,
L² = -CR⁹=CR⁹-,
W, X, L³, R², R³ and R⁹ are as defined for formula (**I**) in Claim 1;
(e) the conversion of aldehydes of the formula (**VII**) in the presence of a base and, if appropriate, a solvent, into alkynes of the formula (**X**) according to the reaction scheme below (Scheme 5): 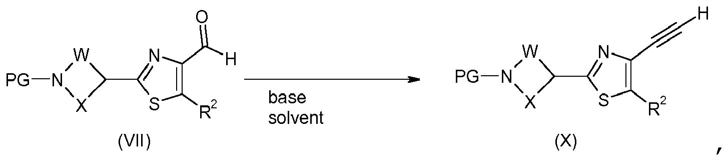 where
PG = unsubstituted or substituted phenylmethyl (where the substituents independently of one another are selected from the group consisting of methyl, methoxy, nitro, dioxolano), acetyl and C₁-C₄-alkoxycarbonyl,
W, X and R² are as defined for formula (**I**) in Claim 1;
(f) the reaction of alkynes of the formula (**X**) with an R³-L³-halide in an optionally catalyzed C-C coupling reaction in the presence of a base and, if appropriate, a solvent, to give compounds of the formula (**IVc**), according to the reaction scheme below (Scheme 6): 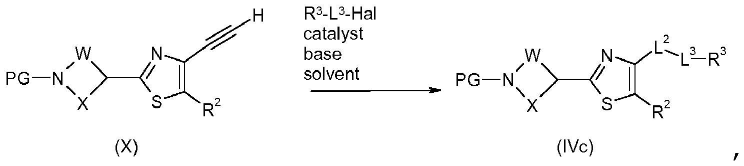 where
PG = unsubstituted or substituted phenylmethyl (where the substituents independently of one another are selected from the group consisting of methyl, methoxy, nitro, dioxolano), acetyl and C₁-C₄-alkoxycarbonyl,
Hal = Cl, Br or I,
L² = -C≡C-,
W, X, L³, R² and R³ are as defined for formula (**I**) in Claim 1;
(g) the reaction of alkynes of the formula (**X**) with a trialkylsilyl chloride (R¹⁰R¹¹R¹²) SiCl or trialkylsilyl triflate (R¹⁰R¹¹R¹²) SiOSO₂CF₃ or other known silylating agents in the presence of a base and, if appropriate, a solvent, to give compounds of the formula (**XI**), according to the reaction scheme below (Scheme 7): 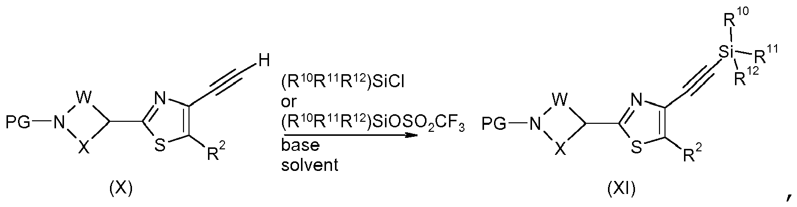 where
PG = unsubstituted or substituted phenylmethyl (where the substituents independently of one another are selected from the group consisting of methyl, methoxy, nitro, dioxolano), acetyl and C₁-C₄-alkoxycarbonyl,
R¹⁰, R¹¹ and R¹² = C₁-C₄ alkyl or phenyl,
W, X and R² are as defined for formula (**I**) in Claim 1;
(h) the reaction of alkynes of the formula **(XI)** with a compound R³-L³-Hal, if appropriate in the presence of a catalyst, in the presence of a base and, if appropriate, a solvent, to give compounds of the formula (**IVc**), according to the reaction scheme below (Scheme 8): 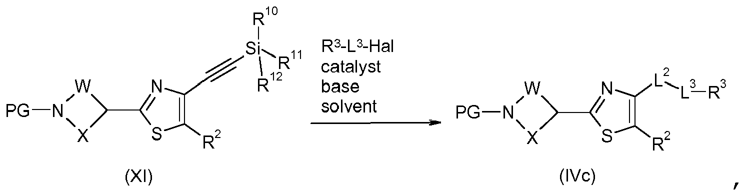 where
PG = unsubstituted or substituted phenylmethyl (where the substituents independently of one another are selected from the group consisting of methyl, methoxy, nitro, dioxolano), acetyl and C₁-C₄-alkoxycarbonyl,
Hal = Cl, Br or I,
R² = hydrogen, C₁-C₂-alkyl or C₁-C₂-haloalkyl,
L² = -C≡C-,
R¹⁰, R¹¹ and R¹² = C₁-C₄-alkyl or phenyl,
W, X, L³ and R³ are as defined for formula (**I**) in Claim 1;
(i) the reaction of thioamides of the formula (**V**) with compounds of the formula **(VIa)** or (**VIb**) in the presence of a base and, if appropriate, in the presence of a solvent, to give compounds of the formula **(IVa)** or (**IVb**), according to the reaction scheme below (Scheme 9): 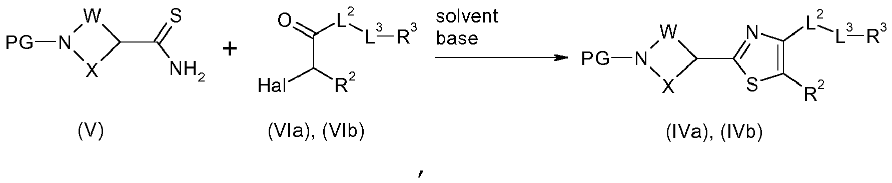 where
PG = unsubstituted or substituted phenylmethyl (where the substituents independently of one another are selected from the group consisting of methyl, methoxy, nitro, dioxolano), acetyl, C₁-C₄-alkoxycarbonyl and benzyloxycarbonyl,
Hal = Cl, Br or I,
L² = -C(R⁸)₂-C(R⁸)₂- for compounds of the formula (**IVa**) and (**VIa**),
L² = -CR⁹=CR⁹- for compounds of the formula (**IVb**) and (**VIb**),
W, X, L³, R², R³, R⁸ and R⁹ are as defined for formula (**I**) in Claim 1;
(j) the conversion of compounds of the formula (**IVb**) or (**IVc**) in the presence of hydrogen, a catalyst and, if appropriate, a solvent, into compounds of the formula (**IVa**), according to the reaction scheme below, (Scheme 10): 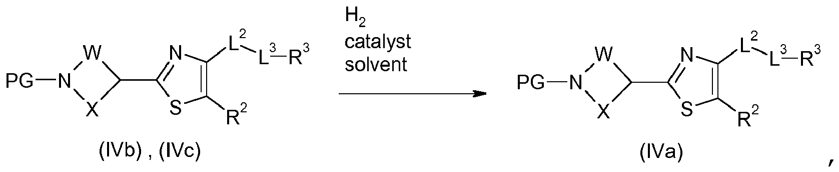 where
PG = acetyl or C₁-C₄-alkoxycarbonyl,
L² = -C(R⁸)₂-C(R⁸)₂- for compounds of the formula (**IVa**),
L² = -CR⁹=CR⁹- for compounds of the formula (**IVb**), L² = -C≡C- for compounds of the formula (**IVc**),
W, X, L³, R², R³, R⁸ and R⁹ are as defined for formula (**I**) in Claim 1;
(k) the conversion of compounds of the formula (**IV**), if appropriate in the presence of a solvent and, if appropriate, in the presence of an acid or, if appropriate, in the presence of a base or, if appropriate, in the presence of a source of hydrogen, into compounds of the formula (**II**), according to the reaction scheme below (Scheme 11): 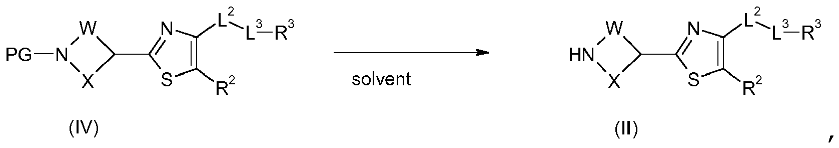 where
PG = acetyl, C₁-C₄-alkoxycarbonyl or benzyloxycarbonyl,
W, X, L², L³, R², and R³ are as defined for formula (**I**) in Claim 1;
(l) the conversion of compounds of the formula (**IIa'**) in the presence of a halogenating agent, if appropriate in the presence of an acid and a solvent, into compounds of the formula (**IIa**), according to the reaction scheme below (Scheme 12): 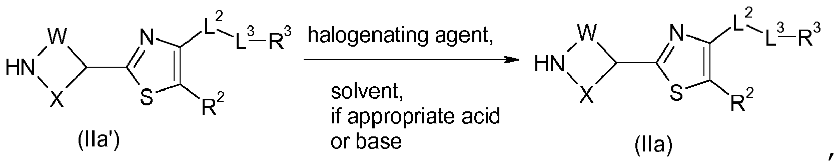 where
L² = -C(R⁸)₂-C(R⁸)₂-,
R² = I, Br or Cl for compounds of the formula (**IIa**),
R² = hydrogen for compounds of the formula (**IIa'**), W, X, L³, R³ and R⁸ are as defined for formula (**I**) in Claim 1;
(m) the reaction of compounds of the formula (**III**) with compounds of the formula (**II**), if appropriate in the presence of a coupling agent, a base and a solvent, to give compounds of the formula (**I**), according to the reaction scheme below (Scheme 13): 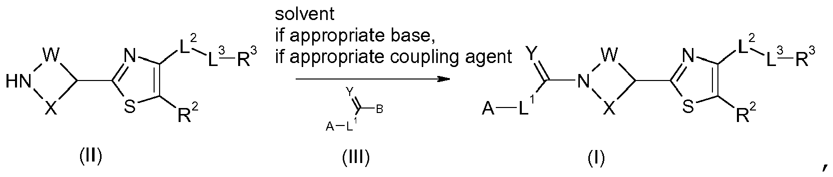 where
B = OH, chlorine, bromine or iodine,
Y = oxygen,
A, W, X, L¹, L², L³, R², and R³ are as defined for formula (**I**) in Claim 1;
(n) the conversion of compounds of the formula (**I**) in the presence of a sulphurizing agent and, if appropriate, in the presence of a solvent, into compounds of the formula (**I**), according to the reaction scheme below (Scheme 14): 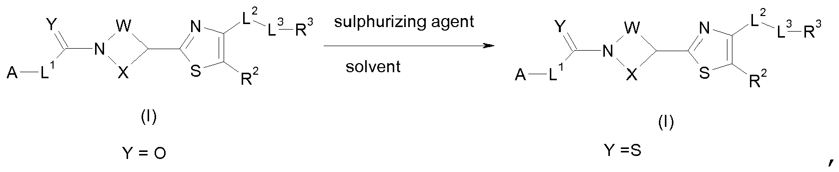 where
A, W, X, L¹, L², L³, R², and R³ are as defined for formula (**I**) in Claim 1.

10. Process for preparing the compounds of the formula (**I**), comprising at least one of steps (o) to (u) below:
(o) the conversion of aldehydes of the formula (**XII**) in the presence of a base and, if appropriate, a solvent, into alkynes of the formula (**XIII**), according to the reaction scheme below (Scheme 17): 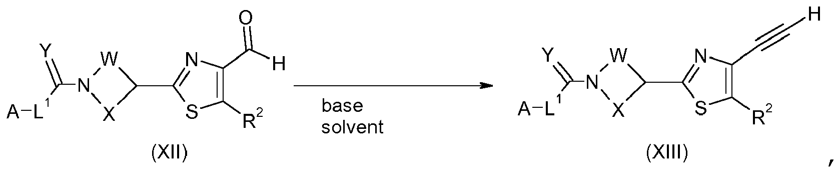 where
A, W, X, Y, L¹ and R² are as defined for formula (**I**) in Claim 1;
(p) the reaction of alkynes of the formula (**XIII**) with a trialkylsilyl chloride (R¹⁰R¹¹R¹²) SiCl or trialkylsilyl triflate (R¹⁰R¹¹R¹²) SiOSO₂CF₃ or other known silylating agents in the presence of a base and, if appropriate, a solvent, to give compounds of the formula (**XIV**), according to the reaction scheme below (Scheme 18): 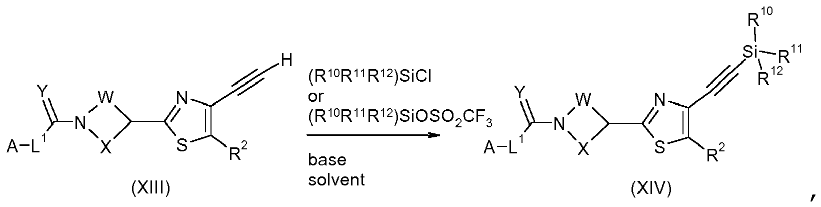 where
R¹⁰, R¹¹ and R¹² = C₁-C₄ alkyl or phenyl,
A, W, X, Y, L¹ and R² are as defined for formula (**I**) in Claim 1;
(q) the reaction of alkynes of the formula (**XIII**) with an R³-L³-halide in an optionally catalyzed C-C coupling reaction in the presence of a base and, if appropriate, a solvent, to give compounds of the formula (**Ic**), according to the reaction scheme below (Scheme 19): 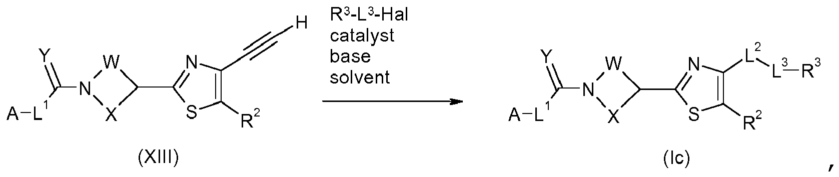 where
Hal = Cl, Br or I,
L² = -C=C-,
A, W, X, Y, L¹ L³, R² and R³ are as defined for formula (**I**) in Claim 1;
(r) the reaction of alkynes of the formula (**XIV**) with a compound R³-L³-Hal, if appropriate in the presence of a catalyst, in the presence of a base and, if appropriate, a solvent, to give compounds of the formula (**Ic**), according to the reaction scheme below (Scheme 20): 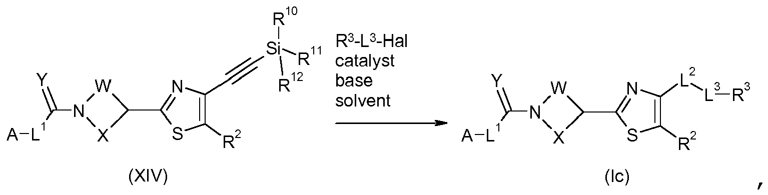 where
Hal = Cl, Br or I,
L² = -C=C-,
R¹⁰, R¹¹ and R¹² = C₁-C₄-alkyl or phenyl, A, W, X, Y, L¹ L³, R² and R³ are as defined for formula (**I**) in Claim 1;
(s) the conversion of aldehydes of the formula (**XII**) in the presence of a base or an acid and, if appropriate, a solvent, into compounds of the formula (**Ib**), according to the reaction scheme below (Scheme 21): 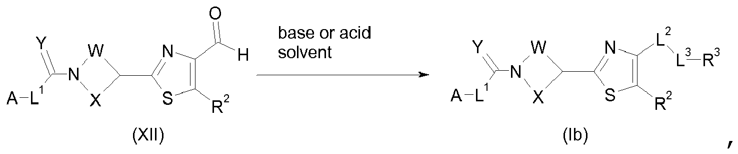 where
L² = -CR⁹=CR⁹-,
A, W, X, Y, L¹ L³, R², R³ and R⁹ are as defined for formula (**I**) in Claim 1;
(t) the conversion of compounds of the formula (**Ic**) in the presence of hydrogen, a catalyst and, if appropriate, a solvent, into compounds of the formula (**Ib**), according to the reaction scheme below (Scheme 22): 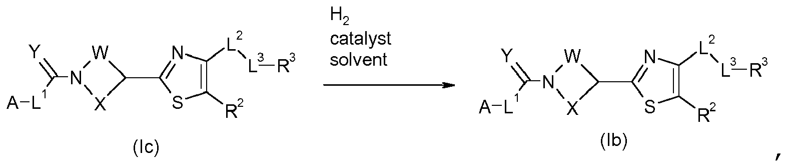 where
L² =-CR⁹=CR⁹- for compounds of the formula (**Ib**), L² = -C≡C- for compounds of the formula (**Ic**),
A, W, X, Y, L¹ L³, R², R³ and R⁹ are as defined for formula (**I**) in Claim 1;
(u) the conversion of compounds of the formula (**Ib**) or (**Ic**) in the presence of hydrogen, a catalyst and, if appropriate, a solvent, into compounds of the formula (**Ia**), according to the reaction scheme below (Scheme 23): 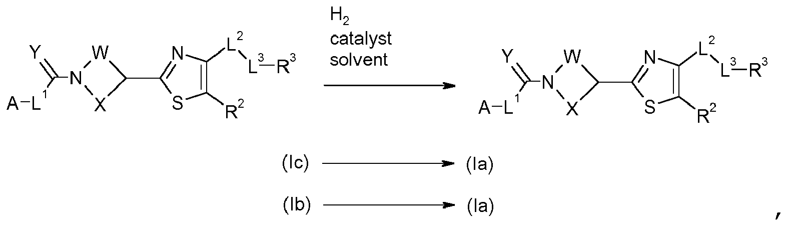 where
L² = -C(R⁸)₂-C(R⁸)₂- for compounds of the formula (**Ia**),
L² = -CR⁹=CR⁹ for compounds of the formula (**Ib**), L² = -C≡C- for compounds of the formula (**Ic**),
A, W, X, Y, L¹ L³, R², R³, R⁸ and R⁹ are as defined for formula (**I**) in Claim 1.

## Revendications

1. Composés de formule (**I**) 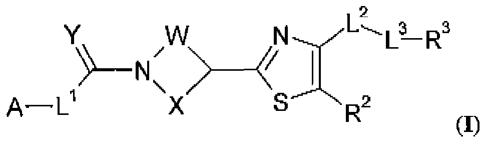 dans laquelle les symboles présentent les significations suivantes :
A représente phényle non substitué ou substitué,
les substituants étant choisis, indépendamment les uns des autres, dans la liste suivante :
cyano, nitro, halogène, C₁-C₆₋alkyle, C₁-C₃-halogénoalkyle, C₃-C₆-cycloalkyle, C₃-C₆-halogénocycloalkyle, C₄-C₁₀-cycloalkylalkyle, C₄-C₁₀-alkylcycloalkyle, C₅-C₁₀-alkylcycloalkylalkyle, C₂-C₆-alcényle, C₂-C₆-halogénoalcényle, C₂-C₆-alcynyle, C₂-C₆-halogénoalcynyle, tri(C₁-C₂-alkyl)silyle, benzyle, phényle, hydroxy, C₁-C₄-alcoxy, C₁-C₄-halogénoalcoxy, OCH₂OCH₃, SH, C₁-C₄-alkylthio, C₁-C₆-halogénoalkylthio, CHO, COOH, (C₁-C₄-alcoxy) carbonyle, CONR⁶R⁷, CR⁶=NOR⁷, (C₁-C₄-alkyl)carbonyle, (C₁-C₄-halogénoalkyl)carbonyle, (C₁-C₄-alkyl)carbonyloxy, (C₁-C₄-alkyl)carbonylthio, C₁-C₄-alkylsulfinyle, C₁-C₄-halogénoalkylsulfinyle, C₁-C₄-alkylsulfonyle, C₁-C₄-halogénoalkylsulfonyle, NR⁶R⁷, NR⁶COR⁷, SF₅, SO₂NR⁶R⁷, C₂-C₄-alcoxyalkyle ou 1-méthoxycyclopropyle ou
A représente hétéroaryle le cas échéant benzocondensé, non substitué ou substitué de 5 ou 6 chaînons, les substituants étant choisis, indépendamment les uns des autres, dans la liste suivante :
substituants sur le carbone :
cyano, nitro, halogène, C₁-C₆-alkyle, C₁-C₃-halogénoalkyle, C₃-C₆-cycloalkyle, C₃-C₆-halogénocycloalkyle, C₄-C₁₀-cycloalkylalkyle, C₄-C₁₀-alkylcycloalkyle, C₅-C₁₀-alkylcycloalkylalkyle, C₂-C₆-alcényle, C₂-C₆-halogénoalcényle, C₂-C₆-alcynyle, C₂-C₆-halogénoalcynyle, tri(C₁-C₂-alkyl)silyle, benzyle, phényle, hydroxy, C₁-C₄-alcoxy, C₁-C₄-halogénoalcoxy, OCH₂OCH₃, SH, C₁-C₄-alkylthio, C₁-C₆-halogénoalkylthio, CHO, COOH, (C₁-C₄-alcoxy) carbonyle, CONR⁶R⁷, CR⁶=NOR⁷, (C₁-C₄-alkyl)carbonyle, (C₁-C₄-halogénoalkyl)carbonyle, (C₁-C₄-alkyl)carbonyloxy, (C₁-C₄-alkyl)carbonylthio, C₁-C₄-alkylsulfinyle, C₁-C₄-halogénoalkylsulfinyle, C₁-C₄-alkylsulfonyle, C₁-C₄-halogénoalkylsulfonyle, NR⁶R⁷, NR⁶COR⁷, SF₅, SO₂NR⁶R⁷, C₂-C₄-alcoxyalkyle ou 1-méthoxycyclopropyle,
substituants sur l'azote :
hydroxy, NR⁶R⁷, C₁-C₆-alkyle, C₁-C₃-halogénoalkyle, C₃-C₆-cycloalkyle, C₃-C₆-halogénocycloalkyle, C₄-C₁₀-alkylcycloalkyle, C₂-C₆-alcényle, C₂-C₆-halogénoalcényle, C₂-C₆-alcynyle ou C₂-C₆-halogénoalcynyle,
L¹ représente (C(R¹)₂)ₙ
avec n = 0 à 3,
R¹ est identique ou différent et représente, indépendamment, hydrogène, halogène, C₁-C₂-alkyle, C₁-C₂-halogénoalkyle ou cyano,
à condition que L¹ puisse contenir au maximum deux R¹ qui sont différents d'hydrogène,
Y représente soufre ou oxygène,
W représente une chaîne carbonée en C₁-C₃, non substituée ou monosubstituée, le substituant étant choisi parmi oxo, hydroxy, cyano ou C₁-C₄-alkyle
X représente une chaîne carbonée en C₁-C₂, non substituée ou monosubstituée, le substituant étant choisi parmi oxo, hydroxy, cyano ou C₁-C₄-alkyle
R² représente hydrogène, C₁-C₂-alkyle, C₁-C₂-halogénoalkyle ou halogène,
L² représente -C(R⁸)₂-C(R⁸)₂- ou -CR⁹=CR⁹- ou -C=C-
L³ représente une liaison directe ou
L³ représente une chaîne carbonée en C₁-C₄, qui peut contenir jusqu'à deux substituants, les substituants étant choisis, indépendamment l'un de l'autre, dans la liste suivante :
halogène, C₁-C₂-alkyle, C₁-C₂-alcoxy, C₁-C₂-halogénoalkyle ou C₃-C₆-cycloalkyle
R³ représente C₃-C₁₀-cycloalkyle non substitué ou monosubstitué, le substituant étant choisi dans la liste suivante :
cyano, halogène, C₁-C₆-alkyle, C₁-C₃-halogénoalkyle, C₃-C₆-cycloalkyle, C₂-C₆-alcényle, C₂-C₆-halogénoalcényle, C₂-C₆-alcynyle, C₂-C₆-halogénoalcynyle, tri(C₁-C₂-alkyl)silyle, phényle, hydroxy, oxo, C₁-C₆-alcoxy, C₁-C₆-halogénoalcoxy, C₂-C₆-alcényloxy, C₂-C₆-alcynyloxy, C₁-C₆-alkylthio ou C₁-C₆-halogénoalkylthio ou
R³ représente phényle non substitué ou substitué,
les substituants étant choisis, indépendamment les uns des autres, dans la liste suivante :
cyano, nitro, halogène, C₁-C₆-alkyle, C₁-C₃-halogénoalkyle, C₃-C₆-cycloalkyle, C₃-C₆-halogénocycloalkyle, C₄-C₁₀-cycloalkylalkyle, C₄-C₁₀-halogénocycloalkylalkyle, C₄-C₁₀-alkylcycloalkyle, C₅-C₁₀-alkylcycloalkylalkyle, C₄-C₁₀-cycloalcoxyalkyle, C₂-C₆-alcényle, C₂-C₆-halogénoalcényle, C₃-C₈-cycloalcényle, C₃-C₈-halogénocycloalcényle, C₂-C₆-alcynyle, C₂-C₆-halogénoalcynyle, C₂-C₆-alcoxyalkyle, C₂-C₆-halogénoalcoxyalkyle, C₃-C₈-alcoxyalcoxyalkyle, tri(C₁-C₂-alkyl)silyle, benzyle, phényle, hydroxy, C₁-C₆-alcoxy, C₂-C₆-alcoxyalcoxy, C₁-C₆-halogénoalcoxy, C₂-C₆-alcényloxy, C₂-C₆-halogénoalcényloxy, C₂-C₆-alcynyloxy, C₂-C₆-halogénoalcynyloxy, C₃-C₆-cycloalcoxy, C₃-C₆-cycloalkyloxy, C₃-C₆-halogénocycloalcoxy, C₄-C₁₀-cycloalkylalkyloxy, NR⁶R⁷, SH, SF₅, C₁-C₆-alkylthio, C₁-C₆-halogénoalkylthio, C₂-C₆-alkylalkylthio, C₃-C₆-cycloalkylthio, CHO, COOH, (C₁-C₆-alcoxy)carbonyle, CONR⁶R⁷, CR⁶=NOR⁷, (C₁-C₆-alkyl) carbonyle, (C₁-C₆-halogénoalkyl)carbonyle, (C₁-C₆-alkyl)carbonyloxy, (C₁-C₆-halogénoalkyl)carbonyloxy, (C₁-C₆-alkyl)carbonylthio, C₁-C₆-alkylsulfinyle, C₁-C₆-halogénoalkylsulfinyle, C₁-C₆-alkylsulfonyle, C₁-C₆-halogénoalkylsulfonyle, NR⁶COR⁷ ou SO₂NR⁶R⁷ ou
R³ représente naphtyle ou indényle saturé ou partiellement ou totalement insaturé,
qui peut contenir jusqu'à deux substituants, les substituants étant choisis, indépendamment l'un de l'autre, dans la liste suivante :
cyano, halogène, C₁-C₆-alkyle, C₁-C₃-halogénoalkyle, C₃-C₆-cycloalkyle, C₂-C₆-alcényle, C₂-C₆-halogénoalcényle, C₂-C₆-alcynyle, C₂-C₆-halogénoalcynyle, tri(C₁-C₂-alkyl)silyle, phényle, hydroxy, C₁-C₆-alcoxy, C₁-C₆-halogénoalcoxy, C₂-C₆-alcényloxy, C₂-C₆-alcynyloxy, C₁-C₆-alkylthio ou C₁-C₆-halogénoalkylthio ou
R³ représente en outre un radical hétéroaryle non substitué ou substitué de 5 ou 6 chaînons,
les substituants étant choisis, indépendamment les uns des autres, dans la liste suivante :
substituants sur le carbone :
cyano, nitro, halogène, C₁-C₆-alkyle, C₁-C₃-halogénoalkyle, C₃-C₆-cycloalkyle, C₃-C₆-halogénocycloalkyle C₄-C₁₀-cycloalkylalkyle, C₄-C₁₀-alkylcycloalkyle, C₅-C₁₀-alkylcycloalkylalkyle, C₂-C₆-alcényle, C₂-C₆-halogénoalcényle, C₂-C₆-alcynyle, C₂-C₆-halogénoalcynyle, tri (C₁-C₂-alkyl)silyle, benzyle, phényle, hydroxy, C₁-C₄-alcoxy, C₁-C₄-halogénoalcoxy, OCH₂OCH₃, SH, C₁-C₄-alkylthio, C₁-C₆-halogénoalkylthio, COOH, (C₁-C₄-alcoxy) carbonyle, CONR⁶R⁷, (C₁-C₄-alkyl)carbonyle, (C₁-C₄-halogénoalkyl)carbonyle, (C₁-C₄-alkyl)carbonyloxy, (C₁-C₄-alkyl)carbonylthio, C₁-C₄-alkylsulfinyle, C₁-C₄-halogénoalkylsulfinyle, C₁-C₄-alkylsulfonyle, C₁-C₄-halogénoalkylsulfonyle, NR⁶R⁷, NR⁶COR⁷, SF₅, SO₂NR⁶R⁷, C₂-C₄-alcoxyalkyle ou 1-méthoxycyclopropyle
substituants sur l'azote :
hydroxy, C₁-C₆-alkyle, C₁-C₃-halogénoalkyle, C₃-C₆-cycloalkyle, C₃-C₆-halogénocycloalkyle, C₄-C₁₀-alkylcycloalkyle, C₂-C₆-alcényle, C₂-C₆-halogénoalcényle, C₂-C₆-alcynyle, C₂-C₆-halogénoalcynyle ou phényle ou
R³ représente hétéroaryle benzocondensé, non substitué ou substitué de 5 ou 6 chaînons, qui peut contenir jusqu'à deux substituants, les substituants étant choisis, indépendamment l'un de l'autre, dans la liste suivante :
substituants sur le carbone :
cyano, nitro, halogène, C₁-C₆-alkyle, C₁-C₃-halogénoalkyle, C₃-C₆-cycloalkyle, C₃-C₆-halogénocycloalkyle, C₄-C₁₀-cycloalkylalkyle, C₄-C₁₀-alkylcycloalkyle, C₅-C₁₀-alkylcycloalkylalkyle, C₂-C₆-alcényle, C₂-C₆-halogénoalcényle, C₂-C₆-alcynyle, C₂-C₆-halogénoalcynyle, tri(C₁-C₂-alkyl)silyle, benzyle, phényle, hydroxy, C₁-C₄-alcoxy, C₁-C₄-halogénoalcoxy, OCH₂OCH₃, SH, C₁-C₄-alkylthio, C₁-C₆-halogénoalkylthio, COOH, (C₁-C₄-alcoxy)carbonyle, CONR⁶R⁷, (C₁-C₄-alkyl)carbonyle, (C₁-C₄-halogénoalkyl)carbonyle, (C₁-C₄-alkyl)carbonyloxy, (C₁-C₄-alkyl)carbonylthio, C₁-C₄-alkylsulfinyle, C₁-C₄-halogénoalkylsulfinyle, C₁-C₄-alkylsulfonyle, C₁-C₄-halogénoalkylsulfonyle, NR⁶R⁷, NR⁶COR⁷, SF₅, SO₂NR⁶R⁷, C₂-C₄-alcoxyalkyle ou 1-méthoxycyclopropyle
substituants sur l'azote :
C₁-C₆-alkyle, C₁-C₃-halogénoalkyle, C₃-C₆-cycloalkyle, C₃-C₆-halogénocycloalkyle, C₄-C₁₀-alkylcycloalkyle, C₂-C₆-alcényle, C₂-C₆-halogénoalcényle, C₂-C₆-alcynyle, C₂-C₆-halogénoalcynyle ou phényle ou
R³ représente un radical hétérocyclyle lié via un atome de carbone, non substitué ou monosubstitué, de 5 à 15 chaînons, qui peut contenir jusqu'à deux autres hétéroatomes, choisis parmi azote, oxygène et soufre,
le substituant étant choisi dans la liste suivante :
substituants sur le carbone :
cyano, halogène, C₁-C₆-alkyle, C₁-C₃-halogénoalkyle, C₃-C₆-cycloalkyle, C₂-C₆-alcényle, C₂-C₆-halogénoalcényle, C₂-C₆-alcynyle, C₂-C₆-halogénoalcynyle, tri(C₁-C₂-alkyl)silyle, phényle, hydroxy, C₁-C₆-alcoxy, C₁-C₆-halogénoalcoxy, C₂-C₆-alcényloxy, C₂-C₆-alcynyloxy, C₁-C₆-alkylthio ou C₁-C₆-halogénoalkylthio
substituants sur l'azote :
C₁-C₆-alkyle, C₁-C₃-halogénoalkyle, C₃-C₆-cycloalkyle, C₂-C₆-alcényle, C₂-C₆-halogénoalcényle, C₂-C₆-alcynyle, C₂-C₆-halogénoalcynyle, tri(C₁-C₂-alkyl)silyle ou phényle
R⁶, R⁷ représentent, indépendamment l'un de l'autre, hydrogène, C₁-C₄-alkyle, C₁-C₃-halogénoalkyle, C₃-C₆-cycloalkyle
R⁸ est identique ou différent et représente, indépendamment, hydrogène, C₁-C₄-alkyle,
R⁹ est identique ou différent et représente, indépendamment, hydrogène, C₁-C₄-alkyle,
ainsi que les sels actifs sur le plan agrochimique correspondants.

2. Composés de formule (I) selon la revendication 1, dans laquelle les symboles ont les significations suivantes :
A représente phényle, qui peut contenir jusqu'à deux substituants, les substituants étant choisis, indépendamment l'un de l'autre, dans la liste suivante :
cyano, nitro, halogène, C₁-C₄-alkyle, C₁-C₃-halogénoalkyle, C₃-C₆-cycloalkyle, C₂-C₄-alcényle, C₂-C₄-alcynyle, hydroxy, C₁-C₄-alcoxy, C₁-C₄-halogénoalcoxy, C₁-C₄-alkylthio, C₁-C₄-halogénoalkylthio, (C₁-C₄-alcoxy)carbonyle, (C₁-C₄-alkyl)carbonyle, (C₁-C₄-halogénoalkyl)carbonyle, C₁-C₄-alkylsulfinyle, C₁-C₄-halogénoalkylsulfinyle, C₁-C₄-alkylsulfonyle ou C₁-C₄-halogénoalkylsulfonyle ou
A représente un radical hétéroaromatique choisi dans le groupe suivant : furann-2-yle, furann-3-yle, thiophén-2-yle, thiophén-3-yle, isoxazol-3-yle, isoxazol-4-yle, isoxazol-5-yle, 1H-pyrrol-1-yle, 1H-pyrrol-2-yle, 1H-pyrrol-3-yle, oxazol-2-yle, oxazol-4-yle, oxazol-5-yle, thiazol-2-yle, thiazol-4-yle, thiazol-5-yle, isothiazol-3-yle, isothiazol-4-yle, isothiazol-5-yle, pyrazol-1-yle, pyrazol-3-yle, pyrazol-4-yle, imidazol-1-yle, imidazol-2-yle, imidazol-4-yle, 1,2,4-oxadiazol-3-yle, 1,2,4-oxadiazol-5-yle, 1,3,4-oxadiazol-2-yle, 1,2,4-thiadiazol-3-yle, 1,2,4-thiadiazol-5-yle, 1,3,4-thiadiazol-2-yle, 1,2,3-triazol-1-yle, 1,2,3-triazol-2-yle, 1,2,3-triazol-4-yle, 1,2,4-triazol-1-yle, 1,2,4-triazol-3-yle, pyridin-2-yle, pyridin-3-yle, pyridin-4-yle, pyridazin-3-yle, pyridazin-4-yle, pyrimidin-2-yle, pyrimidin-4-yle, pyrimidin-5-yle, pyrazin-2-yle, 1,3,5-triazin-2-yle, 1,2,4-triazin-3-yle, 1H-indol-1-yle, 1H-indol-2-yle, 1H-indol-3-yle, 1H-indol-4-yle, 1H-indol-5-yle, 1H-indol-6-yle, 1H-indol-7-yle, 1H-benzimidazol-1-yle, 1H-benzimidazol-2-yle, 1H-benzimidazol-4-yle, benzimidazol-5-yle, 1H-indazol-1-yle, 1H-indazol-3-yle, 1H-indazol-4-yle, 1H-indazol-5-yle, 1H-indazol-6-yle, 1H-indazol-7-yle, 2H-indazol-2-yle, 1-benzofurann-2-yle, 1-benzofurann-3-yle, 1-benzofurann-4-yle, 1-benzofurann-5-yle, 1-benzofurann-6-yle, 1-benzofurann-7-yle, 1-benzothiophén-2-yle, 1-benzothiophén-3-yle, 1-benzothiophén-4-yle, 1-benzothiophén-5-yle, 1-benzothiophén-6-yle, 1-benzothiophén-7-yle, 1,3-benzothiazol-2-yle, 1,3-benzoxazol-2-yle, quinoléin-2-yle, quinoléin-3-yle, quinoléin-4-yle, quinoléin-5-yle, quinoléin-6-yle, quinoléin-7-yle, quinoléin-8-yle, isoquinoléin-1-yle, isoquinoléin-3-yle, isoquinoléin-4-yle, isoquinoléin-5-yle, isoquinoléin-6-yle, isoquinoléin-7-yle ou isoquinoléin-8-yle,
qui peut contenir jusqu'à deux substituants, les substituants étant choisis, indépendamment l'un de l'autre, dans la liste suivante :
substituants sur le carbone :
cyano, halogène, C₁-C₄-alkyle, C₁-C₃-halogénoalkyle, hydroxy, C₁-C₄-alcoxy, C₁-C₄-halogénoalcoxy, C₁-C₄-alkylthio ou C₁-C₄-halogénoalkylthio,
substituants sur l'azote :
C₁-C₄-alkyle, C₁-C₃-halogénoalkyle, cyclopropyle, C₂-C₄-alcényle ou C₂-C₄-alcynyle,
L¹ représente (C(R¹)₂)ₙ
avec n = 0 à 3,
R¹ est identique ou différent et représente, indépendamment, hydrogène, chlore, fluor, méthyle, CF₃ ou cyano,
à condition que L¹ puisse contenir au maximum deux R¹ qui sont différents d'hydrogène,
Y représente soufre ou oxygène,
W représente une chaîne carbonée en C₁-C₂, non substituée ou monosubstituée, le substituant étant choisi parmi cyano ou C₁-C₂-alkyle
X représente une chaîne carbonée en C₁-C₂, non substituée ou monosubstituée, le substituant étant choisi parmi cyano ou C₁-C₂-alkyle
R² représente hydrogène, C₁-C₂-alkyle ou halogène,
L² représente -C(R⁸)₂-C(R⁸)₂- ou -CR⁹=CR⁹- ou -C=C-
L³ représente une liaison directe ou
L³ représente une chaîne carbonée en C₁-C₄, qui peut contenir jusqu'à deux substituants, les substituants étant choisis, indépendamment l'un de l'autre, dans la liste suivante :
C₁-C₂-alkyle, C₁-C₂-alcoxy, C₁-C₂-halogénoalkyle ou cyclopropyle,
R³ représente C₃-C₁₀-cycloalkyle non substitué ou monosubstitué, le substituant étant choisi dans la liste suivante :
halogène, C₁-C₆-alkyle, C₃-C₆-cycloalkyle, C₂-C₆-alcényle, C₂-C₆-alcynyle, tri(C₁-C₂-alkyl)silyle, phényle ou oxo ou
R³ représente phényle, qui peut contenir jusqu'à trois substituants, les substituants étant choisis, indépendamment les uns des autres, dans la liste suivante :
cyano, nitro, halogène, C₁-C₆-alkyle, C₁-C₃-halogénoalkyle, C₃-C₆-cycloalkyle, C₃-C₆-halogénocycloalkyle, C₂-C₆-alcényle, C₂-C₆-halogénoalcényle, C₂-C₆-alcynyle, C₂-C₆-halogénoalcynyle, tri(C₁-C₂-alkyl)silyle, phényle, hydroxy, C₁-C₆-alcoxy, C₁-C₆-halogénoalcoxy, C₁-C₆-alkylthio ou C₁-C₆-halogénoalkylthio ou
R³ représente naphtalén-1-yle, naphtalén-2-yle, 1,2,3,4-tétrahydronaphtalén-1-yle, 1,2,3,4-tétrahydronaphtalén-2-yle, 5,6,7,8-tétrahydronaphtalén-1-yle, 5,6,7,8-tétrahydronaphtalén-2-yle, décalin-1-yle, décalin-2-yle, 1H-indén-1-yle, 1H-indén-2-yle, 1H-indén-3-yle, 1H-indén-4-yle, 1H-indén-5-yle, 1H-indén-6-yle, 1H-indén-7-yle, 2,3-dihydro-1H-indén-1-yle, 2,3-dihydro-1H-indén-2-yle, 2,3-dihydro-1H-indén-4-yle ou 2,3-dihydro-1H-indén-5-yle,
qui peut contenir jusqu'à deux substituants, les substituants étant choisis, indépendamment l'un de l'autre, dans la liste suivante :
cyano, halogène, C₁-C₆-alkyle, C₁-C₃-halogénoalkyle, C₃-C₆-cycloalkyle, phényle, hydroxy, C₁-C₆-alcoxy, C₁-C₃-halogénoalcoxy, C₁-C₆-alkylthio ou C₁-C₃-halogénoalkylthio ou
R³ représente furann-2-yle, furann-3-yle, thiophén-2-yle, thiophén-3-yle, isoxazol-3-yle, isoxazol-4-yle, isoxazol-5-yle, 1H-pyrrol-1-yle, 1H-pyrrol-2-yle, 1H-pyrrol-3-yle, oxazol-2-yle, oxazol-4-yle, oxazol-5-yle, thiazol-2-yle, thiazol-4-yle, thiazol-5-yle, isothiazol-3-yle, isothiazol-4-yle, isothiazol-5-yle, pyrazol-1-yle, pyrazol-3-yle, pyrazol-4-yle, imidazol-1-yle, imidazol-2-yle, imidazol-4-yle, 1,2,4-oxadiazol-3-yle, 1,2,4-oxadiazol-5-yle, 1,3,4-oxadiazol-2-yle, 1,2,4-thiadiazol-3-yle, 1,2,4-thiadiazol-5-yle, 1,3,4-thiadiazol-2-yle, 1,2,3-triazol-1-yle, 1,2,3-triazol-2-yle, 1,2,3-triazol-4-yle, 1,2,4-triazol-1-yle, 1,2,4-triazol-3-yle, pyridin-2-yle, pyridin-3-yle, pyridin-4-yle, pyridazin-3-yle, pyridazin-4-yle, pyrimidin-2-yle, pyrimidin-4-yle, pyrimidin-5-yle, pyrazin-2-yle, 1,3,5-triazin-2-yle, 1,2,4-triazin-3-yle,
qui peut contenir jusqu'à deux substituants, les substituants étant choisis, indépendamment l'un de l'autre, dans la liste suivante :
substituants sur le carbone :
cyano, halogène, C₁-C₆-alkyle, C₁-C₃-halogénoalkyle, C₃-C₆-cycloalkyle, phényle, hydroxy, C₁-C₄-alcoxy, C₁-C₄-halogénoalcoxy, C₁-C₄-alkylthio ou C₁-C₄-halogénoalkylthio,
substituants sur l'azote :
C₁-C₆-alkyle, C₁-C₃-halogénoalkyle, C₃-C₆-cycloalkyle, C₂-C₆-alcényle, C₂-C₆-alcynyle ou phényle ou
R³ représente 1H-indol-1-yle, 1H-indol-2-yle, 1H-indol-3-yle, 1H-indol-4-yle, 1H-indol-5-yle, 1H-indol-6-yle, 1H-indol-7-yle, 1H-benzimidazol-1-yle, 1H-benzimidazol-2-yle, 1H-benzimidazol-4-yle, benzimidazol-5-yle, 1H-indazol-1-yle, 1H-indazol-3-yle, 1H-indazol-4-yle, 1H-indazol-5-yle, 1H-indazol-6-yle, 1H-indazol-7-yle, 2H-indazol-2-yle, 1-benzofurann-2-yle, 1-benzofurann-3-yle, 1-benzofurann-4-yle, 1-benzofurann-5-yle, 1-benzofurann-6-yle, 1-benzofurann-7-yle, 1-benzothiophén-2-yle, 1-benzothiophén-3-yle, 1-benzothiophén-4-yle, 1-benzothiophén-5-yle, 1-benzothiophén-6-yle, 1-benzothiophén-7-yle, quinoléin-2-yle, quinoléin-3-yle, quinoléin-4-yle, quinoléin-5-yle, quinoléin-6-yle, quinoléin-7-yle, quinoléin-8-yle, isoquinoléin-1-yle, isoquinoléin-3-yle, isoquinoléin-4-yle, isoquinoléin-5-yle, isoquinoléin-6-yle, isoquinoléin-7-yle ou isoquinoléin-8-yle,
qui peut contenir jusqu'à deux substituants, les substituants étant choisis, indépendamment l'un de l'autre, dans la liste suivante :
substituants sur le carbone :
cyano, halogène, C₁-C₆-alkyle, C₁-C₃-halogénoalkyle, C₃-C₆-cycloalkyle, phényle, hydroxy, C₁-C₄-alcoxy, C₁-C₄-halogénoalcoxy, C₁-C₄-alkylthio ou C₁-C₄-halogénoalkylthio,
substituants sur l'azote :
C₁-C₆-alkyle, C₁-C₃-halogénoalkyle, C₃-C₆-cycloalkyle, C₂-C₆-alcényle, C₂-C₆-alcynyle ou phényle ou
R³ représente un radical hétérocyclyle lié via un atome de carbone, non substitué ou monosubstitué, de 5 à 6 chaînons, qui peut contenir jusqu'à deux autres hétéroatomes, choisis parmi azote, oxygène et soufre, le substituant étant choisi dans la liste suivante :
substituants sur le carbone :
C₁-C₆-alkyle, C₁-C₃-halogénoalkyle, C₃-C₆-cycloalkyle, C₂-C₆-alcényle, C₂-C₆-alcynyle, tri(C₁-C₂-alkyl)silyle ou phényle,
substituants sur l'azote :
C₁-C₆-alkyle, C₃-C₆-cycloalkyle, C₂-C₆-alcényle, C₂-C₆-alcynyle, tri(C₁-C₂-alkyl)silyle ou phényle,
R⁸ est identique ou différent et représente, indépendamment, hydrogène, C₁-C₂-alkyle,
R⁹ est identique ou différent et représente, indépendamment, hydrogène, C₁-C₂-alkyle,
ainsi que les sels actifs sur le plan agrochimique correspondants.

3. Composés de formule (I) selon l'une ou plusieurs des revendications 1 à 2, dans laquelle les symboles ont les significations suivantes :
A représente phényle, qui peut contenir jusqu'à deux substituants, les substituants étant choisis, indépendamment l'un de l'autre, dans la liste suivante :
cyano, nitro, halogène, C₁-C₂-alkyle, C₁-C₂-halogénoalkyle, hydroxy, C₁-C₂-alcoxy ou C₁-C₂-halogénoalcoxy ou
A représente un radical hétéroaromatique, choisi dans le groupe suivant : furann-2-yle, furann-3-yle, thiophén-2-yle, thiophén-3-yle, oxazol-2-yle, oxazol-4-yle, oxazol-5-yle, thiazol-2-yle, thiazol-4-yle, thiazol-5-yle, pyrazol-1-yle, pyrazol-3-yle, pyrazol-4-yle, imidazol-1-yle, imidazol-2-yle, imidazol-4-yle, 1,2,4-triazol-1-yle, pyridin-2-yle, pyridin-3-yle, pyridin-4-yle, 1,2,4-triazin-3-yle, 1H-benzimidazol-2-yle ou 1,3-benzothiazol-2-yle,
qui peut contenir jusqu'à deux substituants, les substituants étant choisis, indépendamment l'un de l'autre, dans la liste suivant
substituants sur le carbone :
cyano, halogène, C₁-C₂-alkyle, C₁-C₂-halogénoalkyle, hydroxy, C₁-C₂-alcoxy ou C₁-C₂-halogénoalcoxy ou
substituants sur l'azote :
C₁-C₂-alkyle ou C₁-C₂-halogénoalkyle,
L¹ représente (C(R¹)₂)ₙ
avec n = 0 à 3,
R¹ est identique ou différent et représente, indépendamment, hydrogène ou méthyle,
à condition que L¹ puisse contenir au maximum deux substituants méthyle,
Y représente soufre ou oxygène,
W représente -CH₂CH₂-,
X représente -CH₂CH₂-,
R² représente hydrogène, méthyle ou halogène
L² représente -C(R⁸)₂-C(R⁸)₂- ou -CR⁹=CR⁹- ou -C=C-
L³ représente une liaison directe ou
L³ représente une chaîne carbonée en C₁-C₄, qui peut contenir jusqu'à deux substituants, les substituants étant choisis, indépendamment l'un de l'autre, dans la liste suivante :
méthyle, méthoxy ou CF₃,
R³ représente C₃-C₁₀-cycloalkyle non substitué ou monosubstitué, le substituant étant choisi dans la liste suivante :
fluor, chlore, méthyle, éthyle, cyclopropyle, cyclopentyle ou cyclohexyle ou
R³ représente phényle, qui peut contenir jusqu'à trois substituants, les substituants étant choisis, indépendamment les uns des autres, dans la liste suivante :
cyano, halogène, C₁-C₆-alkyle, C₁-C₃-halogénoalkyle, phényle, hydroxy, C₁-C₆-alcoxy, C₁-C₆-halogénoalcoxy, C₁-C₆-alkylthio ou C₁-C₆-halogénoalkylthio ou
R³ représente naphtalén-1-yle, naphtalén-2-yle, 1,2,3,4-tétrahydronaphtalén-1-yle, 1,2,3,4-tétrahydronaphtalén-2-yle, 5,6,7,8-tétrahydronaphtalén-1-yle, 5,6,7,8-tétrahydronaphtalén-2-yle, décalin-1-yle, décalin-2-yle, 1H-indén-1-yle, 1H-indén-2-yle, 1H-indén-3-yle, 1H-indén-4-yle, 1H-indén-5-yle, 1H-indén-6-yle, 1H-indén-7-yle, 2,3-dihydro-1H-indén-1-yle, 2,3-dihydro-1H-indén-2-yle, 2,3-dihydro-1H-indén-4-yle ou 2,3-dihydro-1H-indén-5-yle,
qui peut contenir jusqu'à deux substituants, les substituants étant choisis, indépendamment l'un de l'autre, dans la liste suivante :
fluor, chlore, brome, iode, méthyle, éthyle, CF₃, CHF₂, cyclopropyle, phényle, hydroxy, OMe, OEt, OCF₃, OCHF₂, OC₂F₅, SMe ou SCF₃ ou
R³ représente furann-2-yle, furann-3-yle, thiophén-2-yle, thiophén-3-yle, isoxazol-3-yle, isoxazol-4-yle, isoxazol-5-yle, 1H-pyrrol-1-yle, 1H-pyrrol-2-yle, 1H-pyrrol-3-yle, oxazol-2-yle, oxazol-4-yle, oxazol-5-yle, thiazol-2-yle, thiazol-4-yle, thiazol-5-yle, isothiazol-3-yle, isothiazol-4-yle, isothiazol-5-yle, pyrazol-1-yle, pyrazol-3-yle, pyrazol-4-yle, imidazol-1-yle, imidazol-2-yle, imidazol-4-yle, 1,2,4-oxadiazol-3-yle, 1,2,4-oxadiazol-5-yle, 1,3,4-oxadiazol-2-yle, 1,2,4-thiadiazol-3-yle, 1,2,4-thiadiazol-5-yle, 1,3,4-thiadiazol-2-yle, 1,2,3-triazol-1-yle, 1,2,3-triazol-2-yle, 1,2,3-triazol-4-yle, 1,2,4-triazol-1-yle, 1,2,4-triazol-3-yle, pyridin-2-yle, pyridin-3-yle, pyridin-4-yle, pyridazin-3-yle, pyridazin-4-yle, pyrimidin-2-yle, pyrimidin-4-yle, pyrimidin-5-yle, pyrazin-2-yle, 1,3,5-triazin-2-yle ou 1,2,4-triazin-3-yle,
qui peut contenir jusqu'à deux substituants, les substituants étant choisis, indépendamment l'un de l'autre, dans la liste suivante :
substituants sur le carboné :
cyano, fluor, chlore, brome, iode, méthyle, éthyle, 1-méthyléthyle, 1,1-diméthyléthyle, CF₃, CHF₂, C₂F₅, CCl₃, cyclopropyle, phényle, hydroxy, OMe, OEt, OisoPr, OCF₃, OCHF₂, OC₂F₅, SMe ou SCF₃,
substituants sur l'azote :
méthyle, éthyle, 1-méthyléthyle, 1,1-diméthyléthyle, cyclopropyle ou phényle ou
R³ représente 1H-indol-1-yle, 1H-indol-2-yle, 1H-indol-3-yle, 1H-indol-4-yle, 1H-indol-5-yle, 1H-indol-6-yle, 1H-indol-7-yle, 1H-benzimidazol-1-yle, 1H-benzimidazol-2-yle, 1H-benzimidazol-4-yle, benzimidazol-5-yle, 1H-indazol-1-yle, 1H-indazol-3-yle, 1H-indazol-4-yle, 1H-indazol-5-yle, 1H-indazol-6-yle, 1H-indazol-7-yle, 2H-indazol-2-yle, 1-benzofurann-2-yle, 1-benzofurann-3-yle, 1-benzofurann-4-yle, 1-benzofurann-5-yle, 1-benzofurann-6-yle, 1-benzofurann-7-yle, 1-benzothiophén-2-yle, 1-benzothiophén-3-yle, 1-benzothiophén-4-yle, 1-benzothiophén-5-yle, 1-benzothiophén-6-yle, 1-benzothiophén-7-yle, quinoléin-2-yle, quinoléin-3-yle, quinoléin-4-yle, quinoléin-5-yle, quinoléin-6-yle, quinoléin-7-yle, quinoléin-8-yle, isoquinoléin-1-yle, isoquinoléin-3-yle, isoquinoléin-4-yle, isoquinoléin-5-yle, isoquinoléin-6-yle, isoquinoléin-7-yle ou isoquinoléin-8-yle,
qui peut contenir jusqu'à deux substituants, les substituants étant choisis, indépendamment l'un de l'autre, dans la liste suivant
substituants sur le carboné :
cyano, fluor, chlore, brome, iode, méthyle, éthyle, 1-méthyléthyle, 1,1-diméthyléthyle, CF₃, CHF₂, C₂F₅, CCl₃, cyclopropyle, phényle, hydroxy, OMe, OEt, OisoPr, OCF₃, OCHF₂, OC₂F₅, SMe ou SCF₃,
substituants sur l'azote :
méthyle, éthyle, 1-méthyléthyle, 1,1-diméthyléthyle, cyclopropyle ou phényle ou
R³ représente pyrrolidin-2-yle, pyrrolidin-3-yle, morpholin-3-yle, morpholin-2-yle, pipéridin-2-yle, pipéridin-3-yle ou pipérazin-2-yle, non substitué ou monosubstitué, le substituant étant choisi dans la liste suivante ;
substituants sur le carboné :
méthyle, éthyle, CF₃, cyclopropyle ou phényle,
substituants sur l'azote :
méthyle, éthyle, cyclopropyle ou phényle,
R⁸ est identique ou différent et représente, indépendamment, hydrogène, méthyle, éthyle R⁹ est identique ou différent et représente, indépendamment, hydrogène, méthyle ou éthyle ainsi que les sels actifs sur le plan agrochimique correspondants.

4. Composés de formule (I) selon l'une ou plusieurs des revendications 1 à 3, dans laquelle les symboles ont les significations suivantes :
A représente phényle, qui peut contenir jusqu'à deux substituants, les substituants étant choisis, indépendamment l'un de l'autre, dans la liste suivante :
cyano, fluor, chlore, brome, iode, méthyle, éthyle, CF₃, CHF₂, C₂F₅, CCl₃, hydroxy, OMe, OCF₃, OCHF₂, OCH₂CF₃ ou OC₂F₅ ou
A représente un radical hétéroaromatique choisi dans le groupe suivant : thiophén-3-yle, pyrazol-1-yle, pyrazol-4-yle, pyridin-4-yle, 1H-benzimidazol-2-yle ou 1,3-benzothiazol-2-yle,
qui peut contenir jusqu'à deux substituants, les substituants étant choisis, indépendamment l'un de l'autre, dans la liste suivante :
substituants sur le carboné :
cyano, fluor, chlore, brome, iode, méthyle, éthyle, CF₃, CHF₂, C₂F₅, CCl₃ hydroxy, OMe, OCF₃, OCHF₂, OCH₂CF₃ ou OC₂F₅,
substituants sur l'azote :
méthyle, éthyle ou CF₃,
L¹ représente (C(R¹)₂)ₙ
avec n = 0 à 3,
R¹ est identique ou différent et représente, indépendamment, hydrogène ou méthyle,
à condition que L¹ puisse contenir au maximum un substituant méthyle,
Y représente soufre ou oxygène,
W représente -CH₂CH₂-,
X représente -CH₂CH₂-,
R² représente hydrogène, méthyle, chlore ou brome,
L² représente -C(R⁸)₂-C(R⁵)₂- ou -CR⁹=CR⁹- ou -C=C-,
L³ représente une liaison directe ou
L³ représente une chaîne carbonée en C₁-C₄,
R³ représente cyclopropyle, cyclobutyle, cyclopentyle, cyclohexyle, cycloheptyle ou cyclooctyle ou
R³ représente phényle, qui peut contenir jusqu'à deux substituants, les substituants étant choisis, indépendamment l'un de l'autre, dans la liste suivante :
cyano, fluor, chlore, brome, iode, méthyle, éthyle, 1-méthyléthyle, 1,1-diméthyléthyle, CF₃, CHF₂, C₂F₅, CCl₃, phényle, hydroxy, OMe, OEt, OisoPr, OCF₃, OCHF₂, OC₂F₅, SMe ou SCF₃ ou
R³ représente naphtalén-1-yle, naphtalén-2-yle, 1,2,3,4-tétrahydronaphtalén-1-yle, 1,2,3,4-tétrahydronaphtalén-2-yle, 5,6,7,8-tétrahydronaphtalén-1-yle, 5,6,7,8-tétrahydronaphtalén-2-yle, décalin-1-yle, décalin-2-yle, 1H-indén-1-yle, 1H-indén-2-yle, 1H-indén-3-yle, 1H-indén-4-yle, 1H-indén-5-yle, 1H-indén-6-yle, 1H-indén-7-yle, 2,3-dihydro-1H-indén-1-yle, 2,3-dihydro-1H-indén-2-yle, 2,3-dihydro-1H-indén-4-yle ou 2,3-dihydro-1H-indén-5-yle ou
R³ représente furann-2-yle, furann-3-yle, thiophén-2-yle, thiophén-3-yle, isoxazol-3-yle, isoxazol-4-yle, isoxazol-5-yle, 1H-pyrrol-1-yle, 1H-pyrrol-2-yle, 1H-pyrrol-3-yle, oxazol-2-yle, oxazol-4-yle, oxazol-5-yle, thiazol-2-yle, thiazol-4-yle, thiazol-5-yle, isothiazol-3-yle, isothiazol-4-yle, isothiazol-5-yle, pyrazol-1-yle, pyrazol-3-yle, pyrazol-4-yle, imidazol-1-yle, imidazol-2-yle, imidazol-4-yle, 1,2,4-oxadiazol-3-yle, 1,2,4-oxadiazol-5-yle, 1,3,4-oxadiazol-2-yle, 1,2,4-thiadiazol-3-yle, 1,2,4-thiadiazol-5-yle, 1,3,4-thiadiazol-2-yle, 1,2,3-triazol-1-yle, 1,2,3-triazol-2-yle, 1,2,3-triazol-4-yle, 1,2,4-triazol-1-yle, 1,2,4-triazol-3-yle, pyridin-2-yle, pyridin-3-yle, pyridin-4-yle, pyridazin-3-yle, pyridazin-4-yle, pyrimidin-2-yle, pyrimidin-4-yle, pyrimidin-5-yle, pyrazin-2-yle, 1,3,5-triazin-2-yle ou 1,2,4-triazin-3-yle ou
R³ représente 1H-indol-1-yle, 1H-indol-2-yle, 1H-indol-3-yle, 1H-indol-4-yle, 1H-indol-5-yle, 1H-indol-6-yle, 1H-indol-7-yle, 1H-benzimidazol-1-yle, 1H-benzimidazol-2-yle, 1H-benzimidazol-4-yle, benzimidazol-5-yle, 1H-indazol-1-yle, 1H-indazol-3-yle, 1H-indazol-4-yle, 1H-indazol-5-yle, 1H-indazol-6-yle, 1H-indazol-7-yle, 2H-indazol-2-yle, 1-benzofurann-2-yle, 1-benzofurann-3-yle, 1-benzofurann-4-yle, 1-benzofurann-5-yle, 1-benzofurann-6-yle, 1-benzofurann-7-yle, 1-benzothiophén-2-yle, 1-benzothiophén-3-yle, 1-benzothiophén-4-yle, 1-benzothiophén-5-yle, 1-benzothiophén-6-yle, 1-benzothiophén-7-yle, quinoléin-2-yle, quinoléin-3-yle, quinoléin-4-yle, quinoléin-5-yle, quinoléin-6-yle, quinoléin-7-yle, quinoléin-8-yle, isoquinoléin-1-yle, isoquinoléin-3-yle, isoquinoléin-4-yle, isoquinoléin-5-yle, isoquinoléin-6-yle, isoquinoléin-7-yle ou isoquinoléin-8-yle ou
R³ représente pyrrolidin-2-yle, pyrrolidin-3-yle, morpholin-3-yle, morpholin-2-yle, pipéridin-2-yle, pipéridin-3-yle ou pipérazin-2-yle,
R⁸ est identique ou différent et représente, indépendamment, hydrogène, méthyle ou éthyle
R⁹ est identique ou différent et représente, indépendamment, hydrogène, méthyle ou éthyle
ainsi que les sels actifs sur le plan agrochimique correspondants.

5. Procédé pour lutter contre des champignons nuisibles phytopathogènes dans et/ou sur des plantes ou dans et/ou sur des semences de plantes, **caractérisé en ce qu'**on épand des composés de formule (I) selon l'une ou plusieurs des revendications 1 à 4 sur des champignons nuisibles phytopathogènes et/ou leur espace de vie.

6. Agent pour lutter contre des champignons nuisibles phytopathogènes dans et/ou sur des plantes ou dans et/ou sur des semences de plantes, **caractérisé par** une teneur en au moins un selon l'une ou plusieurs des revendications 1 à 4 outre des agents d'allongement et/ou des substances tensioactives.

7. Utilisation de thiazoles substitués par hétérocyclyle de formule (I) selon l'une ou plusieurs des revendications 1 à 4 pour lutter contre des champignons nuisibles phytopathogènes dans et/ou sur des plantes ou dans et/ou sur des semences de plantes.

8. Procédé pour préparer des agents pour lutter contre des champignons nuisibles phytopathogènes, **caractérisé en ce qu'**on mélange des thiazoles substitués par hétérocyclyle de formule (I) selon l'une ou plusieurs des revendications 1 à 4 avec des agents d'allongement et/ou des substances tensioactives.

9. Procédé pour la préparation des composés de formule (I), comprenant au moins une des étapes (a) à (n) suivantes :
(a) transformation de composés de formule (VII) en composés de formule (VIII) en présence d'un composé métallo-organique R⁹-M et le cas échéant en présence d'un solvant, selon le schéma de réaction suivant (schéma 1) : 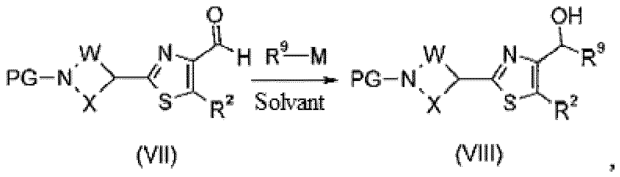 où
PG = représente phénylméthyle non substitué ou substitué (les substituants étant choisis, indépendamment les uns des autres, parmi méthyle, méthoxy, nitro, dioxolano), acétyle, C₁-C₄-alcoxycarbonyle ou benzyloxycarbonyle,
M = par exemple MgCl, MgBr, MgI, Li,
R⁹ = C₁-C₄-alkyle ou C₃-C₆-cycloalkyle,
W, X et R² sont tels que définis pour la formule (I) dans la revendication 1,
(b) transformation d'alcools de formule (VIII) avec un oxydant en présence d'un solvant en cétones de formule (IX) selon le schéma de réaction suivant (schéma 2) : 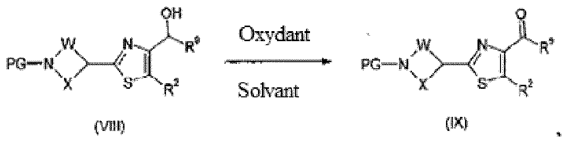 où
PG = représente phénylméthyle non substitué ou substitué (les substituants étant choisis, indépendamment les uns des autres, parmi méthyle, méthoxy, nitro, dioxolano), acétyle ou C₁-C₄-alcoxycarbonyle,
R⁹ = C₁-C₄-alkyle ou C₃-C₆-cycloalkyle,
W, X et R² sont tels que définis pour la formule (I) dans la revendication 1,
(c) transformation de cétones de formule (IX) en composés de formule (IVb) en présence d'une base ou d'un acide et le cas échéant d'un solvant, selon le schéma de réaction suivant (schéma 3) : 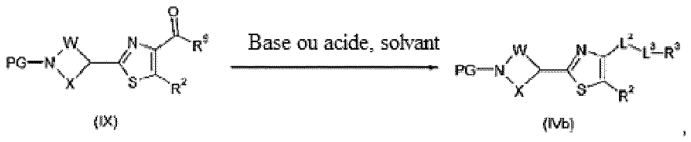 où
PG = représente phénylméthyle non substitué ou substitué (les substituants étant choisis, indépendamment les uns des autres, parmi méthyle, méthoxy, nitro, dioxolano), acétyle ou C₁-C₄-alcoxycarbonyle,
L² = -CR⁹=CR⁹-,
W, X, L³, R², R³ et R⁹ sont tels que définis pour la formule (I) dans la revendication 1,
(d) transformation d'aldéhydes de formule (VII) en composés de formule (IVb) en présence d'une base ou d'un acide et le cas échéant d'un solvant, selon le schéma de réaction suivant (schéma 4) : 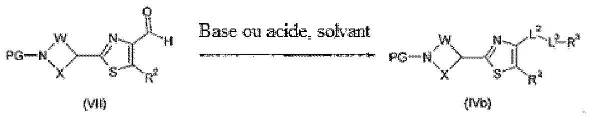 où
PG = représente phénylméthyle non substitué ou substitué (les substituants étant choisis, indépendamment les uns des autres, parmi méthyle, méthoxy, nitro, dioxolano), acétyle ou C₁-C₄-alcoxycarbonyle,
L² = -CR⁹=CR⁹-,
W, X, L³, R², R³ et R⁹ sont tels que définis pour la formule (I) dans la revendication 1,
(e) transformation d'aldéhydes de formule (VII) en alcynes de formule (X) en présence d'une base et le cas échéant d'un solvant, selon le schéma de réaction suivant (schéma 5) : 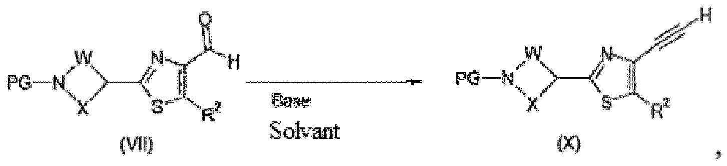 où
PG = représente phénylméthyle non substitué ou substitué (les substituants étant choisis, indépendamment les uns des autres, parmi méthyle, méthoxy, nitro, dioxolano), acétyle ou C₁-C₄-alcoxycarbonyle,
W, X et R² sont tels que définis pour la formule (I) dans la revendication 1,
(f) transformation d'alcynes de formule (X) avec un halogénure de R³-L³ dans une réaction de couplage C-C le cas échéant catalysée en présence d'une base et le cas échéant d'un solvant en composés de formule (IVc) selon le schéma de réaction suivant (schéma 6) : 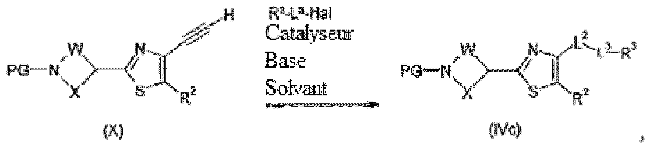 où
PG = représente phénylméthyle non substitué ou substitué (les substituants étant choisis, indépendamment les uns des autres, parmi méthyle, méthoxy, nitro, dioxolano), acétyle ou C₁-C₄-alcoxycarbonyle,
Hal = Cl, Br ou I,
L² = -C=C-
W, X, L³, R² et R³ sont tels que définis pour la formule (I) dans la revendication 1,
(g) transformation d'alcynes de formule (X) avec un chlorure de trialkylsilyle (R¹⁰R¹¹R¹²) SiCl ou un triflate de trialkylsilyle (R¹⁰R¹¹R¹²) SiOSO₂CF₃ ou d'autres réactifs de silylation connus, en présence d'une base et le cas échéant d'un solvant en composés de formule (XI) selon le schéma de réaction suivant (schéma 7) : 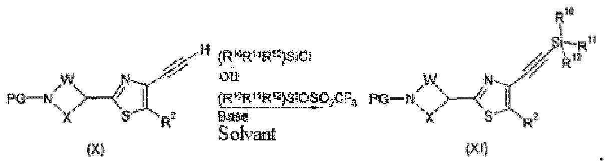 où
PG = représente phénylméthyle non substitué ou substitué (les substituants étant choisis, indépendamment les uns des autres, parmi méthyle, méthoxy, nitro, dioxolano), acétyle ou C₁-C₄-alcoxycarbonyle,
R¹⁰, R¹¹ et R¹² = C₁-C₄-alkyle ou phényle, W, X et R² sont tels que définis pour la formule (I) dans la revendication 1,
(h) transformation d'alcynes de formule (XI) avec un composé R³-L³-Hal, le cas échéant en présence d'un catalyseur, en présence d'une base et le cas échéant d'un solvant en composés de formule (IVc) selon le schéma de réaction suivant (schéma 8) : 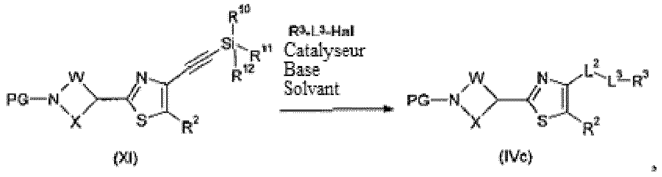 où
PG = représente phénylméthyle non substitué ou substitué (les substituants étant choisis, indépendamment les uns des autres, parmi méthyle, méthoxy, nitro, dioxolano), acétyle ou C₁-C₄-alcoxycarbonyle,
Hal = Cl, Br ou I,
R² = hydrogène, C₁-C₂-alkyle ou C₁-C₂-halogénoalkyle,
L² = -C=C-
R¹⁰, R¹¹ et R¹² = C₁-C₄-alkyle ou phényle,
W, X, L³ et R³ sont tels que définis pour la formule (I) dans la revendication 1,
(i) transformation de thioamides de formule (V) avec des composés des formules (VIa) ou (VIb) en composés des formules (IVa) ou (IVb) en présence d'une base et le cas échéant en présence d'un solvant, selon le schéma de réaction suivant (schéma 9) : 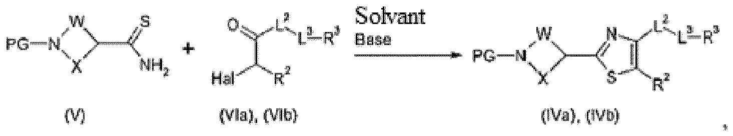 où
PG = phénylméthyle non substitué ou substitué (les substituants étant choisis, indépendamment les uns des autres, parmi méthyle, méthoxy, nitro, dioxolano), acétyle, C₁-C₄-alcoxycarbonyle ou benzyloxycarbonyle,
Hal = Cl, Br ou I,
L² = -C(R⁸)₂-C(R⁸)₂- pour les composés des formules (IVa) et (VIa),
L² = -CR⁹=CR⁹- pour les composés des formules (IVb) et (VIb),
W, X, L³, R², R³, R⁸ et R⁹ sont tels que définis pour la formule (I) dans la revendication 1,
(j) transformation de composés des formules (IVb) ou (IVc) en composés de formule (IVa) en présence d'hydrogène, d'un catalyseur et le cas échéant d'un solvant selon le schéma de réaction suivant (schéma 10) : 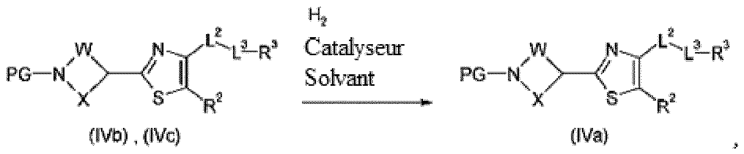 où
PG = acétyle ou C₁-C₄-alcoxycarbonyle,
L² = -C(R⁸)₂-C(R⁸)₂- pour les composés de formule (IVa),
L² = -CR⁹=CR⁹- pour les composés de formule (IVb)
L² = -C=C- pour les composés de formule (IVc)
W, X, L³, R², R³, R⁸ et R⁹ sont tels que définis pour la formule (I) dans la revendication 1,
(k) transformation de composés de formule (IV) en composés de formule (II), le cas échéant en présence d'un solvant ainsi que le cas échéant en présence d'un acide ou le cas échéant en présence d'une base ou le cas échéant en présence d'une source d'hydrogène, selon le schéma de réaction suivant (schéma 11) : 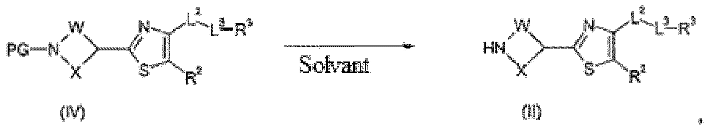 où
PG = acétyle, C₁-C₄-alcoxycarbonyle ou benzyloxycarbonyle,
W, X, L², L³, R², et R³ sont tels que définis pour la formule (I) dans la revendication 1,
(l) transformation de composés de formule (IIa') en composés de formule (IIa) en présence d'un agent d'halogénation, le cas échéant en présence d'un acide et d'un solvant, selon le schéma de réaction suivant (schéma 12) : 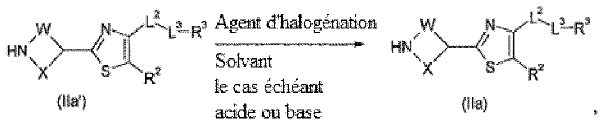 où
L² = -C(R⁸)₂-C(R⁸)₂-,
R² = I, Br ou Cl pour les composés de formule (IIa),
R² = hydrogène pour les composés de formule (IIa') W, X, L³, R³ et R⁸ sont tels que définis pour la formule (I) dans la revendication 1,
(m) transformation de composés de formule (III) avec des composés de formule (II) en composés de formule (I), le cas échéant en présence d'un réactif de couplage, d'une base et d'un solvant, selon le schéma de réaction suivant (schéma 13) : 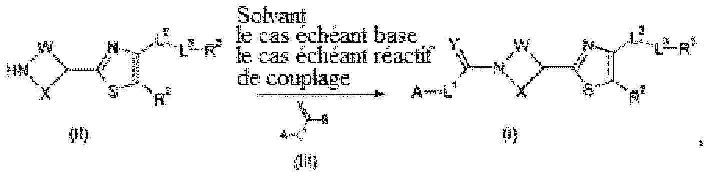 où
B = OH, chlore, brome ou iode,
Y = oxygène ;
A, W, X, L¹, L², L³, R² et R³ sont tels que définis pour la formule (I) dans la revendication 1,
(n) transformation de composés de formule (I) en composés de formule (I) en présence d'un réactif de sulfuration et le cas échéant en présence d'un solvant, selon le schéma de réaction suivant (schéma 14) : 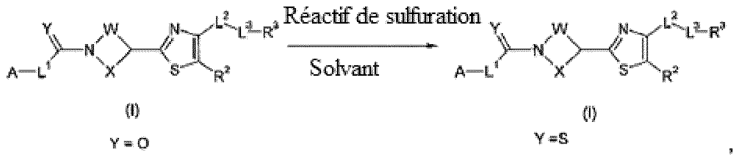 où
A, W, X, L¹, L², L³, R² et R³ sont tels que définis pour la formule (I) dans la revendication 1.

10. Procédé pour la préparation des composés de formule (I), comprenant au moins une des étapes (o) à (u) suivantes :
(o) transformation d'aldéhydes de formule (XII) en alcynes de formule (XIII) en présence d'une base et le cas échéant en présence d'un solvant, selon le schéma de réaction suivant (schéma 17) : 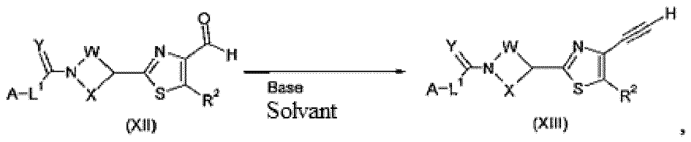 où
A, W, X, Y, L¹ et R² sont tels que définis pour la formule (I) ci-dessus,
(p) transformation d'alcynes de formule (XIII) avec un chlorure de trialkylsilyle (R¹⁰R¹¹R¹²) SiCl ou un triflate de trialkylsilyle (R¹⁰R¹¹R¹²) SiOSO₂CF₃ ou d'autres réactifs de silylation connus, en présence d'une base et le cas échéant d'un solvant en composés de formule (XIV) selon le schéma de réaction suivant (schéma 18) : 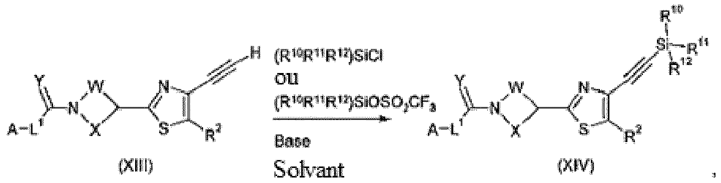 où
R¹⁰, R¹¹ et R¹² = C₁-C₄-alkyle ou phényle,
A, W, X, Y, L¹ et R² sont tels que définis pour la formule (I) ci-dessus,
(q) transformation d'alcynes de formule (XIII) avec un halogénure de R³-L³ dans une réaction de couplage C-C le cas échéant catalysée en présence d'une base et le cas échéant d'un solvant en composés de formule (Ic) selon le schéma de réaction suivant (schéma 19) : 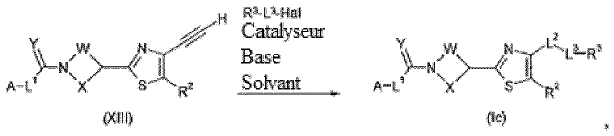 où
Hal = Cl, Br ou I,
L² = -C≡C-,
A, W, X, Y, L¹ L³, R² et R³ sont tels que définis pour la formule (I) ci-dessus,
(r) transformation d'alcynes de formule (XIV) avec un composé R³-L³-Hal, le cas échéant en présence d'un catalyseur, en présence d'une base et le cas échéant d'un solvant en composés de formule (Ic) selon le schéma de réaction suivant (schéma 20) : 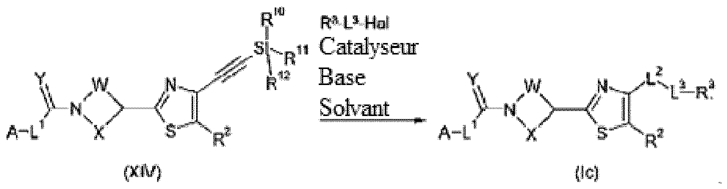 où
Hal = Cl, Br ou I,
L² = -C≡C-,
R¹⁰, R¹¹ et R¹² = C₁-C₄-alkyle ou phényle, A, W, X, Y, L¹ L³, R² et R³ sont tels que définis pour la formule (I) ci-dessus,
(s) transformation d'aldéhydes de formule (XII) en composés de formule (Ib) en présence d'une base ou d'un acide et le cas échéant en présence d'un solvant, selon le schéma de réaction suivant (schéma 21) : 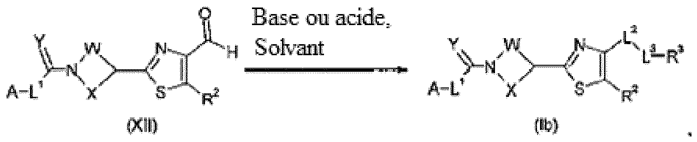 où
L² = -CR⁹=CR⁹-,
A, W, X, Y, L¹ L³, R², R³ et R⁹ sont tels que définis pour la formule (I) ci-dessus,
(t) transformation de composés de formule (Ic) en composés de formule (Ib) en présence d'hydrogène, d'un catalyseur et le cas échéant d'un solvant selon le schéma de réaction suivant (schéma 22) : 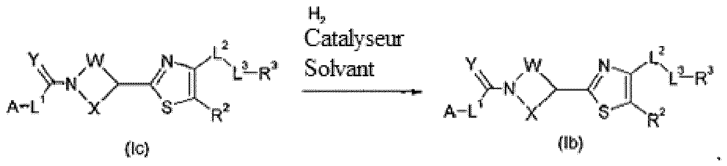 où
L² = -CR⁹=CR⁹- pour les composés de formule (Ib)
L² = -C=C- pour les composés de formule (Ic)
A, W, X, Y, L¹ L³, R², R³ et R⁹ sont tels que définis pour la formule (I) ci-dessus,
(u) transformation de composés des formules (Ib) ou (Ic) en composés de formule (Ia) en présence d'hydrogène, d'un catalyseur et le cas échéant d'un solvant selon le schéma de réaction suivant (schéma 23) : 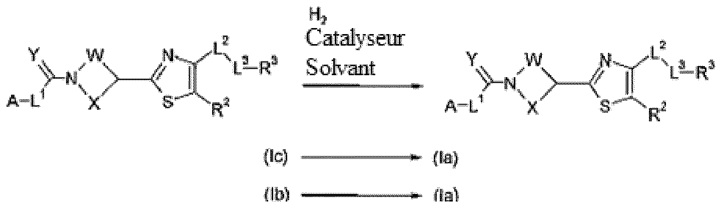 où
L² = -C(R⁸)₂-C(R⁸)₂- pour les composés de formule (Ia),
L² = -CR⁹=CR⁹- pour les composés de formule (Ib)
L² = -C=C- pour les composés de formule (Ic)
A, W, X, Y, L¹ L³, R², R³, R⁸ et R⁹ sont tels que définis pour la formule (I) ci-dessus.
